(19) **Europäisches Patentamt / European Patent Office / Office européen des brevets**

(11) **EP 4 421 171 A1**

(12) **EUROPEAN PATENT APPLICATION**
published in accordance with Art. 153(4) EPC

(43) Date of publication:
**28.08.2024 Bulletin 2024/35**

(21) Application number: **22883622.7**

(22) Date of filing: **20.10.2022**

(51) International Patent Classification (IPC):
**C12N 15/11** (2006.01)    **C12Q 1/6837** (2018.01)
**C12Q 1/6886** (2018.01)

(52) Cooperative Patent Classification (CPC):
**C12N 15/11; C12Q 1/6837; C12Q 1/6886**

(86) International application number:
**PCT/JP2022/039032**

(87) International publication number:
**WO 2023/068318 (27.04.2023 Gazette 2023/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **20.10.2021 JP 2021171945**

(71) Applicant: **Toray Industries, Inc.**
**Tokyo 103-8666 (JP)**

(72) Inventors:
• **WAKAO, Osamu**
  **Kamakura-shi, Kanagawa 248-8555 (JP)**
• **IWASAKI, Chie**
  **Kamakura-shi, Kanagawa 248-8555 (JP)**
• **SUDO, Hiroko**
  **Kamakura-shi, Kanagawa 248-8555 (JP)**

(74) Representative: **Mewburn Ellis LLP**
**Aurora Building**
**Counterslip**
**Bristol BS1 6BX (GB)**

Remarks:
The complete document including Reference Table(s) and the Sequence Listing(s) can be downloaded from the EPO website

(54) **KIT, DEVICE AND METHOD FOR DISTINGUISHING BETWEEN OVARIAN CANCER AND BENIGN OVARIAN TUMORS**

(57)    Provided are a kit or device for detecting ovarian cancer, and an ovarian cancer detection method. The kit or device for detecting ovarian cancer comprises a nucleic acid capable of bonding specifically with miRNA in a sample derived from a subject, and the ovarian cancer detection method comprises measuring the miRNA in vitro.

EP 4 421 171 A1

**Description**

TECHNICAL FIELD

The present invention relates to a kit or a device comprising a probe and/or a primer for detecting a particular miRNA, which is used for discriminating between ovarian cancer and benign ovarian tumor in a subject, and a method for discriminating between ovarian cancer and benign ovarian tumor, comprising measuring an expression level of the miRNA using the nucleic acid.

BACKGROUND ART

The ovary is the female reproductive organ that produces an ovum, and one each is found on both sides of the uterus. Its function is to secrete female hormones such as estrogen and progesterone. The ovary is considered an organ prone to develop tumors, which are roughly classified into surface epithelial-stromal tumors, sex cord-stromal tumor, and germ cell tumor, based on the origin of tumorigenesis. The ovarian tumors are categorized into benign, borderline malignant, and malignant; and the malignant ovarian tumors are called ovarian cancers.

A stage of progression of ovarian cancer is defined according to a tumor size, the presence or absence of infiltration, lymph node metastasis and distant metastasis, and the like; and ovarian cancer is classified into stages IA, IB, IC, IIA, IIB, IIC, IIIA, IIIB, IIIC, and IV. Five-year relative survival rate in ovarian cancer depends largely on a stage of the cancer progression, and it is reported that the rate is 92% in the case of a cancer being localized, 73% in the case of a cancer being observed in adjacent (peripheral) region, and 29% in the case of a distant metastatic cancer being observed. Thus, early detection of ovarian cancer leads to improvement in survival rate and the provision of means for enabling the early detection is strongly required.

Early ovarian cancer and benign ovarian tumor are often asymptomatic, and even progressive ovarian cancer at most cases, only involves general symptoms which could be caused by other reasons such as abdominal bloating sensation and pains, because of which it is difficult to detect ovarian cancer based on subjective symptoms. Extensive studies on the screening test for ovarian cancer have been conducted but no substantial achievement is accomplished, and only two methods are mainly usable in practice: transvaginal ultrasound test and a CA-125 blood marker test. The secondary test for ovarian cancer includes imaging tests such as ultrasonography, CT scanning test, barium enema X-ray examination, and MRI. When an abnormality is detected in these ovarian cancer tests, a benign, borderline malignant, or malignant case is definitively diagnosed by histological diagnosis in which tissue samples obtained by surgery are evaluated. Hence, when an abnormality is detected in the ovarian cancer tests, laparotomy is required for even benign tumors and is considered to be a heavy burden for patients.

As shown in Patent Literatures 1 to 3, there are reports, although they are at a research stage, on the detection of ovarian cancer using the expression levels of microRNAs (miRNAs) in biological samples, including blood.

Patent Literature 1 discloses a kit, a device and a method for detecting ovarian tumor using miR-4525, miR-1908-5p, miR-4674, miR-939-5p, miR-1268a, miR-6789-5p and the like in serum.

Patent Literature 2 discloses miR-193b*, miR-602 and the like in human sample tissues or blood as biomarkers for gynecologic cancers.

Patent Literature 3 discloses a method for diagnosing ovarian cancer using miRNAs in blood.

PRIOR ART LITERATURE

Patent Literature

[0009]

　　Patent Literature 1: International Publication No. WO 2018/199275
　　Patent Literature 2: JP Patent Publication (Kokai) No. 2010-154843 A
　　Patent Literature 3: Published U.S. Patent Application No. 2020-0123614 A

SUMMARY OF INVENTION

Problem to be Solved by Invention

An object of the present invention is to find markers for discriminating between ovarian cancer and benign ovarian tumor and to provide a method for effectively discriminating between ovarian cancer and benign ovarian tumor using probes or primers for detecting the markers.

In general, it is difficult to detect ovarian cancer based on subjective symptoms, and it is further difficult to discriminate between ovarian cancer and benign ovarian tumor. Usually, in the primary screening test method for ovarian cancer, two tests: transvaginal ultrasound test and tumor marker CA-125 test, are used. However, neither performs sufficient effectiveness as test methods. The transvaginal ultrasound test can detect a tumor using ultrasound from within the vagina, but the ultrasound has a limited reachable range and thus may fail to detect a tumor present distant from the vagina. CA-125 is a protein in blood and is known to increase in ovarian cancer patients but more often increases for different reasons from ovarian cancer. Furthermore, a CA-125 level does not increase in some ovarian cancer patients thus making the CA-125 test have low performance (Whitehouse C. et al., 2003, Gynecologic Oncology, Vol. 88, S152).

In the secondary test for ovarian cancer, imaging tests are generally conducted. When an abnormality is detected at this stage, a laparotomy is required, irrespective of malignancy. However, there is a possibility of being diagnosed as a benign ovarian tumor, not ovarian cancer, after the laparotomy, and thus for benign ovarian tumor patients in particular, there are heavy physical invasiveness, emotional distress, and economic burdens.

Further, as described below, there are reports, albeit at a research stage, on the determination of ovarian cancer using the expression levels of miRNAs in biological samples including blood, none of which, however, have yet been brought into practical use.

Patent Literature 1 discloses a kit, a device and a method for detecting ovarian tumor using miR-4525, miR-1908-5p, miR-4674, miR-939-5p, miR-1268a, miR-6789-5p and the like in serum. However, it merely discloses these miRNAs as ovarian tumor markers for detecting ovarian tumor by comparing expression levels of the miRNAs in a patient group encompassing both ovarian cancer patients and benign ovarian tumor patients to expression levels of the miRNAs in healthy subjects and patient groups with other diseases (benign bone and soft tissue tumor and benign breast disease) and does not describe their performance for discriminating between ovarian cancer and benign ovarian tumor.

Patent Literature 2 discloses miRNAs such as miR-193b* and miR-602 in human sample tissues or blood as biomarkers for gynecologic cancers. However, it discloses only miRNA expression differences relevant to uterine cancer tissues and polyps, noncancer tissues, and does not describe the specific detection performance, such as accuracy, sensitivity, or specificity, for discriminating between ovarian cancer and benign ovarian tumor.

Patent Literature 3 discloses a method for diagnosing ovarian cancer using miRNAs in blood, but it does not describe performance for discriminating between ovarian cancer and benign ovarian tumor and is not suitable for discriminating between ovarian cancer and benign ovarian tumor. Additionally, patients with borderline malignant tumor, which has heretofore been excised in the same manner as for ovarian cancer by laparotomy, are discriminated as the same group as benign ovarian tumor patients. Hence, when an abnormality is detected in the test, it is difficult to discriminate borderline malignant tumor patients and it is highly possible to require laparotomy. Therefore, it is considered to bring heavy physical invasiveness, emotional distress, and economic burdens particularly for benign ovarian tumor patients.

As mentioned above, the existing tumor markers exhibit low performance in the detection of ovarian cancer, and no evidence about the performance for discriminating between ovarian cancer and benign ovarian tumor is shown even for the markers at a research stage. The conventional tumor markers might fail to discriminate benign ovarian tumor from ovarian cancer, and thus the false diagnosis of ovarian cancer patients or the missing of a therapeutic opportunity because of overlooking ovarian cancer patients may occur. Furthermore, the conventional diagnosis of benign ovarian tumor, which involves collecting ovarian tissues, is highly invasive to patients and is not favorable. Hence, there is a demand for an ovarian cancer marker with high accuracy, particularly with high specificity, that is capable of correctly discriminating an ovarian cancer patient from a benign ovarian tumor patient and that is detectable using a sample which can be collected in a less invasive manner, such as blood. Particularly, burdens on patients can be reduced by avoiding highly invasive or high-cost surgery if benign ovarian tumor patients can be accurately discriminated. Furthermore, the early detection of ovarian cancer can reduce the risk of recurrence if it can lead to early treatment. Thus, there is a high demand for a biomarker with high performance for discriminating between ovarian cancer and benign ovarian tumor that is capable of highly sensitively discriminating early ovarian cancer at an early stage of progression. Furthermore, when a simpler primary screening test for ovarian cancer is provided, a consultation rate of the secondary test is expected to increase.

Solution to Problem

The present inventors have conducted diligent studies to attain the object and consequently completed the present invention by finding genes usable for discriminating between ovarian cancer and benign ovarian tumor using samples, such as blood, which can be collected with limited invasiveness, and finding that ovarian cancer and benign ovarian tumor can be significantly discriminated by using nucleic acids capable of specifically binding to these genes.

<Summary of Invention>

The present invention has the following features:

(1) A kit for discrimination between ovarian cancer and benign ovarian tumor, comprising a probe(s) and/or a primer(s) for detecting at least one polynucleotide selected from the group consisting of the following markers for discriminating between ovarian cancer and benign ovarian tumor: miR-1908-5p, miR-4723-5p, miR-4674, miR-939-5p, miR-6789-5p, miR-1268a, miR-1202, miR-4525, and miR-128-1-5p.

(2) The kit according to (1), wherein the probe(s) and/or primer(s) is a polynucleotide(s) selected from the group consisting of the following polynucleotides (a) to (e):

(a) a polynucleotide consisting of a nucleotide sequence represented by any of SEQ ID NOs: 1 to 9, or a nucleotide sequence derived from the nucleotide sequence by the replacement of u with t, a variant thereof, a derivative thereof, or a fragment thereof comprising 15 or more consecutive nucleotides;

(b) a polynucleotide comprising a nucleotide sequence represented by any of SEQ ID NOs: 1 to 9;

(c) a polynucleotide consisting of a nucleotide sequence complementary to a nucleotide sequence represented by any of SEQ ID NOs: 1 to 9, or a nucleotide sequence derived from the nucleotide sequence by the replacement of u with t, a variant thereof, a derivative thereof, or a fragment thereof comprising 15 or more consecutive nucleotides;

(d) a polynucleotide comprising a nucleotide sequence complementary to a nucleotide sequence represented by any of SEQ ID NOs: 1 to 9, or a nucleotide sequence derived from the nucleotide sequence by the replacement of u with t; and

(e) a polynucleotide hybridizing under stringent conditions to any of the polynucleotides (a) to (d).

(3) The kit according to (1) or (2), wherein the kit further comprises a probe(s) and/or a primer(s) for detecting at least one polynucleotide selected from the group consisting of the following additional markers for discriminating between ovarian cancer and benign ovarian tumor: miR-6806-5p, miR-7845-5p, miR-4632-5p, miR-10396b-5p, miR-6768-5p, miR-8059, miR-8072, miR-9901, miR-1231, miR-1225-5p, miR-12114, miR-3178, miR-6798-5p, miR-4276, miR-6125, miR-3652, miR-7111-5p, miR-6749-5p, miR-1199-5p, miR-6802-5p, miR-6816-5p, miR-4706, miR-5008-5p, miR-6797-5p, miR-4516, miR-4508, miR-6729-5p, miR-564, miR-1233-5p, miR-6127, miR-1469, miR-6738-5p, miR-6785-5p, miR-10401-5p, miR-4430, miR-6889-5p, miR-1236-5p, miR-3176, miR-3141, miR-3928-3p, miR-1237-5p, miR-1915-3p, miR-5195-3p, miR-6743-5p, miR-6746-5p, miR-4446-3p, miR-1228-5p, miR-1268b, miR-1260a, miR-6879-3p, miR-149-3p, miR-3162-5p, miR-1207-5p, miR-4747-3p, miR-4651, miR-638, miR-4736, miR-6845-5p, miR-1343-3p, miR-6126, miR-92b-5p, miR-6774-5p, miR-7847-3p, miR-6795-5p, miR-7109-5p, miR-3197, miR-6824-5p, miR-6771-5p, miR-11399, miR-2861, miR-4707-3p, miR-4638-5p, miR-8073, miR-328-5p, miR-665, miR-6778-5p, miR-10398-3p, miR-5698, miR-6794-5p, miR-1247-3p, miR-4697-5p, miR-8069, miR-572, miR-6751-5p, miR-3180-3p, miR-486-3p, miR-6086, miR-30c-1-3p, miR-8063, miR-3621, miR-6887-5p, miR-3191-3p, miR-11181-3p, miR-6722-5p, miR-6781-5p, miR-5739, miR-3937, miR-1343-5p, miR-1181, miR-4725-3p, miR-6865-5p, miR-375-5p, miR-3196, miR-6762-5p, miR-4258, miR-5196-5p, miR-10401-3p, miR-675-5p, miR-4488, miR-10527-5p, miR-10396a-5p, miR-4269, miR-6800-5p, miR-6819-5p, miR-10396b-3p, miR-4688, miR-6786-5p, miR-4634, miR-3940-5p, miR-4655-5p, miR-7155-5p, miR-6769b-5p, miR-6810-5p, miR-4665-3p, miR-6727-5p, miR-6803-5p, miR-4640-5p, miR-6735-5p, miR-4535, miR-8089, miR-1292-3p, miR-5088-5p, miR-3622a-5p, miR-6124, miR-6820-5p, miR-6805-3p, miR-4513, miR-760, miR-4665-5p, miR-10400-3p, miR-4298, miR-8085, miR-4463, miR-6807-5p, miR-4433b-3p, miR-3185, miR-12121, miR-671-5p, miR-6752-5p, miR-371a-5p, miR-3917, miR-1224-5p, miR-498-5p, miR-7704, miR-6741-5p, miR-765, miR-4486, miR-6090, miR-718, miR-4767, miR-6851-5p, miR-5572, miR-6850-5p, miR-6089, miR-5787, miR-4534, miR-3665, miR-4787-5p, miR-6754-5p, miR-6825-3p, miR-4728-5p, miR-6088, miR-3154, miR-6869-5p, miR-187-5p, miR-6165, miR-4447, miR-4731-5p, miR-6805-5p, miR-12118, miR-4270, miR-7846-3p, miR-4443, miR-6737-5p, miR-197-5p, miR-1229-5p, miR-6757-5p, miR-6765-5p, miR-4722-5p, miR-6891-5p, miR-5006-5p, miR-345-3p, miR-6726-5p, miR-3195, miR-6877-5p, miR-4462, miR-6812-5p, miR-483-5p, miR-9899, miR-4800-5p, miR-4734, miR-3135b, miR-4433a-3p, miR-6769a-5p, miR-4743-5p, miR-1909-3p, miR-4741, miR-4685-5p, miR-3147, miR-4726-5p, miR-3180, miR-3188, miR-6782-5p, miR-6776-5p, miR-4484, miR-1185-1-3p, miR-6790-3p, miR-4466, miR-10394-3p, miR-1275, miR-4478, miR-3175, miR-7106-5p, miR-4667-5p, miR-193b-5p, and miR-602.

(4) The kit according to (3), wherein the probe(s) and/or primer(s) is a polynucleotide(s) selected from the group consisting of the following polynucleotides (a) to (e):

(a) a polynucleotide consisting of a nucleotide sequence represented by any of SEQ ID NOs: 10 to 235, or a nucleotide sequence derived from the nucleotide sequence by the replacement of u with t, a variant thereof, a derivative thereof, or a fragment thereof comprising 15 or more consecutive nucleotides;

(b) a polynucleotide comprising a nucleotide sequence represented by any of SEQ ID NOs: 10 to 235;

(c) a polynucleotide consisting of a nucleotide sequence complementary to a nucleotide sequence represented by any of SEQ ID NOs: 10 to 235, or a nucleotide sequence derived from the nucleotide sequence by the replacement of u with t, a variant thereof, a derivative thereof, or a fragment thereof comprising 15 or more consecutive nucleotides;

(d) a polynucleotide comprising a nucleotide sequence complementary to a nucleotide sequence represented by any of SEQ ID NOs: 10 to 235, or a nucleotide sequence derived from the nucleotide sequence by the replacement of u with t; and

(e) a polynucleotide hybridizing under stringent conditions to any of the polynucleotides (a) to (d).

(5) A device for discrimination between ovarian cancer and benign ovarian tumor, comprising a probe(s) and/or a primer(s) for detecting at least one polynucleotide selected from the group consisting of the following markers for discriminating between ovarian cancer and benign ovarian tumor: miR-1908-5p, miR-4723-5p, miR-4674, miR-939-5p, miR-6789-5p, miR-1268a, miR-1202, miR-4525, and miR-128-1-5p.

(6) The device according to (5), wherein the probe(s) and/or primer(s) is a polynucleotide(s) selected from the group consisting of the following polynucleotides (a) to (e):

(a) a polynucleotide consisting of a nucleotide sequence represented by any of SEQ ID NOs: 1 to 9, or a nucleotide sequence derived from the nucleotide sequence by the replacement of u with t, a variant thereof, a derivative thereof, or a fragment thereof comprising 15 or more consecutive nucleotides;

(b) a polynucleotide comprising a nucleotide sequence represented by any of SEQ ID NOs: 1 to 9;

(c) a polynucleotide consisting of a nucleotide sequence complementary to a nucleotide sequence represented by any of SEQ ID NOs: 1 to 9, or a nucleotide sequence derived from the nucleotide sequence by the replacement of u with t, a variant thereof, a derivative thereof, or a fragment thereof comprising 15 or more consecutive nucleotides;

(d) a polynucleotide comprising a nucleotide sequence complementary to a nucleotide sequence represented by any of SEQ ID NOs: 1 to 9, or a nucleotide sequence derived from the nucleotide sequence by the replacement of u with t; and

(e) a polynucleotide hybridizing under stringent conditions to any of the polynucleotides (a) to (d).

(7) The device according to (5) or (6), wherein the device further comprises a probe(s) and/or a primer(s) for detecting at least one polynucleotide selected from the group consisting of the following additional markers for discriminating between ovarian cancer and benign ovarian tumor: miR-6806-5p, miR-7845-5p, miR-4632-5p, miR-10396b-5p, miR-6768-5p, miR-8059, miR-8072, miR-9901, miR-1231, miR-1225-5p, miR-12114, miR-3178, miR-6798-5p, miR-4276, miR-6125, miR-3652, miR-7111-5p, miR-6749-5p, miR-1199-5p, miR-6802-5p, miR-6816-5p, miR-4706, miR-5008-5p, miR-6797-5p, miR-4516, miR-4508, miR-6729-5p, miR-564, miR-1233-5p, miR-6127, miR-1469, miR-6738-5p, miR-6785-5p, miR-10401-5p, miR-4430, miR-6889-5p, miR-1236-5p, miR-3176, miR-3141, miR-3928-3p, miR-1237-5p, miR-1915-3p, miR-5195-3p, miR-6743-5p, miR-6746-5p, miR-4446-3p, miR-1228-5p, miR-1268b, miR-1260a, miR-6879-3p, miR-149-3p, miR-3162-5p, miR-1207-5p, miR-4747-3p, miR-4651, miR-638, miR-4736, miR-6845-5p, miR-1343-3p, miR-6126, miR-92b-5p, miR-6774-5p, miR-7847-3p, miR-6795-5p, miR-7109-5p, miR-3197, miR-6824-5p, miR-6771-5p, miR-11399, miR-2861, miR-4707-3p, miR-4638-5p, miR-8073, miR-328-5p, miR-665, miR-6778-5p, miR-10398-3p, miR-5698, miR-6794-5p, miR-1247-3p, miR-4697-5p, miR-8069, miR-572, miR-6751-5p, miR-3180-3p, miR-486-3p, miR-6086, miR-30c-1-3p, miR-8063, miR-3621, miR-6887-5p, miR-3191-3p, miR-11181-3p, miR-6722-5p, miR-6781-5p, miR-5739, miR-3937, miR-1343-5p, miR-1181, miR-4725-3p, miR-6865-5p, miR-375-5p, miR-3196, miR-6762-5p, miR-4258, miR-5196-5p, miR-10401-3p, miR-675-5p, miR-4488, miR-10527-5p, miR-10396a-5p, miR-4269, miR-6800-5p, miR-6819-5p, miR-10396b-3p, miR-4688, miR-6786-5p, miR-4634, miR-3940-5p, miR-4655-5p, miR-7155-5p, miR-6769b-5p, miR-6810-5p, miR-4665-3p, miR-6727-5p, miR-6803-5p, miR-4640-5p, miR-6735-5p, miR-4535, miR-8089, miR-1292-3p, miR-5088-5p, miR-3622a-5p, miR-6124, miR-6820-5p, miR-6805-3p, miR-4513, miR-760, miR-4665-5p, miR-10400-3p, miR-4298, miR-8085, miR-4463, miR-6807-5p, miR-4433b-3p, miR-3185, miR-12121, miR-671-5p, miR-6752-5p, miR-371a-5p, miR-3917, miR-1224-5p, miR-498-5p, miR-7704, miR-6741-5p, miR-765, miR-4486, miR-6090, miR-718, miR-4767, miR-6851-5p, miR-5572, miR-6850-5p, miR-6089, miR-5787, miR-4534, miR-3665, miR-4787-5p, miR-6754-5p, miR-6825-3p, miR-4728-5p, miR-6088, miR-3154, miR-6869-5p, miR-187-5p, miR-6165, miR-4447, miR-4731-5p, miR-6805-5p, miR-12118, miR-4270, miR-7846-3p, miR-4443, miR-6737-5p, miR-197-5p, miR-1229-5p, miR-6757-5p, miR-6765-5p, miR-4722-5p, miR-6891-5p, miR-5006-5p, miR-345-3p, miR-6726-5p, miR-3195, miR-6877-5p, miR-4462, miR-6812-5p, miR-483-5p, miR-9899, miR-4800-5p, miR-4734, miR-3135b, miR-4433a-3p, miR-6769a-5p, miR-4743-5p, miR-1909-3p, miR-4741, miR-4685-5p, miR-3147, miR-4726-5p, miR-3180, miR-3188, miR-6782-5p, miR-6776-5p, miR-4484, miR-1185-1-3p, miR-6790-3p, miR-4466, miR-10394-3p, miR-1275, miR-4478, miR-3175, miR-7106-5p, miR-4667-5p, miR-193b-5p, and miR-602.

(8) The device according to (7), wherein the probe(s) and/or primer(s) is a polynucleotide(s) selected from the group consisting of the following polynucleotides (a) to (e):

(a) a polynucleotide consisting of a nucleotide sequence represented by any of SEq ID NOs: 10 to 235, or a nucleotide sequence derived from the nucleotide sequence by the replacement of u with t, a variant thereof, a derivative thereof, or a fragment thereof comprising 15 or more consecutive nucleotides;

(b) a polynucleotide comprising a nucleotide sequence represented by any of SEQ ID NOs: 10 to 235;

(c) a polynucleotide consisting of a nucleotide sequence complementary to a nucleotide sequence represented by any of SEQ ID NOs: 10 to 235, or a nucleotide sequence derived from the nucleotide sequence by the replacement of u with t, a variant thereof, a derivative thereof, or a fragment thereof comprising 15 or more consecutive nucleotides;

(d) a polynucleotide comprising a nucleotide sequence complementary to a nucleotide sequence represented by any of SEQ ID NOs: 10 to 235, or a nucleotide sequence derived from the nucleotide sequence by the replacement of u with t; and

(e) a polynucleotide hybridizing under stringent conditions to any of the polynucleotides (a) to (d).

(9) The device according to any of (5) to (8), wherein the device is for measurement by a hybridization technique.

(10) The device according to (9), wherein the hybridization technique is a nucleic acid array technique.

(11) A method for discriminating between ovarian cancer and benign ovarian tumor, comprising: measuring an expression level(s) of at least one polynucleotide selected from the group consisting of the following markers for discriminating between ovarian cancer and benign ovarian tumor: miR-1908-5p, miR-4723-5p, miR-4674, miR-939-5p, miR-6789-5p, miR-1268a, miR-1202, miR-4525, and miR-128-1-5p in a sample from a subject; and evaluating *in vitro* whether the subject has ovarian cancer or is a benign ovarian tumor patient using the measured expression level(s) and a control expression level(s) from a benign ovarian tumor patient measured in the same manner.

(12) The method according to (11), comprising assigning the expression level(s) of the target gene(s) in the sample from the subject to a discriminant formula, which is capable of discriminatorily determining ovarian cancer and benign ovarian tumor and which is prepared with gene expression levels in a samples from a patient(s) known to have ovarian cancer and a sample(s) from a benign ovarian tumor patient(s) as a training sample(s), and thereby evaluating the presence of ovarian cancer or the presence of benign ovarian tumor.

(13) The method according to (11) or (12), comprising measuring an expression level(s) of the polynucleotide using a kit according to any of (1) to (4) or a device according to any of (5) to (10), wherein the kit or the device comprises a probe(s) and/or a primer(s) for detecting the polynucleotide.

(14) The method according to (13), wherein the probe(s) and/or primer(s) is a polynucleotide(s) selected from the group consisting of the following polynucleotides (a) to (e):

(a) a polynucleotide consisting of a nucleotide sequence represented by any of SEQ ID NOs: 1 to 9, or a nucleotide sequence derived from the nucleotide sequence by the replacement of u with t, a variant thereof, a derivative thereof, or a fragment thereof comprising 15 or more consecutive nucleotides;

(b) a polynucleotide comprising a nucleotide sequence represented by any of SEQ ID NOs: 1 to 9;

(c) a polynucleotide consisting of a nucleotide sequence complementary to a nucleotide sequence represented by any of SEQ ID NOs: 1 to 9, or a nucleotide sequence derived from the nucleotide sequence by the replacement of u with t, a variant thereof, a derivative thereof, or a fragment thereof comprising 15 or more consecutive nucleotides;

(d) a polynucleotide comprising a nucleotide sequence complementary to a nucleotide sequence represented by any of SEQ ID NOs: 1 to 9, or a nucleotide sequence derived from the nucleotide sequence by the replacement of u with t; and

(e) a polynucleotide hybridizing under stringent conditions to any of the polynucleotides (a) to (d).

(15) The method according to (13) or (14), wherein the probe(s) and/or the primer(s) further comprises a probe(s) and/or a primer(s) for detecting at least one polynucleotide selected from the group consisting of the following additional markers for discriminating between ovarian cancer and benign ovarian tumor: miR-6806-5p, miR-7845-5p, miR-4632-5p, miR-10396b-5p, miR-6768-5p, miR-8059, miR-8072, miR-9901, miR-1231, miR-1225-5p, miR-12114, miR-3178, miR-6798-5p, miR-4276, miR-6125, miR-3652, miR-7111-5p, miR-6749-5p, miR-1199-5p, miR-6802-5p, miR-6816-5p, miR-4706, miR-5008-5p, miR-6797-5p, miR-4516, miR-4508, miR-6729-5p, miR-564, miR-1233-5p, miR-6127, miR-1469, miR-6738-5p, miR-6785-5p, miR-10401-5p, miR-4430, miR-6889-5p, miR-1236-5p, miR-3176, miR-3141, miR-3928-3p, miR-1237-5p, miR-1915-3p, miR-5195-3p, miR-6743-5p, miR-6746-5p, miR-4446-3p, miR-1228-5p, miR-1268b, miR-1260a, miR-6879-3p, miR-149-3p, miR-3162-5p, miR-1207-5p, miR-4747-3p, miR-4651, miR-638, miR-4736, miR-6845-5p, miR-1343-3p, miR-6126, miR-92b-5p, miR-6774-5p, miR-7847-3p, miR-6795-5p, miR-7109-5p, miR-3197, miR-6824-5p, miR-6771-5p, miR-11399, miR-2861, miR-4707-3p, miR-4638-5p, miR-8073, miR-328-5p, miR-665, miR-6778-5p, miR-10398-3p, miR-5698, miR-6794-5p, miR-1247-3p, miR-4697-5p, miR-8069, miR-572, miR-6751-5p, miR-3180-3p, miR-486-3p, miR-6086, miR-30c-1-3p, miR-8063, miR-3621, miR-6887-5p, miR-3191-3p, miR-11181-3p, miR-6722-5p, miR-6781-5p, miR-5739, miR-3937, miR-1343-5p, miR-1181, miR-4725-3p, miR-6865-5p, miR-375-5p, miR-3196, miR-6762-5p, miR-4258, miR-5196-5p, miR-10401-3p, miR-675-5p, miR-4488, miR-10527-5p, miR-10396a-5p, miR-4269, miR-6800-5p, miR-6819-5p, miR-10396b-3p, miR-4688, miR-6786-5p, miR-4634, miR-3940-5p, miR-4655-5p, miR-7155-5p, miR-6769b-5p, miR-6810-5p, miR-4665-3p, miR-6727-5p, miR-6803-5p, miR-4640-5p, miR-6735-5p, miR-4535, miR-8089, miR-1292-3p, miR-5088-5p, miR-3622a-5p, miR-6124, miR-6820-5p, miR-6805-3p, miR-4513, miR-760, miR-4665-5p, miR-10400-3p, miR-4298, miR-8085, miR-4463, miR-6807-5p, miR-4433b-3p, miR-3185, miR-12121, miR-671-5p, miR-6752-5p, miR-371a-5p, miR-3917, miR-1224-5p, miR-498-5p, miR-7704, miR-6741-5p, miR-765, miR-4486, miR-6090, miR-718, miR-4767, miR-6851-5p, miR-5572, miR-6850-5p, miR-6089, miR-5787, miR-4534, miR-3665, miR-4787-5p, miR-6754-5p, miR-6825-3p, miR-4728-5p, miR-6088, miR-3154, miR-6869-5p, miR-187-5p, miR-6165, miR-4447, miR-4731-5p, miR-6805-5p, miR-12118, miR-4270, miR-7846-3p, miR-4443, miR-6737-5p, miR-197-5p, miR-1229-5p, miR-6757-5p, miR-6765-5p, miR-4722-5p, miR-6891-5p, miR-5006-5p, miR-345-3p, miR-6726-5p, miR-3195, miR-6877-5p, miR-4462, miR-6812-5p, miR-483-5p, miR-9899, miR-4800-5p, miR-4734, miR-3135b, miR-4433a-3p, miR-6769a-5p, miR-4743-5p, miR-1909-3p, miR-4741, miR-4685-5p, miR-3147, miR-4726-5p, miR-3180, miR-3188, miR-6782-5p, miR-6776-5p, miR-4484, miR-1185-1-3p, miR-6790-3p, miR-4466, miR-10394-3p, miR-1275, miR-4478, miR-3175, miR-7106-5p, miR-4667-5p, miR-193b-5p, and miR-602.

(16) The method according to (15), wherein the probe(s) and/or primer(s) is a polynucleotide(s) selected from the group consisting of the following polynucleotides (a) to (e):

(a) a polynucleotide consisting of a nucleotide sequence represented by any of SEQ ID NOs: 10 to 235, or a nucleotide sequence derived from the nucleotide sequence by the replacement of u with t, a variant thereof, a derivative thereof, or a fragment thereof comprising 15 or more consecutive nucleotides;
(b) a polynucleotide comprising a nucleotide sequence represented by any of SEQ ID NOs: 10 to 235;
(c) a polynucleotide consisting of a nucleotide sequence complementary to a nucleotide sequence represented by any of SEQ ID NOs: 10 to 235, or a nucleotide sequence derived from the nucleotide sequence by the replacement of u with t, a variant thereof, a derivative thereof, or a fragment thereof comprising 15 or more consecutive nucleotides;
(d) a polynucleotide comprising a nucleotide sequence complementary to a nucleotide sequence represented by any of SEQ ID NOs: 10 to 235, or a nucleotide sequence derived from the nucleotide sequence by the replacement of u with t; and
(e) a polynucleotide hybridizing under stringent conditions to any of the polynucleotides (a) to (d).

(17) The method according to any of (11) to (16), wherein the subject is a human.
(18) The method according to any of (11) to (17), wherein the sample is blood, serum, or plasma.

<Definition of Terms>

The terms used herein are defined as described below.
Abbreviations or terms such as nucleotide, polynucleotide, DNA, and RNA abide by "Guidelines for the preparation of specification which contain nucleotide and/or amino acid sequences" (edited by Japan Patent Office) and common use in the art.
The term "polynucleotide" used herein refers to a nucleic acid including any of RNA, DNA, and RNA/DNA (chimera). The DNA includes any of cDNA, genomic DNA, and synthetic DNA. The RNA includes any of total RNA, mRNA, rRNA, miRNA, siRNA, snoRNA, snRNA, non-coding RNA and synthetic RNA. Here the "synthetic DNA" and the "synthetic RNA" refer to a DNA and an RNA artificially prepared using, for example, an automatic nucleic acid synthesizer, on the basis of predetermined nucleotide sequences (which may be any of natural and non-natural sequences). The "non-natural sequence" is intended to be used in a broad sense and includes, for example, a sequence comprising substitution, deletion, insertion, and/or addition of one or more nucleotides (i.e., a variant sequence) and a sequence comprising one or more modified nucleotides (i.e., a modified sequence), which are different from the natural sequence. Herein, the term "polynucleotide" is used interchangeably with the term "nucleic acid."
The term "fragment" used herein is a polynucleotide having a nucleotide sequence that consists of a consecutive portion of a polynucleotide and desirably has a length of 15 or more nucleotides, preferably 17 or more nucleotides, more preferably 19 or more nucleotides.

The term "gene" used herein is intended to include not only RNA and double-stranded DNA but also each single-stranded DNA such as a plus(+) strand (or a sense strand) or a complementary strand (or an antisense strand) constituting the duplex. The gene is not particularly limited by its length.

Thus, the "gene" used herein includes any of double-stranded DNA including human genomic DNA, single-stranded DNA (plus strand), single-stranded DNA having a sequence complementary to the plus strand (complementary strand), cDNA, microRNA (miRNA), their fragments, and human genome, and their transcripts, unless otherwise specified. The "gene" includes not only a "gene" represented by a particular nucleotide sequence (or SEQ ID NO) but "nucleic acids" encoding RNAs having biological functions equivalent to RNA encoded by the gene, for example, a congener (i.e., a homolog or an ortholog), a variant (e.g., a genetic polymorph), and a derivative. Specific examples of such a "nucleic acid" encoding a congener, a variant, or a derivative can include a "nucleic acid" having a nucleotide sequence hybridizing under stringent conditions described later to a nucleotide sequence represented by any of SEQ ID NOs: 1 to 852, a nucleotide sequence derived from the nucleotide sequence by the replacement of u with t or a complementary sequence thereof. Regardless whether or not there is a difference in functional region, the "gene" can comprise, for example, expression control regions, coding region, exons, or introns. The "gene" may be contained in a cell or may exist alone after being released from a cell. Alternatively, the "gene" may be in a state enclosed in a vesicle called exosome.

The term "exosome" used herein is a vesicle that is encapsulated by lipid bilayer and secreted from a cell. The exosome is derived from a multivesicular endosome and may incorporate biomaterials such as "genes" (e.g., RNA or DNA) or proteins when released into an extracellular environment. The exosome is known to be contained in a body fluid such as blood, serum, plasma, or lymph.

The term "transcript" used herein refers to an RNA synthesized from the DNA sequence of a gene as a template. RNA polymerase binds to a site called promoter located upstream of the gene and adds ribonucleotides complementary to the nucleotide sequence of the DNA to the 3' end to synthesize an RNA. This RNA contains not only the gene itself but the whole sequence from a transcription initiation site to the end of a polyA sequence, including expression control regions, coding region, exons, or introns.

Unless otherwise specified, the term "microRNA (miRNA)" used herein is intended to mean a 15- to 25-nucleotide non-coding RNA that is transcribed as an RNA precursor having a hairpin-like structure, cleaved by a dsRNA-cleaving enzyme having RNase III cleavage activity, and integrated into a protein complex called RISC, and that is involved in the suppression of translation of mRNA. The term "miRNA" used herein includes not only an "miRNA" represented by a particular nucleotide sequence (or SEQ ID NO) but a precursor of the "miRNA" (pre-miRNA or pri-miRNA), an miRNA having biological functions equivalent to the miRNAs, for example, a congener (i.e., a homolog or an ortholog), a variant such as a genetic polymorph, and a derivative. Such a precursor, a congener, a variant, or a derivative can be specifically identified using miRBase Release 22 (http://www.mirbase.org/), and examples thereof can include an "miRNA" having a nucleotide sequence hybridizing under stringent conditions described later to a complementary sequence of any particular nucleotide sequence represented by any of SEQ ID NOs: 1 to 852. The term "miRNA" used herein may be a transcript of an miRNA gene. Such a transcript includes a mature miRNA (e.g., a 15- to 25-nucleotide or 19-to 25-nucleotide non-coding RNA involved in the suppression of translation of mRNA as described above) or an miRNA precursor (e.g., pre-miRNA or pri-miRNA as described above).

The term "probe" used herein includes a polynucleotide capable of specifically recognizing and detecting an RNA resulting from the expression of a gene or a polynucleotide derived from the RNA, a polynucleotide complementary thereto, and an aptamer.

The term "primer" used herein includes a polynucleotide capable of specifically recognizing and amplifying an RNA resulting from the expression of a gene or a polynucleotide derived from the RNA, and a polynucleotide complementary thereto.

In this context, the complementary polynucleotide (complementary strand or reverse strand) means a polynucleotide in a complementary relationship based on A:T (U) and G:C base pairs with a nucleotide sequence defined by any of SEQ ID NOs: 1 to 852 or a nucleotide sequence derived from the nucleotide sequence by the replacement of u with t, the full-length sequence of a polynucleotide consisting of the nucleotide sequence, or a partial sequence thereof (here, this full-length or partial sequence is referred to as a plus strand for the sake of convenience). However, such a complementary strand is not limited to a sequence completely complementary to the nucleotide sequence of the target plus strand and may have a complementary relationship to an extent that permits hybridization under stringent conditions to the target plus strand.

The term "stringent conditions" used herein refers to conditions under which a nucleic acid probe hybridizes to its target sequence to a larger extent (e.g., a measurement value equal to or larger than "(a mean of background measurement values) + (a standard deviation of the background measurement values) $\times$ 2") than that for other sequences. The stringent conditions are dependent on a sequence and differ depending on an environment where hybridization is performed. Specific examples of the "stringent conditions" will be mentioned later.

The term "Tm value" used herein means a temperature at which the double-stranded moiety of a polynucleotide is denatured into single strands so that the double strands and the single strands exist at a ratio of 1:1.

The term "variant" used herein means, in the case of a nucleic acid, a natural variant attributed to polymorphism, mutation, or the like; a variant containing the deletion, substitution, addition, or insertion of 1, 2 or 3 or more (e.g., 1 to multiple) nucleotides in a nucleotide sequence represented by a SEQ ID NO or a nucleotide sequence derived from the nucleotide sequence by the replacement of u with t, or a partial sequence thereof; a variant containing the deletion, substitution, addition, or insertion of 1 or 2 or more nucleotides in a nucleotide sequence of a premature miRNA of the sequence of any of SEQ ID NOs 1 to 235 or a nucleotide sequence derived from the nucleotide sequence by the replacement of u with t, or a partial sequence thereof; a variant that exhibits percent (%) identity of approximately 90% or higher, approximately 95% or higher, approximately 97% or higher, approximately 98% or higher, approximately 99% or higher to each of these nucleotide sequences or the partial sequences thereof; or a nucleic acid hybridizing under the stringent conditions defined above to a polynucleotide or an oligonucleotide comprising each of these nucleotide sequences or the partial sequences thereof.

The term "plurality (multiple)" used herein means an integer of approximately 10, 9, 8, 7, 6, 5, 4, 3, or 2.

The variant as used herein can be prepared by use of a well-known technique such as site-directed mutagenesis or mutagenesis using PCR.

The term "percent (%) identity" used herein can be determined with or without an introduced gap, using a protein or gene search system based on BLAST (https://blast.ncbi.nlm.nih.gov/Blast.cgi) or FASTA (http://www.genome.jp/tools/fasta/) (Zheng Zhang et al., 2000, J. Comput. Biol., Vol. 7, p. 203-214; Altschul, S.F. et al., 1990, Journal of Molecular Biology, Vol. 215, p. 403-410; and Pearson, W.R. et al., 1988, Proc. Natl. Acad. Sci. U. S. A., Vol. 85, p. 2444-2448).

The term "derivative" used herein is meant to include unlimitedly a modified nucleic acid, for example, a derivative labeled with a fluorophore or the like, a derivative containing a modified nucleotide (e.g., a nucleotide containing a group such as halogen, alkyl such as methyl, alkoxy such as methoxy, thio, or carboxymethyl, and a nucleotide that has undergone base rearrangement, double bond saturation, deamination, replacement of an oxygen molecule with a sulfur atom, etc.), PNA (peptide nucleic acid; Nielsen, P.E. et al., 1991, Science, Vol. 254, p. 1497-500), and LNA (locked nucleic acid; Obika, S. et al., 1998, Tetrahedron Lett., Vol. 39, p. 5401-5404).

As used herein, the "nucleic acid" capable of specifically binding to a polynucleotide selected from the marker miRNAs for discriminating between ovarian cancer and benign ovarian tumor described above is a synthesized or prepared nucleic acid and specifically includes a "nucleic acid probe" and/or a "primer", and is utilized directly or indirectly for detecting the presence or absence of benign ovarian tumor in a subject, for diagnosing the presence or absence or the severity of ovarian cancer, the presence or absence or the degree of amelioration of ovarian cancer, or the therapeutic sensitivity of ovarian cancer, or for screening for a candidate substance useful in the prevention, amelioration, or treatment of ovarian cancer. The "nucleic acid" includes a nucleotide, an oligonucleotide, and a polynucleotide capable of specifically recognizing and binding to a transcript represented by any of SEQ ID NOs: 1 to 852 or a synthetic cDNA nucleic acid thereof *in vivo*, particularly, in a sample such as a body fluid (e.g., blood or urine), in relation to the development of ovarian tumor. The nucleotide, the oligonucleotide, and the polynucleotide can be effectively used as probes for detecting the aforementioned nucleic acid expressed *in vivo*, in tissues, in cells, or the like on the basis of the properties described above, or as primers for amplifying the aforementioned nucleic acid expressed *in vivo*.

The term "detection" used herein is interchangeable with the term "examination", "measurement", "detection", or "decision support". As used herein, the term "evaluation" is meant to include diagnosing- or evaluation-supporting on the basis of examination results or measurement results.

The term "subject" used herein is a mammal such as a primate including a human and a chimpanzee, a pet animal including a dog and a cat, a livestock animal including cattle, a horse, sheep, and a goat, a rodent including a mouse and a rat, and animals raised in a zoo and means an animal that is subjected to determination of the presence of ovarian cancer or the presence of benign ovarian tumor. The subject is preferably a human. The term "patient" is a mammal and means an animal known to have ovarian cancer or benign ovarian tumor. The patient is preferably a human. The term "healthy subject" also means such a mammal, which is an animal without the cancer to be detected. The healthy subject is preferably a human.

The term "ovarian cancer" used herein is a malignant tumor which develops in the ovary and includes, for example, but not limited to, epithelial ovarian cancer which develops from the mucosal epithelium, and germ cell stromal ovarian cancers. The malignant tumor refers to a tumor that (will) infiltrates and/or metastasizes at present or in the future. Whether a tumor is a malignant tumor or not may be confirmed based on the following observation: infiltrated cells, high cellular atypia, immature differentiation as to structural atypia, or others, mainly in a histological test (pathological test).

The term "benign ovarian tumor" used herein is a benign tumor which develops in the ovary and includes, for example, but not limited to, mucinous cystadenoma, serous cystadenoma, mature teratoma and fibroma. The benign tumor refers to a tumor that does (or will) not infiltrate and metastasize at present or in the future. Whether a tumor is a benign tumor or not can be determined based on the following observation: no infiltrated cell, being localized, low cellular atypia, mature differentiationas to structural atypia, or others, mainly in a histological test (pathological test). The "cellular atypia" refers to deviation from a normal cell structure and specifically refers to an increase in nuclear-cytoplasmic ratio, different sizes of cells or nuclei (anisokaryosis), an irregular nuclear shape, an increased amount of nuclear chromatin, an increased size or number of nucleolus, an increase of karyomitotic image, and/or appearance of an abnormal karyomitotic image. The "structural atypia" refers to deviation from a normal tissue structure, i.e., an increased irregularity of tissue structure. The structural atypia is roughly classified into immature and mature types depending on the state of differentiation of cells constituting a tissue found to have the structural atypia.

The term "P" or "P value" used herein refers to a probability at which a more extreme statistic than that actually calculated from data under null hypothesis is observed in a statistical test. Thus, smaller "P" or "P value" is regarded as being a more significant difference between subjects to be compared.

The term "sensitivity" used herein means a value of (the number of true positives) / (the number of true positives + the number of false negatives). High sensitivity allows ovarian tumor to be detected early, leading to the complete resection of cancer sites and reduction in the rate of recurrence.

The term "specificity" used herein means a value of (the number of true negatives) / (the number of true negatives + the number of false positives). High specificity prevents needless extra examination for ovarian benign tumor patients misjudged as being ovarian tumor patients, leading to reduction in burden on patients and reduction in medical expense.

The term "accuracy" used herein means a value of (the number of true positives + the number of true negatives) / (the total number of cases). The accuracy indicates the ratio of samples that are identified correctly to all samples, and serves as a primary index for evaluating detection performance.

As used herein, the "sample" that is subjected to determination, detection, or diagnosis refers to a tissue and a biological material in which the expression of the gene of the present invention varies as ovarian tumor or ovarian benign tumor develops, as ovarian tumor progresses, or as therapeutic effects on ovarian tumor are exerted. Specifically, the sample refers to an ovarian tissue and fallopian tube, lymph node and a surrounding organ thereof, an organ suspected of having metastasis, the skin, a body fluid such as blood, urine, saliva, sweat, or tissue exudates, serum or plasma prepared from blood, feces, hair, and the like. The "sample" further refers to a biological sample extracted therefrom, specifically, a sample containing a transcript such as RNA or miRNA.

The term "hsa-miR-1908-5p gene" or "hsa-miR-1908-5p" used herein includes the hsa-miR-1908-5p gene (miRBase Accession No. MIMAT0007881) described in SEQ ID NO: 1, its homolog or ortholog of a different organism species, and the like. The hsa-miR-1908-5p gene can be obtained by a method described in Bar M et al., 2008, Stem Cells, Vol. 26, p. 2496-2505. Also, "hsa-mir-1908" (miRBase Accession No. MI0008329, SEQ ID NO: 236) having a hairpin-like structure is known as a precursor of "hsa-miR-1908-5p".

The term "hsa-miR-4723-5p gene" or "hsa-miR-4723-5p" used herein includes the hsa-miR-4723-5p gene (miRBase Accession No. MIMAT0019838) described in SEQ ID NO: 2, its homolog or ortholog of a different organism species, and the like. The hsa-miR-4723-5p gene can be obtained by a method described in Persson H et al., 2011, Cancer Res, Vol. 71, p. 78-86. Also, "hsa-mir-4723" (miRBase Accession No. MI0017359, SEQ ID NO: 237) having a hairpin-like structure is known as a precursor of "hsa-miR-4723-5p".

The term "hsa-miR-4674 gene" or "hsa-miR-4674" used herein includes the hsa-miR-4674 gene (miRBase Accession No. MIMAT0019756) described in SEQ ID NO: 3, its homolog or ortholog of a different organism species, and the like. The hsa-miR-4674 gene can be obtained by a method described in Persson H et al., 2011, Cancer Res, Vol. 71, p. 78-86. Also, "hsa-mir-4674" (miRBase Accession No. MI0017305, SEQ ID NO: 238) having a hairpin-like structure is known as a precursor of "hsa-miR-4674".

The term "hsa-miR-939-5p gene" or "hsa-miR-939-5p" used herein includes the hsa-miR-939-5p gene (miRBase Accession No. MIMAT0004982) described in SEQ ID NO: 4, its homolog or ortholog of a different organism species, and the like. The hsa-miR-939-5p gene can be obtained by a method described in Lui WO et al., 2007, Cancer Res, Vol. 67, p. 6031-6043. Also, "hsa-mir-939" (miRBase Accession No. MI0005761, SEQ ID NO: 239) having a hairpin-like structure is known as a precursor of "hsa-miR-939-5p".

The term "hsa-miR-6789-5p gene" or "hsa-miR-6789-5p" used herein includes the hsa-miR-6789-5p gene (miRBase Accession No. MIMAT0027478) described in SEQ ID NO: 5, its homolog or ortholog of a different organism species, and the like. The hsa-miR-6789-5p gene can be obtained by a method described in Ladewig E et al., 2012, Genome Res, Vol. 22, p. 1634-1645. Also, "hsa-mir-6789" (miRBase Accession No. MI0022634, SEQ ID NO: 240) having a hairpin-like structure is known as a precursor of "hsa-miR-6789-5p".

The term "hsa-miR-1268a gene" or "hsa-miR-1268a" used herein includes the hsa-miR-1268a gene (miRBase Accession No. MIMAT0005922) described in SEQ ID NO: 6, its homolog or ortholog of a different organism species, and the like. The hsa-miR-1268a gene can be obtained by a method described in Morin RD et al., 2008, Genome Res, Vol. 18, p. 610-621. Also, "hsa-mir-1268a" (miRBase Accession No. MI0006405, SEQ ID NO: 241) having a hairpin-like structure is known as a precursor of "hsa-miR-1268a".

The term "hsa-miR-1202 gene" or "hsa-miR-1202" used herein includes the hsa-miR-1202 gene (miRBase Accession No. MIMAT0005865) described in SEQ ID NO: 7, its homolog or ortholog of a different organism species, and the like. The hsa-miR-1202 gene can be obtained by a method described in Marton S et al., 2008, Leukemia, Vol. 22, p. 330-338. Also, "hsa-mir-1202" (miRBase Accession No. MI0006334, SEQ ID NO: 242) having a hairpin-like structure is known as a precursor of "hsa-miR-1202".

The term "hsa-miR-4525 gene" or "hsa-miR-4525" used herein includes the hsa-miR-4525 gene (miRBase Accession No. MIMAT0019064) described in SEQ ID NO: 8, its homolog or ortholog of a different organism species, and the like. The hsa-miR-4525 gene can be obtained by a method described in Jima DD et al., 2010, Blood, Vol. 116, e118-e127. Also, "hsa-mir-4525" (miRBase Accession No. MI0016892, SEQ ID NO: 243) having a hairpin-like structure is known as a precursor of "hsa-miR-4525".

The term "hsa-miR-128-1-5p gene" or "hsa-miR-128-1-5p" used herein includes the hsa-miR-128-1-5p gene (miRBase Accession No. MIMAT0026477) described in SEQ ID NO: 9, its homolog or ortholog of a different organism species, and the like. The hsa-miR-128-1-5p gene can be obtained by a method described in Lagos-Quintana M et al., 2002, Curr Biol, Vol. 12, p. 735-739. Also, "hsa-mir-128-1" (miRBase Accession No. MI0000447, SEQ ID NO: 244) having a hairpin-like structure is known as a precursor of "hsa-miR-128-1-5p".

The term "hsa-miR-6806-5p gene" or "hsa-miR-6806-5p" used herein includes the hsa-miR-6806-5p gene (miRBase Accession No. MIMAT0027512) described in SEQ ID NO: 10, its homolog or ortholog of a different organism species, and the like. The hsa-miR-6806-5p gene can be obtained by a method described in Ladewig E et al., 2012, Genome Res, Vol. 22, p. 1634-1645. Also, "hsa-mir-6806" (miRBase Accession No. MI0022651, SEQ ID NO: 245) having a hairpin-like structure is known as a precursor of "hsa-miR-6806-5p".

The term "hsa-miR-7845-5p gene" or "hsa-miR-7845-5p" used herein includes the hsa-miR-7845-5p gene (miRBase Accession No. MIMAT0030420) described in SEQ ID NO: 11, its homolog or ortholog of a different organism species, and the like. The hsa-miR-7845-5p gene can be obtained by a method described in Ple H et al., 2012, PLoS One, Vol. 7, e50746. Also, "hsa-mir-7845" (miRBase Accession No. MI0025515, SEQ ID NO: 246) having a hairpin-like structure is known as a precursor of "hsa-miR-7845-5p".

The term "hsa-miR-4632-5p gene" or "hsa-miR-4632-5p" used herein includes the hsa-miR-4632-5p gene (miRBase Accession No. MIMAT0022977) described in SEQ ID NO: 12, its homolog or ortholog of a different organism species, and the like. The hsa-miR-4632-5p gene can be obtained by a method described in Persson H et al., 2011, Cancer Res, Vol. 71, p. 78-86. Also, "hsa-mir-4632" (miRBase Accession No. MI0017259, SEQ ID NO: 247) having a hairpin-like structure is known as a precursor of "hsa-miR-4632-5p".

The term "hsa-miR-10396b-5p gene" or "hsa-miR-10396b-5p" used herein includes the hsa-miR-10396b-5p gene (miRBase Accession No. MIMAT0041635) described in SEQ ID NO: 13, its homolog or ortholog of a different organism species, and the like. The hsa-miR-10396b-5p gene can be obtained by a method described in Lim EL et al., 2015, Genome Biol, Vol. 16, p. 18. Also, "hsa-mir-10396b" (miRBase Accession No. MI0033426, SEQ ID NO: 248) having a hairpin-like structure is known as a precursor of "hsa-miR-10396b-5p".

The term "hsa-miR-6768-5p gene" or "hsa-miR-6768-5p" used herein includes the hsa-miR-6768-5p gene (miRBase Accession No. MIMAT0027436) described in SEQ ID NO: 14, its homolog or ortholog of a different organism species, and the like. The hsa-miR-6768-5p gene can be obtained by a method described in Ladewig E et al., 2012, Genome Res, Vol. 22, p. 1634-1645. Also, "hsa-mir-6768" (miRBase Accession No. MI0022613, SEQ ID NO: 249) having a hairpin-like structure is known as a precursor of "hsa-miR-6768-5p".

The term "hsa-miR-8059 gene" or "hsa-miR-8059" used herein includes the hsa-miR-8059 gene (miRBase Accession No. MIMAT0030986) described in SEQ ID NO: 15, its homolog or ortholog of a different organism species, and the like. The hsa-miR-8059 gene can be obtained by a method described in Wang HJ et al., 2013, Shock, Vol. 39, p. 480-487. Also, "hsa-mir-8059" (miRBase Accession No. MI0025895, SEQ ID NO: 250) having a hairpin-like structure is known as a precursor of "hsa-miR-8059".

The term "hsa-miR-8072 gene" or "hsa-miR-8072" used herein includes the hsa-miR-8072 gene (miRBase Accession No. MIMAT0030999) described in SEQ ID NO: 16, its homolog or ortholog of a different organism species, and the like. The hsa-miR-8072 gene can be obtained by a method described in Wang HJ et al., 2013, Shock, Vol. 39, p. 480-487. Also, "hsa-mir-8072" (miRBase Accession No. MI0025908, SEQ ID NO: 251) having a hairpin-like structure is known as a precursor of "hsa-miR-8072".

The term "hsa-miR-9901 gene" or "hsa-miR-9901" used herein includes the hsa-miR-9901 gene (miRBase Accession No. MIMAT0039321) described in SEQ ID NO: 17, its homolog or ortholog of a different organism species, and the like. The hsa-miR-9901 gene can be obtained by a method described in Boele J et al., 2014, Proc Natl Acad Sci USA, Vol. 111, p. 11467-11472. Also, "hsa-mir-9901" (miRBase Accession No. MI0031829, SEQ ID NO: 252) having a hairpin-like structure is known as a precursor of "hsa-miR-9901".

The term "hsa-miR-1231 gene" or "hsa-miR-1231" used herein includes the hsa-miR-1231 gene (miRBase Accession No. MIMAT0005586) described in SEQ ID NO: 18, its homolog or ortholog of a different organism species, and the like. The hsa-miR-1231 gene can be obtained by a method described in Berezikov E et al., 2007, Mol Cell, Vol. 28, p. 328-336. Also, "hsa-mir-1231" (miRBase Accession No. MI0006321, SEQ ID NO: 253) having a hairpin-like structure is known as a precursor of "hsa-miR-1231".

The term "hsa-miR-1225-5p gene" or "hsa-miR-1225-5p" used herein includes the hsa-miR-1225-5p gene (miRBase Accession No. MIMAT0005572) described in SEQ ID NO: 19, its homolog or ortholog of a different organism species, and the like. The hsa-miR-1225-5p gene can be obtained by a method described in Berezikov E et al., 2007, Mol Cell, Vol. 28, p. 328-336. Also, "hsa-mir-1225" (miRBase Accession No. MI0006311, SEQ ID NO: 254) having a hairpin-like structure is known as a precursor of "hsa-miR-1225-5p".

The term "hsa-miR-12114 gene" or "hsa-miR-12114" used herein includes the hsa-miR-12114 gene (miRBase Accession No. MIMAT0049008) described in SEQ ID NO: 20, its homolog or ortholog of a different organism species, and the like. The hsa-miR-12114 gene can be obtained by a method described in Ozata DM et al., 2017, Cell Death Dis, Vol. 8, e2759. Also, "hsa-mir-12114" (miRBase Accession No. MI0039716, SEQ ID NO: 255) having a hairpin-like structure is known as a precursor of "hsa-miR-12114".

The term "hsa-miR-3178 gene" or "hsa-miR-3178" used herein includes the hsa-miR-3178 gene (miRBase Accession No. MIMAT0015055) described in SEQ ID NO: 21, its homolog or ortholog of a different organism species, and the like. The hsa-miR-3178 gene can be obtained by a method described in Stark MS et al., 2010, PLoS One, Vol. 5, e9685. Also, "hsa-mir-3178" (miRBase Accession No. MI0014212, SEQ ID NO: 256) having a hairpin-like structure is known as a precursor of "hsa-miR-3178".

The term "hsa-miR-6798-5p gene" or "hsa-miR-6798-5p" used herein includes the hsa-miR-6798-5p gene (miRBase Accession No. MIMAT0027496) described in SEQ ID NO: 22, its homolog or ortholog of a different organism species, and the like. The hsa-miR-6798-5p gene can be obtained by a method described in Ladewig E et al., 2012, Genome Res, Vol. 22, p. 1634-1645. Also, "hsa-mir-6798" (miRBase Accession No. MI0022643, SEQ ID NO: 257) having a hairpin-like structure is known as a precursor of "hsa-miR-6798-5p".

The term "hsa-miR-4276 gene" or "hsa-miR-4276" used herein includes the hsa-miR-4276 gene (miRBase Accession No. MIMAT0016904) described in SEQ ID NO: 23, its homolog or ortholog of a different organism species, and the like. The hsa-miR-4276 gene can be obtained by a method described in Goff LA et al., 2009, PLoS One, Vol. 4, e7192. Also, "hsa-mir-4276" (miRBase Accession No. MI0015882, SEQ ID NO: 258) having a hairpin-like structure is known as a precursor of "hsa-miR-4276".

The term "hsa-miR-6125 gene" or "hsa-miR-6125" used herein includes the hsa-miR-6125 gene (miRBase Accession No. MIMAT0024598) described in SEQ ID NO: 24, its homolog or ortholog of a different organism species, and the like. The hsa-miR-6125 gene can be obtained by a method described in Smith JL et al., 2012, J Virol, Vol. 86, p. 5278-5287. Also, "hsa-mir-6125" (miRBase Accession No. MI0021259, SEQ ID NO: 259) having a hairpin-like structure is known as a precursor of "hsa-miR-6125".

The term "hsa-miR-3652 gene" or "hsa-miR-3652" used herein includes the hsa-miR-3652 gene (miRBase Accession No. MIMAT0018072) described in SEQ ID NO: 25, its homolog or ortholog of a different organism species, and the like. The hsa-miR-3652 gene can be obtained by a method described in Meiri E et al., 2010, Nucleic Acids Res, Vol. 38, p. 6234-6246. Also, "hsa-mir-3652" (miRBase Accession No. MI0016052, SEQ ID NO: 260) having a hairpin-like structure is known as a precursor of "hsa-miR-3652".

The term "hsa-miR-7111-Sp gene" or "hsa-miR-7111-Sp" used herein includes the hsa-miR-7111-5p gene (miRBase Accession No. MIMAT0028119) described in SEQ ID NO: 26, its homolog or ortholog of a different organism species, and the like. The hsa-miR-7111-5p gene can be obtained by a method described in Ladewig E et al., 2012, Genome Res, Vol. 22, p. 1634-1645. Also, "hsa-mir-7111" (miRBase Accession No. MI0022962, SEQ ID NO: 261) having a hairpin-like structure is known as a precursor of "hsa-miR-7111-5p".

The term "hsa-miR-6749-5p gene" or "hsa-miR-6749-5p" used herein includes the hsa-miR-6749-5p gene (miRBase Accession No. MIMAT0027398) described in SEQ ID NO: 27, its homolog or ortholog of a different organism species, and the like. The hsa-miR-6749-5p gene can be obtained by a method described in Ladewig E et al., 2012, Genome Res, Vol. 22, p. 1634-1645. Also, "hsa-mir-6749" (miRBase Accession No. MI0022594, SEQ ID NO: 262) having a hairpin-like structure is known as a precursor of "hsa-miR-6749-5p".

The term "hsa-miR-1199-Sp gene" or "hsa-miR-1199-Sp" used herein includes the hsa-miR-1199-Sp gene (miRBase Accession No. MIMAT0031119) described in SEQ ID NO: 28, its homolog or ortholog of a different organism species, and the like. The hsa-miR-1199-5p gene can be obtained by a method described in Salvi A et al., 2013, Int J Oncol, Vol. 42, p. 391-402. Also, "hsa-mir-1199" (miRBase Accession No. MI0020340, SEQ ID NO: 263) having a hairpin-like structure is known as a precursor of "hsa-miR-1199-Sp".

The term "hsa-miR-6802-5p gene" or "hsa-miR-6802-5p" used herein includes the hsa-miR-6802-5p gene (miRBase Accession No. MIMAT0027504) described in SEQ ID NO: 29, its homolog or ortholog of a different organism species, and the like. The hsa-miR-6802-5p gene can be obtained by a method described in Ladewig E et al., 2012, Genome Res, Vol. 22, p. 1634-1645. Also, "hsa-mir-6802" (miRBase Accession No. MI0022647, SEQ ID NO: 264) having a hairpin-like structure is known as a precursor of "hsa-miR-6802-5p".

The term "hsa-miR-6816-5p gene" or "hsa-miR-6816-5p" used herein includes the hsa-miR-6816-5p gene (miRBase Accession No. MIMAT0027532) described in SEQ ID NO: 30, its homolog or ortholog of a different organism species, and the like. The hsa-miR-6816-5p gene can be obtained by a method described in Ladewig E et al., 2012, Genome Res, Vol. 22, p. 1634-1645. Also, "hsa-mir-6816" (miRBase Accession No. MI0022661, SEQ ID NO: 265) having a hairpin-like structure is known as a precursor of "hsa-miR-6816-5p".

The term "hsa-miR-4706 gene" or "hsa-miR-4706" used herein includes the hsa-miR-4706 gene (miRBase Accession No. MIMAT0019806) described in SEQ ID NO: 31, its homolog or ortholog of a different organism species, and the like. The hsa-miR-4706 gene can be obtained by a method described in Persson H et al., 2011, Cancer Res, Vol. 71, p. 78-86. Also, "hsa-mir-4706" (miRBase Accession No. MI0017339, SEQ ID NO: 266) having a hairpin-like structure is known as a precursor of "hsa-miR-4706".

The term "hsa-miR-5008-5p gene" or "hsa-miR-5008-5p" used herein includes the hsa-miR-5008-5p gene (miRBase Accession No. MIMAT0021039) described in SEQ ID NO: 32, its homolog or ortholog of a different organism species, and the like. The hsa-miR-5008-5p gene can be obtained by a method described in Hansen TB et al., 2011, RNA Biol, Vol. 8, p. 378-383. Also, "hsa-mir-5008" (miRBase Accession No. MI0017876, SEQ ID NO: 267) having a hairpin-like structure is known as a precursor of "hsa-miR-5008-5p".

The term "hsa-miR-6797-5p gene" or "hsa-miR-6797-5p" used herein includes the hsa-miR-6797-5p gene (miRBase Accession No. MIMAT0027494) described in SEQ ID NO: 33, its homolog or ortholog of a different organism species, and the like. The hsa-miR-6797-5p gene can be obtained by a method described in Ladewig E et al., 2012, Genome Res, Vol. 22, p. 1634-1645. Also, "hsa-mir-6797" (miRBase Accession No. MI0022642, SEQ ID NO: 268) having a hairpin-like structure is known as a precursor of "hsa-miR-6797-5p".

The term "hsa-miR-4516 gene" or "hsa-miR-4516" used herein includes the hsa-miR-4516 gene (miRBase Accession No. MIMAT0019053) described in SEQ ID NO: 34, its homolog or ortholog of a different organism species, and the like. The hsa-miR-4516 gene can be obtained by a method described in Jima DD et al., 2010, Blood, Vol. 116, e118-e127. Also, "hsa-mir-4516" (miRBase Accession No. MI0016882, SEQ ID NO: 269) having a hairpin-like structure is known as a precursor of "hsa-miR-4516".

The term "hsa-miR-4508 gene" or "hsa-miR-4508" used herein includes the hsa-miR-4508 gene (miRBase Accession No. MIMAT0019045) described in SEQ ID NO: 35, its homolog or ortholog of a different organism species, and the like. The hsa-miR-4508 gene can be obtained by a method described in Jima DD et al., 2010, Blood, Vol. 116, e118-e127. Also, "hsa-mir-4508" (miRBase Accession No. MI0016872, SEQ ID NO: 270) having a hairpin-like structure is known as a precursor of "hsa-miR-4508".

The term "hsa-miR-6729-5p gene" or "hsa-miR-6729-5p" used herein includes the hsa-miR-6729-5p gene (miRBase Accession No. MIMAT0027359) described in SEQ ID NO: 36, its homolog or ortholog of a different organism species, and the like. The hsa-miR-6729-5p gene can be obtained by a method described in Ladewig E et al., 2012, Genome Res, Vol. 22, p. 1634-1645. Also, "hsa-mir-6729" (miRBase Accession No. MI0022574, SEQ ID NO: 271) having a hairpin-like structure is known as a precursor of "hsa-miR-6729-5p".

The term "hsa-miR-564 gene" or "hsa-miR-564" used herein includes the hsa-miR-564 gene (miRBase Accession No. MIMAT0003228) described in SEQ ID NO: 37, its homolog or ortholog of a different organism species, and the like. The hsa-miR-564 gene can be obtained by a method described in Cummins JM et al., 2006, Proc Natl Acad Sci USA, Vol. 103, p. 3687-3692. Also, "hsa-mir-564" (miRBase Accession No. MI0003570, SEQ ID NO: 272) having a hairpin-like structure is known as a precursor of "hsa-miR-564".

The term "hsa-miR-1233-5p gene" or "hsa-miR-1233-5p" used herein includes the hsa-miR-1233-5p gene (miRBase Accession No. MIMAT0022943) described in SEQ ID NO: 38, its homolog or ortholog of a different organism species, and the like. The hsa-miR-1233-5p gene can be obtained by a method described in Berezikov E et al., 2007, Mol Cell, Vol. 28, p. 328-336. Also, "hsa-mir-1233-1" (miRBase Accession No. MI0006323, SEQ ID NO: 273) having a hairpin-like structure is known as a precursor of "hsa-miR-1233-5p".

The term "hsa-miR-6127 gene" or "hsa-miR-6127" used herein includes the hsa-miR-6127 gene (miRBase Accession No. MIMAT0024610) described in SEQ ID NO: 39, its homolog or ortholog of a different organism species, and the like. The hsa-miR-6127 gene can be obtained by a method described in Dannemann M et al., 2012, Genome Biol Evol, Vol. 4, p. 552-564. Also, "hsa-mir-6127" (miRBase Accession No. MI0021271, SEQ ID NO: 274) having a hairpin-like structure is known as a precursor of "hsa-miR-6127".

The term "hsa-miR-1469 gene" or "hsa-miR-1469" used herein includes the hsa-miR-1469 gene (miRBase Accession No. MIMAT0007347) described in SEQ ID NO: 40, its homolog or ortholog of a different organism species, and the like. The hsa-miR-1469 gene can be obtained by a method described in Kawaji H et al., 2008, BMC Genomics, Vol. 9, p. 157. Also, "hsa-mir-1469" (miRBase Accession No. MI0007074, SEQ ID NO: 275) having a hairpin-like structure is known as a precursor of "hsa-miR-1469".

The term "hsa-miR-6738-5p gene" or "hsa-miR-6738-5p" used herein includes the hsa-miR-6738-5p gene (miRBase Accession No. MIMAT0027377) described in SEQ ID NO: 41, its homolog or ortholog of a different organism species, and the like. The hsa-miR-6738-5p gene can be obtained by a method described in Ladewig E et al., 2012, Genome Res, Vol. 22, p. 1634-1645. Also, "hsa-mir-6738" (miRBase Accession No. MI0022583, SEQ ID NO: 276) having a hairpin-like structure is known as a precursor of "hsa-miR-6738-5p".

The term "hsa-miR-6785-5p gene" or "hsa-miR-6785-5p" used herein includes the hsa-miR-6785-5p gene (miRBase Accession No. MIMAT0027470) described in SEQ ID NO: 42, its homolog or ortholog of a different organism species, and the like. The hsa-miR-6785-5p gene can be obtained by a method described in Ladewig E et al., 2012, Genome Res, Vol. 22, p. 1634-1645. Also, "hsa-mir-6785" (miRBase Accession No. MI0022630, SEQ ID NO: 277) having a hairpin-like structure is known as a precursor of "hsa-miR-6785-5p".

The term "hsa-miR-10401-5p gene" or "hsa-miR-10401-5p" used herein includes the hsa-miR-10401-5p gene (miRBase Accession No. MIMAT0041633) described in SEQ ID NO: 43, its homolog or ortholog of a different organism species, and the like. The hsa-miR-10401-5p gene can be obtained by a method described in Lim EL et al., 2015, Genome Biol, Vol. 16, p. 18. Also, "hsa-mir-10401" (miRBase Accession No. MI0033425, SEQ ID NO: 278) having a hairpin-like structure is known as a precursor of "hsa-miR-10401-5p".

The term "hsa-miR-4430 gene" or "hsa-miR-4430" used herein includes the hsa-miR-4430 gene (miRBase Accession No. MIMAT0018945) described in SEQ ID NO: 44, its homolog or ortholog of a different organism species, and the like. The hsa-miR-4430 gene can be obtained by a method described in Jima DD et al., 2010, Blood, Vol. 116, e118-e127. Also, "hsa-mir-4430" (miRBase Accession No. MI0016769, SEQ ID NO: 279) having a hairpin-like structure is known as a precursor of "hsa-miR-4430".

The term "hsa-miR-6889-5p gene" or "hsa-miR-6889-5p" used herein includes the hsa-miR-6889-5p gene (miRBase Accession No. MIMAT0027678) described in SEQ ID NO: 45, its homolog or ortholog of a different organism species, and the like. The hsa-miR-6889-5p gene can be obtained by a method described in Ladewig E et al., 2012, Genome Res, Vol. 22, p. 1634-1645. Also, "hsa-mir-6889" (miRBase Accession No. MI0022736, SEQ ID NO: 280) having a hairpin-like structure is known as a precursor of "hsa-miR-6889-5p".

The term "hsa-miR-1236-5p gene" or "hsa-miR-1236-5p" used herein includes the hsa-miR-1236-5p gene (miRBase Accession No. MIMAT0022945) described in SEQ ID NO: 46, its homolog or ortholog of a different organism species, and the like. The hsa-miR-1236-5p gene can be obtained by a method described in Berezikov E et al., 2007, Mol Cell, Vol. 28, p. 328-336. Also, "hsa-mir-1236" (miRBase Accession No. MI0006326, SEQ ID NO: 281) having a hairpin-like structure is known as a precursor of "hsa-miR-1236-5p".

The term "hsa-miR-3176 gene" or "hsa-miR-3176" used herein includes the hsa-miR-3176 gene (miRBase Accession No. MIMAT0015053) described in SEQ ID NO: 47, its homolog or ortholog of a different organism species, and the like. The hsa-miR-3176 gene can be obtained by a method described in Stark MS et al., 2010, PLoS One, Vol. 5, e9685. Also, "hsa-mir-3176" (miRBase Accession No. MI0014210, SEQ ID NO: 282) having a hairpin-like structure is known as a precursor of "hsa-miR-3176".

The term "hsa-miR-3141 gene" or "hsa-miR-3141" used herein includes the hsa-miR-3141 gene (miRBase Accession No. MIMAT0015010) described in SEQ ID NO: 48, its homolog or ortholog of a different organism species, and the like. The hsa-miR-3141 gene can be obtained by a method described in Stark MS et al., 2010, PLoS One, Vol. 5, e9685. Also, "hsa-mir-3141" (miRBase Accession No. MI0014165, SEQ ID NO: 283) having a hairpin-like structure is known as a precursor of "hsa-miR-3141".

The term "hsa-miR-3928-3p gene" or "hsa-miR-3928-3p" used herein includes the hsa-miR-3928-3p gene (miRBase Accession No. MIMAT0018205) described in SEQ ID NO: 49, its homolog or ortholog of a different organism species, and the like. The hsa-miR-3928-3p gene can be obtained by a method described in Creighton CJ et al., 2010, PLoS One, Vol. 5, e9637. Also, "hsa-mir-3928" (miRBase Accession No. MI0016438, SEQ ID NO: 284) having a hairpin-like structure is known as a precursor of "hsa-miR-3928-3p".

The term "hsa-miR-1237-5p gene" or "hsa-miR-1237-5p" used herein includes the hsa-miR-1237-5p gene (miRBase Accession No. MIMAT0022946) described in SEQ ID NO: 50, its homolog or ortholog of a different organism species, and the like. The hsa-miR-1237-5p gene can be obtained by a method described in Berezikov E et al., 2007, Mol Cell, Vol. 28, p. 328-336. Also, "hsa-mir-1237" (miRBase Accession No. MI0006327, SEQ ID NO: 285) having a hairpin-like structure is known as a precursor of "hsa-miR-1237-5p".

The term "hsa-miR-1915-3p gene" or "hsa-miR-1915-3p" used herein includes the hsa-miR-1915-3p gene (miRBase Accession No. MIMAT0007892) described in SEQ ID NO: 51, its homolog or ortholog of a different organism species, and the like. The hsa-miR-1915-3p gene can be obtained by a method described in Bar M et al., 2008, Stem Cells, Vol. 26, p. 2496-2505. Also, "hsa-mir-1915" (miRBase Accession No. MI0008336, SEQ ID NO: 286) having a hairpin-like structure is known as a precursor of "hsa-miR-1915-3p".

The term "hsa-miR-5195-3p gene" or "hsa-miR-5195-3p" used herein includes the hsa-miR-5195-3p gene (miRBase Accession No. MIMAT0021127) described in SEQ ID NO: 52, its homolog or ortholog of a different organism species, and the like. The hsa-miR-5195-3p gene can be obtained by a method described in Schotte D et al., 2011, Leukemia, Vol. 25, p. 1389-1399. Also, "hsa-mir-5195" (miRBase Accession No. MI0018174, SEQ ID NO: 287) having a hairpin-like structure is known as a precursor of "hsa-miR-5195-3p".

The term "hsa-miR-6743-5p gene" or "hsa-miR-6743-5p" used herein includes the hsa-miR-6743-5p gene (miRBase Accession No. MIMAT0027387) described in SEQ ID NO: 53, its homolog or ortholog of a different organism species, and the like. The hsa-miR-6743-5p gene can be obtained by a method described in Ladewig E et al., 2012, Genome Res, Vol. 22, p. 1634-1645. Also, "hsa-mir-6743" (miRBase Accession No. MI0022588, SEQ ID NO: 288) having a hairpin-like structure is known as a precursor of "hsa-miR-6743-5p".

The term "hsa-miR-6746-5p gene" or "hsa-miR-6746-5p" used herein includes the hsa-miR-6746-5p gene (miRBase Accession No. MIMAT0027392) described in SEQ ID NO: 54, its homolog or ortholog of a different organism species, and the like. The hsa-miR-6746-5p gene can be obtained by a method described in Ladewig E et al., 2012, Genome Res, Vol. 22, p. 1634-1645. Also, "hsa-mir-6746" (miRBase Accession No. MI0022591, SEQ ID NO: 289) having a hairpin-like structure is known as a precursor of "hsa-miR-6746-5p".

The term "hsa-miR-4446-3p gene" or "hsa-miR-4446-3p" used herein includes the hsa-miR-4446-3p gene (miRBase Accession No. MIMAT0018965) described in SEQ ID NO: 55, its homolog or ortholog of a different organism species, and the like. The hsa-miR-4446-3p gene can be obtained by a method described in Jima DD et al., 2010, Blood, Vol. 116, e118-e127. Also, "hsa-mir-4446" (miRBase Accession No. MI0016789, SEQ ID NO: 290) having a hairpin-like structure is known as a precursor of "hsa-miR-4446-3p".

The term "hsa-miR-1228-5p gene" or "hsa-miR-1228-5p" used herein includes the hsa-miR-1228-5p gene (miRBase Accession No. MIMAT0005582) described in SEQ ID NO: 56, its homolog or ortholog of a different organism species, and the like. The hsa-miR-1228-5p gene can be obtained by a method described in Berezikov E et al., 2007, Mol Cell, Vol. 28, p. 328-336. Also, "hsa-mir-1228" (miRBase Accession No. MI0006318, SEQ ID NO: 291) having a hairpin-like structure is known as a precursor of "hsa-miR-1228-5p".

The term "hsa-miR-1268b gene" or "hsa-miR-1268b" used herein includes the hsa-miR-1268b gene (miRBase Accession No. MIMAT0018925) described in SEQ ID NO: 57, its homolog or ortholog of a different organism species, and the like. The hsa-miR-1268b gene can be obtained by a method described in Jima DD et al., 2010, Blood, Vol. 116, e118-e127. Also, "hsa-mir-1268b" (miRBase Accession No. MI0016748, SEQ ID NO: 292) having a hairpin-like structure is known as a precursor of "hsa-miR-1268b".

The term "hsa-miR-1260a gene" or "hsa-miR-1260a" used herein includes the hsa-miR-1260a gene (miRBase Accession No. MIMAT0005911) described in SEQ ID NO: 58, its homolog or ortholog of a different organism species, and the like. The hsa-miR-1260a gene can be obtained by a method described in Morin RD et al., 2008, Genome Res, Vol. 18, p. 610-621. Also, "hsa-mir-1260a" (miRBase Accession No. MI0006394, SEQ ID NO: 293) having a hairpin-like structure is known as a precursor of "hsa-miR-1260a".

The term "hsa-miR-6879-3p gene" or "hsa-miR-6879-3p" used herein includes the hsa-miR-6879-3p gene (miRBase Accession No. MIMAT0027659) described in SEQ ID NO: 59, its homolog or ortholog of a different organism species, and the like. The hsa-miR-6879-3p gene can be obtained by a method described in Ladewig E et al., 2012, Genome Res, Vol. 22, p. 1634-1645. Also, "hsa-mir-6879" (miRBase Accession No. MI0022726, SEQ ID NO: 294) having a hairpin-like structure is known as a precursor of "hsa-miR-6879-3p".

The term "hsa-miR-149-3p gene" or "hsa-miR-149-3p" used herein includes the hsa-miR-149-3p gene (miRBase Accession No. MIMAT0004609) described in SEQ ID NO: 60, its homolog or ortholog of a different organism species, and the like. The hsa-miR-149-3p gene can be obtained by a method described in Lagos-Quintana M et al., 2002, Curr Biol, Vol. 12, p. 735-739. Also, "hsa-mir-149" (miRBase Accession No. MI0000478, SEQ ID NO: 295) having a hairpin-like structure is known as a precursor of "hsa-miR-149-3p".

The term "hsa-miR-3162-5p gene" or "hsa-miR-3162-5p" used herein includes the hsa-miR-3162-5p gene (miRBase Accession No. MIMAT0015036) described in SEQ ID NO: 61, its homolog or ortholog of a different organism species, and the like. The hsa-miR-3162-5p gene can be obtained by a method described in Stark MS et al., 2010, PLoS One, Vol. 5, e9685. Also, "hsa-mir-3162" (miRBase AccessionNo. MI0014192, SEQ ID NO: 296) having a hairpin-like structure is known as a precursor of "hsa-miR-3162-5p".

The term "hsa-miR-1207-5p gene" or "hsa-miR-1207-5p" used herein includes the hsa-miR-1207-5p gene (miRBase Accession No. MIMAT0005871) described in SEQ ID NO: 62, its homolog or ortholog of a different organism species, and the like. The hsa-miR-1207-5p gene can be obtained by a method described in Huppi K et al., 2008, Mol Cancer Res, Vol. 6, p. 212-221. Also, "hsa-mir-1207" (miRBase Accession No. MI0006340, SEQ ID NO: 297) having a hairpin-like structure is known as a precursor of "hsa-miR-1207-5p".

The term "hsa-miR-4747-3p gene" or "hsa-miR-4747-3p" used herein includes the hsa-miR-4747-3p gene (miRBase Accession No. MIMAT0019883) described in SEQ ID NO: 63, its homolog or ortholog of a different organism species, and the like. The hsa-miR-4747-3p gene can be obtained by a method described in Persson H et al., 2011, Cancer Res, Vol. 71, p. 78-86. Also, "hsa-mir-4747" (miRBase Accession No. MI0017386, SEQ ID NO: 298) having a hairpin-like structure is known as a precursor of "hsa-miR-4747-3p".

The term "hsa-miR-4651 gene" or "hsa-miR-4651" used herein includes the hsa-miR-4651 gene (miRBase Accession No. MIMAT0019715) described in SEQ ID NO: 64, its homolog or ortholog of a different organism species, and the like. The hsa-miR-4651 gene can be obtained by a method described in Persson H et al., 2011, Cancer Res, Vol. 71, p. 78-86. Also, "hsa-mir-4651" (miRBase Accession No. MI0017279, SEQ ID NO: 299) having a hairpin-like structure is known as a precursor of "hsa-miR-4651".

The term "hsa-miR-638 gene" or "hsa-miR-638" used herein includes the hsa-miR-638 gene (miRBase Accession No. MIMAT0003308) described in SEQ ID NO: 65, its homolog or ortholog of a different organism species, and the like. The hsa-miR-638 gene can be obtained by a method described in Cummins JM et al., 2006, Proc Natl Acad Sci USA, Vol. 103, p. 3687-3692. Also, "hsa-mir-638" (miRBase Accession No. MI0003653, SEQ ID NO: 300) having a hairpin-like structure is known as a precursor of "hsa-miR-638".

The term "hsa-miR-4736 gene" or "hsa-miR-4736" used herein includes the hsa-miR-4736 gene (miRBase Accession No. MIMAT0019862) described in SEQ ID NO: 66, its homolog or ortholog of a different organism species, and the like. The hsa-miR-4736 gene can be obtained by a method described in Persson H et al., 2011, Cancer Res, Vol. 71, p. 78-86. Also, "hsa-mir-4736" (miRBase Accession No. MI0017373, SEQ ID NO: 301) having a hairpin-like structure is known as a precursor of "hsa-miR-4736".

The term "hsa-miR-6845-5p gene" or "hsa-miR-6845-5p" used herein includes the hsa-miR-6845-5p gene (miRBase Accession No. MIMAT0027590) described in SEQ ID NO: 67, its homolog or ortholog of a different organism species, and the like. The hsa-miR-6845-5p gene can be obtained by a method described in Ladewig E et al., 2012, Genome Res, Vol. 22, p. 1634-1645. Also, "hsa-mir-6845" (miRBase Accession No. MI0022691, SEQ ID NO: 302) having a hairpin-like structure is known as a precursor of "hsa-miR-6845-5p".

The term "hsa-miR-1343-3p gene" or "hsa-miR-1343-3p" used herein includes the hsa-miR-1343-3p gene (miRBase Accession No. MIMAT0019776) described in SEQ ID NO: 68, its homolog or ortholog of a different organism species, and the like. The hsa-miR-1343-3p gene can be obtained by a method described in Persson H et al., 2011, Cancer Res, Vol. 71, p. 78-86. Also, "hsa-mir-1343" (miRBase Accession No. MI0017320, SEQ ID NO: 303) having a hairpin-like structure is known as a precursor of "hsa-miR-1343-3p".

The term "hsa-miR-6126 gene" or "hsa-miR-6126" used herein includes the hsa-miR-6126 gene (miRBase Accession No. MIMAT0024599) described in SEQ ID NO: 69, its homolog or ortholog of a different organism species, and the like. The hsa-miR-6126 gene can be obtained by a method described in Smith JL et al., 2012, J Virol, Vol. 86, p. 5278-5287. Also, "hsa-mir-6126" (miRBase Accession No. MI0021260, SEQ ID NO: 304) having a hairpin-like structure is known as a precursor of "hsa-miR-6126".

The term "hsa-miR-92b-5p gene" or "hsa-miR-92b-5p" used herein includes the hsa-miR-92b-5p gene (miRBase Accession No. MIMAT0004792) described in SEQ ID NO: 70, its homolog or ortholog of a different organism species, and the like. The hsa-miR-92b-5p gene can be obtained by a method described in Cummins JM et al., 2006, Proc Natl Acad Sci USA, Vol. 103, p. 3687-3692. Also, "hsa-mir-92b" (miRBase Accession No. MI0003560, SEQ ID NO: 305) having a hairpin-like structure is known as a precursor of "hsa-miR-92b-5p".

The term "hsa-miR-6774-5p gene" or "hsa-miR-6774-5p" used herein includes the hsa-miR-6774-5p gene (miRBase Accession No. MIMAT0027448) described in SEQ ID NO: 71, its homolog or ortholog of a different organism species, and the like. The hsa-miR-6774-5p gene can be obtained by a method described in Ladewig E et al., 2012, Genome Res, Vol. 22, p. 1634-1645. Also, "hsa-mir-6774" (miRBase Accession No. MI0022619, SEQ ID NO: 306) having a hairpin-like structure is known as a precursor of "hsa-miR-6774-5p".

The term "hsa-miR-7847-3p gene" or "hsa-miR-7847-3p" used herein includes the hsa-miR-7847-3p gene (miRBase Accession No. MIMAT0030422) described in SEQ ID NO: 72, its homolog or ortholog of a different organism species, and the like. The hsa-miR-7847-3p gene can be obtained by a method described in Ple H et al., 2012, PLoS One, Vol. 7, e50746. Also, "hsa-mir-7847" (miRBase Accession No. MI0025517, SEQ ID NO: 307) having a hairpin-like structure is known as a precursor of "hsa-miR-7847-3p".

The term "hsa-miR-6795-5p gene" or "hsa-miR-6795-5p" used herein includes the hsa-miR-6795-5p gene (miRBase Accession No. MIMAT0027490) described in SEQ ID NO: 73, its homolog or ortholog of a different organism species, and the like. The hsa-miR-6795-5p gene can be obtained by a method described in Ladewig E et al., 2012, Genome Res, Vol. 22, p. 1634-1645. Also, "hsa-mir-6795" (miRBase Accession No. MI0022640, SEQ ID NO: 308) having a hairpin-like structure is known as a precursor of "hsa-miR-6795-5p".

The term "hsa-miR-7109-5p gene" or "hsa-miR-7109-5p" used herein includes the hsa-miR-7109-5p gene (miRBase Accession No. MIMAT0028115) described in SEQ ID NO: 74, its homolog or ortholog of a different organism species, and the like. The hsa-miR-7109-5p gene can be obtained by a method described in Ladewig E et al., 2012, Genome Res, Vol. 22, p. 1634-1645. Also, "hsa-mir-7109" (miRBase Accession No. MI0022960, SEQ ID NO: 309) having a hairpin-like structure is known as a precursor of "hsa-miR-7109-5p".

The term "hsa-miR-3197 gene" or "hsa-miR-3197" used herein includes the hsa-miR-3197 gene (miRBase Accession No. MIMAT0015082) described in SEQ ID NO: 75, its homolog or ortholog of a different organism species, and the like. The hsa-miR-3197 gene can be obtained by a method described in Stark MS et al., 2010, PLoS One, Vol. 5, e9685. Also, "hsa-mir-3197" (miRBase Accession No. MI0014245, SEQ ID NO: 310) having a hairpin-like structure is known as a precursor of "hsa-miR-3197".

The term "hsa-miR-6824-5p gene" or "hsa-miR-6824-5p" used herein includes the hsa-miR-6824-5p gene (miRBase Accession No. MIMAT0027548) described in SEQ ID NO: 76, its homolog or ortholog of a different organism species, and the like. The hsa-miR-6824-5p gene can be obtained by a method described in Ladewig E et al., 2012, Genome Res, Vol. 22, p. 1634-1645. Also, "hsa-mir-6824" (miRBase Accession No. MI0022669, SEQ ID NO: 311) having a hairpin-like structure is known as a precursor of "hsa-miR-6824-5p".

The term "hsa-miR-6771-5p gene" or "hsa-miR-6771-5p" used herein includes the hsa-miR-6771-5p gene (miRBase Accession No. MIMAT0027442) described in SEQ ID NO: 77, its homolog or ortholog of a different organism species, and the like. The hsa-miR-6771-5p gene can be obtained by a method described in Ladewig E et al., 2012, Genome Res, Vol. 22, p. 1634-1645. Also, "hsa-mir-6771" (miRBase Accession No. MI0022616, SEQ ID NO: 312) having a hairpin-like structure is known as a precursor of "hsa-miR-6771-5p".

The term "hsa-miR-11399 gene" or "hsa-miR-11399" used herein includes the hsa-miR-11399 gene (miRBase Accession No. MIMAT0044656) described in SEQ ID NO: 78, its homolog or ortholog of a different organism species, and the like. The hsa-miR-11399 gene can be obtained by a method described in Sun Q et al., 2015, Exp Cell Res, Vol. 333, p. 220-227. Also, "hsa-mir-11399" (miRBase Accession No. MI0036558, SEQ ID NO: 313) having a hairpin-like structure is known as a precursor of "hsa-miR-11399".

The term "hsa-miR-2861 gene" or "hsa-miR-2861" used herein includes the hsa-miR-2861 gene (miRBase Accession No. MIMAT0013802) described in SEQ ID NO: 79, its homolog or ortholog of a different organism species, and the like. The hsa-miR-2861 gene can be obtained by a method described in Li H, 2009, J Clin Invest, Vol. 119, p. 3666-3677. Also, "hsa-mir-2861" (miRBase Accession No. MI0013006, SEQ ID NO: 314) having a hairpin-like structure is known as a precursor of "hsa-miR-2861".

The term "hsa-miR-4707-3p gene" or "hsa-miR-4707-3p" used herein includes the hsa-miR-4707-3p gene (miRBase Accession No. MIMAT0019808) described in SEQ ID NO: 80, its homolog or ortholog of a different organism species, and the like. The hsa-miR-4707-3p gene can be obtained by a method described in Persson H et al., 2011, Cancer Res, Vol. 71, p. 78-86. Also, "hsa-mir-4707" (miRBase Accession No. MI0017340, SEQ ID NO: 315) having a hairpin-like structure is known as a precursor of "hsa-miR-4707-3p".

The term "hsa-miR-4638-5p gene" or "hsa-miR-4638-5p" used herein includes the hsa-miR-4638-5p gene (miRBase Accession No. MIMAT0019695) described in SEQ ID NO: 81, its homolog or ortholog of a different organism species, and the like. The hsa-miR-4638-5p gene can be obtained by a method described in Persson H et al., 2011, Cancer Res, Vol. 71, p. 78-86. Also, "hsa-mir-4638" (miRBase Accession No. MI0017265, SEQ ID NO: 316) having a hairpin-like structure is known as a precursor of "hsa-miR-4638-5p".

The term "hsa-miR-8073 gene" or "hsa-miR-8073" used herein includes the hsa-miR-8073 gene (miRBase Accession No. MIMAT0031000) described in SEQ ID NO: 82, its homolog or ortholog of a different organism species, and the like. The hsa-miR-8073 gene can be obtained by a method described in Wang HJ et al., 2013, Shock, Vol. 39, p. 480-487. Also, "hsa-mir-8073" (miRBase Accession No. MI0025909, SEQ ID NO: 317) having a hairpin-like structure is known as a precursor of "hsa-miR-8073".

The term "hsa-miR-328-5p gene" or "hsa-miR-328-5p" used herein includes the hsa-miR-328-5p gene (miRBase Accession No. MIMAT0026486) described in SEQ ID NO: 83, its homolog or ortholog of a different organism species, and the like. The hsa-miR-328-5p gene can be obtained by a method described in Kim J et al., 2004, Proc Natl Acad Sci USA, Vol. 101, p. 360-365. Also, "hsa-mir-328" (miRBase Accession No. MI0000804, SEQ ID NO: 318) having a hairpin-like structure is known as a precursor of "hsa-miR-328-5p".

The term "hsa-miR-665 gene" or "hsa-miR-665" used herein includes the hsa-miR-665 gene (miRBase Accession No. MIMAT0004952) described in SEQ ID NO: 84, its homolog or ortholog of a different organism species, and the like. The hsa-miR-665 gene can be obtained by a method described in Berezikov E et al., 2006, Genome Res, Vol. 16, p. 1289-1298. Also, "hsa-mir-665" (miRBase Accession No. MI0005563, SEQ ID NO: 319) having a hairpin-like structure is known as a precursor of "hsa-miR-665".

The term "hsa-miR-6778-5p gene" or "hsa-miR-6778-5p" used herein includes the hsa-miR-6778-5p gene (miRBase Accession No. MIMAT0027456) described in SEQ ID NO: 85, its homolog or ortholog of a different organism species, and the like. The hsa-miR-6778-5p gene can be obtained by a method described in Ladewig E et al., 2012, Genome Res, Vol. 22, p. 1634-1645. Also, "hsa-mir-6778" (miRBase Accession No. MI0022623, SEQ ID NO: 320) having a hairpin-like structure is known as a precursor of "hsa-miR-6778-5p".

The term "hsa-miR-10398-3p gene" or "hsa-miR-10398-3p" used herein includes the hsa-miR-10398-3p gene (miRBase Accession No. MIMAT0041628) described in SEQ ID NO: 86, its homolog or ortholog of a different organism species, and the like. The hsa-miR-10398-3p gene can be obtained by a method described in Lim EL et al., 2015, Genome Biol, Vol. 16, p. 18. Also, "hsa-mir-10398" (miRBase Accession No. MI0033422, SEQ ID NO: 321) having a hairpin-like structure is known as a precursor of "hsa-miR-10398-3p".

The term "hsa-miR-5698 gene" or "hsa-miR-5698" used herein includes the hsa-miR-5698 gene (miRBase Accession No. MIMAT0022491) described in SEQ ID NO: 87, its homolog or ortholog of a different organism species, and the like. The hsa-miR-5698 gene can be obtained by a method described in Watahiki A, 2011, PLoS One, Vol. 6, e24950. Also, "hsa-mir-5698" (miRBase Accession No. MI0019305, SEQ ID NO: 322) having a hairpin-like structure is known as a precursor of "hsa-miR-5698".

The term "hsa-miR-6794-5p gene" or "hsa-miR-6794-5p" used herein includes the hsa-miR-6794-5p gene (miRBase Accession No. MIMAT0027488) described in SEQ ID NO: 88, its homolog or ortholog of a different organism species, and the like. The hsa-miR-6794-5p gene can be obtained by a method described in Ladewig E et al., 2012, Genome Res, Vol. 22, p. 1634-1645. Also, "hsa-mir-6794" (miRBase Accession No. MI0022639, SEQ ID NO: 323) having a hairpin-like structure is known as a precursor of "hsa-miR-6794-5p".

The term "hsa-miR-1247-3p gene" or "hsa-miR-1247-3p" used herein includes the hsa-miR-1247-3p gene (miRBase Accession No. MIMAT0022721) described in SEQ ID NO: 89, its homolog or ortholog of a different organism species, and the like. The hsa-miR-1247-3p gene can be obtained by a method described in Morin RD et al., 2008, Genome Res, Vol. 18, p. 610-621. Also, "hsa-mir-1247" (miRBase Accession No. MI0006382, SEQ ID NO: 324) having a hairpin-like structure is known as a precursor of "hsa-miR-1247-3p".

The term "hsa-miR-4697-5p gene" or "hsa-miR-4697-5p" used herein includes the hsa-miR-4697-5p gene (miRBase Accession No. MIMAT0019791) described in SEQ ID NO: 90, its homolog or ortholog of a different organism species, and the like. The hsa-miR-4697-5p gene can be obtained by a method described in Persson H et al., 2011, Cancer Res, Vol. 71, p. 78-86. Also, "hsa-mir-4697" (miRBase Accession No. MI0017330, SEQ ID NO: 325) having a hairpin-like structure is known as a precursor of "hsa-miR-4697-5p".

The term "hsa-miR-8069 gene" or "hsa-miR-8069" used herein includes the hsa-miR-8069 gene (miRBase Accession No. MIMAT0030996) described in SEQ ID NO: 91, its homolog or ortholog of a different organism species, and the like. The hsa-miR-8069 gene can be obtained by a method described in Wang HJ et al., 2013, Shock, Vol. 39, p. 480-487. Also, "hsa-mir-8069-1" (miRBase Accession No. MI0025905, SEQ ID NO: 326) having a hairpin-like structure is known as a precursor of "hsa-miR-8069".

The term "hsa-miR-572 gene" or "hsa-miR-572" used herein includes the hsa-miR-572 gene (miRBase Accession No. MIMAT0003237) described in SEQ ID NO: 92, its homolog or ortholog of a different organism species, and the like. The hsa-miR-572 gene can be obtained by a method described in Cummins JM et al., 2006, Proc Natl Acad Sci USA, Vol. 103, p. 3687-3692. Also, "hsa-mir-572" (miRBase Accession No. MI0003579, SEQ ID NO: 327) having a hairpin-like structure is known as a precursor of "hsa-miR-572".

The term "hsa-miR-6751-5p gene" or "hsa-miR-6751-5p" used herein includes the hsa-miR-6751-5p gene (miRBase Accession No. MIMAT0027402) described in SEQ ID NO: 93, its homolog or ortholog of a different organism species, and the like. The hsa-miR-6751-5p gene can be obtained by a method described in Ladewig E et al., 2012, Genome Res, Vol. 22, p. 1634-1645. Also, "hsa-mir-6751" (miRBase Accession No. MI0022596, SEQ ID NO: 328) having a hairpin-like structure is known as a precursor of "hsa-miR-6751-5p".

The term "hsa-miR-3180-3p gene" or "hsa-miR-3180-3p" used herein includes the hsa-miR-3180-3p gene (miRBase Accession No. MIMAT0015058) described in SEQ ID NO: 94, its homolog or ortholog of a different organism species, and the like. The hsa-miR-3180-3p gene can be obtained by a method described in Stark MS et al., 2010, PLoS One, Vol. 5, e9685. Also, "hsa-mir-3180-1" (miRBase Accession No. MI0014214, SEQ ID NO: 329) having a hairpin-like structure is known as a precursor of "hsa-miR-3180-3p".

The term "hsa-miR-486-3p gene" or "hsa-miR-486-3p" used herein includes the hsa-miR-486-3p gene (miRBase Accession No. MIMAT0004762) described in SEQ ID NO: 95, its homolog or ortholog of a different organism species, and the like. The hsa-miR-486-3p gene can be obtained by a method described in Fu H et al., 2005, FEBS Lett, Vol. 579, p. 3849-3854. Also, "hsa-mir-486-1" (miRBase Accession No. MI0002470, SEQ ID NO: 330) having a hairpin-like structure is known as a precursor of "hsa-miR-486-3p".

The term "hsa-miR-6086 gene" or "hsa-miR-6086" used herein includes the hsa-miR-6086 gene (miRBase Accession No. MIMAT0023711) described in SEQ ID NO: 96, its homolog or ortholog of a different organism species, and the like. The hsa-miR-6086 gene can be obtained by a method described in Yoo JK et al., 2012, Stem Cells Dev, Vol. 21, p. 2049-2057. Also, "hsa-mir-6086" (miRBase Accession No. MI0020363, SEQ ID NO: 331) having a hairpin-like structure is known as a precursor of "hsa-miR-6086".

The term "hsa-miR-30c-1-3p gene" or "hsa-miR-30c-1-3p" used herein includes the hsa-miR-30c-1-3p gene (miRBase Accession No. MIMAT0004674) described in SEQ ID NO: 97, its homolog or ortholog of a different organism species, and the like. The hsa-miR-30c-1-3p gene can be obtained by a method described in Lagos-Quintana M et al., 2002, Curr Biol, Vol. 12, p. 735-739. Also, "hsa-mir-30c-1" (miRBase Accession No. MI0000736, SEQ ID NO: 332) having a hairpin-like structure is known as a precursor of "hsa-miR-30c-1-3p".

The term "hsa-miR-8063 gene" or "hsa-miR-8063" used herein includes the hsa-miR-8063 gene (miRBase Accession No. MIMAT0030990) described in SEQ ID NO: 98, its homolog or ortholog of a different organism species, and the like. The hsa-miR-8063 gene can be obtained by a method described in Wang HJ et al., 2013, Shock, Vol. 39, p. 480-487. Also, "hsa-mir-8063" (miRBase Accession No. MI0025899, SEQ ID NO: 333) having a hairpin-like structure is known as a precursor of "hsa-miR-8063".

The term "hsa-miR-3621 gene" or "hsa-miR-3621" used herein includes the hsa-miR-3621 gene (miRBase Accession No. MIMAT0018002) described in SEQ ID NO: 99, its homolog or ortholog of a different organism species, and the like. The hsa-miR-3621 gene can be obtained by a method described in Witten D et al., 2010, BMC Biol, Vol. 8, p. 58. Also, "hsa-mir-3621" (miRBase Accession No. MI0016012, SEQ ID NO: 334) having a hairpin-like structure is known as a precursor of "hsa-miR-3621".

The term "hsa-miR-6887-5p gene" or "hsa-miR-6887-5p" used herein includes the hsa-miR-6887-5p gene (miRBase Accession No. MIMAT0027674) described in SEQ ID NO: 100, its homolog or ortholog of a different organism species, and the like. The hsa-miR-6887-5p gene can be obtained by a method described in Ladewig E et al., 2012, Genome Res, Vol. 22, p. 1634-1645. Also, "hsa-mir-6887" (miRBase Accession No. MI0022734, SEQ ID NO: 335) having a hairpin-like structure is known as a precursor of "hsa-miR-6887-5p".

The term "hsa-miR-3191-3p gene" or "hsa-miR-3191-3p" used herein includes the hsa-miR-3191-3p gene (miRBase Accession No. MIMAT0015075) described in SEQ ID NO: 101, its homolog or ortholog of a different organism species, and the like. The hsa-miR-3191-3p gene can be obtained by a method described in Stark MS et al., 2010, PLoS One, Vol. 5, e9685. Also, "hsa-mir-3191" (miRBase AccessionNo. MI0014236, SEQ ID NO: 336) having a hairpin-like structure is known as a precursor of "hsa-miR-3191-3p".

The term "hsa-miR-11181-3p gene" or "hsa-miR-11181-3p" used herein includes the hsa-miR-11181-3p gene (miRBase Accession No. MIMAT0043997) described in SEQ ID NO: 102, its homolog or ortholog of a different organism species, and the like. The hsa-miR-11181-3p gene can be obtained by a method described in Dokanehiifard S et al., 2015, Cell Mol Life Sci, Vol. 72, p2613-2625. Also, "hsa-mir-11181" (miRBase Accession No. MI0035972, SEQ ID NO: 337) having a hairpin-like structure is known as a precursor of "hsa-miR-11181-3p".

The term "hsa-miR-6722-5p gene" or "hsa-miR-6722-5p" used herein includes the hsa-miR-6722-5p gene (miRBase Accession No. MIMAT0025853) described in SEQ ID NO: 103, its homolog or ortholog of a different organism species, and the like. The hsa-miR-6722-5p gene can be obtained by a method described in Li Y et al., 2012, Gene, Vol. 497, p. 330-335. Also, "hsa-mir-6722" (miRBase Accession No. MI0022557, SEQ ID NO: 338) having a hairpin-like structure is known as a precursor of "hsa-miR-6722-5p".

The term "hsa-miR-6781-5p gene" or "hsa-miR-6781-5p" used herein includes the hsa-miR-6781-5p gene (miRBase Accession No. MIMAT0027462) described in SEQ ID NO: 104, its homolog or ortholog of a different organism species, and the like. The hsa-miR-6781-5p gene can be obtained by a method described in Ladewig E et al., 2012, Genome Res, Vol. 22, p. 1634-1645. Also, "hsa-mir-6781" (miRBase Accession No. MI0022626, SEQ ID NO: 339) having a hairpin-like structure is known as a precursor of "hsa-miR-6781-5p".

The term "hsa-miR-5739 gene" or "hsa-miR-5739" used herein includes the hsa-miR-5739 gene (miRBase Accession No. MIMAT0023116) described in SEQ ID NO: 105, its homolog or ortholog of a different organism species, and the like. The hsa-miR-5739 gene can be obtained by a method described in Yoo JK et al., 2011, Biochem Biophys Res Commun, Vol. 415, p. 258-262. Also, "hsa-mir-5739" (miRBase Accession No. MI0019412, SEQ ID NO: 340) having a hairpin-like structure is known as a precursor of "hsa-miR-5739".

The term "hsa-miR-3937 gene" or "hsa-miR-3937" used herein includes the hsa-miR-3937 gene (miRBase Accession No. MIMAT0018352) described in SEQ ID NO: 106, its homolog or ortholog of a different organism species, and the like. The hsa-miR-3937 gene can be obtained by a method described in Liao JY et al., 2010, PLoS One, Vol. 5, e10563. Also, "hsa-mir-3937" (miRBase Accession No. MI0016593, SEQ ID NO: 341) having a hairpin-like structure is known as a precursor of "hsa-miR-3937".

The term "hsa-miR-1343-5p gene" or "hsa-miR-1343-5p" used herein includes the hsa-miR-1343-Sp gene (miRBase Accession No. MIMAT0027038) described in SEQ ID NO: 107, its homolog or ortholog of a different organism species, and the like. The hsa-miR-1343-5p gene can be obtained by a method described in Persson H et al., 2011, Cancer Res, Vol. 71, p. 78-86. Also, "hsa-mir-1343" (miRBase Accession No. MI0017320, SEQ ID NO: 303) having a hairpin-like structure is known as a precursor of "hsa-miR-1343-5p".

The term "hsa-miR-1181 gene" or "hsa-miR-1181" used herein includes the hsa-miR-1181 gene (miRBase Accession No. MIMAT0005826) described in SEQ ID NO: 108, its homolog or ortholog of a different organism species, and the like. The hsa-miR-1181 gene can be obtained by a method described in Subramanian S et al., 2008, Oncogene, Vol. 27, p. 2015-2026. Also, "hsa-mir-1181" (miRBase Accession No. MI0006274, SEQ ID NO: 342) having a hairpin-like structure is known as a precursor of "hsa-miR-1181".

The term "hsa-miR-4725-3p gene" or "hsa-miR-4725-3p" used herein includes the hsa-miR-4725-3p gene (miRBase Accession No. MIMAT0019844) described in SEQ ID NO: 109, its homolog or ortholog of a different organism species, and the like. The hsa-miR-4725-3p gene can be obtained by a method described in Persson H et al., 2011, Cancer Res, Vol. 71, p. 78-86. Also, "hsa-mir-4725" (miRBase Accession No. MI0017362, SEQ ID NO: 343) having a hairpin-like structure is known as a precursor of "hsa-miR-4725-3p".

The term "hsa-miR-6865-5p gene" or "hsa-miR-6865-5p" used herein includes the hsa-miR-6865-5p gene (miRBase Accession No. MIMAT0027630) described in SEQ ID NO: 110, its homolog or ortholog of a different organism species, and the like. The hsa-miR-6865-5p gene can be obtained by a method described in Ladewig E et al., 2012, Genome Res, Vol. 22, p. 1634-1645. Also, "hsa-mir-6865" (miRBase Accession No. MI0022712, SEQ ID NO: 344) having a hairpin-like structure is known as a precursor of "hsa-miR-6865-5p".

The term "hsa-miR-375-5p gene" or "hsa-miR-375-5p" used herein includes the hsa-miR-375-5p gene (miRBase Accession No. MIMAT0037313) described in SEQ ID NO: 111, its homolog or ortholog of a different organism species, and the like. The hsa-miR-375-5p gene can be obtained by a method described in Poy MN et al., 2004, Nature, Vol. 432, p. 226-230. Also, "hsa-mir-375" (miRBase Accession No. MI0000783, SEQ ID NO: 345) having a hairpin-like structure is known as a precursor of "hsa-miR-375-5p".

The term "hsa-miR-3196 gene" or "hsa-miR-3196" used herein includes the hsa-miR-3196 gene (miRBase Accession No. MIMAT0015080) described in SEQ ID NO: 112, its homolog or ortholog of a different organism species, and the like. The hsa-miR-3196 gene can be obtained by a method described in Stark MS et al., 2010, PLoS One, Vol. 5, e9685. Also, "hsa-mir-3196" (miRBase Accession No. MI0014241, SEQ ID NO: 346) having a hairpin-like structure is known as a precursor of "hsa-miR-3196".

The term "hsa-miR-6762-5p gene" or "hsa-miR-6762-5p" used herein includes the hsa-miR-6762-5p gene (miRBase Accession No. MIMAT0027424) described in SEQ ID NO: 113, its homolog or ortholog of a different organism species, and the like. The hsa-miR-6762-5p gene can be obtained by a method described in Ladewig E et al., 2012, Genome Res, Vol. 22, p. 1634-1645. Also, "hsa-mir-6762" (miRBase Accession No. MI0022607, SEQ ID NO: 347) having a hairpin-like structure is known as a precursor of "hsa-miR-6762-5p".

The term "hsa-miR-4258 gene" or "hsa-miR-4258" used herein includes the hsa-miR-4258 gene (miRBase Accession No. MIMAT0016879) described in SEQ ID NO: 114, its homolog or ortholog of a different organism species, and the like. The hsa-miR-4258 gene can be obtained by a method described in Goff LA et al., 2009, PLoS One, Vol. 4, e7192. Also, "hsa-mir-4258" (miRBase Accession No. MI0015857, SEQ ID NO: 348) having a hairpin-like structure is known as a precursor of "hsa-miR-4258".

The term "hsa-miR-5196-5p gene" or "hsa-miR-5196-5p" used herein includes the hsa-miR-5196-5p gene (miRBase Accession No. MIMAT0021128) described in SEQ ID NO: 115, its homolog or ortholog of a different organism species, and the like. The hsa-miR-5196-5p gene can be obtained by a method described in Schotte D et al., 2011, Leukemia, Vol. 25, p. 1389-1399. Also, "hsa-mir-5196" (miRBase Accession No. MI0018175, SEQ ID NO: 349) having a hairpin-like structure is known as a precursor of "hsa-miR-5196-5p".

The term "hsa-miR-10401-3p gene" or "hsa-miR-10401-3p" used herein includes the hsa-miR-10401-3p gene (miRBase Accession No. MIMAT0041634) described in SEQ ID NO: 116, its homolog or ortholog of a different organism species, and the like. The hsa-miR-10401-3p gene can be obtained by a method described in Lim EL et al., 2015, Genome Biol, Vol. 16, p. 18. Also, "hsa-mir-10401" (miRBase Accession No. MI0033425, SEQ ID NO: 278) having a hairpin-like structure is known as a precursor of "hsa-miR-10401-3p".

The term "hsa-miR-675-5p gene" or "hsa-miR-675-5p" used herein includes the hsa-miR-675-5p gene (miRBase Accession No. MIMAT0004284) described in SEQ ID NO: 117, its homolog or ortholog of a different organism species, and the like. The hsa-miR-675-5p gene can be obtained by a method described in Cai X et al., 2007, RNA, Vol. 13, p. 313-316. Also, "hsa-mir-675" (miRBase Accession No. MI0005416, SEQ ID NO: 350) having a hairpin-like structure is known as a precursor of "hsa-miR-675-5p".

The term "hsa-miR-4488 gene" or "hsa-miR-4488" used herein includes the hsa-miR-4488 gene (miRBase Accession No. MIMAT0019022) described in SEQ ID NO: 118, its homolog or ortholog of a different organism species, and the like. The hsa-miR-4488 gene can be obtained by a method described in Jima DD et al., 2010, Blood, Vol. 116, e118-e127. Also, "hsa-mir-4488" (miRBase Accession No. MI0016849, SEQ ID NO: 351) having a hairpin-like structure is known as a precursor of "hsa-miR-4488".

The term "hsa-miR-10527-5p gene" or "hsa-miR-10527-5p" used herein includes the hsa-miR-10527-5p gene (miRBase Accession No. MIMAT0041997) described in SEQ ID NO: 119, its homolog or ortholog of a different organism species, and the like. The hsa-miR-10527-5p gene can be obtained by a method described in Asikainen S et al., 2015, PLoS One, Vol. 10, e0116668. Also, "hsa-mir-10527" (miRBase Accession No. MI0033674, SEQ ID NO: 352) having a hairpin-like structure is known as a precursor of "hsa-miR-10527-5p".

The term "hsa-miR-10396a-5p gene" or "hsa-miR-10396a-5p" used herein includes the hsa-miR-10396a-5p gene (miRBase Accession No. MIMAT0041623) described in SEQ ID NO: 120, its homolog or ortholog of a different organism species, and the like. The hsa-miR-10396a-5p gene can be obtained by a method described in Lim EL et al., 2015, Genome Biol, Vol. 16, p. 18. Also, "hsa-mir-10396a" (miRBase Accession No. MI0033420, SEQ ID NO: 353) having a hairpin-like structure is known as a precursor of "hsa-miR-10396a-5p".

The term "hsa-miR-4269 gene" or "hsa-miR-4269" used herein includes the hsa-miR-4269 gene (miRBase Accession No. MIMAT0016897) described in SEQ ID NO: 121, its homolog or ortholog of a different organism species, and the like. The hsa-miR-4269 gene can be obtained by a method described in Goff LA et al., 2009, PLoS One, Vol. 4, e7192. Also, "hsa-mir-4269" (miRBase Accession No. MI0015875, SEQ ID NO: 354) having a hairpin-like structure is known as a precursor of "hsa-miR-4269".

The term "hsa-miR-6800-5p gene" or "hsa-miR-6800-5p" used herein includes the hsa-miR-6800-5p gene (miRBase Accession No. MIMAT0027500) described in SEQ ID NO: 122, its homolog or ortholog of a different organism species, and the like. The hsa-miR-6800-5p gene can be obtained by a method described in Ladewig E et al., 2012, Genome Res, Vol. 22, p. 1634-1645. Also, "hsa-mir-6800" (miRBase Accession No. MI0022645, SEQ ID NO: 355) having a hairpin-like structure is known as a precursor of "hsa-miR-6800-5p".

The term "hsa-miR-6819-5p gene" or "hsa-miR-6819-5p" used herein includes the hsa-miR-6819-5p gene (miRBase Accession No. MIMAT0027538) described in SEQ ID NO: 123, its homolog or ortholog of a different organism species, and the like. The hsa-miR-6819-5p gene can be obtained by a method described in Ladewig E et al., 2012, Genome Res, Vol. 22, p. 1634-1645. Also, "hsa-mir-6819" (miRBase Accession No. MI0022664, SEQ ID NO: 356) having a hairpin-like structure is known as a precursor of "hsa-miR-6819-5p".

The term "hsa-miR-10396b-3p gene" or "hsa-miR-10396b-3p" used herein includes the hsa-miR-10396b-3p gene (miRBase Accession No. MIMAT0041636) described in SEQ ID NO: 124, its homolog or ortholog of a different organism species, and the like. The hsa-miR-10396b-3p gene can be obtained by a method described in Lim EL et al., 2015, Genome Biol, Vol. 16, p. 18. Also, "hsa-mir-10396b" (miRBase Accession No. MI0033426, SEQ ID NO: 248) having a hairpin-like structure is known as a precursor of "hsa-miR-10396b-3p".

The term "hsa-miR-4688 gene" or "hsa-miR-4688" used herein includes the hsa-miR-4688 gene (miRBase Accession No. MIMAT0019777) described in SEQ ID NO: 125, its homolog or ortholog of a different organism species, and the like. The hsa-miR-4688 gene can be obtained by a method described in Persson H et al., 2011, Cancer Res, Vol. 71, p. 78-86. Also, "hsa-mir-4688" (miRBase Accession No. MI0017321, SEQ ID NO: 357) having a hairpin-like structure is known as a precursor of "hsa-miR-4688".

The term "hsa-miR-6786-5p gene" or "hsa-miR-6786-5p" used herein includes the hsa-miR-6786-5p gene (miRBase Accession No. MIMAT0027472) described in SEQ ID NO: 126, its homolog or ortholog of a different organism species, and the like. The hsa-miR-6786-5p gene can be obtained by a method described in Ladewig E et al., 2012, Genome Res, Vol. 22, p. 1634-1645. Also, "hsa-mir-6786" (miRBase Accession No. MI0022631, SEQ ID NO: 358) having a hairpin-like structure is known as a precursor of "hsa-miR-6786-5p".

The term "hsa-miR-4634 gene" or "hsa-miR-4634" used herein includes the hsa-miR-4634 gene (miRBase Accession No. MIMAT0019691) described in SEQ ID NO: 127, its homolog or ortholog of a different organism species, and the like. The hsa-miR-4634 gene can be obtained by a method described in Persson H et al., 2011, Cancer Res, Vol. 71, p. 78-86. Also, "hsa-mir-4634" (miRBase Accession No. MI0017261, SEQ ID NO: 359) having a hairpin-like structure is known as a precursor of "hsa-miR-4634".

The term "hsa-miR-3940-5p gene" or "hsa-miR-3940-5p" used herein includes the hsa-miR-3940-5p gene (miRBase Accession No. MIMAT0019229) described in SEQ ID NO: 128, its homolog or ortholog of a different organism species, and the like. The hsa-miR-3940-5p gene can be obtained by a method described in Liao JY et al., 2010, PLoS One, Vol. 5, e10563. Also, "hsa-mir-3940" (miRBase AccessionNo. MI0016597, SEQ ID NO: 360) having a hairpin-like structure is known as a precursor of "hsa-miR-3940-5p".

The term "hsa-miR-4655-5p gene" or "hsa-miR-4655-5p" used herein includes the hsa-miR-4655-5p gene (miRBase Accession No. MIMAT0019721) described in SEQ ID NO: 129, its homolog or ortholog of a different organism species, and the like. The hsa-miR-4655-5p gene can be obtained by a method described in Persson H et al., 2011, Cancer Res, Vol. 71, p. 78-86. Also, "hsa-mir-4655" (miRBase Accession No. MI0017283, SEQ ID NO: 361) having a hairpin-like structure is known as a precursor of "hsa-miR-4655-5p".

The term "hsa-miR-7155-5p gene" or "hsa-miR-7155-5p" used herein includes the hsa-miR-7155-5p gene (miRBase Accession No. MIMAT0028220) described in SEQ ID NO: 130, its homolog or ortholog of a different organism species, and the like. The hsa-miR-7155-5p gene can be obtained by a method described in Meunier J et al., 2013, Genome Res, Vol. 23, p. 34-45. Also, "hsa-mir-7155" (miRBase Accession No. MI0023615, SEQ ID NO: 362) having a hairpin-like structure is known as a precursor of "hsa-miR-7155-5p".

The term "hsa-miR-6769b-5p gene" or "hsa-miR-6769b-5p" used herein includes the hsa-miR-6769b-5p gene (miRBase Accession No. MIMAT0027620) described in SEQ ID NO: 131, its homolog or ortholog of a different organism species, and the like. The hsa-miR-6769b-5p gene can be obtained by a method described in Ladewig E et al., 2012, Genome Res, Vol. 22, p. 1634-1645. Also, "hsa-mir-6769b" (miRBase Accession No. MI0022706, SEQ ID NO: 363) having a hairpin-like structure is known as a precursor of "hsa-miR-6769b-5p".

The term "hsa-miR-6810-5p gene" or "hsa-miR-6810-5p" used herein includes the hsa-miR-6810-5p gene (miRBase Accession No. MIMAT0027520) described in SEQ ID NO: 132, its homolog or ortholog of a different organism species, and the like. The hsa-miR-6810-5p gene can be obtained by a method described in Ladewig E et al., 2012, Genome Res, Vol. 22, p. 1634-1645. Also, "hsa-mir-6810" (miRBase Accession No. MI0022655, SEQ ID NO: 364) having a hairpin-like structure is known as a precursor of "hsa-miR-6810-5p".

The term "hsa-miR-4665-3p gene" or "hsa-miR-4665-3p" used herein includes the hsa-miR-4665-3p gene (miRBase Accession No. MIMAT0019740) described in SEQ ID NO: 133, its homolog or ortholog of a different organism species, and the like. The hsa-miR-4665-3p gene can be obtained by a method described in Persson H et al., 2011, Cancer Res, Vol. 71, p. 78-86. Also, "hsa-mir-4665" (miRBase Accession No. MI0017295, SEQ ID NO: 365) having a hairpin-like structure is known as a precursor of "hsa-miR-4665-3p".

The term "hsa-miR-6727-5p gene" or "hsa-miR-6727-5p" used herein includes the hsa-miR-6727-5p gene (miRBase Accession No. MIMAT0027355) described in SEQ ID NO: 134, its homolog or ortholog of a different organism species, and the like. The hsa-miR-6727-5p gene can be obtained by a method described in Ladewig E et al., 2012, Genome Res, Vol. 22, p. 1634-1645. Also, "hsa-mir-6727" (miRBase Accession No. MI0022572, SEQ ID NO: 366) having a hairpin-like structure is known as a precursor of "hsa-miR-6727-5p".

The term "hsa-miR-6803-5p gene" or "hsa-miR-6803-5p" used herein includes the hsa-miR-6803-5p gene (miRBase Accession No. MIMAT0027506) described in SEQ ID NO: 135, its homolog or ortholog of a different organism species, and the like. The hsa-miR-6803-5p gene can be obtained by a method described in Ladewig E et al., 2012, Genome Res, Vol. 22, p. 1634-1645. Also, "hsa-mir-6803" (miRBase Accession No. MI0022648, SEQ ID NO: 367) having a hairpin-like structure is known as a precursor of "hsa-miR-6803-5p".

The term "hsa-miR-4640-5p gene" or "hsa-miR-4640-5p" used herein includes the hsa-miR-4640-5p gene (miRBase Accession No. MIMAT0019699) described in SEQ ID NO: 136, its homolog or ortholog of a different organism species, and the like. The hsa-miR-4640-5p gene can be obtained by a method described in Persson H et al., 2011, Cancer Res, Vol. 71, p. 78-86. Also, "hsa-mir-4640" (miRBase Accession No. MI0017267, SEQ ID NO: 368) having a hairpin-like structure is known as a precursor of "hsa-miR-4640-5p".

The term "hsa-miR-6735-5p gene" or "hsa-miR-6735-5p" used herein includes the hsa-miR-6735-5p gene (miRBase Accession No. MIMAT0027371) described in SEQ ID NO: 137, its homolog or ortholog of a different organism species, and the like. The hsa-miR-6735-5p gene can be obtained by a method described in Ladewig E et al., 2012, Genome Res, Vol. 22, p. 1634-1645. Also, "hsa-mir-6735" (miRBase Accession No. MI0022580, SEQ ID NO: 369) having a hairpin-like structure is known as a precursor of "hsa-miR-6735-5p".

The term "hsa-miR-4535 gene" or "hsa-miR-4535" used herein includes the hsa-miR-4535 gene (miRBase Accession No. MIMAT0019075) described in SEQ ID NO: 138, its homolog or ortholog of a different organism species, and the like. The hsa-miR-4535 gene can be obtained by a method described in Jima DD et al., 2010, Blood, Vol. 116, e118-e127. Also, "hsa-mir-4535" (miRBase Accession No. MI0016903, SEQ ID NO: 370) having a hairpin-like structure is known as a precursor of "hsa-miR-4535".

The term "hsa-miR-8089 gene" or "hsa-miR-8089" used herein includes the hsa-miR-8089 gene (miRBase Accession No. MIMAT0031016) described in SEQ ID NO: 139, its homolog or ortholog of a different organism species, and the like. The hsa-miR-8089 gene can be obtained by a method described in Wang HJ et al., 2013, Shock, Vol. 39, p. 480-487. Also, "hsa-mir-8089" (miRBase Accession No. MI0025925, SEQ ID NO: 371) having a hairpin-like structure is known as a precursor of "hsa-miR-8089".

The term "hsa-miR-1292-3p gene" or "hsa-miR-1292-3p" used herein includes the hsa-miR-1292-3p gene (miRBase Accession No. MIMAT0022948) described in SEQ ID NO: 140, its homolog or ortholog of a different organism species, and the like. The hsa-miR-1292-3p gene can be obtained by a method described in Morin RD et al., 2008, Genome Res, Vol. 18, p. 610-621. Also, "hsa-mir-1292" (miRBase Accession No. MI0006433, SEQ ID NO: 372) having a hairpin-like structure is known as a precursor of "hsa-miR-1292-3p".

The term "hsa-miR-5088-5p gene" or "hsa-miR-5088-5p" used herein includes the hsa-miR-5088-5p gene (miRBase Accession No. MIMAT0021080) described in SEQ ID NO: 141, its homolog or ortholog of a different organism species, and the like. The hsa-miR-5088-5p gene can be obtained by a method described in Ding N et al., 2011, J Radiat Res, Vol. 52, p. 425-432. Also, "hsa-mir-5088" (miRBase Accession No. MI0017977, SEQ ID NO: 373) having a hairpin-like structure is known as a precursor of "hsa-miR-5088-5p".

The term "hsa-miR-3622a-5p gene" or "hsa-miR-3622a-5p" used herein includes the hsa-miR-3622a-5p gene (miRBase Accession No. MIMAT0018003) described in SEQ ID NO: 142, its homolog or ortholog of a different organism species, and the like. The hsa-miR-3622a-5p gene can be obtained by a method described in Witten D et al., 2010, BMC Biol, Vol. 8, p. 58. Also, "hsa-mir-3622a" (miRBase Accession No. MI0016013, SEQ ID NO: 374) having a hairpin-like structure is known as a precursor of "hsa-miR-3622a-5p".

The term "hsa-miR-6124 gene" or "hsa-miR-6124" used herein includes the hsa-miR-6124 gene (miRBase Accession No. MIMAT0024597) described in SEQ ID NO: 143, its homolog or ortholog of a different organism species, and the like. The hsa-miR-6124 gene can be obtained by a method described in Smith JL et al., 2012, J Virol, Vol. 86, p. 5278-5287. Also, "hsa-mir-6124" (miRBase Accession No. MI0021258, SEQ ID NO: 375) having a hairpin-like structure is known as a precursor of "hsa-miR-6124".

The term "hsa-miR-6820-5p gene" or "hsa-miR-6820-5p" used herein includes the hsa-miR-6820-5p gene (miRBase Accession No. MIMAT0027540) described in SEQ ID NO: 144, its homolog or ortholog of a different organism species, and the like. The hsa-miR-6820-5p gene can be obtained by a method described in Ladewig E et al., 2012, Genome Res, Vol. 22, p. 1634-1645. Also, "hsa-mir-6820" (miRBase Accession No. MI0022665, SEQ ID NO: 376) having a hairpin-like structure is known as a precursor of "hsa-miR-6820-5p".

The term "hsa-miR-6805-3p gene" or "hsa-miR-6805-3p" used herein includes the hsa-miR-6805-3p gene (miRBase Accession No. MIMAT0027511) described in SEQ ID NO: 145, its homolog or ortholog of a different organism species, and the like. The hsa-miR-6805-3p gene can be obtained by a method described in Ladewig E et al., 2012, Genome Res, Vol. 22, p. 1634-1645. Also, "hsa-mir-6805" (miRBase Accession No. MI0022650, SEQ ID NO: 377) having a hairpin-like structure is known as a precursor of "hsa-miR-6805-3p".

The term "hsa-miR-4513 gene" or "hsa-miR-4513" used herein includes the hsa-miR-4513 gene (miRBase Accession No. MIMAT0019050) described in SEQ ID NO: 146, its homolog or ortholog of a different organism species, and the like. The hsa-miR-4513 gene can be obtained by a method described in Jima DD et al., 2010, Blood, Vol. 116, e118-e127. Also, "hsa-mir-4513" (miRBase Accession No. MI0016879, SEQ ID NO: 378) having a hairpin-like structure is known as a precursor of "hsa-miR-4513".

The term "hsa-miR-760 gene" or "hsa-miR-760" used herein includes the hsa-miR-760 gene (miRBase Accession No. MIMAT0004957) described in SEQ ID NO: 147, its homolog or ortholog of a different organism species, and the like. The hsa-miR-760 gene can be obtained by a method described in Berezikov E et al., 2006, Genome Res, Vol. 16, p. 1289-1298. Also, "hsa-mir-760" (miRBase Accession No. MI0005567, SEQ ID NO: 379) having a hairpin-like structure is known as a precursor of "hsa-miR-760".

The term "hsa-miR-4665-5p gene" or "hsa-miR-4665-5p" used herein includes the hsa-miR-4665-5p gene (miRBase Accession No. MIMAT0019739) described in SEQ ID NO: 148, its homolog or ortholog of a different organism species, and the like. The hsa-miR-4665-5p gene can be obtained by a method described in Persson H et al., 2011, Cancer Res, Vol. 71, p. 78-86. Also, "hsa-mir-4665" (miRBase Accession No. MI0017295, SEQ ID NO: 365) having a hairpin-like structure is known as a precursor of "hsa-miR-4665-5p".

The term "hsa-miR-10400-3p gene" or "hsa-miR-10400-3p" used herein includes the hsa-miR-10400-3p gene (miRBase Accession No. MIMAT0041632) described in SEQ ID NO: 149, its homolog or ortholog of a different organism species, and the like. The hsa-miR-10400-3p gene can be obtained by a method described in Lim EL et al., 2015, Genome Biol, Vol. 16, p. 18. Also, "hsa-mir-10400" (miRBase Accession No. MI0033424, SEQ ID NO: 380) having a hairpin-like structure is known as a precursor of "hsa-miR-10400-3p".

The term "hsa-miR-4298 gene" or "hsa-miR-4298" used herein includes the hsa-miR-4298 gene (miRBase Accession No. MIMAT0016852) described in SEQ ID NO: 150, its homolog or ortholog of a different organism species, and the like. The hsa-miR-4298 gene can be obtained by a method described in Goff LA et al., 2009, PLoS One, Vol. 4, e7192. Also, "hsa-mir-4298" (miRBase Accession No. MI0015830, SEQ ID NO: 381) having a hairpin-like structure is known as a precursor of "hsa-miR-4298".

The term "hsa-miR-8085 gene" or "hsa-miR-8085" used herein includes the hsa-miR-8085 gene (miRBase Accession No. MIMAT0031012) described in SEQ ID NO: 151, its homolog or ortholog of a different organism species, and the like. The hsa-miR-8085 gene can be obtained by a method described in Wang HJ et al., 2013, Shock, Vol. 39, p. 480-487. Also, "hsa-mir-8085" (miRBase Accession No. MI0025921, SEQ ID NO: 382) having a hairpin-like structure is known as a precursor of "hsa-miR-8085".

The term "hsa-miR-4463 gene" or "hsa-miR-4463" used herein includes the hsa-miR-4463 gene (miRBase Accession No. MIMAT0018987) described in SEQ ID NO: 152, its homolog or ortholog of a different organism species, and the like. The hsa-miR-4463 gene can be obtained by a method described in Jima DD et al., 2010, Blood, Vol. 116, e118-e127. Also, "hsa-mir-4463" (miRBase Accession No. MI0016811, SEQ ID NO: 383) having a hairpin-like structure is known as a precursor of "hsa-miR-4463".

The term "hsa-miR-6807-5p gene" or "hsa-miR-6807-5p" used herein includes the hsa-miR-6807-5p gene (miRBase Accession No. MIMAT0027514) described in SEQ ID NO: 153, its homolog or ortholog of a different organism species, and the like. The hsa-miR-6807-5p gene can be obtained by a method described in Ladewig E et al., 2012, Genome Res, Vol. 22, p. 1634-1645. Also, "hsa-mir-6807" (miRBase Accession No. MI0022652, SEQ ID NO: 384) having a hairpin-like structure is known as a precursor of "hsa-miR-6807-5p".

The term "hsa-miR-4433b-3p gene" or "hsa-miR-4433b-3p" used herein includes the hsa-miR-4433b-3p gene (miRBase Accession No. MIMAT0030414) described in SEQ ID NO: 154, its homolog or ortholog of a different organism species, and the like. The hsa-miR-4433b-3p gene can be obtained by a method described in Ple H et al., 2012, PLoS One, Vol. 7, e50746. Also, "hsa-mir-4433b" (miRBase Accession No. MI0025511, SEQ ID NO: 385) having a hairpin-like structure is known as a precursor of "hsa-miR-4433b-3p".

The term "hsa-miR-3185 gene" or "hsa-miR-3185" used herein includes the hsa-miR-3185 gene (miRBase Accession No. MIMAT0015065) described in SEQ ID NO: 155, its homolog or ortholog of a different organism species, and the like. The hsa-miR-3185 gene can be obtained by a method described in Stark MS et al., 2010, PLoS One, Vol. 5, e9685. Also, "hsa-mir-3185" (miRBase Accession No. MI0014227, SEQ ID NO: 386) having a hairpin-like structure is known as a precursor of "hsa-miR-3185".

The term "hsa-miR-12121 gene" or "hsa-miR-12121" used herein includes the hsa-miR-12121 gene (miRBase Accession No. MIMAT0049015) described in SEQ ID NO: 156, its homolog or ortholog of a different organism species, and the like. The hsa-miR-12121 gene can be obtained by a method described in Ozata DM et al., 2017, Cell Death Dis, Vol. 8, e2759. Also, "hsa-mir-12121" (miRBase Accession No. MI0039723, SEQ ID NO: 387) having a hairpin-like structure is known as a precursor of "hsa-miR-12121".

The term "hsa-miR-671-5p gene" or "hsa-miR-671-5p" used herein includes the hsa-miR-671-5p gene (miRBase Accession No. MIMAT0003880) described in SEQ ID NO: 157, its homolog or ortholog of a different organism species, and the like. The hsa-miR-671-5p gene can be obtained by a method described in Berezikov E et al., 2006, Genome Res, Vol. 16, p. 1289-1298. Also, "hsa-mir-671" (miRBase Accession No. MI0003760, SEQ ID NO: 388) having a hairpin-like structure is known as a precursor of "hsa-miR-671-5p".

The term "hsa-miR-6752-5p gene" or "hsa-miR-6752-5p" used herein includes the hsa-miR-6752-5p gene (miRBase Accession No. MIMAT0027404) described in SEQ ID NO: 158, its homolog or ortholog of a different organism species, and the like. The hsa-miR-6752-5p gene can be obtained by a method described in Ladewig E et al., 2012, Genome Res, Vol. 22, p. 1634-1645. Also, "hsa-mir-6752" (miRBase Accession No. MI0022597, SEQ ID NO: 389) having a hairpin-like structure is known as a precursor of "hsa-miR-6752-5p".

The term "hsa-miR-371a-5p gene" or "hsa-miR-371a-5p" used herein includes the hsa-miR-371a-5p gene (miRBase Accession No. MIMAT0004687) described in SEQ ID NO: 159, its homolog or ortholog of a different organism species, and the like. The hsa-miR-371a-5p gene can be obtained by a method described in Suh MR et al., 2004, Dev Biol, Vol. 270, p. 488-498. Also, "hsa-mir-371a" (miRBase Accession No. MI0000779, SEQ ID NO: 390) having a hairpin-like structure is known as a precursor of "hsa-miR-371a-5p".

The term "hsa-miR-3917 gene" or "hsa-miR-3917" used herein includes the hsa-miR-3917 gene (miRBase Accession No. MIMAT0018191) described in SEQ ID NO: 160, its homolog or ortholog of a different organism species, and the like. The hsa-miR-3917 gene can be obtained by a method described in Creighton CJ et al., 2010, PLoS One, Vol. 5, e9637. Also, "hsa-mir-3917" (miRBase Accession No. MI0016423, SEQ ID NO: 391) having a hairpin-like structure is known as a precursor of "hsa-miR-3917".

The term "hsa-miR-1224-5p gene" or "hsa-miR-1224-5p" used herein includes the hsa-miR-1224-5p gene (miRBase Accession No. MIMAT0005458) described in SEQ ID NO: 161, its homolog or ortholog of a different organism species, and the like. The hsa-miR-1224-5p gene can be obtained by a method described in Berezikov E et al., 2006, Genome Res, Vol. 16, p. 1289-1298. Also, "hsa-mir-1224" (miRBase Accession No. MI0003764, SEQ ID NO: 392) having a hairpin-like structure is known as a precursor of "hsa-miR-1224-5p".

The term "hsa-miR-498-5p gene" or "hsa-miR-498-5p" used herein includes the hsa-miR-498-5p gene (miRBase Accession No. MIMAT0002824) described in SEQ ID NO: 162, its homolog or ortholog of a different organism species, and the like. The hsa-miR-498-5p gene can be obtained by a method described in Bentwich I et al., 2005, Nat Genet, Vol. 37, p. 766-770. Also, "hsa-mir-498" (miRBase Accession No. MI0003142, SEQ ID NO: 393) having a hairpin-like structure is known as a precursor of "hsa-miR-498-5p".

The term "hsa-miR-7704 gene" or "hsa-miR-7704" used herein includes the hsa-miR-7704 gene (miRBase Accession No. MIMAT0030019) described in SEQ ID NO: 163, its homolog or ortholog of a different organism species, and the like. The hsa-miR-7704 gene can be obtained by a method described in Swaminathan S et al., 2013, Biochem Biophys Res Commun, Vol. 434, p. 228-234. Also, "hsa-mir-7704" (miRBase Accession No. MI0025240, SEQ ID NO: 394) having a hairpin-like structure is known as a precursor of "hsa-miR-7704".

The term "hsa-miR-6741-5p gene" or "hsa-miR-6741-5p" used herein includes the hsa-miR-6741-5p gene (miRBase Accession No. MIMAT0027383) described in SEQ ID NO: 164, its homolog or ortholog of a different organism species, and the like. The hsa-miR-6741-5p gene can be obtained by a method described in Ladewig E et al., 2012, Genome Res, Vol. 22, p. 1634-1645. Also, "hsa-mir-6741" (miRBase Accession No. MI0022586, SEQ ID NO: 395) having a hairpin-like structure is known as a precursor of "hsa-miR-6741-5p".

The term "hsa-miR-765 gene" or "hsa-miR-765" used herein includes the hsa-miR-765 gene (miRBase Accession No. MIMAT0003945) described in SEQ ID NO: 165, its homolog or ortholog of a different organism species, and the like. The hsa-miR-765 gene can be obtained by a method described in Berezikov E et al., 2006, Genome Res, Vol. 16, p. 1289-1298. Also, "hsa-mir-765" (miRBase Accession No. MI0005116, SEQ ID NO: 396) having a hairpin-like structure is known as a precursor of "hsa-miR-765".

The term "hsa-miR-4486 gene" or "hsa-miR-4486" used herein includes the hsa-miR-4486 gene (miRBase Accession No. MIMAT0019020) described in SEQ ID NO: 166, its homolog or ortholog of a different organism species, and the like. The hsa-miR-4486 gene can be obtained by a method described in Jima DD et al., 2010, Blood, Vol. 116, e118-e127. Also, "hsa-mir-4486" (miRBase Accession No. MI0016847, SEQ ID NO: 397) having a hairpin-like structure is known as a precursor of "hsa-miR-4486".

The term "hsa-miR-6090 gene" or "hsa-miR-6090" used herein includes the hsa-miR-6090 gene (miRBase Accession No. MIMAT0023715) described in SEQ ID NO: 167, its homolog or ortholog of a different organism species, and the like. The hsa-miR-6090 gene can be obtained by a method described in Yoo JK et al., 2012, Stem Cells Dev, Vol. 21, p. 2049-2057. Also, "hsa-mir-6090" (miRBase Accession No. MI0020367, SEQ ID NO: 398) having a hairpin-like structure is known as a precursor of "hsa-miR-6090".

The term "hsa-miR-718 gene" or "hsa-miR-718" used herein includes the hsa-miR-718 gene (miRBase Accession No. MIMAT0012735) described in SEQ ID NO: 168, its homolog or ortholog of a different organism species, and the like. The hsa-miR-718 gene can be obtained by a method described in Artzi S et al., 2008, BMC Bioinformatics, Vol. 9, p. 39. Also, "hsa-mir-718" (miRBase Accession No. MI0012489, SEQ ID NO: 399) having a hairpin-like structure is known as a precursor of "hsa-miR-718".

The term "hsa-miR-4767 gene" or "hsa-miR-4767" used herein includes the hsa-miR-4767 gene (miRBase Accession No. MIMAT0019919) described in SEQ ID NO: 169, its homolog or ortholog of a different organism species, and the like. The hsa-miR-4767 gene can be obtained by a method described in Persson H et al., 2011, Cancer Res, Vol. 71, p. 78-86. Also, "hsa-mir-4767" (miRBase Accession No. MI0017408, SEQ ID NO: 400) having a hairpin-like structure is known as a precursor of "hsa-miR-4767".

The term "hsa-miR-6851-5p gene" or "hsa-miR-6851-5p" used herein includes the hsa-miR-6851-5p gene (miRBase Accession No. MIMAT0027602) described in SEQ ID NO: 170, its homolog or ortholog of a different organism species, and the like. The hsa-miR-6851-5p gene can be obtained by a method described in Ladewig E et al., 2012, Genome Res, Vol. 22, p. 1634-1645. Also, "hsa-mir-6851" (miRBase Accession No. MI0022697, SEQ ID NO: 401) having a hairpin-like structure is known as a precursor of "hsa-miR-6851-5p".

The term "hsa-miR-5572 gene" or "hsa-miR-5572" used herein includes the hsa-miR-5572 gene (miRBase Accession No. MIMAT0022260) described in SEQ ID NO: 171, its homolog or ortholog of a different organism species, and the like. The hsa-miR-5572 gene can be obtained by a method described in Tandon M et al., 2012, Oral Dis, Vol. 18, p. 127-131. Also, "hsa-mir-5572" (miRBase Accession No. MI0019117, SEQ ID NO: 402) having a hairpin-like structure is known as a precursor of "hsa-miR-5572".

The term "hsa-miR-6850-5p gene" or "hsa-miR-6850-5p" used herein includes the hsa-miR-6850-5p gene (miRBase Accession No. MIMAT0027600) described in SEQ ID NO: 172, its homolog or ortholog of a different organism species, and the like. The hsa-miR-6850-5p gene can be obtained by a method described in Ladewig E et al., 2012, Genome Res, Vol. 22, p. 1634-1645. Also, "hsa-mir-6850" (miRBase Accession No. MI0022696, SEQ ID NO: 403) having a hairpin-like structure is known as a precursor of "hsa-miR-6850-5p".

The term "hsa-miR-6089 gene" or "hsa-miR-6089" used herein includes the hsa-miR-6089 gene (miRBase Accession No. MIMAT0023714) described in SEQ ID NO: 173, its homolog or ortholog of a different organism species, and the like. The hsa-miR-6089 gene can be obtained by a method described in Yoo JK et al., 2012, Stem Cells Dev, Vol. 21, p. 2049-2057. Also, "hsa-mir-6089-1" (miRBase Accession No. MI0020366, SEQ ID NO: 404) having a hairpin-like structure is known as a precursor of "hsa-miR-6089".

The term "hsa-miR-5787 gene" or "hsa-miR-5787" used herein includes the hsa-miR-5787 gene (miRBase Accession No. MIMAT0023252) described in SEQ ID NO: 174, its homolog or ortholog of a different organism species, and the like. The hsa-miR-5787 gene can be obtained by a method described in Yoo H et al., 2011, Biochem Biophys Res Commun, Vol. 415, p. 567-572. Also, "hsa-mir-5787" (miRBase Accession No. MI0019797, SEQ ID NO: 405) having a hairpin-like structure is known as a precursor of "hsa-miR-5787".

The term "hsa-miR-4534 gene" or "hsa-miR-4534" used herein includes the hsa-miR-4534 gene (miRBase Accession No. MIMAT0019073) described in SEQ ID NO: 175, its homolog or ortholog of a different organism species, and the like. The hsa-miR-4534 gene can be obtained by a method described in Jima DD et al., 2010, Blood, Vol. 116, e118-e127. Also, "hsa-mir-4534" (miRBase Accession No. MI0016901, SEQ ID NO: 406) having a hairpin-like structure is known as a precursor of "hsa-miR-4534".

The term "hsa-miR-3665 gene" or "hsa-miR-3665" used herein includes the hsa-miR-3665 gene (miRBase Accession No. MIMAT0018087) described in SEQ ID NO: 176, its homolog or ortholog of a different organism species, and the like. The hsa-miR-3665 gene can be obtained by a method described in Xie X et al., 2005, Nature, Vol. 434, p. 338-345. Also, "hsa-mir-3665" (miRBase Accession No. MI0016066, SEQ ID NO: 407) having a hairpin-like structure is known as a precursor of "hsa-miR-3665".

The term "hsa-miR-4787-5p gene" or "hsa-miR-4787-5p" used herein includes the hsa-miR-4787-5p gene (miRBase Accession No. MIMAT0019956) described in SEQ ID NO: 177, its homolog or ortholog of a different organism species, and the like. The hsa-miR-4787-5p gene can be obtained by a method described in Persson H et al., 2011, Cancer Res, Vol. 71, p. 78-86. Also, "hsa-mir-4787" (miRBase Accession No. MI0017434, SEQ ID NO: 408) having a hairpin-like structure is known as a precursor of "hsa-miR-4787-5p".

The term "hsa-miR-6754-5p gene" or "hsa-miR-6754-5p" used herein includes the hsa-miR-6754-5p gene (miRBase Accession No. MIMAT0027408) described in SEQ ID NO: 178, its homolog or ortholog of a different organism species, and the like. The hsa-miR-6754-5p gene can be obtained by a method described in Ladewig E et al., 2012, Genome Res, Vol. 22, p. 1634-1645. Also, "hsa-mir-6754" (miRBase Accession No. MI0022599, SEQ ID NO: 409) having a hairpin-like structure is known as a precursor of "hsa-miR-6754-5p".

The term "hsa-miR-6825-3p gene" or "hsa-miR-6825-3p" used herein includes the hsa-miR-6825-3p gene (miRBase Accession No. MIMAT0027551) described in SEQ ID NO: 179, its homolog or ortholog of a different organism species, and the like. The hsa-miR-6825-3p gene can be obtained by a method described in Ladewig E et al., 2012, Genome Res, Vol. 22, p. 1634-1645. Also, "hsa-mir-6825" (miRBase Accession No. MI0022670, SEQ ID NO: 410) having a hairpin-like structure is known as a precursor of "hsa-miR-6825-3p".

The term "hsa-miR-4728-5p gene" or "hsa-miR-4728-5p" used herein includes the hsa-miR-4728-5p gene (miRBase Accession No. MIMAT0019849) described in SEQ ID NO: 180, its homolog or ortholog of a different organism species, and the like. The hsa-miR-4728-5p gene can be obtained by a method described in Persson H et al., 2011, Cancer Res, Vol. 71, p. 78-86. Also, "hsa-mir-4728" (miRBase Accession No. MI0017365, SEQ ID NO: 411) having a hairpin-like structure is known as a precursor of "hsa-miR-4728-5p".

The term "hsa-miR-6088 gene" or "hsa-miR-6088" used herein includes the hsa-miR-6088 gene (miRBase Accession No. MIMAT0023713) described in SEQ ID NO: 181, its homolog or ortholog of a different organism species, and the like. The hsa-miR-6088 gene can be obtained by a method described in Yoo JK et al., 2012, Stem Cells Dev, Vol. 21, p. 2049-2057. Also, "hsa-mir-6088" (miRBase Accession No. MI0020365, SEQ ID NO: 412) having a hairpin-like structure is known as a precursor of "hsa-miR-6088".

The term "hsa-miR-3154 gene" or "hsa-miR-3154" used herein includes the hsa-miR-3154 gene (miRBase Accession No. MIMAT0015028) described in SEQ ID NO: 182, its homolog or ortholog of a different organism species, and the like. The hsa-miR-3154 gene can be obtained by a method described in Berezikov E et al., 2006, Genome Res, Vol. 16, p. 1289-1298. Also, "hsa-mir-3154" (miRBase Accession No. MI0014182, SEQ ID NO: 413) having a hairpin-like structure is known as a precursor of "hsa-miR-3154".

The term "hsa-miR-6869-5p gene" or "hsa-miR-6869-5p" used herein includes the hsa-miR-6869-5p gene (miRBase Accession No. MIMAT0027638) described in SEQ ID NO: 183, its homolog or ortholog of a different organism species, and the like. The hsa-miR-6869-5p gene can be obtained by a method described in Ladewig E et al., 2012, Genome Res, Vol. 22, p. 1634-1645. Also, "hsa-mir-6869" (miRBase Accession No. MI0022716, SEQ ID NO: 414) having a hairpin-like structure is known as a precursor of "hsa-miR-6869-5p".

The term "hsa-miR-187-5p gene" or "hsa-miR-187-5p" used herein includes the hsa-miR-187-5p gene (miRBase Accession No. MIMAT0004561) described in SEQ ID NO: 184, its homolog or ortholog of a different organism species, and the like. The hsa-miR-187-5p gene can be obtained by a method described in Lim LP et al., 2003, Science, Vol. 299, p. 1540. Also, "hsa-mir-187" (miRBase Accession No. MI0000274, SEQ ID NO: 415) having a hairpin-like structure is known as a precursor of "hsa-miR-187-5p".

The term "hsa-miR-6165 gene" or "hsa-miR-6165" used herein includes the hsa-miR-6165 gene (miRBase Accession No. MIMAT0024782) described in SEQ ID NO: 185, its homolog or ortholog of a different organism species, and the like. The hsa-miR-6165 gene can be obtained by a method described in Parsi S et al., 2012, PLoS One, Vol. 7, e35561. Also, "hsa-mir-6165" (miRBase Accession No. MI0021472, SEQ ID NO: 416) having a hairpin-like structure is known as a precursor of "hsa-miR-6165".

The term "hsa-miR-4447 gene" or "hsa-miR-4447" used herein includes the hsa-miR-4447 gene (miRBase Accession No. MIMAT0018966) described in SEQ ID NO: 186, its homolog or ortholog of a different organism species, and the like. The hsa-miR-4447 gene can be obtained by a method described in Jima DD et al., 2010, Blood, Vol. 116, e118-e127. Also, "hsa-mir-4447" (miRBase Accession No. MI0016790, SEQ ID NO: 417) having a hairpin-like structure is known as a precursor of "hsa-miR-4447".

The term "hsa-miR-4731-5p gene" or "hsa-miR-4731-5p" used herein includes the hsa-miR-4731-5p gene (miRBase Accession No. MIMAT0019853) described in SEQ ID NO: 187, its homolog or ortholog of a different organism species, and the like. The hsa-miR-4731-5p gene can be obtained by a method described in Persson H et al., 2011, Cancer Res, Vol. 71, p. 78-86. Also, "hsa-mir-4731" (miRBase Accession No. MI0017368, SEQ ID NO: 418) having a hairpin-like structure is known as a precursor of "hsa-miR-4731-5p".

The term "hsa-miR-6805-5p gene" or "hsa-miR-6805-5p" used herein includes the hsa-miR-6805-5p gene (miRBase Accession No. MIMAT0027510) described in SEQ ID NO: 188, its homolog or ortholog of a different organism species, and the like. The hsa-miR-6805-5p gene can be obtained by a method described in Ladewig E et al., 2012, Genome Res, Vol. 22, p. 1634-1645. Also, "hsa-mir-6805" (miRBase Accession No. MI0022650, SEQ ID NO: 377) having a hairpin-like structure is known as a precursor of "hsa-miR-6805-5p".

The term "hsa-miR-12118 gene" or "hsa-miR-12118" used herein includes the hsa-miR-12118 gene (miRBase Accession No. MIMAT0049012) described in SEQ ID NO: 189, its homolog or ortholog of a different organism species, and the like. The hsa-miR-12118 gene can be obtained by a method described in Ozata DM et al., 2017, Cell Death Dis, Vol. 8, e2759. Also, "hsa-mir-12118" (miRBase Accession No. MI0039720, SEQ ID NO: 419) having a hairpin-like structure is known as a precursor of "hsa-miR-12118".

The term "hsa-miR-4270 gene" or "hsa-miR-4270" used herein includes the hsa-miR-4270 gene (miRBase Accession No. MIMAT0016900) described in SEQ ID NO: 190, its homolog or ortholog of a different organism species, and the like. The hsa-miR-4270 gene can be obtained by a method described in Goff LA et al., 2009, PLoS One, Vol. 4, e7192. Also, "hsa-mir-4270" (miRBase Accession No. MI0015878, SEQ ID NO: 420) having a hairpin-like structure is known as a precursor of "hsa-miR-4270".

The term "hsa-miR-7846-3p gene" or "hsa-miR-7846-3p" used herein includes the hsa-miR-7846-3p gene (miRBase Accession No. MIMAT0030421) described in SEQ ID NO: 191, its homolog or ortholog of a different organism species, and the like. The hsa-miR-7846-3p gene can be obtained by a method described in Ple H et al., 2012, PLoS One, Vol. 7, e50746. Also, "hsa-mir-7846" (miRBase AccessionNo. MI0025516, SEQ ID NO: 421) having a hairpin-like structure is known as a precursor of "hsa-miR-7846-3p".

The term "hsa-miR-4443 gene" or "hsa-miR-4443" used herein includes the hsa-miR-4443 gene (miRBase Accession No. MIMAT0018961) described in SEQ ID NO: 192, its homolog or ortholog of a different organism species, and the like. The hsa-miR-4443 gene can be obtained by a method described in Jima DD et al., 2010, Blood, Vol. 116, e118-e127. Also, "hsa-mir-4443" (miRBase Accession No. MI0016786, SEQ ID NO: 422) having a hairpin-like structure is known as a precursor of "hsa-miR-4443".

The term "hsa-miR-6737-5p gene" or "hsa-miR-6737-5p" used herein includes the hsa-miR-6737-5p gene (miRBase Accession No. MIMAT0027375) described in SEQ ID NO: 193, its homolog or ortholog of a different organism species, and the like. The hsa-miR-6737-5p gene can be obtained by a method described in Ladewig E et al., 2012, Genome Res, Vol. 22, p. 1634-1645. Also, "hsa-mir-6737" (miRBase Accession No. MI0022582, SEQ ID NO: 423) having a hairpin-like structure is known as a precursor of "hsa-miR-6737-5p".

The term "hsa-miR-197-5p gene" or "hsa-miR-197-5p" used herein includes the hsa-miR-197-5p gene (miRBase Accession No. MIMAT0022691) described in SEQ ID NO: 194, its homolog or ortholog of a different organism species, and the like. The hsa-miR-197-5p gene can be obtained by a method described in Lagos-Quintana M et al., 2003, RNA, Vol. 9, p. 175-179. Also, "hsa-mir-197" (miRBase Accession No. MI0000239, SEQ ID NO: 424) having a hairpin-like structure is known as a precursor of "hsa-miR-197-5p".

The term "hsa-miR-1229-5p gene" or "hsa-miR-1229-5p" used herein includes the hsa-miR-1229-5p gene (miRBase Accession No. MIMAT0022942) described in SEQ ID NO: 195, its homolog or ortholog of a different organism species, and the like. The hsa-miR-1229-5p gene can be obtained by a method described in Berezikov E et al., 2007, Mol Cell, Vol. 28, p. 328-336. Also, "hsa-mir-1229" (miRBase Accession No. MI0006319, SEQ ID NO: 425) having a hairpin-like structure is known as a precursor of "hsa-miR-1229-5p".

The term "hsa-miR-6757-5p gene" or "hsa-miR-6757-5p" used herein includes the hsa-miR-6757-5p gene (miRBase Accession No. MIMAT0027414) described in SEQ ID NO: 196, its homolog or ortholog of a different organism species, and the like. The hsa-miR-6757-5p gene can be obtained by a method described in Ladewig E et al., 2012, Genome Res, Vol. 22, p. 1634-1645. Also, "hsa-mir-6757" (miRBase Accession No. MI0022602, SEQ ID NO: 426) having a hairpin-like structure is known as a precursor of "hsa-miR-6757-5p".

The term "hsa-miR-6765-5p gene" or "hsa-miR-6765-5p" used herein includes the hsa-miR-6765-5p gene (miRBase Accession No. MIMAT0027430) described in SEQ ID NO: 197, its homolog or ortholog of a different organism species, and the like. The hsa-miR-6765-5p gene can be obtained by a method described in Ladewig E et al., 2012, Genome Res, Vol. 22, p. 1634-1645. Also, "hsa-mir-6765" (miRBase Accession No. MI0022610, SEQ ID NO: 427) having a hairpin-like structure is known as a precursor of "hsa-miR-6765-5p".

The term "hsa-miR-4722-5p gene" or "hsa-miR-4722-5p" used herein includes the hsa-miR-4722-5p gene (miRBase Accession No. MIMAT0019836) described in SEQ ID NO: 198, its homolog or ortholog of a different organism species, and the like. The hsa-miR-4722-5p gene can be obtained by a method described in Persson H et al., 2011, Cancer Res, Vol. 71, p. 78-86. Also, "hsa-mir-4722" (miRBase Accession No. MI0017357, SEQ ID NO: 428) having a hairpin-like structure is known as a precursor of "hsa-miR-4722-5p".

The term "hsa-miR-6891-5p gene" or "hsa-miR-6891-5p" used herein includes the hsa-miR-6891-5p gene (miRBase Accession No. MIMAT0027682) described in SEQ ID NO: 199, its homolog or ortholog of a different organism species, and the like. The hsa-miR-6891-5p gene can be obtained by a method described in Ladewig E et al., 2012, Genome Res, Vol. 22, p. 1634-1645. Also, "hsa-mir-6891" (miRBase Accession No. MI0022738, SEQ ID NO: 429) having a hairpin-like structure is known as a precursor of "hsa-miR-6891-5p".

The term "hsa-miR-5006-5p gene" or "hsa-miR-5006-5p" used herein includes the hsa-miR-5006-5p gene (miRBase Accession No. MIMAT0021033) described in SEQ ID NO: 200, its homolog or ortholog of a different organism species, and the like. The hsa-miR-5006-5p gene can be obtained by a method described in Hansen TB et al., 2011, RNA Biol, Vol. 8, p. 378-383. Also, "hsa-mir-5006" (miRBase Accession No. MI0017873, SEQ ID NO: 430) having a hairpin-like structure is known as a precursor of "hsa-miR-5006-5p".

The term "hsa-miR-345-3p gene" or "hsa-miR-345-3p" used herein includes the hsa-miR-345-3p gene (miRBase Accession No. MIMAT0022698) described in SEQ ID NO: 201, its homolog or ortholog of a different organism species, and the like. The hsa-miR-345-3p gene can be obtained by a method described in Kim J et al., 2004, Proc Natl Acad Sci USA, Vol. 101, p. 360-365. Also, "hsa-mir-345" (miRBase Accession No. MI0000825, SEQ ID NO: 431) having a hairpin-like structure is known as a precursor of "hsa-miR-345-3p".

The term "hsa-miR-6726-5p gene" or "hsa-miR-6726-5p" used herein includes the hsa-miR-6726-5p gene (miRBase Accession No. MIMAT0027353) described in SEQ ID NO: 202, its homolog or ortholog of a different organism species, and the like. The hsa-miR-6726-5p gene can be obtained by a method described in Ladewig E et al., 2012, Genome Res, Vol. 22, p. 1634-1645. Also, "hsa-mir-6726" (miRBase Accession No. MI0022571, SEQ ID NO: 432) having a hairpin-like structure is known as a precursor of "hsa-miR-6726-5p".

The term "hsa-miR-3195 gene" or "hsa-miR-3195" used herein includes the hsa-miR-3195 gene (miRBase Accession No. MIMAT0015079) described in SEQ ID NO: 203, its homolog or ortholog of a different organism species, and the like. The hsa-miR-3195 gene can be obtained by a method described in Stark MS et al., 2010, PLoS One, Vol. 5, e9685. Also, "hsa-mir-3195" (miRBase Accession No. MI0014240, SEQ ID NO: 433) having a hairpin-like structure is known as a precursor of "hsa-miR-3195".

The term "hsa-miR-6877-5p gene" or "hsa-miR-6877-5p" used herein includes the hsa-miR-6877-5p gene (miRBase Accession No. MIMAT0027654) described in SEQ ID NO: 204, its homolog or ortholog of a different organism species, and the like. The hsa-miR-6877-5p gene can be obtained by a method described in Ladewig E et al., 2012, Genome Res, Vol. 22, p. 1634-1645. Also, "hsa-mir-6877" (miRBase Accession No. MI0022724, SEQ ID NO: 434) having a hairpin-like structure is known as a precursor of "hsa-miR-6877-5p".

The term "hsa-miR-4462 gene" or "hsa-miR-4462" used herein includes the hsa-miR-4462 gene (miRBase Accession No. MIMAT0018986) described in SEQ ID NO: 205, its homolog or ortholog of a different organism species, and the like. The hsa-miR-4462 gene can be obtained by a method described in Jima DD et al., 2010, Blood, Vol. 116, e118-e127. Also, "hsa-mir-4462" (miRBase Accession No. MI0016810, SEQ ID NO: 435) having a hairpin-like structure is known as a precursor of "hsa-miR-4462".

The term "hsa-miR-6812-5p gene" or "hsa-miR-6812-5p" used herein includes the hsa-miR-6812-5p gene (miRBase Accession No. MIMAT0027524) described in SEQ ID NO: 206, its homolog or ortholog of a different organism species, and the like. The hsa-miR-6812-5p gene can be obtained by a method described in Ladewig E et al., 2012, Genome Res, Vol. 22, p. 1634-1645. Also, "hsa-mir-6812" (miRBase Accession No. MI0022657, SEQ ID NO: 436) having a hairpin-like structure is known as a precursor of "hsa-miR-6812-5p".

The term "hsa-miR-483-5p gene" or "hsa-miR-483-5p" used herein includes the hsa-miR-483-5p gene (miRBase Accession No. MIMAT0004761) described in SEQ ID NO: 207, its homolog or ortholog of a different organism species, and the like. The hsa-miR-483-5p gene can be obtained by a method described in Fu H et al., 2005, FEBS Lett, Vol. 579, p. 3849-3854. Also, "hsa-mir-483" (miRBase Accession No. MI0002467, SEQ ID NO: 437) having a hairpin-like structure is known as a precursor of "hsa-miR-483-5p".

The term "hsa-miR-9899 gene" or "hsa-miR-9899" used herein includes the hsa-miR-9899 gene (miRBase Accession No. MIMAT0039319) described in SEQ ID NO: 208, its homolog or ortholog of a different organism species, and the like. The hsa-miR-9899 gene can be obtained by a method described in Boele J et al., 2014, Proc Natl Acad Sci USA, Vol. 111, p. 11467-11472. Also, "hsa-mir-9899" (miRBase Accession No. MI0031827, SEQ ID NO: 438) having a hairpin-like structure is known as a precursor of "hsa-miR-9899".

The term "hsa-miR-4800-5p gene" or "hsa-miR-4800-5p" used herein includes the hsa-miR-4800-5p gene (miRBase Accession No. MIMAT0019978) described in SEQ ID NO: 209, its homolog or ortholog of a different organism species, and the like. The hsa-miR-4800-5p gene can be obtained by a method described in Persson H et al., 2011, Cancer Res, Vol. 71, p. 78-86. Also, "hsa-mir-4800" (miRBase Accession No. MI0017448, SEQ ID NO: 439) having a hairpin-like structure is known as a precursor of "hsa-miR-4800-5p".

The term "hsa-miR-4734 gene" or "hsa-miR-4734" used herein includes the hsa-miR-4734 gene (miRBase Accession No. MIMAT0019859) described in SEQ ID NO: 210, its homolog or ortholog of a different organism species, and the like. The hsa-miR-4734 gene can be obtained by a method described in Persson H et al., 2011, Cancer Res, Vol. 71, p. 78-86. Also, "hsa-mir-4734" (miRBase Accession No. MI0017371, SEQ ID NO: 440) having a hairpin-like structure is known as a precursor of "hsa-miR-4734".

The term "hsa-miR-3135b gene" or "hsa-miR-3135b" used herein includes the hsa-miR-3135b gene (miRBase Accession No. MIMAT0018985) described in SEQ ID NO: 211, its homolog or ortholog of a different organism species, and the like. The hsa-miR-3135b gene can be obtained by a method described in Jima DD et al., 2010, Blood, Vol. 116, e118-e127. Also, "hsa-mir-3135b" (miRBase Accession No. MI0016809, SEQ ID NO: 441) having a hairpin-like structure is known as a precursor of "hsa-miR-3135b".

The term "hsa-miR-4433a-3p gene" or "hsa-miR-4433a-3p" used herein includes the hsa-miR-4433a-3p gene (miRBase Accession No. MIMAT0018949) described in SEQ ID NO: 212, its homolog or ortholog of a different organism species, and the like. The hsa-miR-4433a-3p gene can be obtained by a method described in Jima DD et al., 2010, Blood, Vol. 116, e118-e127. Also, "hsa-mir-4433a" (miRBase Accession No. MI0016773, SEQ ID NO: 442) having a hairpin-like structure is known as a precursor of "hsa-miR-4433a-3p".

The term "hsa-miR-6769a-5p gene" or "hsa-miR-6769a-5p" used herein includes the hsa-miR-6769a-5p gene (miRBase Accession No. MIMAT0027438) described in SEQ ID NO: 213, its homolog or ortholog of a different organism species, and the like. The hsa-miR-6769a-5p gene can be obtained by a method described in Ladewig E et al., 2012, Genome Res, Vol. 22, p. 1634-1645. Also, "hsa-mir-6769a" (miRBase Accession No. MI0022614, SEQ ID NO: 443) having a hairpin-like structure is known as a precursor of "hsa-miR-6769a-5p".

The term "hsa-miR-4743-5p gene" or "hsa-miR-4743-5p" used herein includes the hsa-miR-4743-5p gene (miRBase Accession No. MIMAT0019874) described in SEQ ID NO: 214, its homolog or ortholog of a different organism species, and the like. The hsa-miR-4743-5p gene can be obtained by a method described in Persson H et al., 2011, Cancer Res, Vol. 71, p. 78-86. Also, "hsa-mir-4743" (miRBase Accession No. MI0017381, SEQ ID NO: 444) having a hairpin-like structure is known as a precursor of "hsa-miR-4743-5p".

The term "hsa-miR-1909-3p gene" or "hsa-miR-1909-3p" used herein includes the hsa-miR-1909-3p gene (miRBase Accession No. MIMAT0007883) described in SEQ ID NO: 215, its homolog or ortholog of a different organism species, and the like. The hsa-miR-1909-3p gene can be obtained by a method described in Bar M et al., 2008, Stem Cells, Vol. 26, p. 2496-2505. Also, "hsa-mir-1909" (miRBase Accession No. MI0008330, SEQ ID NO: 445) having a hairpin-like structure is known as a precursor of "hsa-miR-1909-3p".

The term "hsa-miR-4741 gene" or "hsa-miR-4741" used herein includes the hsa-miR-4741 gene (miRBase Accession No. MIMAT0019871) described in SEQ ID NO: 216, its homolog or ortholog of a different organism species, and the like. The hsa-miR-4741 gene can be obtained by a method described in Persson H et al., 2011, Cancer Res, Vol. 71, p. 78-86. Also, "hsa-mir-4741" (miRBase Accession No. MI0017379, SEQ ID NO: 446) having a hairpin-like structure is known as a precursor of "hsa-miR-4741".

The term "hsa-miR-4685-5p gene" or "hsa-miR-4685-5p" used herein includes the hsa-miR-4685-5p gene (miRBase Accession No. MIMAT0019771) described in SEQ ID NO: 217, its homolog or ortholog of a different organism species, and the like. The hsa-miR-4685-5p gene can be obtained by a method described in Persson H et al., 2011, Cancer Res, Vol. 71, p. 78-86. Also, "hsa-mir-4685" (miRBase Accession No. MI0017317, SEQ ID NO: 447) having a hairpin-like structure is known as a precursor of "hsa-miR-4685-5p".

The term "hsa-miR-3147 gene" or "hsa-miR-3147" used herein includes the hsa-miR-3147 gene (miRBase Accession No. MIMAT0015019) described in SEQ ID NO: 218, its homolog or ortholog of a different organism species, and the like. The hsa-miR-3147 gene can be obtained by a method described in Stark MS et al., 2010, PLoS One, Vol. 5, e9685. Also, "hsa-mir-3147" (miRBase Accession No. MI0014173, SEQ ID NO: 448) having a hairpin-like structure is known as a precursor of "hsa-miR-3147".

The term "hsa-miR-4726-5p gene" or "hsa-miR-4726-5p" used herein includes the hsa-miR-4726-5p gene (miRBase Accession No. MIMAT0019845) described in SEQ ID NO: 219, its homolog or ortholog of a different organism species, and the like. The hsa-miR-4726-5p gene can be obtained by a method described in Persson H et al., 2011, Cancer Res, Vol. 71, p. 78-86. Also, "hsa-mir-4726" (miRBase Accession No. MI0017363, SEQ ID NO: 449) having a hairpin-like structure is known as a precursor of "hsa-miR-4726-5p".

The term "hsa-miR-3180 gene" or "hsa-miR-3180" used herein includes the hsa-miR-3180 gene (miRBase Accession No. MIMAT0018178) described in SEQ ID NO: 220, its homolog or ortholog of a different organism species, and the like. The hsa-miR-3180 gene can be obtained by a method described in Creighton CJ et al., 2010, PLoS One, Vol. 5, e9637. Also, "hsa-mir-3180-4" (miRBase Accession No. MI0016408, SEQ ID NO: 450) having a hairpin-like structure is known as a precursor of "hsa-miR-3180".

The term "hsa-miR-3188 gene" or "hsa-miR-3188" used herein includes the hsa-miR-3188 gene (miRBase Accession No. MIMAT0015070) described in SEQ ID NO: 221, its homolog or ortholog of a different organism species, and the like. The hsa-miR-3188 gene can be obtained by a method described in Stark MS et al., 2010, PLoS One, Vol. 5, e9685. Also, "hsa-mir-3188" (miRBase Accession No. MI0014232, SEQ ID NO: 451) having a hairpin-like structure is known as a precursor of "hsa-miR-3188".

The term "hsa-miR-6782-5p gene" or "hsa-miR-6782-5p" used herein includes the hsa-miR-6782-5p gene (miRBase Accession No. MIMAT0027464) described in SEQ ID NO: 222, its homolog or ortholog of a different organism species, and the like. The hsa-miR-6782-5p gene can be obtained by a method described in Ladewig E et al., 2012, Genome Res, Vol. 22, p. 1634-1645. Also, "hsa-mir-6782" (miRBase Accession No. MI0022627, SEQ ID NO: 452) having a hairpin-like structure is known as a precursor of "hsa-miR-6782-5p".

The term "hsa-miR-6776-5p gene" or "hsa-miR-6776-5p" used herein includes the hsa-miR-6776-5p gene (miRBase Accession No. MIMAT0027452) described in SEQ ID NO: 223, its homolog or ortholog of a different organism species, and the like. The hsa-miR-6776-5p gene can be obtained by a method described in Ladewig E et al., 2012, Genome Res, Vol. 22, p. 1634-1645. Also, "hsa-mir-6776" (miRBase Accession No. MI0022621, SEQ ID NO: 453) having a hairpin-like structure is known as a precursor of "hsa-miR-6776-5p".

The term "hsa-miR-4484 gene" or "hsa-miR-4484" used herein includes the hsa-miR-4484 gene (miRBase Accession No. MIMAT0019018) described in SEQ ID NO: 224, its homolog or ortholog of a different organism species, and the like. The hsa-miR-4484 gene can be obtained by a method described in Jima DD et al., 2010, Blood, Vol. 116, e118-e127. Also, "hsa-mir-4484" (miRBase Accession No. MI0016845, SEQ ID NO: 454) having a hairpin-like structure is known as a precursor of "hsa-miR-4484".

The term "hsa-miR-1185-1-3p gene" or "hsa-miR-1185-1-3p" used herein includes the hsa-miR-1185-1-3p gene (miRBase Accession No. MIMAT0022838) described in SEQ ID NO: 225, its homolog or ortholog of a different organism species, and the like. The hsa-miR-1185-1-3p gene can be obtained by a method described in Berezikov E et al., 2006, Genome Res, Vol. 16, p. 1289-1298. Also, "hsa-mir-1185-1" (miRBase Accession No. MI0003844, SEQ ID NO: 455) having a hairpin-like structure is known as a precursor of "hsa-miR-1185-1-3p".

The term "hsa-miR-6790-3p gene" or "hsa-miR-6790-3p" used herein includes the hsa-miR-6790-3p gene (miRBase Accession No. MIMAT0027481) described in SEQ ID NO: 226, its homolog or ortholog of a different organism species, and the like. The hsa-miR-6790-3p gene can be obtained by a method described in Ladewig E et al., 2012, Genome Res, Vol. 22, p. 1634-1645. Also, "hsa-mir-6790" (miRBase Accession No. MI0022635, SEQ ID NO: 456) having a hairpin-like structure is known as a precursor of "hsa-miR-6790-3p".

The term "hsa-miR-4466 gene" or "hsa-miR-4466" used herein includes the hsa-miR-4466 gene (miRBase Accession No. MIMAT0018993) described in SEQ ID NO: 227, its homolog or ortholog of a different organism species, and the like. The hsa-miR-4466 gene can be obtained by a method described in Jima DD et al., 2010, Blood, Vol. 116, e118-e127. Also, "hsa-mir-4466" (miRBase Accession No. MI0016817, SEQ ID NO: 457) having a hairpin-like structure is known as a precursor of "hsa-miR-4466".

The term "hsa-miR-10394-3p gene" or "hsa-miR-10394-3p" used herein includes the hsa-miR-10394-3p gene (miRBase Accession No. MIMAT0041620) described in SEQ ID NO: 228, its homolog or ortholog of a different organism species, and the like. The hsa-miR-10394-3p gene can be obtained by a method described in Lim EL et al., 2015, Genome Biol, Vol. 16, p. 18. Also, "hsa-mir-10394" (miRBase Accession No. MI0033418, SEQ ID NO: 458) having a hairpin-like structure is known as a precursor of "hsa-miR-10394-3p".

The term "hsa-miR-1275 gene" or "hsa-miR-1275" used herein includes the hsa-miR-1275 gene (miRBase Accession No. MIMAT0005929) described in SEQ ID NO: 229, its homolog or ortholog of a different organism species, and the like. The hsa-miR-1275 gene can be obtained by a method described in Morin RD et al., 2008, Genome Res, Vol. 18, p. 610-621. Also, "hsa-mir-1275" (miRBase Accession No. MI0006415, SEQ ID NO: 459) having a hairpin-like structure is known as a precursor of "hsa-miR-1275".

The term "hsa-miR-4478 gene" or "hsa-miR-4478" used herein includes the hsa-miR-4478 gene (miRBase Accession No. MIMAT0019006) described in SEQ ID NO: 230, its homolog or ortholog of a different organism species, and the like. The hsa-miR-4478 gene can be obtained by a method described in Jima DD et al., 2010, Blood, Vol. 116, e118-e127. Also, "hsa-mir-4478" (miRBase Accession No. MI0016831, SEQ ID NO: 460) having a hairpin-like structure is known as a precursor of "hsa-miR-4478".

The term "hsa-miR-3175 gene" or "hsa-miR-3175" used herein includes the hsa-miR-3175 gene (miRBase Accession No. MIMAT0015052) described in SEQ ID NO: 231, its homolog or ortholog of a different organism species, and the like. The hsa-miR-3175 gene can be obtained by a method described in Stark MS et al., 2010, PLoS One, Vol. 5, e9685. Also, "hsa-mir-3175" (miRBase Accession No. MI0014209, SEQ ID NO: 461) having a hairpin-like structure is known as a precursor of "hsa-miR-3175".

The term "hsa-miR-7106-5p gene" or "hsa-miR-7106-5p" used herein includes the hsa-miR-7106-5p gene (miRBase Accession No. MIMAT0028109) described in SEQ ID NO: 232, its homolog or ortholog of a different organism species, and the like. The hsa-miR-7106-5p gene can be obtained by a method described in Ladewig E et al., 2012, Genome Res, Vol. 22, p. 1634-1645. Also, "hsa-mir-7106" (miRBase Accession No. MI0022957, SEQ ID NO: 462) having a hairpin-like structure is known as a precursor of "hsa-miR-7106-5p".

The term "hsa-miR-4667-5p gene" or "hsa-miR-4667-5p" used herein includes the hsa-miR-4667-5p gene (miRBase Accession No. MIMAT0019743) described in SEQ ID NO: 233, its homolog or ortholog of a different organism species, and the like. The hsa-miR-4667-5p gene can be obtained by a method described in Persson H et al., 2011, Cancer Res, Vol. 71, p. 78-86. Also, "hsa-mir-4667" (miRBase Accession No. MI0017297, SEQ ID NO: 463) having a hairpin-like structure is known as a precursor of "hsa-miR-4667-5p".

The term "hsa-miR-193b-5p gene" or "hsa-miR-193b-5p" used herein includes the hsa-miR-193b-5p gene (miRBase Accession No. MIMAT0004767) described in SEQ ID NO: 234, its homolog or ortholog of a different organism species, and the like. The hsa-miR-193b-5p gene can be obtained by a method described in Bentwich I et al., 2005, Nat Genet, Vol. 37, p. 766-770. Also, "hsa-mir-193b" (miRBase Accession No. MI0003137, SEQ ID NO: 464) having a hairpin-like structure is known as a precursor of "hsa-miR-193b-5p".

The term "hsa-miR-602 gene" or "hsa-miR-602" used herein includes the hsa-miR-602 gene (miRBase Accession No. MIMAT0003270) described in SEQ ID NO: 235, its homolog or ortholog of a different organism species, and the like. The hsa-miR-602 gene can be obtained by a method described in Cummins JM et al., 2006, Proc Natl Acad Sci USA, Vol. 103, p. 3687-3692. Also, "hsa-mir-602" (miRBase Accession No. MI0003615, SEQ ID NO: 465) having a hairpin-like structure is known as a precursor of "hsa-miR-602".

A mature miRNA may become a variant due to the sequence cleaved shorter or longer by one to several flanking nucleotides, or due to substitution of nucleotides, when cut out as the mature miRNA from its RNA precursor having a hairpin-like structure. This variant is called isomiR (Morin RD. et al., 2008, Genome Res., Vol. 18, p. 610-621). The miRBase Release 22 shows the nucleotide sequences represented by SEQ ID NOs: 1 to 235 as well as a large number of the nucleotide sequence variants and fragments represented by SEQ ID NOs: 466 to 852, called isomiRs. These variants can also be obtained as miRNAs having a nucleotide sequence represented by any of SEQ ID NOs: 1 to 235. Specifically, among the variants of polynucleotides consisting of the nucleotide sequence represented by any of SEQ ID NOs: 1, 2, 3, 4, 6, 7, 8, 9, 11, 12, 13, 14, 16, 17, 18, 19, 20, 21, 24, 25, 26, 29, 31, 32, 33, 34, 35, 36, 37, 38, 41, 42, 43, 44, 45, 46, 47, 48, 49, 50, 51, 52, 53, 54, 55, 56, 57, 58, 59, 60, 61, 64, 65, 67, 68, 69, 70, 73, 75, 76, 79, 80, 81, 83, 84, 85, 87, 88, 89, 93, 94, 95, 97, 98, 100, 101, 104, 106, 107, 108, 109, 111, 112, 115, 116, 117, 118, 119, 120, 122, 123, 124, 125, 127, 128, 129, 130, 132, 134, 136, 137, 142, 143, 144, 145, 146, 147, 148, 150, 151, 152, 153, 154, 155, 156, 157, 159, 160, 161, 162, 163, 164, 165, 166, 168, 169, 170, 171, 172, 173, 174, 176, 177, 178, 179, 180, 181, 182, 183, 184, 187, 188, 189, 191, 192, 194, 195, 196, 198, 199, 200, 202, 203, 204, 207, 209, 210, 211, 212, 214, 215, 216, 219, 220, 221, 223, 224, 225, 227, 228, 229, 231, 232, 233, and 234 or a nucleotide sequence derived from the nucleotide sequence by the replacement of u with t according to the present invention, examples of the longest variants registered in miRBase Release 22 include polynucleotides represented by SEQ ID NOs: 466, 468, 470, 472, 475, 477, 479, 481, 483, 485, 487, 489, 491, 493, 495, 497, 499, 501, 504, 506, 508, 511, 514, 516, 518, 520, 522, 524, 526, 528, 530, 532, 534, 536, 538, 540, 542, 544, 546, 548, 550, 552, 554, 556, 558, 560, 562, 564, 566, 568, 570, 573, 575, 578, 580, 582, 584, 588, 591, 593, 597, 599, 601, 604, 606, 608, 611, 613, 615, 619, 621, 623, 625, 627, 629, 631, 633, 635, 637, 639, 641, 644, 646, 649, 651, 653, 655, 657, 659, 661, 663, 665, 667, 670, 672, 674, 676, 678, 681, 684, 686, 690, 692, 694, 696, 698, 700, 702, 704, 706, 708, 710, 712, 714, 716, 718, 721, 723, 725, 727, 729, 731, 733, 735, 738, 740, 742, 744, 746, 748, 750, 752, 754, 756, 758, 760, 762, 764, 766, 768, 772, 774, 776, 777, 779, 782, 784, 786, 789, 791, 793, 796, 798, 800, 803, 806, 808, 810, 812, 815, 817, 819, 823, 621, 827, 830, 832, 834, 837, 839, 841, 844, 846, 848, and 850, respectively. Also, among the variants of polynucleotides consisting of a nucleotide sequence represented by any of SEQ ID NOs: 1, 2, 3, 4, 5, 6, 7, 8, 9, 11, 12, 13, 14, 16, 17, 18, 19, 20, 21, 22, 24, 25, 26, 27, 29, 30, 31, 32, 33, 34, 35, 36, 37, 38, 41, 42, 43, 44, 45, 46, 47, 48, 49, 50, 51, 52, 53, 54, 55, 56, 57, 58, 59, 60, 61, 63, 64, 65, 66, 67, 68, 69, 70, 71, 72, 73, 74, 75, 76, 77, 78, 79, 80, 81, 82, 83, 84, 85, 86, 87, 88, 89, 90, 92, 93, 94, 95, 97, 99, 100, 101, 104, 106, 107, 108, 109, 110, 111, 112, 113, 115, 116, 117, 118, 119, 120, 122, 123, 124, 125, 126, 127, 128, 129, 131, 132, 133, 134, 135, 136, 137, 140, 141, 142, 143, 144, 145, 146, 147, 148, 150, 151, 152, 153, 154, 155, 156, 157, 158, 159, 160, 161, 162, 163, 164, 165, 166, 167, 168, 169, 170, 171, 172, 173, 174, 176, 177, 178, 179, 180, 181, 182, 183, 184, 185, 186, 187, 188, 191, 192, 193, 194, 195, 196, 197, 198, 199, 200, 201, 202, 203, 204, 206, 207, 208, 209, 210, 211, 212, 213, 214, 215, 216, 217, 218, 219, 220, 221, 222, 223, 224, 225, 226, 227, 228, 229, 230, 231, 232, 233, 234, and 235 or a nucleotide sequence derived from the nucleotide sequence by the replacement of u with t according to the present invention, examples of shortest variants registered in the miRBase Release 22 include polynucleotides having sequences represented by SEQ ID NOs: 467, 469, 471, 473, 474, 476, 478, 480, 482, 484, 485, 488, 490, 492, 494, 496, 498, 500, 502, 503, 505, 507, 509, 510, 512, 513, 515, 517, 519, 521, 523, 525, 526, 529, 531, 533, 535, 537, 539, 541, 543, 545, 547, 548, 550, 552, 555, 557, 559, 561, 563, 565, 567, 569, 571, 572, 574, 576, 577, 579, 581, 583, 585, 586, 587, 589, 590, 591, 594, 595, 596, 598, 600, 602, 603, 605, 607, 609, 610, 612, 614, 616, 617, 618, 620, 622, 624, 626, 628, 630, 631, 634, 636, 638, 640, 642, 643, 645, 647, 648, 650, 652, 654, 656, 658, 660, 662, 664, 666, 668, 669, 670, 672, 675, 677, 679, 680, 682, 683, 685, 687, 688, 689, 691, 693, 695, 697, 699, 701, 702, 704, 707, 709, 711, 713, 714, 717, 719, 720, 722, 724, 726, 728, 730, 732, 734, 736, 737, 738, 741, 743, 744, 747, 749, 750, 753, 755, 757, 759, 760, 762, 764, 767, 769, 770, 771, 773, 775, 778, 780, 781, 783, 785, 787, 788, 790, 792, 794, 795, 797, 799, 801, 802, 804, 805, 807, 809, 811, 813, 814, 816, 818, 820, 821, 822, 824, 826, 828, 829, 831, 833, 835, 836, 838, 840, 842, 843, 845, 847, 849, 851, and 852, respectively. In addition to these variants and fragments, examples thereof include a large number of isomiR polynucleotides of SEQ ID NOs: 1 to 235 registered in the miRBase. Examples of the polynucleotide comprising a nucleotide sequence represented by any of SEQ ID NOs: 1 to 235 include a polynucleotide represented by any of SEQ ID NOs: 236 to 465, which are their respective precursors.

The names and miRBase Accession Nos. (registration numbers) of the genes represented by SEQ ID NOs: 1 to 852 are shown in Table 1.

As used herein, the term "capable of specifically binding" means that the nucleic acid probe or the primer used in the present invention binds to a particular target nucleic acid and cannot substantially bind to other nucleic acids.

[Table 1]

| SEQ ID NO. | Name of gene | Accession No. of miRBase (Accession No.) |
|---|---|---|
| 1 | hsa-miR-1908-5p | MIMAT0007881 |
| 2 | hsa-miR-4723-5p | MIMAT0019838 |
| 3 | hsa-miR-4674 | MIMAT0019756 |
| 4 | hsa-miR-939-5p | MIMAT0004982 |
| 5 | hsa-miR-6789-5p | MIMAT0027478 |
| 6 | hsa-miR-1268a | MIMAT0005922 |
| 7 | hsa-miR-1202 | MIMAT0005865 |
| 8 | hsa-miR-4525 | MIMAT0019064 |
| 9 | hsa-miR-128-1-5p | MIMAT0026477 |
| 10 | hsa-mi R-6806-5p | MIMAT0027512 |
| 11 | hsa-miR-7845-5p | MIMAT0030420 |
| 12 | hsa-miR-4632-5p | MIMAT0022977 |
| 13 | hsa-miR-10396b-5p | MIMAT0041635 |

(continued)

| SEQ ID NO. | Name of gene | Accession No. of miRBase (Accession No.) |
|---|---|---|
| 14 | hsa-miR-6768-5p | MIMAT0027436 |
| 15 | hsa-miR-8059 | MIMAT0030986 |
| 16 | hsa-miR-8072 | MIMAT0030999 |
| 17 | hsa-miR-9901 | MIMAT0039321 |
| 18 | hsa-miR-1231 | MIMAT0005586 |
| 19 | hsa-miR-1225-5p | MIMAT0005572 |
| 20 | hsa-miR-12114 | MIMAT0049008 |
| 21 | hsa-miR-3178 | MIMAT0015055 |
| 22 | hsa-miR-6798-5p | MIMAT0027496 |
| 23 | hsa-miR-4276 | MIMAT0016904 |
| 24 | hsa-miR-6125 | MIMAT0024598 |
| 25 | hsa-miR-3652 | MIMAT0018072 |
| 26 | hsa-miR-7111-5p | MIMAT0028119 |
| 27 | hsa-miR-6749-5p | MIMAT0027398 |
| 28 | hsa-miR-1199-5p | MIMAT0031119 |
| 29 | hsa-miR-6802-5p | MIMAT0027504 |
| 30 | hsa-miR-6816-5p | MIMAT0027532 |
| 31 | hsa-miR-4706 | MIMAT0019806 |
| 32 | hsa-miR-5008-5p | MIMAT0021039 |
| 33 | hsa-miR-6797-5p | MIMAT0027494 |
| 34 | hsa-miR-4516 | MIMAT0019053 |
| 35 | hsa-miR-4508 | MIMAT0019045 |
| 36 | hsa-miR-6729-5p | MIMAT0027359 |
| 37 | hsa-miR-564 | MIMAT0003228 |
| 38 | hsa-miR-1233-5p | MIMAT0022943 |
| 39 | hsa-miR-6127 | MIMAT0024610 |
| 40 | hsa-miR-1469 | MIMAT0007347 |
| 41 | hsa-miR-6738-5p | MIMAT0027377 |
| 42 | hsa-miR-6785-5p | MIMAT0027470 |
| 43 | hsa-miR-10401-5p | MIMAT0041633 |
| 44 | hsa-miR-4430 | MIMAT0018945 |
| 45 | hsa-mi R-6889-5p | MIMAT0027678 |
| 46 | hsa-miR-1236-5p | MIMAT0022945 |
| 47 | hsa-miR-3176 | MIMAT0015053 |
| 48 | hsa-miR-3141 | MIMAT0015010 |
| 49 | hsa-miR-3928-3p | MIMAT0018205 |
| 50 | hsa-miR-1237-5p | MIMAT0022946 |
| 51 | hsa-miR-1915-3p | MIMAT0007892 |

(continued)

| SEQ ID NO. | Name of gene | Accession No. of miRBase (Accession No.) |
|---|---|---|
| 52 | hsa-mi R-5195-3p | MIMAT0021127 |
| 53 | hsa-miR-6743-5p | MIMAT0027387 |
| 54 | hsa-miR-6746-5p | MIMAT0027392 |
| 55 | hsa-miR-4446-3p | MIMAT0018965 |
| 56 | hsa-miR-1228-5p | MIMAT0005582 |
| 57 | hsa-miR-1268b | MIMAT0018925 |
| 58 | hsa-miR-1260a | MIMAT0005911 |
| 59 | hsa-miR-6879-3p | MIMAT0027659 |
| 60 | hsa-miR-149-3p | MIMAT0004609 |
| 61 | hsa-miR-3162-5p | MIMAT0015036 |
| 62 | hsa-miR-1207-5p | MIMAT0005871 |
| 63 | hsa-miR-4747-3p | MIMAT0019883 |
| 64 | hsa-miR-4651 | MIMAT0019715 |
| 65 | hsa-miR-638 | MIMAT0003308 |
| 66 | hsa-miR-4736 | MIMAT0019862 |
| 67 | hsa-miR-6845-5p | MIMAT0027590 |
| 68 | hsa-miR-1343-3p | MIMAT0019776 |
| 69 | hsa-miR-6126 | MIMAT0024599 |
| 70 | hsa-miR-92b-5p | MIMAT0004792 |
| 71 | hsa-miR-6774-5p | MIMAT0027448 |
| 72 | hsa-miR-7847-3p | MIMAT0030422 |
| 73 | hsa-miR-6795-5p | MIMAT0027490 |
| 74 | hsa-miR-7109-5p | MIMAT0028115 |
| 75 | hsa-miR-3197 | MIMAT0015082 |
| 76 | hsa-miR-6824-5p | MIMAT0027548 |
| 77 | hsa-miR-6771-5p | MIMAT0027442 |
| 78 | hsa-miR-11399 | MIMAT0044656 |
| 79 | hsa-miR-2861 | MIMAT0013802 |
| 80 | hsa-mi R-4707-3p | MIMAT0019808 |
| 81 | hsa-miR-4638-5p | MIMAT0019695 |
| 82 | hsa-miR-8073 | MIMAT0031000 |
| 83 | hsa-miR-328-5p | MIMAT0026486 |
| 84 | hsa-miR-665 | MIMAT0004952 |
| 85 | hsa-miR-6778-5p | MIMAT0027456 |
| 86 | hsa-miR-10398-3p | MIMAT0041628 |
| 87 | hsa-miR-5698 | MIMAT0022491 |
| 88 | hsa-miR-6794-5p | MIMAT0027488 |
| 89 | hsa-miR-1247-3p | MIMAT0022721 |

(continued)

| SEQ ID NO. | Name of gene | Accession No. of miRBase (Accession No.) |
|---|---|---|
| 90 | hsa-miR-4697-5p | MIMAT0019791 |
| 91 | hsa-miR-8069 | MIMAT0030996 |
| 92 | hsa-miR-572 | MIMAT0003237 |
| 93 | hsa-miR-6751-5p | MIMAT0027402 |
| 94 | hsa-miR-3180-3p | MIMAT0015058 |
| 95 | hsa-miR-486-3p | MIMAT0004762 |
| 96 | hsa-miR-6086 | MIMAT0023711 |
| 97 | hsa-miR-30c-1-3p | MIMAT0004674 |
| 98 | hsa-miR-8063 | MIMAT0030990 |
| 99 | hsa-miR-3621 | MIMAT0018002 |
| 100 | hsa-miR-6887-5p | MIMAT0027674 |
| 101 | hsa-miR-3191-3p | MIMAT0015075 |
| 102 | hsa-miR-11181-3p | MIMAT0043997 |
| 103 | hsa-miR-6722-5p | MIMAT0025853 |
| 104 | hsa-miR-6781-5p | MIMAT0027462 |
| 105 | hsa-miR-5739 | MIMAT0023116 |
| 106 | hsa-miR-3937 | MIMAT0018352 |
| 107 | hsa-miR-1343-5p | MIMAT0027038 |
| 108 | hsa-miR-1181 | MIMAT0005826 |
| 109 | hsa-miR-4725-3p | MIMAT0019844 |
| 110 | hsa-miR-6865-5p | MIMAT0027630 |
| 111 | hsa-miR-375-5p | MIMAT0037313 |
| 112 | hsa-miR-3196 | MIMAT0015080 |
| 113 | hsa-miR-6762-5p | MIMAT0027424 |
| 114 | hsa-miR-4258 | MIMAT0016879 |
| 115 | hsa-miR-5196-5p | MIMAT0021128 |
| 116 | hsa-miR-10401-3p | MIMAT0041634 |
| 117 | hsa-miR-675-5p | MIMAT0004284 |
| 118 | hsa-miR-4488 | MIMAT0019022 |
| 119 | hsa-miR-10527-5p | MIMAT0041997 |
| 120 | hsa-miR-10396a-5p | MIMAT0041623 |
| 121 | hsa-miR-4269 | MIMAT0016897 |
| 122 | hsa-miR-6800-5p | MIMAT0027500 |
| 123 | hsa-miR-6819-5p | MIMAT0027538 |
| 124 | hsa-miR-10396b-3p | MIMAT0041636 |
| 125 | hsa-miR-4688 | MIMAT0019777 |
| 126 | hsa-miR-6786-5p | MIMAT0027472 |
| 127 | hsa-miR-4634 | MIMAT0019691 |

(continued)

| SEQ ID NO. | Name of gene | Accession No. of miRBase (Accession No.) |
| --- | --- | --- |
| 128 | hsa-miR-3940-5p | MIMAT0019229 |
| 129 | hsa-miR-4655-5p | MIMAT0019721 |
| 130 | hsa-miR-7155-5p | MIMAT0028220 |
| 131 | hsa-miR-6769b-5p | MIMAT0027620 |
| 132 | hsa-miR-6810-5p | MIMAT0027520 |
| 133 | hsa-miR-4665-3p | MIMAT0019740 |
| 134 | hsa-miR-6727-5p | MIMAT0027355 |
| 135 | hsa-miR-6803-5p | MIMAT0027506 |
| 136 | hsa-miR-4640-5p | MIMAT0019699 |
| 137 | hsa-miR-6735-5p | MIMAT0027371 |
| 138 | hsa-miR-4535 | MIMAT0019075 |
| 139 | hsa-miR-8089 | MIMAT0031016 |
| 140 | hsa-miR-1292-3p | MIMAT0022948 |
| 141 | hsa-miR-5088-5p | MIMAT0021080 |
| 142 | hsa-miR-3622a-5p | MIMAT0018003 |
| 143 | hsa-miR-6124 | MIMAT0024597 |
| 144 | hsa-miR-6820-5p | MIMAT0027540 |
| 145 | hsa-miR-6805-3p | MIMAT0027511 |
| 146 | hsa-miR-4513 | MIMAT0019050 |
| 147 | hsa-miR-760 | MIMAT0004957 |
| 148 | hsa-miR-4665-5p | MIMAT0019739 |
| 149 | hsa-miR-10400-3p | MIMAT0041632 |
| 150 | hsa-miR-4298 | MIMAT0016852 |
| 151 | hsa-miR-8085 | MIMAT0031012 |
| 152 | hsa-miR-4463 | MIMAT0018987 |
| 153 | hsa-miR-6807-5p | MIMAT0027514 |
| 154 | hsa-miR-4433b-3p | MIMAT0030414 |
| 155 | hsa-miR-3185 | MIMAT0015065 |
| 156 | hsa-miR-12121 | MIMAT0049015 |
| 157 | hsa-miR-671-5p | MIMAT0003880 |
| 158 | hsa-miR-6752-5p | MIMAT0027404 |
| 159 | hsa-miR-371a-5p | MIMAT0004687 |
| 160 | hsa-miR-3917 | MIMAT0018191 |
| 161 | hsa-miR-1224-5p | MIMAT0005458 |
| 162 | hsa-miR-498-5p | MIMAT0002824 |
| 163 | hsa-miR-7704 | MIMAT0030019 |
| 164 | hsa-miR-6741-5p | MIMAT0027383 |
| 165 | hsa-miR-765 | MIMAT0003945 |

(continued)

| SEQ ID NO. | Name of gene | Accession No. of miRBase (Accession No.) |
|---|---|---|
| 166 | hsa-miR-4486 | MIMAT0019020 |
| 167 | hsa-miR-6090 | MIMAT0023715 |
| 168 | hsa-miR-718 | MIMAT0012735 |
| 169 | hsa-miR-4767 | MIMAT0019919 |
| 170 | hsa-miR-6851-5p | MIMAT0027602 |
| 171 | hsa-miR-5572 | MIMAT0022260 |
| 172 | hsa-miR-6850-5p | MIMAT0027600 |
| 173 | hsa-miR-6089 | MIMAT0023714 |
| 174 | hsa-miR-5787 | MIMAT0023252 |
| 175 | hsa-miR-4534 | MIMAT0019073 |
| 176 | hsa-miR-3665 | MIMAT0018087 |
| 177 | hsa-miR-4787-5p | MIMAT0019956 |
| 178 | hsa-miR-6754-5p | MIMAT0027408 |
| 179 | hsa-miR-6825-3p | MIMAT0027551 |
| 180 | hsa-miR-4728-5p | MIMAT0019849 |
| 181 | hsa-miR-6088 | MIMAT0023713 |
| 182 | hsa-miR-3154 | MIMAT0015028 |
| 183 | hsa-miR-6869-5p | MIMAT0027638 |
| 184 | hsa-miR-187-5p | MIMAT0004561 |
| 185 | hsa-miR-6165 | MIMAT0024782 |
| 186 | hsa-miR-4447 | MIMAT0018966 |
| 187 | hsa-miR-4731-5p | MIMAT0019853 |
| 188 | hsa-miR-6805-5p | MIMAT0027510 |
| 189 | hsa-miR-12118 | MIMAT0049012 |
| 190 | hsa-miR-4270 | MIMAT0016900 |
| 191 | hsa-miR-7846-3p | MIMAT0030421 |
| 192 | hsa-miR-4443 | MIMAT0018961 |
| 193 | hsa-miR-6737-5p | MIMAT0027375 |
| 194 | hsa-miR-197-5p | MIMAT0022691 |
| 195 | hsa-miR-1229-5p | MIMAT0022942 |
| 196 | hsa-miR-6757-5p | MIMAT0027414 |
| 197 | hsa-miR-6765-5p | MIMAT0027430 |
| 198 | hsa-miR-4722-5p | MIMAT0019836 |
| 199 | hsa-miR-6891-5p | MIMAT0027682 |
| 200 | hsa-miR-5006-5p | MIMAT0021033 |
| 201 | hsa-miR-345-3p | MIMAT0022698 |
| 202 | hsa-miR-6726-5p | MIMAT0027353 |
| 203 | hsa-miR-3195 | MIMAT0015079 |

(continued)

| SEQ ID NO. | Name of gene | Accession No. of miRBase (Accession No.) |
|---|---|---|
| 204 | hsa-miR-6877-5p | MIMAT0027654 |
| 205 | hsa-miR-4462 | MIMAT0018986 |
| 206 | hsa-miR-6812-5p | MIMAT0027524 |
| 207 | hsa-miR-483-5p | MIMAT0004761 |
| 208 | hsa-miR-9899 | MIMAT0039319 |
| 209 | hsa-miR-4800-5p | MIMAT0019978 |
| 210 | hsa-miR-4734 | MIMAT0019859 |
| 211 | hsa-miR-3135b | MIMAT0018985 |
| 212 | hsa-miR-4433a-3p | MIMAT0018949 |
| 213 | hsa-miR-6769a-5p | MIMAT0027438 |
| 214 | hsa-miR-4743-5p | MIMAT0019874 |
| 215 | hsa-miR-1909-3p | MIMAT0007883 |
| 216 | hsa-miR-4741 | MIMAT0019871 |
| 217 | hsa-miR-4685-5p | MIMAT0019771 |
| 218 | hsa-miR-3147 | MIMAT0015019 |
| 219 | hsa-miR-4726-5p | MIMAT0019845 |
| 220 | hsa-miR-3180 | MIMAT0018178 |
| 221 | hsa-miR-3188 | MIMAT0015070 |
| 222 | hsa-miR-6782-5p | MIMAT0027464 |
| 223 | hsa-miR-6776-5p | MIMAT0027452 |
| 224 | hsa-miR-4484 | MIMAT0019018 |
| 225 | hsa-miR-1185-1-3p | MIMAT0022838 |
| 226 | hsa-miR-6790-3p | MIMAT0027481 |
| 227 | hsa-miR-4466 | MIMAT0018993 |
| 228 | hsa-miR-10394-3p | MIMAT0041620 |
| 229 | hsa-miR-1275 | MIMAT0005929 |
| 230 | hsa-miR-4478 | MIMAT0019006 |
| 231 | hsa-miR-3175 | MIMAT0015052 |
| 232 | hsa-miR-7106-5p | MIMAT0028109 |
| 233 | hsa-miR-4667-5p | MIMAT0019743 |
| 234 | hsa-miR-193b-5p | MIMAT0004767 |
| 235 | hsa-miR-602 | MIMAT0003270 |
| 236 | hsa-mir-1908 | MI0008329 |
| 237 | hsa-mir-4723 | MI0017359 |
| 238 | hsa-mir-4674 | MI0017305 |
| 239 | hsa-mir-939 | MI0005761 |
| 240 | hsa-mir-6789 | MI0022634 |
| 241 | hsa-mir-1268a | MI0006405 |

(continued)

| SEQ ID NO. | Name of gene | Accession No. of miRBase (Accession No.) |
|---|---|---|
| 242 | hsa-mir-1202 | MI0006334 |
| 243 | hsa-mir-4525 | MI0016892 |
| 244 | hsa-mir-128-1 | MI0000447 |
| 245 | hsa-mir-6806 | MI0022651 |
| 246 | hsa-mir-7845 | MI0025515 |
| 247 | hsa-mir-4632 | MI0017259 |
| 248 | hsa-mir-10396b | MI0033426 |
| 249 | hsa-mir-6768 | MI0022613 |
| 250 | hsa-mir-8059 | MI0025895 |
| 251 | hsa-mir-8072 | MI0025908 |
| 252 | hsa-mir-9901 | MI0031829 |
| 253 | hsa-mir-1231 | MI0006321 |
| 254 | hsa-mir-1225 | MI0006311 |
| 255 | hsa-mir-12114 | MI0039716 |
| 256 | hsa-mir-3178 | MI0014212 |
| 257 | hsa-mir-6798 | MI0022643 |
| 258 | hsa-mir-4276 | MI0015882 |
| 259 | hsa-mir-6125 | MI0021259 |
| 260 | hsa-mir-3652 | MI0016052 |
| 261 | hsa-mir-7111 | MI0022962 |
| 262 | hsa-mir-6749 | MI0022594 |
| 263 | hsa-mir-1199 | MI0020340 |
| 264 | hsa-mir-6802 | MI0022647 |
| 265 | hsa-mir-6816 | MI0022661 |
| 266 | hsa-mir-4706 | MI0017339 |
| 267 | hsa-mir-5008 | MI0017876 |
| 268 | hsa-mir-6797 | MI0022642 |
| 269 | hsa-mir-4516 | MI0016882 |
| 270 | hsa-mir-4508 | MI0016872 |
| 271 | hsa-mir-6729 | MI0022574 |
| 272 | hsa-mir-564 | MI0003570 |
| 273 | hsa-mir-1233-1 | MI0006323 |
| 274 | hsa-mir-6127 | MI0021271 |
| 275 | hsa-mir-1469 | MI0007074 |
| 276 | hsa-mir-6738 | MI0022583 |
| 277 | hsa-mir-6785 | MI0022630 |
| 278 | hsa-mir-10401 | MI0033425 |
| 279 | hsa-mir-4430 | MI0016769 |

(continued)

| SEQ ID NO. | Name of gene | Accession No. of miRBase (Accession No.) |
|---|---|---|
| 280 | hsa-mir-6889 | MI0022736 |
| 281 | hsa-mir-1236 | MI0006326 |
| 282 | hsa-mir-3176 | MI0014210 |
| 283 | hsa-mir-3141 | MI0014165 |
| 284 | hsa-mir-3928 | MI0016438 |
| 285 | hsa-mir-1237 | MI0006327 |
| 286 | hsa-mir-1915 | MI0008336 |
| 287 | hsa-mir-5195 | MI0018174 |
| 288 | hsa-mir-6743 | MI0022588 |
| 289 | hsa-mir-6746 | MI0022591 |
| 290 | hsa-mir-4446 | MI0016789 |
| 291 | hsa-mir-1228 | MI0006318 |
| 292 | hsa-mir-1268b | MI0016748 |
| 293 | hsa-mir-1260a | MI0006394 |
| 294 | hsa-mir-6879 | MI0022726 |
| 295 | hsa-mir-149 | MI0000478 |
| 296 | hsa-mir-3162 | MI0014192 |
| 297 | hsa-mir-1207 | MI0006340 |
| 298 | hsa-mir-4747 | MI0017386 |
| 299 | hsa-mir-4651 | MI0017279 |
| 300 | hsa-mir-638 | MI0003653 |
| 301 | hsa-mir-4736 | MI0017373 |
| 302 | hsa-mir-6845 | MI0022691 |
| 303 | hsa-mir-1343 | MI0017320 |
| 304 | hsa-mir-6126 | MI0021260 |
| 305 | hsa-mir-92b | MI0003560 |
| 306 | hsa-mir-6774 | MI0022619 |
| 307 | hsa-mir-7847 | MI0025517 |
| 308 | hsa-mir-6795 | MI0022640 |
| 309 | hsa-mir-7109 | MI0022960 |
| 310 | hsa-mir-3197 | MI0014245 |
| 311 | hsa-mir-6824 | MI0022669 |
| 312 | hsa-mir-6771 | MI0022616 |
| 313 | hsa-mir-11399 | MI0036558 |
| 314 | hsa-mir-2861 | MI0013006 |
| 315 | hsa-mir-4707 | MI0017340 |
| 316 | hsa-mir-4638 | MI0017265 |
| 317 | hsa-mir-8073 | MI0025909 |

(continued)

| SEQ ID NO. | Name of gene | Accession No. of miRBase (Accession No.) |
|---|---|---|
| 318 | hsa-mir-328 | MI0000804 |
| 319 | hsa-mir-665 | MI0005563 |
| 320 | hsa-mir-6778 | MI0022623 |
| 321 | hsa-mir-10398 | MI0033422 |
| 322 | hsa-mir-5698 | MI0019305 |
| 323 | hsa-mir-6794 | MI0022639 |
| 324 | hsa-mir-1247 | MI0006382 |
| 325 | hsa-mir-4697 | MI0017330 |
| 326 | hsa-mir-8069-1 | MI0025905 |
| 327 | hsa-mir-572 | MI0003579 |
| 328 | hsa-mir-6751 | MI0022596 |
| 329 | hsa-mir-3180-1 | MI0014214 |
| 330 | hsa-mir-486-1 | MI0002470 |
| 331 | hsa-mir-6086 | MI0020363 |
| 332 | hsa-mir-30c-1 | MI0000736 |
| 333 | hsa-mir-8063 | MI0025899 |
| 334 | hsa-mir-3621 | MI0016012 |
| 335 | hsa-mir-6887 | MI0022734 |
| 336 | hsa-mir-3191 | MI0014236 |
| 337 | hsa-mir-11181 | MI0035972 |
| 338 | hsa-mir-6722 | MI0022557 |
| 339 | hsa-mir-6781 | MI0022626 |
| 340 | hsa-mir-5739 | MI0019412 |
| 341 | hsa-mir-3937 | MI0016593 |
| 342 | hsa-mir-1181 | MI0006274 |
| 343 | hsa-mir-4725 | MI0017362 |
| 344 | hsa-mir-6865 | MI0022712 |
| 345 | hsa-mir-375 | MI0000783 |
| 346 | hsa-mir-3196 | MI0014241 |
| 347 | hsa-mir-6762 | MI0022607 |
| 348 | hsa-mir-4258 | MI0015857 |
| 349 | hsa-mir-5196 | MI0018175 |
| 350 | hsa-mir-675 | MI0005416 |
| 351 | hsa-mir-4488 | MI0016849 |
| 352 | hsa-mir-10527 | MI0033674 |
| 353 | hsa-mir-10396a | MI0033420 |
| 354 | hsa-mir-4269 | MI0015875 |
| 355 | hsa-mir-6800 | MI0022645 |

(continued)

| SEQ ID NO. | Name of gene | Accession No. of miRBase (Accession No.) |
|---|---|---|
| 356 | hsa-mir-6819 | MI0022664 |
| 357 | hsa-mir-4688 | MI0017321 |
| 358 | hsa-mir-6786 | MI0022631 |
| 359 | hsa-mir-4634 | MI0017261 |
| 360 | hsa-mir-3940 | MI0016597 |
| 361 | hsa-mir-4655 | MI0017283 |
| 362 | hsa-mir-7155 | MI0023615 |
| 363 | hsa-mir-6769b | MI0022706 |
| 364 | hsa-mir-6810 | MI0022655 |
| 365 | hsa-mir-4665 | MI0017295 |
| 366 | hsa-mir-6727 | MI0022572 |
| 367 | hsa-mir-6803 | MI0022648 |
| 368 | hsa-mir-4640 | MI0017267 |
| 369 | hsa-mir-6735 | MI0022580 |
| 370 | hsa-mir-4535 | MI0016903 |
| 371 | hsa-mir-8089 | MI0025925 |
| 372 | hsa-mir-1292 | MI0006433 |
| 373 | hsa-mir-5088 | MI0017977 |
| 374 | hsa-mir-3622a | MI0016013 |
| 375 | hsa-mir-6124 | MI0021258 |
| 376 | hsa-mir-6820 | MI0022665 |
| 377 | hsa-mir-6805 | MI0022650 |
| 378 | hsa-mir-4513 | MI0016879 |
| 379 | hsa-mir-760 | MI0005567 |
| 380 | hsa-mir-10400 | MI0033424 |
| 381 | hsa-mir-4298 | MI0015830 |
| 382 | hsa-mir-8085 | MI0025921 |
| 383 | hsa-mir-4463 | MI0016811 |
| 384 | hsa-mir-6807 | MI0022652 |
| 385 | hsa-mir-4433b | MI0025511 |
| 386 | hsa-mir-3185 | MI0014227 |
| 387 | hsa-mir-12121 | MI0039723 |
| 388 | hsa-mir-671 | MI0003760 |
| 389 | hsa-mir-6752 | MI0022597 |
| 390 | hsa-mir-371a | MI0000779 |
| 391 | hsa-mir-3917 | MI0016423 |
| 392 | hsa-mir-1224 | MI0003764 |
| 393 | hsa-mir-498 | MI0003142 |

(continued)

| SEQ ID NO. | Name of gene | Accession No. of miRBase (Accession No.) |
|---|---|---|
| 394 | hsa-mir-7704 | MI0025240 |
| 395 | hsa-mir-6741 | MI0022586 |
| 396 | hsa-mir-765 | MI0005116 |
| 397 | hsa-mir-4486 | MI0016847 |
| 398 | hsa-mir-6090 | MI0020367 |
| 399 | hsa-mir-718 | MI0012489 |
| 400 | hsa-mir-4767 | MI0017408 |
| 401 | hsa-mir-6851 | MI0022697 |
| 402 | hsa-mir-5572 | MI0019117 |
| 403 | hsa-mir-6850 | MI0022696 |
| 404 | hsa-mir-6089-1 | MI0020366 |
| 405 | hsa-mir-5787 | MI0019797 |
| 406 | hsa-mir-4534 | MI0016901 |
| 407 | hsa-mir-3665 | MI0016066 |
| 408 | hsa-mir-4787 | MI0017434 |
| 409 | hsa-mir-6754 | MI0022599 |
| 410 | hsa-mir-6825 | MI0022670 |
| 411 | hsa-mir-4728 | MI0017365 |
| 412 | hsa-mir-6088 | MI0020365 |
| 413 | hsa-mir-3154 | MI0014182 |
| 414 | hsa-mir-6869 | MI0022716 |
| 415 | hsa-mir-187 | MI0000274 |
| 416 | hsa-mir-6165 | MI0021472 |
| 417 | hsa-mir-4447 | MI0016790 |
| 418 | hsa-mir-4731 | MI0017368 |
| 419 | hsa-mir-12118 | MI0039720 |
| 420 | hsa-mir-4270 | MI0015878 |
| 421 | hsa-mir-7846 | MI0025516 |
| 422 | hsa-mir-4443 | MI0016786 |
| 423 | hsa-mir-6737 | MI0022582 |
| 424 | hsa-mir-197 | MI0000239 |
| 425 | hsa-mir-1229 | MI0006319 |
| 426 | hsa-mir-6757 | MI0022602 |
| 427 | hsa-mir-6765 | MI0022610 |
| 428 | hsa-mir-4722 | MI0017357 |
| 429 | hsa-mir-6891 | MI0022738 |
| 430 | hsa-mir-5006 | MI0017873 |
| 431 | hsa-mir-345 | MI0000825 |

(continued)

| SEQ ID NO. | Name of gene | Accession No. of miRBase (Accession No.) |
|---|---|---|
| 432 | hsa-mir-6726 | MI0022571 |
| 433 | hsa-mir-3195 | MI0014240 |
| 434 | hsa-mir-6877 | MI0022724 |
| 435 | hsa-mir-4462 | MI0016810 |
| 436 | hsa-mir-6812 | MI0022657 |
| 437 | hsa-mir-483 | MI0002467 |
| 438 | hsa-mir-9899 | MI0031827 |
| 439 | hsa-mir-4800 | MI0017448 |
| 440 | hsa-mir-4734 | MI0017371 |
| 441 | hsa-mir-3135b | MI0016809 |
| 442 | hsa-mir-4433a | MI0016773 |
| 443 | hsa-mir-6769a | MI0022614 |
| 444 | hsa-mir-4743 | MI0017381 |
| 445 | hsa-mir-1909 | MI0008330 |
| 446 | hsa-mir-4741 | MI0017379 |
| 447 | hsa-mir-4685 | MI0017317 |
| 448 | hsa-mir-3147 | MI0014173 |
| 449 | hsa-mir-4726 | MI0017363 |
| 450 | hsa-mir-3180-4 | MI0016408 |
| 451 | hsa-mir-3188 | MI0014232 |
| 452 | hsa-mir-6782 | MI0022627 |
| 453 | hsa-mir-6776 | MI0022621 |
| 454 | hsa-mir-4484 | MI0016845 |
| 455 | hsa-mir-1185-1 | MI0003844 |
| 456 | hsa-mir-6790 | MI0022635 |
| 457 | hsa-mir-4466 | MI0016817 |
| 458 | hsa-mir-10394 | MI0033418 |
| 459 | hsa-mir-1275 | MI0006415 |
| 460 | hsa-mir-4478 | MI0016831 |
| 461 | hsa-mir-3175 | MI0014209 |
| 462 | hsa-mir-7106 | MI0022957 |
| 463 | hsa-mir-4667 | MI0017297 |
| 464 | hsa-mir-193b | MI0003137 |
| 465 | hsa-mir-602 | MI0003615 |
| 466 | isomiR Example 1 of SEQ ID NO: 1 | - |
| 467 | isomiR Example 2 of SEQ ID NO: 1 | - |
| 468 | isomiR Example 1 of SEQ ID NO: 2 | - |
| 469 | isomiR Example 2 of SEQ ID NO: 2 | - |

(continued)

| SEQ ID NO. | Name of gene | Accession No. of miRBase (Accession No.) |
|---|---|---|
| 470 | isomiR Example 1 of SEQ ID NO: 3 | - |
| 471 | isomiR Example 2 of SEQ ID NO: 3 | - |
| 472 | isomiR Example 1 of SEQ ID NO: 4 | - |
| 473 | isomiR Example 2 of SEQ ID NO: 4 | - |
| 474 | isomiR Example 1 of SEQ ID NO: 5 | - |
| 475 | isomiR Example 1 of SEQ ID NO: 6 | - |
| 476 | isomiR Example 2 of SEQ ID NO: 6 | - |
| 477 | isomiR Example 1 of SEQ ID NO: 7 | - |
| 478 | isomiR Example 2 of SEQ ID NO: 7 | - |
| 479 | isomiR Example 1 of SEQ ID NO: 8 | - |
| 480 | isomiR Example 2 of SEQ ID NO: 8 | - |
| 481 | isomiR Example 1 of SEQ ID NO: 9 | - |
| 482 | isomiR Example 2 of SEQ ID NO: 9 | - |
| 483 | isomiR Example 1 of SEQ ID NO: 11 | - |
| 484 | isomiR Example 2 of SEQ ID NO: 11 | - |
| 485 | isomiR Example 1 of SEQ ID NO: 12 | - |
| 486 | isomiR Example 2 of SEQ ID NO: 12 | - |
| 487 | isomiR Example 1 of SEQ ID NO: 13 | - |
| 488 | isomiR Example 2 of SEQ ID NO: 13 | - |
| 489 | isomiR Example 1 of SEQ ID NO: 14 | - |
| 490 | isomiR Example 2 of SEQ ID NO: 14 | - |
| 491 | isomiR Example 1 of SEQ ID NO: 16 | - |
| 492 | isomiR Example 2 of SEQ ID NO: 16 | - |
| 493 | isomiR Example 1 of SEQ ID NO: 17 | - |
| 494 | isomiR Example 2 of SEQ ID NO: 17 | - |
| 495 | isomiR Example 1 of SEQ ID NO: 18 | - |
| 496 | isomiR Example 2 of SEQ ID NO: 18 | - |
| 497 | isomiR Example 1 of SEQ ID NO: 19 | - |
| 498 | isomiR Example 2 of SEQ ID NO: 19 | - |
| 499 | isomiR Example 1 of SEQ ID NO: 20 | - |
| 500 | isomiR Example 2 of SEQ ID NO: 20 | - |
| 501 | isomiR Example 1 of SEQ ID NO: 21 | - |
| 502 | isomiR Example 2 of SEQ ID NO: 21 | - |
| 503 | isomiR Example 1 of SEQ ID NO: 22 | - |
| 504 | isomiR Example 1 of SEQ ID NO: 24 | - |
| 505 | isomiR Example 2 of SEQ ID NO: 24 | - |
| 506 | isomiR Example 1 of SEQ ID NO: 25 | - |
| 507 | isomiR Example 2 of SEQ ID NO: 25 | - |

(continued)

| SEQ ID NO. | Name of gene | Accession No. of miRBase (Accession No.) |
|---|---|---|
| 508 | isomiR Example 1 of SEQ ID NO: 26 | - |
| 509 | isomiR Example 2 of SEQ ID NO: 26 | - |
| 510 | isomiR Example 1 of SEQ ID NO: 27 | - |
| 511 | isomiR Example 1 of SEQ ID NO: 29 | - |
| 512 | isomiR Example 2 of SEQ ID NO: 29 | - |
| 513 | isomiR Example 1 of SEQ ID NO: 30 | - |
| 514 | isomiR Example 1 of SEQ ID NO: 31 | - |
| 515 | isomiR Example 2 of SEQ ID NO: 31 | - |
| 516 | isomiR Example 1 of SEQ ID NO: 32 | - |
| 517 | isomiR Example 2 of SEQ ID NO: 32 | - |
| 518 | isomiR Example 1 of SEQ ID NO: 33 | - |
| 519 | isomiR Example 2 of SEQ ID NO: 33 | - |
| 520 | isomiR Example 1 of SEQ ID NO: 34 | - |
| 521 | isomiR Example 2 of SEQ ID NO: 34 | - |
| 522 | isomiR Example 1 of SEQ ID NO: 35 | - |
| 523 | isomiR Example 2 of SEQ ID NO: 35 | - |
| 524 | isomiR Example 1 of SEQ ID NO: 36 | - |
| 525 | isomiR Example 2 of SEQ ID NO: 36 | - |
| 526 | isomiR Example 1 of SEQ ID NO: 37 | - |
| 527 | isomiR Example 2 of SEQ ID NO: 37 | - |
| 528 | isomiR Example 1 of SEQ ID NO: 38 | - |
| 529 | isomiR Example 2 of SEQ ID NO: 38 | - |
| 530 | isomiR Example 1 of SEQ ID NO: 41 | - |
| 531 | isomiR Example 2 of SEQ ID NO: 41 | - |
| 532 | isomiR Example 1 of SEQ ID NO: 42 | - |
| 533 | isomiR Example 2 of SEQ ID NO: 42 | - |
| 534 | isomiR Example 1 of SEQ ID NO: 43 | - |
| 535 | isomiR Example 2 of SEQ ID NO: 43 | - |
| 536 | isomiR Example 1 of SEQ ID NO: 44 | - |
| 537 | isomiR Example 2 of SEQ ID NO: 44 | - |
| 538 | isomiR Example 1 of SEQ ID NO: 45 | - |
| 539 | isomiR Example 2 of SEQ ID NO: 45 | - |
| 540 | isomiR Example 1 of SEQ ID NO: 46 | - |
| 541 | isomiR Example 2 of SEQ ID NO: 46 | - |
| 542 | isomiR Example 1 of SEQ ID NO: 47 | - |
| 543 | isomiR Example 2 of SEQ ID NO: 47 | - |
| 544 | isomiR Example 1 of SEQ ID NO: 48 | - |
| 545 | isomiR Example 2 of SEQ ID NO: 48 | - |

(continued)

| SEQ ID NO. | Name of gene | Accession No. of miRBase (Accession No.) |
|---|---|---|
| 546 | isomiR Example 1 of SEQ ID NO: 49 | - |
| 547 | isomiR Example 2 of SEQ ID NO: 49 | - |
| 548 | isomiR Example 1 SEQ ID NO: 50 | - |
| 549 | isomiR Example 2 of SEQ ID NO: 50 | - |
| 550 | isomiR Example 1 of SEQ ID NO: 51 | - |
| 551 | isomiR Example 2 of SEQ ID NO: 51 | - |
| 552 | isomiR Example 1 of SEQ ID NO: 52 | - |
| 553 | isomiR Example 2 of SEQ ID NO: 52 | - |
| 554 | isomiR Example 1 of SEQ ID NO: 53 | - |
| 555 | isomiR Example 2 of SEQ ID NO: 53 | - |
| 556 | isomiR Example 1 of SEQ ID NO: 54 | - |
| 557 | isomiR Example 2 of SEQ ID NO: 54 | - |
| 558 | isomiR Example 1 of SEQ ID NO: 55 | - |
| 559 | isomiR Example 2 of SEQ ID NO: 55 | - |
| 560 | isomiR Example 1 of SEQ ID NO: 56 | - |
| 561 | isomiR Example 2 of SEQ ID NO: 56 | - |
| 562 | isomiR Example 1 of SEQ ID NO: 57 | - |
| 563 | isomiR Example 2 of SEQ ID NO: 57 | - |
| 564 | isomiR Example 1 of SEQ ID NO: 58 | - |
| 565 | isomiR Example 2 of SEQ ID NO: 58 | - |
| 566 | isomiR Example 1 of SEQ ID NO: 59 | - |
| 567 | isomiR Example 2 of SEQ ID NO: 59 | - |
| 568 | isomiR Example 1 of SEQ ID NO: 60 | - |
| 569 | isomiR Example 2 of SEQ ID NO: 60 | - |
| 570 | isomiR Example 1 of SEQ ID NO: 61 | - |
| 571 | isomiR Example 2 of SEQ ID NO: 61 | - |
| 572 | isomiR Example 1 of SEQ ID NO: 63 | - |
| 573 | isomiR Example 1 of SEQ ID NO: 64 | - |
| 574 | isomiR Example 2 of SEQ ID NO: 64 | - |
| 575 | isomiR Example 1 of SEQ ID NO: 65 | - |
| 576 | isomiR Example 2 of SEQ ID NO: 65 | - |
| 577 | isomiR Example 1 of SEQ ID NO: 66 | - |
| 578 | isomiR Example 1 of SEQ ID NO: 67 | - |
| 579 | isomiR Example 2 of SEQ ID NO: 67 | - |
| 580 | isomiR Example 1 of SEQ ID NO: 68 | - |
| 581 | isomiR Example 2 of SEQ ID NO: 68 | - |
| 582 | isomiR Example 1 of SEQ ID NO: 69 | - |
| 583 | isomiR Example 2 of SEQ ID NO: 69 | - |

(continued)

| SEQ ID NO. | Name of gene | Accession No. of miRBase (Accession No.) |
|---|---|---|
| 584 | isomiR Example 1 of SEQ ID NO: 70 | - |
| 585 | isomiR Example 2 of SEQ ID NO: 70 | - |
| 586 | isomiR Example 1 of SEQ ID NO: 71 | - |
| 587 | isomiR Example 1 of SEQ ID NO: 72 | - |
| 588 | isomiR Example 1 of SEQ ID NO: 73 | - |
| 589 | isomiR Example 2 of SEQ ID NO: 73 | - |
| 590 | isomiR Example 1 of SEQ ID NO: 74 | - |
| 591 | isomiR Example 1 of SEQ ID NO: 75 | - |
| 592 | isomiR Example 2 of SEQ ID NO: 75 | - |
| 593 | isomiR Example 1 of SEQ ID NO: 76 | - |
| 594 | isomiR Example 2 of SEQ ID NO: 76 | - |
| 595 | isomiR Example 1 of SEQ ID NO: 77 | - |
| 596 | isomiR Example 1 of SEQ ID NO: 78 | - |
| 597 | isomiR Example 1 of SEQ ID NO: 79 | - |
| 598 | isomiR Example 2 of SEQ ID NO: 79 | - |
| 599 | isomiR Example 1 of SEQ ID NO: 80 | - |
| 600 | isomiR Example 2 of SEQ ID NO: 80 | - |
| 601 | isomiR Example 1 of SEQ ID NO: 81 | - |
| 602 | isomiR Example 2 of SEQ ID NO: 81 | - |
| 603 | isomiR Example 1 of SEQ ID NO: 82 | - |
| 604 | isomiR Example 1 of SEQ ID NO: 83 | - |
| 605 | isomiR Example 2 of SEQ ID NO: 83 | - |
| 606 | isomiR Example 1 of SEQ ID NO: 84 | - |
| 607 | isomiR Example 2 of SEQ ID NO: 84 | - |
| 608 | isomiR Example 1 of SEQ ID NO: 85 | - |
| 609 | isomiR Example 2 of SEQ ID NO: 85 | - |
| 610 | isomiR Example 1 of SEQ ID NO: 86 | - |
| 611 | isomiR Example 1 of SEQ ID NO: 87 | - |
| 612 | isomiR Example 2 of SEQ ID NO: 87 | - |
| 613 | isomiR Example 1 of SEQ ID NO: 88 | - |
| 614 | isomiR Example 2 of SEQ ID NO: 88 | - |
| 615 | isomiR Example 1 of SEQ ID NO: 89 | - |
| 616 | isomiR Example 2 of SEQ ID NO: 89 | - |
| 617 | isomiR Example 1 of SEQ ID NO: 90 | - |
| 618 | isomiR Example 1 of SEQ ID NO: 92 | - |
| 619 | isomiR Example 1 of SEQ ID NO: 93 | - |
| 620 | isomiR Example 2 of SEQ ID NO: 93 | - |
| 621 | isomiR Example 1 of SEQ ID NO: 94 | - |

(continued)

| SEQ ID NO. | Name of gene | Accession No. of miRBase (Accession No.) |
|---|---|---|
| 622 | isomiR Example 2 of SEQ ID NO: 94 | - |
| 623 | isomiR Example 1 of SEQ ID NO: 95 | - |
| 624 | isomiR Example 2 of SEQ ID NO: 95 | - |
| 625 | isomiR Example 1 of SEQ ID NO: 97 | - |
| 626 | isomiR Example 2 of SEQ ID NO: 97 | - |
| 627 | isomiR Example 1 of SEQ ID NO: 98 | - |
| 628 | isomiR Example 1 of SEQ ID NO: 99 | - |
| 629 | isomiR Example 1 of SEQ ID NO: 100 | - |
| 630 | isomiR Example 2 of SEQ ID NO: 100 | - |
| 631 | isomiR Example 1 of SEQ ID NO: 101 | - |
| 632 | isomiR Example 2 of SEQ ID NO: 101 | - |
| 633 | isomiR Example 1 of SEQ ID NO: 104 | - |
| 634 | isomiR Example 2 of SEQ ID NO: 104 | - |
| 635 | isomiR Example 1 of SEQ ID NO: 106 | - |
| 636 | isomiR Example 2 of SEQ ID NO: 106 | - |
| 637 | isomiR Example 1 of SEQ ID NO: 107 | - |
| 638 | isomiR Example 2 of SEQ ID NO: 107 | - |
| 639 | isomiR Example 1 of SEQ ID NO: 108 | - |
| 640 | isomiR Example 2 of SEQ ID NO: 108 | - |
| 641 | isomiR Example 1 of SEQ ID NO: 109 | - |
| 642 | isomiR Example 2 of SEQ ID NO: 109 | - |
| 643 | isomiR Example 1 of SEQ ID NO: 110 | - |
| 644 | isomiR Example 1 of SEQ ID NO: 111 | - |
| 645 | isomiR Example 2 of SEQ ID NO: 111 | - |
| 646 | isomiR Example 1 of SEQ ID NO: 112 | - |
| 647 | isomiR Example 2 of SEQ ID NO: 112 | - |
| 648 | isomiR Example 1 of SEQ ID NO: 113 | - |
| 649 | isomiR Example 1 of SEQ ID NO: 115 | - |
| 650 | isomiR Example 2 of SEQ ID NO: 115 | - |
| 651 | isomiR Example 1 of SEQ ID NO: 116 | - |
| 652 | isomiR Example 2 of SEQ ID NO: 116 | - |
| 653 | isomiR Example 1 of SEQ ID NO: 117 | - |
| 654 | isomiR Example 2 of SEQ ID NO: 117 | - |
| 655 | isomiR Example 1 of SEQ ID NO: 118 | - |
| 656 | isomiR Example 2 of SEQ ID NO: 118 | - |
| 657 | isomiR Example 1 of SEQ ID NO: 119 | - |
| 658 | isomiR Example 2 of SEQ ID NO: 119 | - |
| 659 | isomiR Example 1 of SEQ ID NO: 120 | - |

(continued)

| SEQ ID NO. | Name of gene | Accession No. of miRBase (Accession No.) |
|---|---|---|
| 660 | isomiR Example 2 of SEQ ID NO: 120 | - |
| 661 | isomiR Example 1 of SEQ ID NO: 122 | - |
| 662 | isomiR Example 2 of SEQ ID NO: 122 | - |
| 663 | isomiR Example 1 of SEQ ID NO: 123 | - |
| 664 | isomiR Example 2 of SEQ ID NO: 123 | - |
| 665 | isomiR Example 1 of SEQ ID NO: 124 | - |
| 666 | isomiR Example 2 of SEQ ID NO: 124 | - |
| 667 | isomiR Example 1 of SEQ ID NO: 125 | - |
| 668 | isomiR Example 2 of SEQ ID NO: 125 | - |
| 669 | isomiR Example 1 of SEQ ID NO: 126 | - |
| 670 | isomiR Example 1 of SEQ ID NO: 127 | - |
| 671 | isomiR Example 2 of SEQ ID NO: 127 | - |
| 672 | isomiR Example 1 of SEQ ID NO: 128 | - |
| 673 | isomiR Example 2 of SEQ ID NO: 128 | - |
| 674 | isomiR Example 1 of SEQ ID NO: 129 | - |
| 675 | isomiR Example 2 of SEQ ID NO: 129 | - |
| 676 | isomiR Example 1 of SEQ ID NO: 130 | - |
| 677 | isomiR Example 1 of SEQ ID NO: 131 | - |
| 678 | isomiR Example 1 of SEQ ID NO: 132 | - |
| 679 | isomiR Example 2 of SEQ ID NO: 132 | - |
| 680 | isomiR Example 1 of SEQ ID NO: 133 | - |
| 681 | isomiR Example 1 of SEQ ID NO: 134 | - |
| 682 | isomiR Example 2 of SEQ ID NO: 134 | - |
| 683 | isomiR Example 1 of SEQ ID NO: 135 | - |
| 684 | isomiR Example 1 of SEQ ID NO: 136 | - |
| 685 | isomiR Example 2 of SEQ ID NO: 136 | - |
| 686 | isomiR Example 1 of SEQ ID NO: 137 | - |
| 687 | isomiR Example 2 of SEQ ID NO: 137 | - |
| 688 | isomiR Example 1 of SEQ ID NO: 140 | - |
| 689 | isomiR Example 1 of SEQ ID NO: 141 | - |
| 690 | isomiR Example 1 of SEQ ID NO: 142 | - |
| 691 | isomiR Example 2 of SEQ ID NO: 142 | - |
| 692 | isomiR Example 1 of SEQ ID NO: 143 | - |
| 693 | isomiR Example 2 of SEQ ID NO: 143 | - |
| 694 | isomiR Example 1 of SEQ ID NO: 144 | - |
| 695 | isomiR Example 2 of SEQ ID NO: 144 | - |
| 696 | isomiR Example 1 of SEQ ID NO: 145 | - |
| 697 | isomiR Example 2 of SEQ ID NO: 145 | - |

(continued)

| SEQ ID NO. | Name of gene | Accession No. of miRBase (Accession No.) |
|---|---|---|
| 698 | isomiR Example 1 of SEQ ID NO: 146 | - |
| 699 | isomiR Example 2 of SEQ ID NO: 146 | - |
| 700 | isomiR Example 1 of SEQ ID NO: 147 | - |
| 701 | isomiR Example 2 of SEQ ID NO: 147 | - |
| 702 | isomiR Example 1 of SEQ ID NO: 148 | - |
| 703 | isomiR Example 2 of SEQ ID NO: 148 | - |
| 704 | isomiR Example 1 of SEQ ID NO: 150 | - |
| 705 | isomiR Example 2 of SEQ ID NO: 150 | - |
| 706 | isomiR Example 1 of SEQ ID NO: 151 | - |
| 707 | isomiR Example 2 of SEQ ID NO: 151 | - |
| 708 | isomiR Example 1 of SEQ ID NO: 152 | - |
| 709 | isomiR Example 2 of SEQ ID NO: 152 | - |
| 710 | isomiR Example 1 of SEQ ID NO: 153 | - |
| 711 | isomiR Example 2 of SEQ ID NO: 153 | - |
| 712 | isomiR Example 1 of SEQ ID NO: 154 | - |
| 713 | isomiR Example 2 of SEQ ID NO: 154 | - |
| 714 | isomiR Example 1 of SEQ ID NO: 155 | - |
| 715 | isomiR Example 2 of SEQ ID NO: 155 | - |
| 716 | isomiR Example 1 of SEQ ID NO: 156 | - |
| 717 | isomiR Example 2 of SEQ ID NO: 156 | - |
| 718 | isomiR Example 1 of SEQ ID NO: 157 | - |
| 719 | isomiR Example 2 of SEQ ID NO: 157 | - |
| 720 | isomiR Example 1 of SEQ ID NO: 158 | - |
| 721 | isomiR Example 1 of SEQ ID NO: 159 | - |
| 722 | isomiR Example 2 of SEQ ID NO: 159 | - |
| 723 | isomiR Example 1 of SEQ ID NO: 160 | - |
| 724 | isomiR Example 2 of SEQ ID NO: 160 | - |
| 725 | isomiR Example 1 of SEQ ID NO: 161 | - |
| 726 | isomiR Example 2 of SEQ ID NO: 161 | - |
| 727 | isomiR Example 1 of SEQ ID NO: 162 | - |
| 728 | isomiR Example 2 of SEQ ID NO: 162 | - |
| 729 | isomiR Example 1 of SEQ ID NO: 163 | - |
| 730 | isomiR Example 2 of SEQ ID NO: 163 | - |
| 731 | isomiR Example 1 of SEQ ID NO: 164 | - |
| 732 | isomiR Example 2 of SEQ ID NO: 164 | - |
| 733 | isomiR Example 1 of SEQ ID NO: 165 | - |
| 734 | isomiR Example 2 of SEQ ID NO: 165 | - |
| 735 | isomiR Example 1 of SEQ ID NO: 166 | - |

(continued)

| SEQ ID NO. | Name of gene | Accession No. of miRBase (Accession No.) |
|---|---|---|
| 736 | isomiR Example 2 of SEQ ID NO: 166 | - |
| 737 | isomiR Example 1 of SEQ ID NO: 167 | - |
| 738 | isomiR Example 1 of SEQ ID NO: 168 | - |
| 739 | isomiR Example 2 of SEQ ID NO: 168 | - |
| 740 | isomiR Example 1 of SEQ ID NO: 169 | - |
| 741 | isomiR Example 2 of SEQ ID NO: 169 | - |
| 742 | isomiR Example 1 of SEQ ID NO: 170 | - |
| 743 | isomiR Example 2 of SEQ ID NO: 170 | - |
| 744 | isomiR Example 1 of SEQ ID NO: 171 | - |
| 745 | isomiR Example 2 of SEQ ID NO: 171 | - |
| 746 | isomiR Example 1 of SEQ ID NO: 172 | - |
| 747 | isomiR Example 2 of SEQ ID NO: 172 | - |
| 748 | isomiR Example 1 of SEQ ID NO: 173 | - |
| 749 | isomiR Example 2 of SEQ ID NO: 173 | - |
| 750 | isomiR Example 1 of SEQ ID NO: 174 | - |
| 751 | isomiR Example 2 of SEQ ID NO: 174 | - |
| 752 | isomiR Example 1 of SEQ ID NO: 176 | - |
| 753 | isomiR Example 2 of SEQ ID NO: 176 | - |
| 754 | isomiR Example 1 of SEQ ID NO: 177 | - |
| 755 | isomiR Example 2 of SEQ ID NO: 177 | - |
| 756 | isomiR Example 1 of SEQ ID NO: 178 | - |
| 757 | isomiR Example 2 of SEQ ID NO: 178 | - |
| 758 | isomiR Example 1 of SEQ ID NO: 179 | - |
| 759 | isomiR Example 2 of SEQ ID NO: 179 | - |
| 760 | isomiR Example 1 of SEQ ID NO: 180 | - |
| 761 | isomiR Example 2 of SEQ ID NO: 180 | - |
| 762 | isomiR Example 1 of SEQ ID NO: 181 | - |
| 763 | isomiR Example 2 of SEQ ID NO: 181 | - |
| 764 | isomiR Example 1 of SEQ ID NO: 182 | - |
| 765 | isomiR Example 2 of SEQ ID NO: 182 | - |
| 766 | isomiR Example 1 of SEQ ID NO: 183 | - |
| 767 | isomiR Example 2 of SEQ ID NO: 183 | - |
| 768 | isomiR Example 1 of SEQ ID NO: 184 | - |
| 769 | isomiR Example 2 of SEQ ID NO: 184 | - |
| 770 | isomiR Example 1 of SEQ ID NO: 185 | - |
| 771 | isomiR Example 1 of SEQ ID NO: 186 | - |
| 772 | isomiR Example 1 of SEQ ID NO: 187 | - |
| 773 | isomiR Example 2 of SEQ ID NO: 187 | - |

(continued)

| SEQ ID NO. | Name of gene | Accession No. of miRBase (Accession No.) |
|---|---|---|
| 774 | isomiR Example 1 of SEQ ID NO: 188 | - |
| 775 | isomiR Example 2 of SEQ ID NO: 188 | - |
| 776 | isomiR Example 1 of SEQ ID NO: 189 | - |
| 777 | isomiR Example 1 of SEQ ID NO: 191 | - |
| 778 | isomiR Example 2 of SEQ ID NO: 191 | - |
| 779 | isomiR Example 1 of SEQ ID NO: 192 | - |
| 780 | isomiR Example 2 of SEQ ID NO: 192 | - |
| 781 | isomiR Example 1 of SEQ ID NO: 193 | - |
| 782 | isomiR Example 1 of SEQ ID NO: 194 | - |
| 783 | isomiR Example 2 of SEQ ID NO: 194 | - |
| 784 | isomiR Example 1 of SEQ ID NO: 195 | - |
| 785 | isomiR Example 2 of SEQ ID NO: 195 | - |
| 786 | isomiR Example 1 of SEQ ID NO: 196 | - |
| 787 | isomiR Example 2 of SEQ ID NO: 196 | - |
| 788 | isomiR Example 1 of SEQ ID NO: 197 | - |
| 789 | isomiR Example 1 of SEQ ID NO: 198 | - |
| 790 | isomiR Example 2 of SEQ ID NO: 198 | - |
| 791 | isomiR Example 1 of SEQ ID NO: 199 | - |
| 792 | isomiR Example 2 of SEQ ID NO: 199 | - |
| 793 | isomiR Example 1 of SEQ ID NO: 200 | - |
| 794 | isomiR Example 2 of SEQ ID NO: 200 | - |
| 795 | isomiR Example 1 of SEQ ID NO: 201 | - |
| 796 | isomiR Example 1 of SEQ ID NO: 202 | - |
| 797 | isomiR Example 2 of SEQ ID NO: 202 | - |
| 798 | isomiR Example 1 of SEQ ID NO: 203 | - |
| 799 | isomiR Example 2 of SEQ ID NO: 203 | - |
| 800 | isomiR Example 1 of SEQ ID NO: 204 | - |
| 801 | isomiR Example 2 of SEQ ID NO: 204 | - |
| 802 | isomiR Example 1 of SEQ ID NO: 206 | - |
| 803 | isomiR Example 1 of SEQ ID NO: 207 | - |
| 804 | isomiR Example 2 of SEQ ID NO: 207 | - |
| 805 | isomiR Example 1 of SEQ ID NO: 208 | - |
| 806 | isomiR Example 1 of SEQ ID NO: 209 | - |
| 807 | isomiR Example 2 of SEQ ID NO: 209 | - |
| 808 | isomiR Example 1 of SEQ ID NO: 210 | - |
| 809 | isomiR Example 2 of SEQ ID NO: 210 | - |
| 810 | isomiR Example 1 of SEQ ID NO: 211 | - |
| 811 | isomiR Example 2 of SEQ ID NO: 211 | - |

(continued)

| SEQ ID NO. | Name of gene | Accession No. of miRBase (Accession No.) |
|---|---|---|
| 812 | isomiR Example 1 of SEQ ID NO: 212 | - |
| 813 | isomiR Example 2 of SEQ ID NO: 212 | - |
| 814 | isomiR Example 1 of SEQ ID NO: 213 | - |
| 815 | isomiR Example 1 of SEQ ID NO: 214 | - |
| 816 | isomiR Example 2 of SEQ ID NO: 214 | - |
| 817 | isomiR Example 1 of SEQ ID NO: 215 | - |
| 818 | isomiR Example 2 of SEQ ID NO: 215 | - |
| 819 | isomiR Example 1 of SEQ ID NO: 216 | - |
| 820 | isomiR Example 2 of SEQ ID NO: 216 | - |
| 821 | isomiR Example 1 of SEQ ID NO: 217 | - |
| 822 | isomiR Example 1 of SEQ ID NO: 218 | - |
| 823 | isomiR Example 1 of SEQ ID NO: 219 | - |
| 824 | isomiR Example 2 of SEQ ID NO: 219 | - |
| 825 | isomiR Example 1 of SEQ ID NO: 220 | - |
| 826 | isomiR Example 2 of SEQ ID NO: 220 | - |
| 827 | isomiR Example 1 of SEQ ID NO: 221 | - |
| 828 | isomiR Example 2 of SEQ ID NO: 221 | - |
| 829 | isomiR Example 1 of SEQ ID NO: 222 | - |
| 830 | isomiR Example 1 of SEQ ID NO: 223 | - |
| 831 | isomiR Example 2 of SEQ ID NO: 223 | - |
| 832 | isomiR Example 1 of SEQ ID NO: 224 | - |
| 833 | isomiR Example 2 of SEQ ID NO: 224 | - |
| 834 | isomiR Example 1 of SEQ ID NO: 225 | - |
| 835 | isomiR Example 2 of SEQ ID NO: 225 | - |
| 836 | isomiR Example 1 of SEQ ID NO: 226 | - |
| 837 | isomiR Example 1 of SEQ ID NO: 227 | - |
| 838 | isomiR Example 2 of SEQ ID NO: 227 | - |
| 839 | isomiR Example 1 of SEQ ID NO: 228 | - |
| 840 | isomiR Example 2 of SEQ ID NO: 228 | - |
| 841 | isomiR Example 1 of SEQ ID NO: 229 | - |
| 842 | isomiR Example 2 of SEQ ID NO: 229 | - |
| 843 | isomiR Example 1 of SEQ ID NO: 230 | - |
| 844 | isomiR Example 1 of SEQ ID NO: 231 | - |
| 845 | isomiR Example 2 of SEQ ID NO: 231 | - |
| 846 | isomiR Example 1 of SEQ ID NO: 232 | - |
| 847 | isomiR Example 2 of SEQ ID NO: 232 | - |
| 848 | isomiR Example 1 of SEQ ID NO: 233 | - |
| 849 | isomiR Example 2 of SEQ ID NO: 233 | - |

(continued)

| SEQ ID NO. | Name of gene | Accession No. of miRBase (Accession No.) |
|---|---|---|
| 850 | isomiR Example 1 of SEQ ID NO: 234 | - |
| 851 | isomiR Example 2 of SEQ ID NO: 234 | - |
| 852 | isomiR Example 1 of SEQ ID NO: 235 | - |

The present specification encompasses the contents disclosed in Japanese Patent Application No. 2021-171945 from which the present application claims priority.

Advantageous Effects of Invention

According to the present invention, ovarian cancer and benign ovarian tumor can be discriminated easily and in high accuracy. For example, the presence of ovarian cancer or the presence of benign ovarian tumor in subjects can be easily determined by using, as indicators, the measured expression values of one or multiple miRNAs in body fluids from the subjects, which can be collected with limited invasiveness.

Brief Description of Drawings

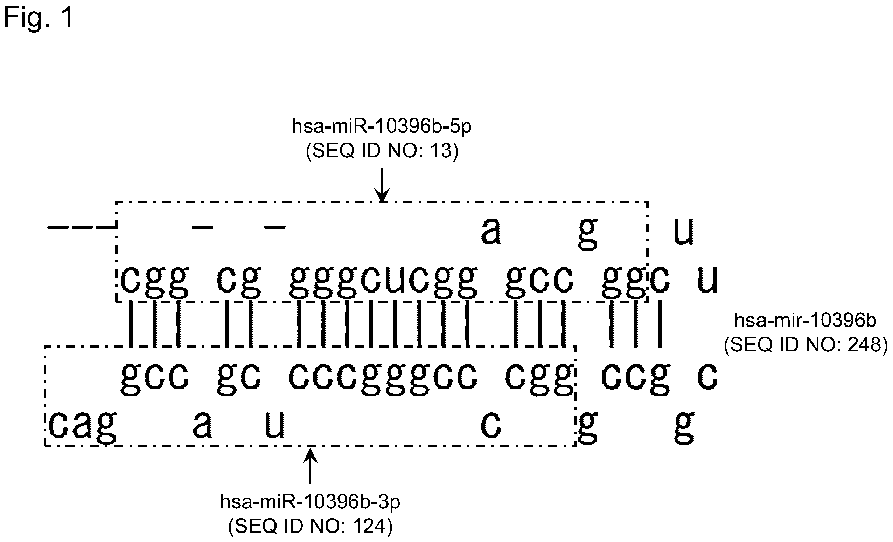

[Figure 1] This figure shows the relationship between the nucleotide sequences of hsa-miR-10396b-5p represented by SEQ ID NO: 13 and hsa-miR-10396b-3p represented by SEQ ID NO: 124, which are produced from the precursor hsa-mir-10396b represented by SEQ ID NO: 248.

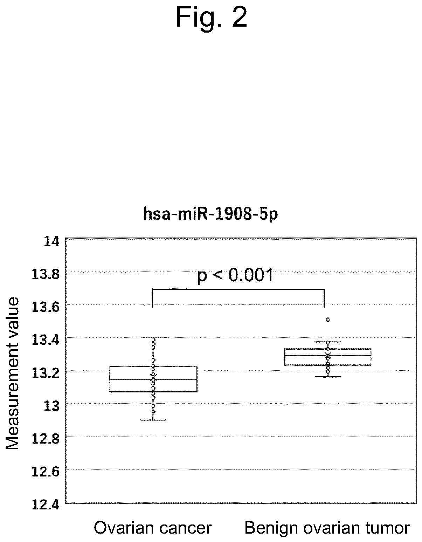

[Figure 2] This figure shows the measured expression values of hsa-miR-1908-5p (SEQ ID NO: 1) in sera from ovarian cancer patients (44 people) and benign ovarian tumor patients (15 people). As a result of performing a two-sided t-test assuming equal variance, a P value was less than 0.001, showing a statistically significant difference.

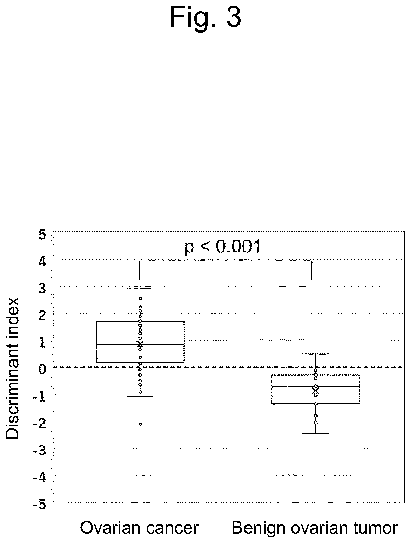

[Figure 3] A discriminant formula (-8.773 × hsa-miR-1908-5p + 6.124 × hsa-miR-7845-5p + 4.909 × hsa-miR-8072 - 1.012 × hsa-miR-7111-5p - 0.974 × hsa-miR-4525) was prepared by use of Fisher's discriminant analysis from the measured expression values of hsa-miR-1908-5p (SEQ ID NO: 1), hsa-miR-7845-5p (SEQ ID NO: 11), hsa-miR-8072 (SEQ ID NO: 16), hsa-miR-7111-5p (SEQ ID NO: 26), and hsa-miR-4525 (SEQ ID NO: 8) in sera from ovarian cancer patients (44 people) and benign ovarian tumor patients (15 people), and discriminant indexes obtained from the discriminant formula were plotted on the ordinate against the ovarian cancer patient group and the benign ovarian tumor patient group on the abscissa. The dotted line in the panel depicts a discriminant boundary discriminating between the two groups and depicts a position that offered a discriminant index of 0.

MODES FOR CARRYING OUT THE INVENTION

Hereinafter, the present invention will be further described in detail.

1. Target nucleic acid for ovarian tumor

Primary markers for discriminating the presence and/or absence of ovarian cancer or ovarian cancer cells from benign ovarian tumor, which are detected using the nucleic acid probes or the primers for the discrimination between ovarian cancer and benign ovarian tumor defined above according to the present invention comprise at least one miRNA selected from the following group A:
Group A:
hsa-miR-1908-5p, hsa-miR-4723-5p, hsa-miR-4674, hsa-miR-939-5p, hsa-miR-6789-5p, hsa-miR-1268a, hsa-miR-1202, hsa-miR-4525, and hsa-miR-128-1-5p
Additional markers that can discriminate between ovarian cancer and benign ovarian tumor in combination with these miRNAs comprise at least one miRNA selected from the following group B:
Group B:
hsa-miR-6806-5p, hsa-miR-7845-5p, hsa-miR-4632-5p, hsa-miR-10396b-5p, hsa-miR-6768-5p, hsa-miR-8059, hsa-miR-8072, hsa-miR-9901, hsa-miR-1231, hsa-miR-1225-5p, hsa-miR-12114, hsa-miR-3178, hsa-miR-6798-5p, hsa-miR-4276, hsa-miR-6125, hsa-miR-3652, hsa-miR-7111-5p, hsa-miR-6749-5p, hsa-miR-1199-5p, hsa-miR-6802-5p, hsa-miR-6816-5p, hsa-miR-4706, hsa-miR-5008-5p, hsa-miR-6797-5p, hsa-miR-4516, hsa-miR-4508, hsa-miR-6729-5p, hsa-miR-564, hsa-miR-1233-5p, hsa-miR-6127, hsa-miR-1469, hsa-miR-6738-5p, hsa-miR-6785-5p, hsa-miR-10401-5p, hsa-miR-4430, hsa-miR-6889-5p, hsa-miR-1236-5p, hsa-miR-3176, hsa-miR-3141, hsa-miR-3928-3p, hsa-miR-1237-5p, hsa-miR-1915-3p, hsa-miR-5195-3p, hsa-miR-6743-5p, hsa-miR-6746-5p, hsa-miR-4446-3p, hsa-miR-1228-5p, hsa-miR-1268b, hsa-miR-1260a, hsa-miR-6879-3p, hsa-miR-149-3p, hsa-miR-3162-5p, hsa-miR-1207-5p, hsa-miR-4747-3p, hsa-miR-4651, hsa-miR-638, hsa-miR-4736, hsa-miR-6845-5p, hsa-miR-1343-3p, hsa-miR-6126, hsa-miR-92b-5p, hsa-miR-6774-5p, hsa-miR-7847-3p, hsa-miR-6795-5p, hsa-miR-7109-5p, hsa-miR-3197, hsa-miR-6824-5p, hsa-miR-6771-5p, hsa-miR-11399, hsa-miR-2861, hsa-miR-4707-3p, hsa-miR-4638-5p, hsa-miR-8073, hsa-miR-328-5p, hsa-miR-665, hsa-miR-6778-5p, hsa-miR-10398-3p, hsa-miR-5698, hsa-miR-6794-5p, hsa-miR-1247-3p, hsa-miR-4697-5p, hsa-miR-8069, hsa-miR-572, hsa-miR-6751-5p, hsa-miR-3180-3p, hsa-miR-486-3p, hsa-miR-6086, hsa-miR-30c-1-3p, hsa-miR-8063, hsa-miR-3621, hsa-miR-6887-5p, hsa-miR-3191-3p, hsa-miR-11181-3p, hsa-miR-6722-5p, hsa-miR-6781-5p, hsa-miR-5739, hsa-miR-3937, hsa-miR-1343-5p, hsa-miR-1181, hsa-miR-4725-3p, hsa-miR-6865-5p, hsa-miR-375-5p, hsa-miR-3196, hsa-miR-6762-5p, hsa-miR-4258, hsa-miR-5196-5p, hsa-miR-10401-3p, hsa-miR-675-5p, hsa-miR-4488, hsa-miR-10527-5p, hsa-miR-10396a-5p, hsa-miR-4269, hsa-miR-6800-5p, hsa-miR-6819-5p, hsa-miR-10396b-3p, hsa-miR-4688, hsa-miR-6786-5p, hsa-miR-4634, hsa-miR-3940-5p, hsa-miR-4655-5p, hsa-miR-7155-5p, hsa-miR-6769b-5p, hsa-miR-6810-5p, hsa-miR-4665-3p, hsa-miR-6727-5p, hsa-miR-6803-5p, hsa-miR-4640-5p, hsa-miR-6735-5p, hsa-miR-4535, hsa-miR-8089, hsa-miR-1292-3p, hsa-miR-5088-5p, hsa-miR-3622a-5p, hsa-miR-6124, hsa-miR-6820-5p, hsa-miR-6805-3p, hsa-miR-4513, hsa-miR-760, hsa-miR-4665-5p, hsa-miR-10400-3p, hsa-miR-4298, hsa-miR-8085, hsa-miR-4463, hsa-miR-6807-5p, hsa-miR-4433b-3p, hsa-miR-3185, hsa-miR-12121, hsa-miR-671-5p, hsa-miR-6752-5p, hsa-miR-371a-5p, hsa-miR-3917, hsa-miR-1224-5p, hsa-miR-498-5p, hsa-miR-7704, hsa-miR-6741-5p, hsa-miR-765, hsa-miR-4486, hsa-miR-6090, hsa-miR-718, hsa-miR-4767, hsa-miR-6851-5p, hsa-miR-5572, hsa-miR-6850-5p, hsa-miR-6089, hsa-miR-5787, hsa-miR-4534, hsa-miR-3665, hsa-miR-4787-5p, hsa-miR-6754-5p, hsa-miR-6825-3p, hsa-miR-4728-5p, hsa-miR-6088, hsa-miR-3154, hsa-miR-6869-5p, hsa-miR-187-5p, hsa-miR-6165, hsa-miR-4447, hsa-miR-4731-5p, hsa-miR-6805-5p, hsa-miR-12118, hsa-miR-4270, hsa-miR-7846-3p, hsa-miR-4443, hsa-miR-6737-5p, hsa-miR-197-5p, hsa-miR-1229-5p, hsa-miR-6757-5p, hsa-miR-6765-5p, hsa-miR-4722-5p, hsa-miR-6891-5p, hsa-miR-5006-5p, hsa-miR-345-3p, hsa-miR-6726-5p, hsa-miR-3195, hsa-miR-6877-5p, hsa-miR-4462, hsa-miR-6812-5p, hsa-miR-483-5p, hsa-miR-9899, hsa-miR-4800-5p, hsa-miR-4734, hsa-miR-3135b, hsa-miR-4433a-3p, hsa-miR-6769a-5p, hsa-miR-4743-5p, hsa-miR-1909-3p, hsa-miR-4741, hsa-miR-4685-5p, hsa-miR-3147, hsa-miR-4726-5p, hsa-miR-3180, hsa-miR-3188, hsa-miR-6782-5p, hsa-miR-6776-5p, hsa-miR-4484, hsa-miR-1185-1-3p, hsa-miR-6790-3p, hsa-miR-4466, hsa-miR-10394-3p, hsa-miR-1275, hsa-miR-4478, hsa-miR-3175, hsa-miR-7106-5p, hsa-miR-4667-5p, hsa-miR-193b-5p, and hsa-miR-602

These miRNAs include, for example, a human gene encoding any of the miRNAs, a congener thereof, a transcript thereof, or/and a variant or a derivative thereof. In this context, the gene, the congener, the transcript, the variant, and the derivative are as defined above.

The target nucleic acid is preferably a human gene comprising a nucleotide sequence represented by any of SEQ ID NOs: 1 to 852 or a transcript thereof, more preferably the transcript, i.e., an miRNA, or its precursor RNA, pri-miRNA or pre-miRNA.

The target genes encoding the first to the 233rd markers are the hsa-miR-1908-5p gene, hsa-miR-4723-5p gene, hsa-miR-4674 gene, hsa-miR-939-5p gene, hsa-miR-6789-5p gene, hsa-miR- 1268a gene, hsa-miR-1202 gene, hsa-miR-4525 gene, hsa-miR-128-1-5p gene, hsa-miR-6806-5p gene, hsa-miR-7845-5p gene, hsa-miR-4632-5p gene, hsa-miR-10396b-5p gene, hsa-miR-6768-5p gene, hsa-miR-8059 gene, hsa-miR-8072 gene, hsa-miR-9901 gene, hsa-miR-1231 gene, hsa-miR-1225-5p gene, hsa-miR-12114 gene, hsa-miR-3178 gene, hsa-miR-6798-5p gene, hsa-miR-4276 gene, hsa-miR-6125 gene, hsa-miR-3652 gene, hsa-miR-7111-5p gene, hsa-miR-6749-5p gene, hsa-miR-1199-5p gene, hsa-miR-6802-5p gene, hsa-miR-6816-5p gene, hsa-miR-4706 gene, hsa-miR-5008-5p gene, hsa-miR-6797-5p gene, hsa-miR-4516 gene, hsa-miR-4508 gene, hsa-miR-6729-5p gene, hsa-miR-564 gene, hsa-miR-1233-5p gene, hsa-miR-6127 gene, hsa-miR-1469 gene, hsa-miR-6738-5p gene, hsa-miR-6785-5p gene, hsa-miR-10401-5p gene, hsa-miR-4430 gene, hsa-miR-6889-5p gene, hsa-miR-1236-5p gene, hsa-miR-3176 gene, hsa-miR-3141 gene, hsa-miR-3928-3p gene, hsa-miR-1237-5p gene, hsa-miR-1915-3p gene, hsa-miR-5195-3p gene, hsa-miR-6743-5p gene, hsa-miR-6746-5p gene, hsa-miR-4446-3p gene, hsa-miR-1228-5p gene, hsa-miR-1268b gene, hsa-miR-1260a gene, hsa-miR-6879-3p gene, hsa-miR-149-3p gene, hsa-miR-3162-5p gene, hsa-miR-1207-5p gene, hsa-miR-4747-3p gene, hsa-miR-4651 gene, hsa-miR-638 gene, hsa-miR-4736 gene, hsa-miR-6845-5p gene, hsa-miR-1343-3p gene, hsa-miR-6126 gene, hsa-miR-92b-5p gene, hsa-miR-6774-5p gene, hsa-miR-7847-3p gene, hsa-miR-6795-5p gene, hsa-miR-7109-5p gene, hsa-miR-3197 gene, hsa-miR-6824-5p gene, hsa-miR-6771-5p gene, hsa-miR-11399 gene, hsa-miR-2861 gene, hsa-miR-4707-3p gene, hsa-miR-4638-5p gene, hsa-miR-8073 gene, hsa-miR-328-5p gene, hsa-miR-665 gene, hsa-miR-6778-5p gene, hsa-miR-10398-3p gene, hsa-miR-5698 gene, hsa-miR-6794-5p gene, hsa-miR-1247-3p gene, hsa-miR-4697-5p gene, hsa-miR-8069 gene, hsa-miR-572 gene, hsa-miR-6751-5p gene, hsa-miR-3180-3p gene, hsa-miR-486-3p gene, hsa-miR-6086 gene, hsa-miR-30c-1-3p gene, hsa-miR-8063 gene, hsa-miR-3621 gene, hsa-miR-6887-5p gene, hsa-miR-3191-3p gene, hsa-miR-11181-3p gene, hsa-miR-6722-5p gene, hsa-miR-6781-5p gene, hsa-miR-5739 gene, hsa-miR-3937 gene, hsa-miR-1343-5p gene, hsa-miR-1181 gene, hsa-miR-4725-3p gene, hsa-miR-6865-5p gene, hsa-miR-375-5p gene, hsa-miR-3196 gene, hsa-miR-6762-5p gene, hsa-miR-4258 gene, hsa-miR-5196-5p gene, hsa-miR-10401-3p gene, hsa-miR-675-5p gene, hsa-miR-4488 gene, hsa-miR-10527-5p gene, hsa-miR-10396a-5p gene, hsa-miR-4269 gene, hsa-miR-6800-5p gene, hsa-miR-6819-5p gene, hsa-miR-10396b-3p gene, hsa-miR-4688 gene, hsa-miR-6786-5p gene, hsa-miR-4634 gene, hsa-miR-3940-5p gene, hsa-miR-4655-5p gene, hsa-miR-7155-5p gene, hsa-miR-6769b-5p gene, hsa-miR-6810-5p gene, hsa-miR-4665-3p gene, hsa-miR-6727-5p gene, hsa-miR-6803-5p gene, hsa-miR-4640-5p gene, hsa-miR-6735-5p gene, hsa-miR-4535 gene, hsa-miR-8089 gene, hsa-miR-1292-3p gene, hsa-miR-5088-5p gene, hsa-miR-3622a-5p gene, hsa-miR-6124 gene, hsa-miR-6820-5p gene, hsa-miR-6805-3p gene, hsa-miR-4513 gene, hsa-miR-760 gene, hsa-miR-4665-5p gene, hsa-miR-10400-3p gene, hsa-miR-4298 gene, hsa-miR-8085 gene, hsa-miR-4463 gene, hsa-miR-6807-5p gene, hsa-miR-4433b-3p gene, hsa-miR-3185 gene, hsa-miR-12121 gene, hsa-miR-671-5p gene, hsa-miR-6752-5p gene, hsa-miR-371a-5p gene, hsa-miR-3917 gene, hsa-miR-1224-5p gene, hsa-miR-498-5p gene, hsa-miR-7704 gene, hsa-miR-6741-5p gene, hsa-miR-765 gene, hsa-miR-4486 gene, hsa-miR-6090 gene, hsa-miR-718 gene, hsa-miR-4767 gene, hsa-miR-6851-5p gene, hsa-miR-5572 gene, hsa-miR-6850-5p gene, hsa-miR-6089 gene, hsa-miR-5787 gene, hsa-miR-4534 gene, hsa-miR-3665 gene, hsa-miR-4787-5p gene, hsa-miR-6754-5p gene, hsa-miR-6825-3p gene, hsa-miR-4728-5p gene, hsa-miR-6088 gene, hsa-miR-3154 gene, hsa-miR-6869-5p gene, hsa-miR-187-5p gene, hsa-miR-6165 gene, hsa-miR-4447 gene, hsa-miR-4731-5p gene, hsa-miR-6805-5p gene, hsa-miR-12118 gene, hsa-miR-4270 gene, hsa-miR-7846-3p gene, hsa-miR-4443 gene, hsa-miR-6737-5p gene, hsa-miR-197-5p gene, hsa-miR-1229-5p gene, hsa-miR-6757-5p gene, hsa-miR-6765-5p gene, hsa-miR-4722-5p gene, hsa-miR-6891-5p gene, hsa-miR-5006-5p gene, hsa-miR-345-3p gene, hsa-miR-6726-5p gene, hsa-miR-3195 gene, hsa-miR-6877-5p gene, hsa-miR-4462 gene, hsa-miR-6812-5p gene, hsa-miR-483-5p gene, hsa-miR-9899 gene, hsa-miR-4800-5p gene, hsa-miR-4734 gene, hsa-miR-3135b gene, hsa-miR-4433a-3p gene, hsa-miR-6769a-5p gene, hsa-miR-4743-5p gene, hsa-miR-1909-3p gene, hsa-miR-4741 gene, hsa-miR-4685-5p gene, hsa-miR-3147 gene, hsa-miR-4726-5p gene, hsa-miR-3180 gene, hsa-miR-3188 gene, hsa-miR-6782-5p gene, hsa-miR-6776-5p gene, hsa-miR-4484 gene, hsa-miR-1185-1-3p gene, hsa-miR-6790-3p gene, hsa-miR-4466 gene, hsa-miR-10394-3p gene, hsa-miR-1275 gene, hsa-miR-4478 gene, hsa-miR-3175 gene, hsa-miR-7106-5p gene, hsa-miR-4667-5p gene, congeners thereof, or variants or derivatives thereof. None of the previously known report shows that change in the expression of these genes or the transcripts thereof can serve as a marker for ovarian tumor.

The target genes encoding the 234th and 235th markers are the hsa-miR-193b-5p gene and the hsa-miR-602 gene, congeners thereof, transcripts thereof, or variants or derivatives thereof. As for these genes, the previously known report shows that change in the expression of the genes or the transcripts thereof can serve as a marker for the discrimination of benign ovarian tumor (Patent Literature 2).

2. Nucleic acid probe or primer for detection of ovarian tumor

In the present invention, the nucleic acid probes or the primers that can be used for detecting ovarian cancer or for discriminately diagnosing ovarian cancer and benign ovarian tumor enable qualitative and/or quantitative measurement of the presence, expression levels, or existing amounts (abundance) of miRNAs represented by SEQ ID NOs: 1 to 9 shown in the group A as target nucleic acids capable of discriminating between ovarian cancer and benign ovarian tumor, or a combination thereof; and miRNAs represented by SEQ ID NOs: 10 to 235 shown in the group B as additional target nucleic acids that can optionally be combined therewith, or combinations thereof; congeners thereof; transcripts thereof; or variants or derivatives thereof.

The expression levels of the target nucleic acids described above are increased or decreased (hereinafter, referred to as "increased/decreased") depending on their types in ovarian cancer patients as compared to benign ovarian tumor patients. Hence, the kit or device of the present invention can be effectively used for measuring expression levels of the target nucleic acids in samples (e.g., body fluids) from subjects (e.g., humans) suspected of having ovarian cancer and samples from ovarian cancer and/or benign ovarian tumor patients, comparing the expression levels, and thereby discriminating between ovarian cancer and benign ovarian tumor.

The nucleic acid probe or primer(s) that can be used in the present invention is, for example, a nucleic acid probe capable of specifically binding to a polynucleotide consisting of a nucleotide sequence represented by at least one of SEQ ID NOs: 1 to 9 or a primer(s) for amplifying a polynucleotide consisting of a nucleotide sequence represented by at least one of SEQ ID NOs: 1 to 9.

The nucleic acid probe or primer(s) that can be used in the present invention may further comprise a nucleic acid probe capable of specifically binding to a polynucleotide consisting of a nucleotide sequence represented by at least one of SEQ ID NOs: 10 to 235; or a primer(s) for amplifying a polynucleotide consisting of a nucleotide sequence represented by at least one of SEQ ID NOs: 10 to 235.

Specifically, these nucleic acid probes or primers comprise a combination of one or multiple polynucleotides selected from: a group of polynucleotides comprising nucleotide sequences represented by any of SEQ ID NOs: 1 to 852 or nucleotide sequences derived from the nucleotide sequences by the replacement of u with t, and a group of complementary polynucleotides thereof; a group of polynucleotides respectively hybridizing under stringent conditions (mentioned later) to DNAs consisting of nucleotide sequences complementary to these nucleotide sequences, and a group of complementary polynucleotides thereof; and a group of polynucleotides comprising 15 or more, preferably 17 or more consecutive nucleotides and being from the nucleotide sequences of these polynucleotide groups. These polynucleotides can be used as nucleic acid probes and/or primers for detecting the markers for discriminating between ovarian cancer and benign ovarian tumor as target nucleic acids.

More specifically, examples of the nucleic acid probes and/or the primers that can be used in the present invention include one or multiple polynucleotides selected from the group consisting of the following polynucleotides (a) to (e):

(a) a polynucleotide consisting of a nucleotide sequence represented by any of SEQ ID NOs: 1 to 9, or a nucleotide sequence derived from the nucleotide sequence by the replacement of u with t, a variant thereof, a derivative thereof, or a fragment of any of these nucleotide sequences, comprising 15 or more consecutive nucleotides;
(b) a polynucleotide comprising a nucleotide sequence represented by any of SEQ ID NOs: 1 to 9;
(c) a polynucleotide consisting of a nucleotide sequence complementary to a nucleotide sequence represented by any of SEQ ID NOs: 1 to 9, or a nucleotide sequence derived from the nucleotide sequence by the replacement of u with t, a variant thereof, a derivative thereof, or a fragment of any of these nucleotide sequences, comprising 15 or more consecutive nucleotides;
(d) a polynucleotide comprising a nucleotide sequence complementary to a nucleotide sequence represented by any of SEQ ID NOs: 1 to 9, or a nucleotide sequence derived from the nucleotide sequence by the replacement of u with t; and
(e) a polynucleotide hybridizing under stringent conditions to any of the polynucleotides (a) to (d).

In addition to at least one polynucleotide selected from the polynucleotides (a) to (e), the nucleic acid probes and/or the primers that can be used in the present invention may further comprise any of the following polynucleotides (f) to (j):

(f) a polynucleotide consisting of a nucleotide sequence represented by any of SEQ ID NOs: 10 to 235, or a nucleotide sequence derived from the nucleotide sequence by the replacement of u with t, a variant thereof, a derivative thereof, or fragment of any of these nucleotide sequences, comprising 15 or more consecutive nucleotides;
(g) a polynucleotide comprising a nucleotide sequence represented by any of SEQ ID NOs: 10 to 235;
(h) a polynucleotide consisting of a nucleotide sequence complementary to a nucleotide sequence represented by any of SEQ ID NOs: 10 to 235, or a nucleotide sequence derived from the nucleotide sequence by the replacement of u with t, a variant thereof, a derivative thereof, or fragment of any of these nucleotide sequences, comprising 15 or more consecutive nucleotides;
(i) a polynucleotide comprising a nucleotide sequence complementary to a nucleotide sequence represented by any of SEQ ID NOs: 10 to 235, or a nucleotide sequence derived from the nucleotide sequence by the replacement of u with t; and
(j) a polynucleotide hybridizing under stringent conditions to any of the polynucleotides (f) to (i).

These polynucleotides or fragments thereof used in the present invention may each be DNA or may each be RNA.
The polynucleotides that can be used in the present invention can be prepared by use of a general technique such as a DNA recombination technique, a PCR method, or a method using an automatic DNA/RNA synthesizer.
The DNA recombination technique and the PCR method may employ techniques described in, for example, Ausubel et al., Current Protocols in Molecular Biology, John Willey & Sons, US (1993); and Sambrook et al., Molecular Cloning - A Laboratory Manual, Cold Spring Harbor Laboratory Press, US (1989).
The target nucleic acids represented by SEQ ID NOs: 1 to 235 are known in the art, and their obtainment methods are also known as mentioned above. Therefore, each polynucleotide that can be used as a nucleic acid probe or a primer in the present invention can be prepared by cloning the gene.
Such nucleic acid probes or primers can be chemically synthesized using an automatic DNA synthesizer. In general, the phosphoramidite method is used in this synthesis, and single-stranded DNA up to approximately 100 nucleotides can be automatically synthesized by this method. The automatic DNA synthesizer is commercially available from, for example, Polygen GmbH, ABI, or Thermo Fisher Scientific Inc.
Alternatively, the polynucleotides of the present invention can also be prepared by cDNA cloning methods. The cDNA cloning technique may employ, for example, microRNA Cloning Kit Wako.
In this context, the sequences of the nucleic acid probes and/or the primers for detecting the polynucleotide consisting of a nucleotide sequence represented by any of SEQ ID NOs: 1 to 235 do not exist as miRNAs or precursors thereof in the living body or *in vivo*. For example, the nucleotide sequences represented by SEQ ID NO: 13 and SEQ ID NO: 124 are produced from the precursor represented by SEQ ID NO: 248. This precursor has a hairpin-like structure as shown in Figure 1, and the nucleotide sequences represented by SEQ ID NO: 13 and SEQ ID NO: 124 have mismatch sequences with each other. As such, a nucleotide sequence completely complementary to the nucleotide sequence represented by SEQ ID NO: 13 or SEQ ID NO: 124 does not naturally occur *in vivo*. Therefore, the nucleic acid probes and the primers for detecting the nucleotide sequence represented by any of SEQ ID NOs: 1 to 235 have artificial nucleotide sequences that do not exist in the living body or *in vivo*.

3. Kit or device for discrimination between ovarian cancer and benign ovarian tumor

The present invention also provides a kit or a device for detection of ovarian cancer, comprising one or multiple polynucleotides (which may include a variant, a fragment, or a derivative thereof) that can be used as nucleic acid probes or primers for measuring target nucleic acids as markers for discriminating between ovarian cancer and benign ovarian tumor.

The target nucleic acids as markers for the discrimination between ovarian cancer and benign ovarian tumor according to the present invention are preferably selected from the group A mentioned above.

Additional target nucleic acids that may be optionally used in the measurement are preferably selected from the group B mentioned above.

The kit or the device of the present invention comprises a probe(s) and/or a primer(s) for detecting any of the target nucleic acids as the markers for the discrimination between ovarian cancer and benign ovarian tumor described above and comprises, for example, one or multiple nucleic acids capable of specifically binding to any of the markers, preferably one or multiple polynucleotides selected from the polynucleotides described in the preceding Section 2, or variants thereof.

Specifically, the kit or the device of the present invention can comprise at least one polynucleotide comprising (or consisting of) a nucleotide sequence represented by any of SEQ ID NOs: 1 to 9 or a nucleotide sequence derived from the nucleotide sequence by the replacement of u with t, a polynucleotide(s) comprising (or consisting of) a complementary sequence thereof, a polynucleotide(s) hybridizing under stringent conditions to any of these polynucleotides, or a variant(s), or a fragment(s) comprising 15 or more consecutive nucleotides of any of these polynucleotide sequences.

The kit or the device of the present invention can further comprise one or more polynucleotides comprising (or consisting of) a nucleotide sequence represented by any of SEQ ID NOs: 10 to 235, or a nucleotide sequence derived from the nucleotide sequence by the replacement of u with t, a polynucleotide(s) comprising (or consisting of) a complementary sequence thereof, a polynucleotide(s) hybridizing under stringent conditions to any of these polynucleotides, a variant(s), or a fragment(s) comprising 15 or more consecutive nucleotides of any of these polynucleotide sequences.

The fragment or fragments that can be comprised in the kit or the device of the present invention is/are, for example, one or more polynucleotides, preferably two or more polynucleotides, selected from the group consisting of the following polynucleotides (1) and (2):

(1) a polynucleotide comprising 15 or more consecutive nucleotides that are from a nucleotide sequence derived from a nucleotide sequence represented by any of SEQ ID NOs: 1 to 9 by the replacement of u with t or a complementary sequence thereof; and

(2) a polynucleotide comprising 15 or more consecutive nucleotides that are from a nucleotide sequence derived from a nucleotide sequence represented by any of SEQ ID NOs: 10 to 235 by the replacement of u with t or a complementary sequence thereof.

In a preferred embodiment, the polynucleotide is a polynucleotide consisting of a nucleotide sequence represented by any of SEQ ID NOs: 1 to 9, or a nucleotide sequence derived from the nucleotide sequence by the replacement of u with t, a polynucleotide consisting of a complementary sequence thereof, a polynucleotide hybridizing under stringent conditions to any of these polynucleotides, or a variant thereof comprising 15 or more, preferably 17 or more, more preferably 19 or more consecutive nucleotides of any of these polynucleotide sequences.

In a preferred embodiment, the polynucleotide is a polynucleotide consisting of a nucleotide sequence represented by any of SEQ ID NOs: 10 to 235, or a nucleotide sequence derived from the nucleotide sequence by the replacement of u with t, a polynucleotide consisting of a complementary sequence thereof, a polynucleotide hybridizing under stringent conditions to any of these polynucleotides, or a variant thereof comprising 15 or more, preferably 17 or more, more preferably 19 or more consecutive nucleotides of any of these polynucleotide sequences.

In a preferred embodiment, the fragment can be a polynucleotide comprising 15 or more, preferably 17 or more, more preferably 19 or more consecutive nucleotides of the original polynucleotide sequence.

In the present invention, the size of the fragment is the number of nucleotides in the range from, for example, 15 or more nucleotides to less than the total number of nucleotides of the sequence, from 17 or more nucleotides to less than the total number of nucleotides of the sequence, or from 19 or more nucleotides to less than the total number of nucleotides of the sequence, in the nucleotide sequence of each original polynucleotide.

Examples of the combination of the above-mentioned polynucleotides constituting the kit or the device of the present invention can specifically include a single (one) polynucleotide or combinations of 2, 3, 4, 5, 6, 7, 8, 9, 10 or more of the above-mentioned polynucleotides consisting of the nucleotide sequences represented by SEQ ID NOs: 1 to 235 as shown in Table 1 above. However, these are given merely for illustrative purposes, and all of various other possible combinations are included in the present invention.

Examples of the above-mentioned combinations constituting the kit or the device for discriminating between ovarian cancer patient and benign ovarian tumor patient according to the present invention can include combinations of two or more of the polynucleotides consisting of the nucleotide sequences represented by SEQ ID NOs shown in Table 1. Further, for example, at least one of the polynucleotides consisting of the nucleotide sequences represented by SEQ ID NOs shown in Table 2 can be combined.

Non-limiting examples of the combination of two polynucleotides comprising at least one of the polynucleotides consisting of the nucleotides represented by SEQ ID NOs: 1 to 9, or complementary sequences thereof are listed below:

(1) a combination of SEQ ID NO: 1 and any one of SEQ ID NOs: 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, and 235;

(2) a combination of SEQ ID NO: 2 and any one of SEQ ID NOs: 3, 4, 5, 6, 7, 8, 9, 11, 12, 13, 15, 19, 21, 22, 24, 25, 26, 32, 33, 34, 37, 38, 39, 41, 43, 46, 49, 50, 53, 56, 59, 63, 64, 65, 66, 70, 74, 75, 77, 80, 82, 86, 92, 94, 96, 99, 103, 108, 109, 110, 116, 117, 119, 121, 122, 124, 125, 126, 130, 131, 135, 137, 140, 142, 146, 148, 150, 152, 154, 156, 157, 158, 159, 161, 165, 167, 171, 173, 175, 176, 177, 182, 185, 186, 187, 190, 192, 196, 200, 201, 202, 207, 208, 226, 228, and 231;

(3) a combination of SEQ ID NO: 3 and any one of SEQ ID NOs: 4, 5, 6, 7, 8, 9, 10, 13, 14, 16, 17, 18, 19, 20, 22, 23, 24, 25, 26, 29, 30, 31, 32, 33, 34, 35, 36, 37, 38, 39, 40, 41, 42, 43, 44, 45, 46, 48, 50, 51, 53, 54, 55, 56, 57, 58, 59, 60, 61, 62, 63, 64, 65, 66, 68, 69, 71, 73, 74, 75, 76, 77, 78, 79, 80, 81, 82, 83, 87, 88, 89, 90, 91, 92, 93, 94, 95, 96, 97, 98, 99, 100, 101, 102, 103, 105, 106, 108, 109, 110, 111, 112, 114, 115, 116, 117, 118, 119, 120, 121, 122, 123, 124, 125, 126, 128, 129, 130, 131, 132, 133, 135, 136, 137, 138, 139, 141, 142, 143, 144, 145, 146, 147, 148, 149, 150, 151, 152, 153, 154, 155, 156, 157, 158, 160, 161, 163, 164, 165, 166, 167, 168, 169, 170, 171, 172, 173, 174, 175, 176, 177, 178, 180, 181, 182, 183, 184, 187, 188, 189, 190, 191, 192, 193, 194, 195, 196, 197, 198, 199, 200, 201, 202, 203, 204, 205, 206, 207, 208, 209, 210, 213, 214, 215, 216, 217, 219, 220, 221, 222, 223, 224, 225, 226, 227, 229, 231, 233, 234, and 235;

(4) a combination of SEQ ID NO: 4 and any one of SEQ ID NOs: 7, 8, 9, 10, 11, 12, 13, 14, 15, 16, 19, 20, 22, 24, 25, 26, 29, 30, 32, 34, 36, 38, 40, 41, 43, 45, 46, 49, 50, 51, 53, 55, 56, 57, 59, 61, 63, 64, 65, 66, 68, 69, 71, 73, 75, 77, 79, 80, 81, 82, 83, 84, 87, 88, 89, 91, 94, 96, 97, 98, 99, 100, 101, 103, 104, 105, 106, 107, 108, 109, 110, 112, 116, 117, 119, 120, 121, 122, 123, 124, 125, 130, 131, 132, 133, 136, 137, 139, 140, 144, 145, 147, 149, 151, 155, 156, 157, 161, 162, 165, 166, 167, 168, 169, 171, 173, 174, 175, 176, 177, 179, 181, 182, 184, 185, 186, 187, 189, 190, 191, 193, 196, 198, 199, 200, 201, 202, 203, 206, 207, 208, 212, 213, 214, 216, 217, 218, 219, 220, 221, 223, 226, 228, 229, 231, 232, 234, and 235;

(5) a combination of SEQ ID NO: 5 and any one of SEQ ID NOs: 6, 8, 9, 12, 13, 14, 15, 16, 17, 18, 19, 20, 21, 22, 23, 24, 25, 32, 34, 36, 41, 44, 47, 48, 49, 51, 53, 54, 56, 57, 59, 60, 61, 64, 65, 66, 69, 70, 74, 76, 77, 79, 80, 81, 82, 87, 93, 96, 98, 103, 105, 107, 109, 110, 115, 118, 121, 125, 129, 130, 131, 136, 137, 139, 142, 149, 156, 158, 163, 165, 166, 167, 168, 169, 170, 174, 176, 177, 179, 181, 184, 187, 191, 194, 199, 200, 202, 203, 204, 208, 214, 220, 226, 227, 228, 231, and 234;

(6) a combination of SEQ ID NO: 6 and any one of SEQ ID NOs: 7, 8, 9, 11, 13, 15, 16, 20, 23, 25, 31, 32, 35, 36, 37, 38, 44, 47, 50, 51, 53, 54, 55, 56, 59, 63, 69, 70, 74, 75, 77, 80, 81, 87, 89, 92, 94, 97, 101, 103, 106, 108, 109, 110, 114, 116, 117, 119, 121, 122, 124, 125, 128, 130, 131, 132, 135, 140, 141, 142, 146, 147, 148, 149, 152, 154, 155, 156, 160, 161, 163, 165, 166, 167, 174, 176, 177, 180, 181, 182, 185, 186, 188, 190, 193, 195, 196, 197, 198, 199, 201, 207, 208, 210, 211, 212, 214, 217, 220, 226, and 233;

(7) a combination of SEQ ID NO: 7 and any one of SEQ ID NOs: 8, 10, 12, 18, 22, 24, 27, 30, 31, 32, 33, 34, 35, 36, 37, 41, 42, 43, 46, 47, 49, 50, 51, 53, 55, 56, 57, 61, 63, 65, 66, 74, 76, 78, 80, 81, 83, 85, 89, 91, 92, 95, 96, 99, 100, 101, 103, 105, 107, 109, 110, 115, 116, 117, 119, 121, 122, 123, 124, 125, 129, 130, 131, 132, 134, 136, 138, 139, 142, 144, 145, 151, 154, 155, 157, 158, 161, 163, 164, 165, 167, 169, 171, 173, 175, 176, 177, 181, 182, 183, 186, 187, 191, 194, 196, 198, 199, 201, 202, 204, 206, 209, 211, 212, 213, 214, 218, 219, 220, 222, 226, 231, 232, and 234;

(8) a combination of SEQ ID NO: 8 and any one of SEQ ID NOs: 9, 10, 11, 12, 13, 14, 15, 16, 19, 20, 21, 23, 24, 25, 26, 27, 28, 29, 30, 31, 32, 33, 34, 35, 36, 37, 38, 39, 40, 41, 43, 44, 45, 46, 47, 48, 49, 50, 51, 53, 54, 55, 56, 57, 58, 59, 60, 61, 63, 64, 65, 66, 67, 68, 69, 70, 71, 74, 75, 76, 78, 79, 80, 81, 82, 83, 87, 88, 90, 91, 92, 93, 94, 95, 96, 97, 100, 103, 104, 105, 106, 107, 108, 110, 111, 112, 113, 114, 115, 119, 121, 122, 124, 126, 128, 129, 130, 131, 132, 134, 135, 136, 137, 138, 139, 141, 142, 143, 144, 145, 148, 149, 151, 152, 154, 155, 156, 158, 159, 160, 161, 162, 163, 164, 165, 166, 167, 168, 169, 170, 171, 172, 173, 176, 177, 179, 183, 184, 185, 186, 188, 189, 190, 191, 193, 194, 195, 196, 199, 200, 202, 203, 204, 205, 208, 209, 210, 212, 213, 214, 215, 216, 217, 218, 220, 222, 223, 224, 225, 226, 227, 228, 229, 231, and 234; and

(9) a combination of SEQ ID NO: 9 and any one of SEQ ID NOs: 12, 13, 14, 17, 18, 22, 23, 24, 27, 30, 36, 37, 38, 41, 46, 51, 53, 57, 80, 87, 92, 99, 110, 114, 121, 130, 156, 160, 161, 163, 167, 170, 176, 177, 178, 183, 193, 198, 200, 205, 214, 220, 221, 222, and 223.

Non-limiting examples of the combination of a plurality of polynucleotides comprising at least one of the polynucleotides consisting of the nucleotides represented by SEQ ID NOs: 1 to 9 or complementary sequences thereof are also listed:

(1) a combination of SEQ ID NOs: 1, 11, 16 and 7;
(2) a combination of SEQ ID NOs: 1, 11, 16, 26, and 8;
(3) a combination of SEQ ID NOs: 1, 2, 11, 16 and 8;
(4) a combination of SEQ ID NOs: 1, 11, 16, 18 and 7;
(5) a combination of SEQ ID NOs: 1, 11, 16, 8 and 29;
(6) a combination of SEQ ID NOs: 1, 12, 13, 16 and 8;
(7) a combination of SEQ ID NOs: 1, 11, 16, 7 and 26; and
(8) a combination of SEQ ID NOs: 1, 10, 11, 16 and 7.

The kit or device of the present invention can also comprise polynucleotide(s) which can discriminate between ovarian cancer and benign ovarian tumor and are known in the art or will be found in the future in addition to the polynucleotide(s) (that can comprise variant(s), fragments, or derivative(s)) according to the present invention as described above.

The kit or device of the present invention can also comprise an antibody and the like for measuring a marker or markers for ovarian cancer examination known in the art, such as CA-125, in addition to the polynucleotide(s) according to the present invention as described above, and a variant or variants thereof or a fragment or fragments thereof.

These polynucleotides and variants thereof or fragments thereof contained in the kit or device of the present invention may be packaged in different containers either individually or in any combination.

The kit or device of the present invention may comprise a reagent for extracting nucleic acids (e.g., total RNA) from body fluids, cells, or tissues, a fluorescent material for labeling, an enzyme for nucleic acid amplification and a medium, an instruction manual, etc.

The device of the present invention is a device for measurement of cancer markers in which nucleic acids such as the polynucleotides according to the present invention described above, variants thereof, derivatives thereof, or fragments thereof are bonded or attached to, for example, a solid phase. Examples of the material for the solid phase include plastics, paper, glass, and silicon. The material for the solid phase is preferably a plastic from the viewpoint of easy processability. The solid phase has any shape and is, for example, square, round, reed-shaped, or film-shaped. The device of the present invention includes, for example, a device for measurement by a hybridization technique or a quantitative amplification technique. Specific examples thereof include blotting devices, nucleic acid arrays (e.g., microarrays, DNA chips, and RNA chips), and nucleic acid amplifiers.

The nucleic acid array technique is a technique which involves bonding or attaching the nucleic acids one by one by use of a method [e.g., a method of spotting the nucleic acids using a high-density dispenser called spotter or arrayer onto the surface of the solid phase surface-treated, if necessary, by coating with L-lysine or the introduction of a functional group such as an amino group or a carboxyl group, a method of spraying the nucleic acids onto the solid phase using an inkjet which injects very small liquid droplets by a piezoelectric element or the like from a nozzle, or a method of sequentially synthesizing nucleotides on the solid phase] to prepare an array such as a chip and measuring target nucleic acids through the use of hybridization using this array.

The kit or the device of the present invention comprises nucleic acids capable of specifically binding to at least one, at least two, at least three, or at least five or more, for example, all, of the ovarian cancer marker polynucleotides, respectively, of the group A described above. The kit or the device of the present invention can optionally further comprise nucleic acids capable of specifically binding to the polynucleotides of at least one, at least two, at least three, or at least five or more, for example, all, of the ovarian cancer marker miRNAs for discrimination, respectively, of the group B described above.

The kit or the device of the present invention can be used for discriminating between ovarian cancer and benign ovarian tumor as described in Section 4 below.

4. Method for discriminating between ovarian cancer and benign ovarian tumor

The present invention further provides a method of using the kit or the device (comprising the above-mentioned nucleic acid(s) that can be used in the present invention) of the present invention as described in Section 3 above to measure an expression level(s) of at least one target nucleic acid selected from the group A described above and optionally an expression level(s) of at least one additional target nucleic acid selected from the group B described above in a sample *in vitro,* and further using the expression level(s) of the target nucleic acid(s) and a control expression level(s) of a benign ovarian tumor patient in samples to discriminate between ovarian cancer and benign ovarian tumor as to the samples, such as blood, serum, or plasma, collected from a subject, and a benign ovarian tumor patient and/or an ovarian cancer patient. The method comprises, for example, evaluating the subject as having ovarian cancer when the expression level(s) of the target nucleic acid(s) in a sample is different from a control by comparing the expression level(s) in the sample with the control expression level(s), or evaluating the subject as not having ovarian cancer when the expression level(s) of the target nucleic acid(s) in the sample is not different from the control.

This method of the present invention enables a limitedly invasive, early diagnosis and preoperative diagnosis of the cancer with high sensitivity and high specificity and thereby brings about early treatment and improved prognosis. In addition, exacerbation of the disease or the effectiveness of surgical, radiotherapeutic, and chemotherapeutic treatments can be monitored by the present invention.

In the method of the present invention, the method for extracting the nucleic acid(s) from the sample may be prepared, for example, by the addition of a reagent for RNA extraction in 3D-Gene(™) RNA extraction reagent from liquid sample kit (Toray Industries, Inc.). Alternatively, a general acidic phenol method (acid guanidinium-phenol-chloroform (AGPC)) may be used, or the nucleic acid(s) may be prepared by the addition of a reagent for RNA extraction containing acidic phenol, such as Trizol(™) (Thermo Fisher Scientific Inc.) or Isogen (Nippon Gene Co., Ltd.). Alternatively, a kit such as miRNeasy(™) Mini Kit (Qiagen N.V.) may be used, though the method is not limited thereto.

The present invention also provides use of the kit or the device of the present invention for detecting *in vitro* an expression product(s) of an ovarian cancer- or benign ovarian tumor-related miRNA gene(s) in a sample from a subject.

The kit or the device described above comprises a single polynucleotide or any possible combination of polynucleotides that can be used in the present invention.

In the discrimination or (genetic) diagnosis between ovarian cancer and benign ovarian tumor according to the present invention, each polynucleotide contained in the kit or device of the present invention can be used as a probe or a primer. In the case of using the polynucleotides as primers, TaqMan(™) MicroRNA Assays from Thermo Fisher Scientific Inc., miScript PCR System from Qiagen N.V., RNA amplification reagent kit (RT-LAMP) from Eiken Chemical Co., Ltd., or the like can be used, though the method is not limited thereto.

Each polynucleotide contained in the kit or device of the present invention can be used as a probe or a primer according to a routine method in a method known in the art for specifically capable of detecting the particular genes, for example, a hybridization technique such as Northern blot, Southern blot, *in situ* hybridization, Northern hybridization, or Southern hybridization, a quantitative amplification technique such as quantitative RT-PCR, or a next-generation sequencer. The sample to be assayed can be selected according to the type of the detection method used, and a sample collected from the subject can be used. Alternatively, for example, total RNA prepared from the thus-collected sample by the method described above may be subjected to be assayed, and various polynucleotides including cDNA prepared on the basis of the RNA may be subjected to be assayed.

The kit or device of the present invention is useful for the discrimination between ovarian cancer and benign ovarian tumor or the detection of the presence or absence of ovarian cancer. Specifically, the discrimination between ovarian cancer and benign ovarian tumor using the kit or device can be performed by detecting *in vitro* an expression level(s) of a gene(s) which is detected using the nucleic acid probe(s) or the primer(s) contained in the kit or device, in a sample from a subject, for example, a subject suspected of having ovarian cancer. The subject can be evaluated as having ovarian cancer when the expression level(s) of a polynucleotide(s) consisting of a nucleotide sequence(s) represented by at least one of, for example, SEQ ID NOs: 1 to 9, and optionally a nucleotide sequence(s) represented by one or more of SEQ ID NOs: 10 to 235, in the sample from the subject, is statistically significantly high compared to an expression level(s) thereof in the sample from a benign ovarian tumor patient.

The method of the present invention can be combined with a diagnostic imaging method such as ultrasonography, CT scanning test, barium enema X-ray examination, or MRI test.

The detection method using the kit or the device of the present invention comprises a method of collecting a sample from a subject, and measuring the expression level(s) of the target gene(s) (or target nucleic acid(s)) contained therein using a single polynucleotide or a plurality of polynucleotides (including a variant(s), a fragment(s), or a derivative(s)) selected from the groups of polynucleotides of the present invention, to discriminate between ovarian cancer and benign ovarian tumor or to detect ovarian cancer. The method for discriminating between ovarian cancer and benign ovarian tumor according to the present invention can also be used to evaluate or diagnose, for example, the presence or absence of amelioration of the disease or the degree of amelioration thereof in a ovarian cancer patient as a subject in the case that an ovarian cancer-related therapeutic drug which is known in the art or on a development stage (including cisplatin, cyclophosphamide, doxorubicin, etoposide, carboplatin, paclitaxel, bevacizumab, olaparib, and combination drugs thereof as non-limiting examples) is administered to the patient for treatment or amelioration of the disease.

The method of the present invention can comprise, for example, the following steps (a), (b), and (c):

(a) a step of contacting *in vitro* a sample from a subject with a polynucleotide(s) contained in the kit or device of the present invention;
(b) a step of measuring an expression level(s) of the target nucleic acid(s) in the sample using the polynucleotide(s) as a nucleic acid probe(s) or primer(s); and
(c) a step of evaluating the presence or absence of ovarian cancer (cells) in the subject on the basis of the measurement results in the step (b).

Specifically, the present invention provides a method for discriminating between ovarian cancer and benign ovarian tumor, comprising: measuring an expression level(s) of a target nucleic acid(s) in a sample from a subject using a probe(s) or a primer(s) capable of detecting at least one polynucleotide or at least two polynucleotides selected from the group consisting of miR-1908-5p, miR-4723-5p, miR-4674, miR-939-5p, miR-6789-5p, miR-1268a, miR-1202, miR-4525, and miR-128-1-5p; and evaluating *in vitro* whether the subject has ovarian cancer, is a benign ovarian tumor patient, or is a healthy subjects without ovarian cancer and benign ovarian tumor using the measured expression levels and control expression levels of a benign ovarian tumor patient(s) measured in the same way as above.

As used herein, the term "evaluating" may be physician's judgment, but may be evaluation support based on results of *in vitro* examination, not physician's judgment.

The nucleic acid(s) in the method of the present invention can further comprise a nucleic acid(s) capable of specifically binding to at least one target nucleic acid selected from the group consisting of miR-6806-5p, miR-7845-5p, miR-4632-5p, miR-10396b-5p, miR-6768-5p, miR-8059, miR-8072, miR-9901, miR-1231, miR-1225-5p, miR-12114, miR-3178, miR-6798-5p, miR-4276, miR-6125, miR-3652, miR-7111-5p, miR-6749-5p, miR-1199-5p, miR-6802-5p, miR-6816-5p, miR-4706, miR-5008-5p, miR-6797-5p, miR-4516, miR-4508, miR-6729-5p, miR-564, miR-1233-5p, miR-6127, miR-1469, miR-6738-5p, miR-6785-5p, miR-10401-5p, miR-4430, miR-6889-5p, miR-1236-5p, miR-3176, miR-3141, miR-3928-3p, miR-1237-5p, miR-1915-3p, miR-5195-3p, miR-6743-5p, miR-6746-5p, miR-4446-3p, miR-1228-5p, miR-1268b, miR-1260a, miR-6879-3p, miR-149-3p, miR-3162-5p, miR-1207-5p, miR-4747-3p, miR-4651, miR-638, miR-4736, miR-6845-5p, miR-1343-3p, miR-6126, miR-92b-5p, miR-6774-5p, miR-7847-3p, miR-6795-5p, miR-7109-5p, miR-3197, miR-6824-5p, miR-6771-5p, miR-11399, miR-2861, miR-4707-3p, miR-4638-5p, miR-8073, miR-328-5p, miR-665, miR-6778-5p, miR-10398-3p, miR-5698, miR-6794-5p, miR-1247-3p, miR-4697-5p, miR-8069, miR-572, miR-6751-5p, miR-3180-3p, miR-486-3p, miR-6086, miR-30c-1-3p, miR-8063, miR-3621, miR-6887-5p, miR-3191-3p, miR-11181-3p, miR-6722-5p, miR-6781-5p, miR-5739, miR-3937, miR-1343-5p, miR-1181, miR-4725-3p, miR-6865-5p, miR-375-5p, miR-3196, miR-6762-5p, miR-4258, miR-5196-5p, miR-10401-3p, miR-675-5p, miR-4488, miR-10527-5p, miR-10396a-5p, miR-4269, miR-6800-5p, miR-6819-5p, miR-10396b-3p, miR-4688, miR-6786-5p, miR-4634, miR-3940-5p, miR-4655-5p, miR-7155-5p, miR-6769b-5p, miR-6810-5p, miR-4665-3p, miR-6727-5p, miR-6803-5p, miR-4640-5p, miR-6735-5p, miR-4535, miR-8089, miR-1292-3p, miR-5088-5p, miR-3622a-5p, miR-6124, miR-6820-5p, miR-6805-3p, miR-4513, miR-760, miR-4665-5p, miR-10400-3p, miR-4298, miR-8085, miR-4463, miR-6807-5p, miR-4433b-3p, miR-3185, miR-12121, miR-671-5p, miR-6752-5p, miR-371a-5p, miR-3917, miR-1224-5p, miR-498-5p, miR-7704, miR-6741-5p, miR-765, miR-4486, miR-6090, miR-718, miR-4767, miR-6851-5p, miR-5572, miR-6850-5p, miR-6089, miR-5787, miR-4534, miR-3665, miR-4787-5p, miR-6754-5p, miR-6825-3p, miR-4728-5p, miR-6088, miR-3154, miR-6869-5p, miR-187-5p, miR-6165, miR-4447, miR-4731-5p, miR-6805-5p, miR-12118, miR-4270, miR-7846-3p, miR-4443, miR-6737-5p, miR-197-5p, miR-1229-5p, miR-6757-5p, miR-6765-5p, miR-4722-5p, miR-6891-5p, miR-5006-5p, miR-345-3p, miR-6726-5p, miR-3195, miR-6877-5p, miR-4462, miR-6812-5p, miR-483-5p, miR-9899, miR-4800-5p, miR-4734, miR-3135b, miR-4433a-3p, miR-6769a-5p, miR-4743-5p, miR-1909-3p, miR-4741, miR-4685-5p, miR-3147, miR-4726-5p, miR-3180, miR-3188, miR-6782-5p, miR-6776-5p, miR-4484, miR-1185-1-3p, miR-6790-3p, miR-4466, miR-10394-3p, miR-1275, miR-4478, miR-3175, miR-7106-5p, miR-4667-5p, miR-193b-5p, and miR-602.

Specifically, the nucleic acid(s) (specifically, a probe(s) and/or a primer(s)) in the method of the present invention is selected from the group consisting of the following polynucleotides (a) to (e):

(a) a polynucleotide consisting of a nucleotide sequence represented by any of SEQ ID NOs: 1 to 235, or a nucleotide sequence derived from the nucleotide sequence by the replacement of u with t, a variant thereof, a derivative thereof, or a fragment of any of these nucleotide sequences, comprising 15 or more consecutive nucleotides;

(b) a polynucleotide comprising a nucleotide sequence represented by any of SEQ ID NOs: 1 to 235;

(c) a polynucleotide consisting of a nucleotide sequence complementary to a nucleotide sequence represented by any of SEQ ID NOs: 1 to 235, or a nucleotide sequence derived from the nucleotide sequence by the replacement of u with t, a variant thereof, a derivative thereof, or a fragment of any of these nucleotide sequences, comprising 15 or more consecutive nucleotides;

(d) a polynucleotide comprising a nucleotide sequence complementary to a nucleotide sequence represented by any of SEQ ID NOs: 1 to 235, or a nucleotide sequence derived from the nucleotide sequence by the replacement of u with t; and

(e) a polynucleotide hybridizing under stringent conditions to any of the polynucleotides (a) to (d).

Examples of the sample used in the method of the present invention can include samples prepared from living tissues (preferably ovarian tissues or fallopian tube tissues) or body fluids such as blood, serum, plasma, and urine from patients or subjects. Specifically, for example, an RNA-containing sample prepared from the tissue, a polynucleotide-containing sample further prepared therefrom, for example, a body fluid such as blood, serum, plasma, or urine, a portion or the whole of a living tissue collected from the subject by biopsy or the like, or a living tissue excised by surgery can be used, and the sample for measurement can be prepared therefrom.

The steps of the method of the present invention can be changed according to the type of the sample to be measured.

In the case of using RNA as an analyte, the discrimination between ovarian cancer and benign ovarian tumor can comprise, for example, the following steps (a), (b), and (c):

(a) a step of binding RNA prepared from a sample from a subject or complementary DNAs (cDNAs) reverse-transcribed from the RNA to a polynucleotide(s) in the kit or the device of the present invention;

(b) a step of measuring the sample-derived RNA or the cDNAs synthesized from the RNA, which is/are bound to the polynucleotide(s), by hybridization using the polynucleotide(s) as a nucleic acid probe(s) or by quantitative RT-PCR using the polynucleotide(s) as a primer(s); and

(c) a step of evaluating the presence of ovarian cancer (-related gene expression) or the presence of benign ovarian tumor (-related gene expression) on the basis of the measurement results of the step (b).

For example, various hybridization methods can be used for discriminating, detecting, examining, evaluating, or diagnosing ovarian cancer (-related gene expression) and benign ovarian tumor (-relate gene expression) *in vitro* according to the present invention. For example, Northern blot, Southern blot, RT-PCR, isothermal nucleic acid amplification, DNA chip analysis, *in situ* hybridization, Northern hybridization, or Southern hybridization can be used as such a hybridization method.

In the case of using the Northern blot, the presence or absence of expression of each gene or the expression level thereof in the RNA can be detected or measured by use of the nucleic acid probe(s) that can be used in the present invention. Specific examples thereof can include a method which comprises labeling the nucleic acid probe (or a complementary strand) with a radioisotope ($^{32}$P, $^{33}$P, $^{35}$S, etc.), a fluorescent material, or the like, hybridizing the labeled product with the tissue-derived RNA from a subject, which is transferred to a nylon membrane or the like according to a routine method, and then detecting and measuring a signal derived from the label (radioisotope or fluorescent material) on the formed DNA/RNA duplex using a radiation detector (examples thereof can include BAS-1800 II (Fujifilm Corp.)) or a fluorescence detector (examples thereof can include STORM 865 (GE Healthcare Japan Corp.)).

In the case of using the quantitative RT-PCR, the presence or absence of expression of each gene or the expression level thereof in the RNA can be detected or measured by use of the primer that can be used in the present invention. Specific examples thereof can include a method which comprises preparing cDNAs from the tissue-derived RNA from a subject according to a routine method, hybridizing a pair of prepared primers (consisting of a plus strand and a reverse strand binding to the cDNA) of the present invention with the cDNA such that the region of each target gene can be amplified with the cDNA as a template, and performing PCR according to a routine method to detect the obtained double-stranded DNA. The method for detecting the double-stranded DNA can include a method of performing the PCR using the primers labeled in advance with a radioisotope or a fluorescent material, a method of electrophoresing the PCR product on an agarose gel and staining the double-stranded DNA with ethidium bromide or the like for detection, and a method of transferring the produced double-stranded DNA to a nylon membrane or the like according to a routine method and hybridizing the double-stranded DNA to a labeled nucleic acid probe for detection.

In the case of using the nucleic acid array analysis, an RNA chip or a DNA chip in which the polynucleotides for detection of the present invention is attached as nucleic acid probes (single-stranded or double-stranded) to a substrate (solid phase) is used. Regions having the attached nucleic acid probes are referred to as probe spots, and regions having no attached nucleic acid probe are referred to as blank spots. A group of genes immobilized on a solid-phase substrate is generally called a nucleic acid chip, a nucleic acid array, a microarray, or the like. The DNA or RNA array includes a DNA or RNA macroarray and a DNA or RNA microarray. In the present specification, the term "chip" includes all of these arrays. 3D-Gene(™) Human miRNA Oligo chip (Toray Industries, Inc., Japan) can be used as the DNA chip, though the DNA chip is not limited thereto.

Examples of the method for measuring signals of the chip can include, but are not limited to, a method of detecting and measuring a signal derived from the label on the composition for detection using an image detector (examples thereof can include Typhoon 9410 (GE Healthcare) and 3D-Gene(™) scanner (Toray Industries, Inc.)).

The "stringent conditions" used herein are, as mentioned above, conditions under which a nucleic acid probe hybridizes to its target sequence to a large extent (e.g., a measurement value equal to or larger than "(a mean of background measurement values) + (a standard error of the background measurement values) $\times$ 2)") than that for other sequences.

The stringent conditions are defined by hybridization and subsequent washing conditions. Examples of the hybridization conditions include, but not limited to, 30°C to 60°C for 1 to 24 hours in a solution containing SSC, a surfactant, formamide, dextran sulfate, a blocking agent(s), etc. In this context, $1 \times$ SSC is an aqueous solution (pH 7.0) containing 150 mM sodium chloride and 15 mM sodium citrate. The surfactant includes, for example, SDS (sodium dodecyl sulfate), Triton, or Tween. The hybridization conditions more preferably comprise 3-10 $\times$ SSC and 0.1-1% SDS. The highly stringent conditions involve, for example, a temperature of 50°C to 65°C or 55°C to 60°C. Also, the highly stringent conditions involve, for example, 1.5 to 3.5 $\times$ SSC, 2 to 3 $\times$ SSC, or 2 to 2.5 $\times$ SSC. Examples of the conditions for the washing, following the hybridization, which is another condition to define the stringent conditions, can include conditions comprising continuous washing at 30°C in a solution containing 0.5 $\times$ SSC and 0.1% SDS, at 30°C in a solution containing 0.2 $\times$ SSC and 0.1% SDS, and at 30°C in a 0.05 $\times$ SSC solution. It is desirable that the complementary strand should maintain its hybridized state with a target plus strand even by washing under such conditions. Specifically, examples of such a complementary strand can include a strand consisting of a nucleotide sequence in a completely complementary relationship with the nucleotide sequence of the target plus (+) strand, and a strand consisting of a nucleotide sequence having at least 80%, at least 85%, at least 90%, at least 95%, at least 98%, or at least 99% identity to the strand.

Other examples of the "stringent conditions" for the hybridization are described in, for example, Sambrook, J. & Russel, D., Molecular Cloning, A LABORATORY MANUAL, Cold Spring Harbor Laboratory Press, published on January 15, 2001, Vol. 1, 7.42 to 7.45 and Vol. 2, 8.9 to 8.17, and can be used in the present invention.

Examples of the conditions for carrying out PCR using polynucleotide fragments in the kit of the present invention as primers include treatment for approximately 15 seconds to 1 minute at 5 to 10°C plus a Tm value calculated from the sequences of the primers, using a PCR buffer having composition such as 10 mM Tris-HCL (pH 8.3), 50 mM KCL, and 1 to 2 mM MgCl$_2$. Examples of the method for calculating such a Tm value include Tm value = 2 $\times$ (the number of adenine residues + the number of thymine residues) + 4 $\times$ (the number of guanine residues + the number of cytosine residues).

In the case of using the quantitative RT-PCR, a commercially available kit for measurement specially designed for quantitatively measuring miRNA, such as TaqMan(™) MicroRNA Assays (Thermo Fisher Scientific Inc.), LNA(™)-based MicroRNA PCR (Exiqon), or Ncode(™) miRNA qRT-PCT kit (Invitrogen Corp.) may be used.

For the calculation of gene expression levels, statistical treatment described in, for example, Statistical analysis of gene expression microarray data (Speed T., Chapman and Hall/CRC), and A beginner's guide Microarray gene expression data analysis (Causton H.C. et al., Blackwell publishing) can be used in the present invention, though the calculation method is not limited thereto. For example, twice, 3 times, or 6 times the standard deviation of the measurement values of the blank spots are added to the average measurement value of the blank spots on the DNA chip, and probe spots having a signal value equal to or larger than the resulting value can be regarded as detection spots. Alternatively, the average measurement value of the blank spots is regarded as a background and can be subtracted from the measurement values of the probe spots to determine gene expression levels. A missing value for a gene expression level can be excluded from the analyte, preferably replaced with the smallest value of the gene expression level in each DNA chip, or more preferably replaced with a value obtained by subtracting 0.1 from a logarithmic value of the smallest value of the gene expression level. In order to eliminate low-signal genes, only a gene having a gene expression level of $2^6$, $2^8$, or $2^{10}$ or larger in 20% or more, 50% or more, or 80% or more of the number of measurement samples can be selected as the analyte. Examples of the normalization of the gene expression level include, but are not limited to, global normalization, quantile normalization (Bolstad, B. M. et al., 2003, Bioinformatics, Vol. 19, p. 185-193), and normalization using an internal control gene. Further, correction (e.g., correction based on a calibration curve) for properly analyzing measurement values may be carried out.

The present invention also provides a method of discriminating between ovarian cancer and benign ovarian tumor (or assisting discrimination thereof) in a subject, comprising measuring expression levels of target nucleic acids or genes in a sample from the subject using the polynucleotides for detection, the kit, or the device (e.g., chip) of the present invention or a combination thereof; and assigning the expression levels of the target genes in a sample from the subject to a discriminant formula (discriminant function), which is prepared using gene expression levels of a sample(s) from a patient(s) known to have ovarian cancer and a sample(s) from a patient(s) known to have benign ovarian tumor, as a training sample(s), and which can determine the presence of ovarian cancer or the presence of benign ovarian tumor, thereby evaluating the presence or absence of the ovarian cancer.

Specifically, the present invention further provides the method comprising a first step of measuring *in vitro* expression levels of target genes in a plurality of patient-derived samples, which are known to have ovarian cancer and/or to have benign ovarian tumor, using the polynucleotides for diagnosis, the kit, or the device (e.g., chip) of the present invention or a combination thereof; a second step of preparing a discriminant formula with the measured expression values of the target genes obtained in the first step as training samples; a third step of measuring *in vitro* the expression levels of the target genes in a sample from the subject in the same manner as in the first step; and a fourth step of assigning the measured expresssion levels of the target genes obtained in the third step to the discriminant formula obtained in the second step, and determining or evaluating whether the subject has ovarian cancer or has benign ovarian tumor on the basis of the results obtained from the discriminant formula, wherein the expression of the target genes can be detected by the polynucleotides, the polynucleotides contained in the kit or device, and variants thereof or fragments thereof.

The discriminant formula that discriminates between ovarian cancer and benign ovarian tumor herein can be prepared by use of any discriminant analysis method, such as Fisher's discriminant analysis, nonlinear discriminant analysis based on the Mahalanobis' distance, neural network or Support Vector Machine (SVM), though the analysis method is not limited thereto.

The linear discriminant analysis is a method for determining the belonging of a cluster using Formula 1 as a discriminant formula when a clustering boundary is a straight line or a hyperplane. In Formula 1, x represents an explanatory variable or an explanatory variable vector (hereinafter, both are collectively referred to as an "explanatory variable"), w represents a coefficient of the explanatory variable, and $w_0$ represents a constant term.

[Equation 1]

$$f(x) = w_0 + \sum_{i=1}^{n} w_i x_i \qquad \text{Formula 1}$$

Values obtained from the discriminant formula are referred to as discriminant indexes. The measurement values of a newly offered data set can be assigned as explanatory variables to the discriminant formula to determine clusters by the signs of the discriminant indexes.

The Fisher's discriminant analysis, a type of linear discriminant analysis, is a dimensionality reduction method for selecting a dimension suitable for discriminating classes, and constructs a highly discriminating synthetic variable by focusing on the variance of the synthetic variables and minimizing the variance of data having the same label (Venables, W.N. et al., Modern Applied Statistics with S. Fourth edition. Springer, 2002). In the Fisher's discriminant analysis, direction w of projection is determined so as to maximize Formula 2. In this formula, $\mu$ represents an average input, ng represents the number of data belonging to class g, and $\mu$g represents an average input of the data belonging to class g. The numerator and the denominator are the interclass variance and the intraclass variance, respectively, when each of data is projected in the direction of the vector w. Discriminant coefficient wi is determined by maximizing this ratio (Takafumi Kanamori et al., "Pattern Recognition", KYORITSU SHUPPAN CO., LTD. (Tokyo, Japan) (2009); Richard O. et al., Pattern Classification, Second Edition., Wiley-Interscience, 2000).

[Equation 2]

$$J(w) = \frac{\sum_{g=1}^{G} n_g \left( w^T \mu_g - w^T \mu \right)\left( w^T \mu_g - w^T \mu \right)^T}{\sum_{g=1}^{G} \sum_{i:y_i=g} \left( w^T x_i - w^T \mu_g \right)\left( w^T x_i - w^T \mu_g \right)} \qquad \text{Formula 2}$$

$$\mu = \sum_{i=1}^{n} \frac{x_i}{n}, \quad \mu_g = \sum_{i:u_i=g}^{n} \frac{x_i}{n_g}$$

The Mahalanobis' distance is calculated according to Formula 3 in consideration of data correlation and can be used as nonlinear discriminant analysis for determining a cluster in which a data point belongs to, based on a short Mahalanobis' distance from the data point to that cluster. In Formula 3, $\mu$ represents a central vector of each cluster, and $S^{-1}$ represents an inverse matrix of the variance-covariance matrix of the cluster. The central vector is calculated from explanatory variable x, and an average vector, a median value vector, or the like can be used.
[Equation 3]

$$D(x,\mu) = \left\{ (x-\mu)^t S^{-1} (x-\mu) \right\}^{\frac{1}{2}} \quad \text{Formula 3}$$

SVM is a discriminant analysis method devised by V. Vapnik (The Nature of Statistical Leaning Theory, Springer, 1995). Particular data points of a data set having known classes are defined as explanatory variables, and classes to be classified are defined as objective variables. A boundary plane called hyperplane for correctly classifying the data set into the known classes is determined, and a discriminant formula for data classification is determined using the boundary plane. Then, the measurement values of a newly offered data set can be assigned as explanatory variables to the discriminant formula to determine classes. In this respect, the result of the discriminant analysis may be classes to be classified, may be a probability of being classified into correct classes, or may be the distance from the hyperplane. In SVM, a method of nonlinearly converting a feature vector to a high dimension and performing linear discriminant analysis in the space is known as a method for tackling nonlinear problems. An expression in which an inner product (scalar product) of two factors in a nonlinearly mapped space is expressed only by inputs in their original spaces is called kernel. Examples of the kernel can include a linear kernel, an RBF (Radial Basis Function) kernel, and a Gaussian kernel. According to the kernel, while highly dimensional mapping is performed, the optimum discriminant formula, i.e., a discriminant formula, can be actually prepared by mere calculation according to the kernel, which avoids calculating features in the mapped space (e.g., Hideki Aso et al., Frontier of Statistical Science 6 "Statistics of pattern recognition and learning - New concepts and approaches", Iwanami Shoten, Publishers (Tokyo, Japan) (2004); Nello Cristianini et al., Introduction to SVM, Kyoritsu Shuppan Co., Ltd. (Tokyo, Japan) (2008)).
C-support vector classification (C-SVC), a type of SVM, comprises preparing a hyperplane by training a data set with the explanatory variables of two groups and classifying an unknown data set into either of the groups (C. Cortes et al., 1995, Machine Learning, Vol. 20, p. 273-297).
Exemplary development of the C-SVC discriminant formula that can be used in the method of the present invention will be given below. First, all patients are divided into, for example, two groups, a group of ovarian cancer patients and a group of benign ovarian tumor patients. For example, ovarian tissue examination may be used for a reference under which each patient is confirmed to have ovarian cancer or to have benign ovarian tumor.
Next, a data set consisting of comprehensive gene expression levels of serum-derived samples of the two divided groups (hereinafter, this data set is referred to as a training cohort) is prepared, and a C-SVC discriminant formula is determined by using genes found to differ clearly in their gene expression levels between the two groups as explanatory variables and this grouping as objective variables (e.g., -1 and +1). An optimizing objective function is represented by Formula 4, wherein e represents all input vectors, y represents an objective variable, a represents a Lagrange's undetermined multiplier vector, Q represents a positive definite matrix, and C represents a parameter for adjusting constrained conditions.
[Equation 4]

$$\min_{a} \quad \frac{1}{2} a^T Q a - e^T a \quad\quad \text{Formula 4}$$

subject to $y^T a = 0$, $0 \leq a_i \leq C$, $i = 1,..., l$,
[0374]   Formula 5 is a finally obtained discriminant formula, and a group in which the data point belongs to can be determined on the basis of the sign of a value obtained according to the discriminant formula. In this formula, x represents a support vector, y represents a label indicating the belonging of a group, a represents the corresponding coefficient, b represents a constant term, and K represents a kernel function.
[Equation 5]

$$f(x) = \text{sgn}\left( \sum_{i=1}^{l} y_i a_i K(x_i, x) + b \right) \quad \text{Formula 5}$$

For example, an RBF kernel defined by Formula 6 can be used as the kernel function. In this formula, x represents a support vector, and r represents a kernel parameter for adjusting the complexity of the hyperplane.
[Equation 6]

$$K(x_i, x_j) = \exp\left(-r\|x_i - x_j\|^2\right) \quad r < 0 \qquad \text{Formula 6}$$

In addition, an approach such as neural network, k-nearest neighbor algorithms, decision trees, or logistic regression analysis can be selected as a method for determining or evaluating the presence of ovarian cancer or the presence of benign ovarian tumor in a sample from a subject.

The method of the present invention can comprise, for example, the following steps (a), (b) and (c):

(a) a step of measuring an expression level(s) of a target gene(s) in samples already known to be from ovarian cancer patients and to be from benign ovarian tumor patients, using the polynucleotide(s), the kit, or the device (e.g., DNA chip) for detection according to the present invention;
(b) a step of preparing the discriminant formulas of Formulas 1, 3, 5 and 6 described above from the measurement values of the expression level determined in the step (a), and
(c) a step of measuring an expression level(s) of the target gene(s) in a sample from a subject using the polynucleotide(s), the kit, or the device (e.g., DNA chip) for diagnosis (detection) according to the present invention, and assigning the obtained measurement value(s) to the explanatory variables of the discriminant formulas prepared in the step (b), or comparing the expression level(s) of the gene(s) in the sample from an ovarian cancer patient to the expression level(s) of the gene(s) in a control sample from a benign ovarian tumor patient, and determining or evaluating that the subject has ovarian cancer or has benign ovarian tumor on the basis of the obtained results.

In this context, in the discriminant formulas of Formulas 1 to 3, 5 and 6, x represents an explanatory variable and includes a value obtained by measuring a polynucleotide(s) selected from the polynucleotides described in Section 2 above or a fragment thereof. Specifically, the explanatory variable of the present invention for discriminating the presence of ovarian cancer or the presence of benign ovarian tumor is a gene expression level(s) selected from, for example, the following expression levels (1) and (2).

(1) a gene expression level(s) in the serum of an ovarian cancer patient and a benign ovarian tumor patient and/or a healthy subject measured by any DNA comprising 15 or more consecutive nucleotides in the nucleotide sequence represented by any of SEQ ID NOs: 1 to 9, or a complementary sequence thereof; and
(2) a gene expression level(s) in the serum of an ovarian cancer patient, and a benign ovarian tumor patient and/or a healthy subject measured by any DNA comprising 15 or more consecutive nucleotides in the nucleotide sequence represented by any of SEQ ID NOs: 10 to 235, or a complementary sequence thereof.

As described above, as the method for determining or evaluating whether a subject has ovarian cancer or has benign ovarian tumor in a sample from the subject, it is possible to use a discriminant formula employing one or more gene expression levels as an explanatory variable(s). In particular, for enhancing the accuracy of the discriminant formula using a single gene expression level alone, it is necessary to use a gene having a clear difference in expression level between two groups consisting of a group of ovarian cancer patients and a group of benign ovarian tumor patients, in a discriminant formula.

The gene that is used for an explanatory variable of a discriminant formula is preferably determined as follows. First, using comprehensive gene expression levels of a group of ovarian cancer patients and comprehensive gene expression levels of a group of benign ovarian tumor patients, both of which are in a training cohort, as a data set, the degree of difference in the expression level of each gene between the two groups is obtained by use of, for example, the P value of a parametric analysis such as t-test, the P value of a nonparametric analysis such as the Mann-Whitney's U test or the P value of the Wilcoxon test.

The gene can be regarded as being statistically significant when the critical rate (significance level) as the P value obtained by the test is smaller than, for example, 5%, 1%, or 0.01%.

In order to correct an increased probability of type I error attributed to the repetition of a test, a method known in the art, for example, Bonferroni or Holm method, can be used for the correction (e.g., Yasushi Nagata et al., "Basics of statistical multiple comparison methods", Scientist Press Co., Ltd. (Tokyo, Japan) (2007)). As an example of the Bonferroni correction, for example, the P value obtained by a test is multiplied by the number of repetitions of the test, i.e., the number of genes used in the analysis, and the obtained value can be compared with a desired significance level to suppress a probability of causing type I error in the whole test.

Instead of the test, the absolute value of a ratio of a median value (fold change) of each gene expression level between gene expression levels of a group of ovarian cancer patients and gene expression levels of a group of benign ovarian tumor patients may be calculated to select a gene that is used for an explanatory variable in a discriminant formula. Alternatively, ROC curves may be prepared using gene expression levels of a group of ovarian cancer patients and a group of benign ovarian tumor patients, and a gene that is used for an explanatory variable in a discriminant formula can be selected on the basis of an AUC value.

Next, a discriminant formula that can be developed by various methods described above is prepared using any number of genes having large difference in their gene expression levels determined here. Examples of the method for preparing a discriminant formula that produces the largest discrimination accuracy include a method of constructing a discriminant formula by use of any combination of expression levels of genes that satisfy the significance level of the P value as an explanatory variable, and a method of repetitively evaluating the genes for use in the preparation of a discriminant formula while increasing the number of genes one by one in a descending order of difference in gene expression level (Furey TS. et al., 2000, Bioinformatics., Vol. 16, p. 906-14). To the discriminant formula thus prepared, the gene expression level of another independent ovarian cancer patient or a benign ovarian tumor patient is assigned as an explanatory variable to calculate discrimination results of the group to which the independent ovarian cancer patient or the benign ovarian tumor patient belongs. Specifically, the gene set for diagnosis found from a sample cohort and the discriminant formula prepared from the gene set for diagnosis can be evaluated in an independent sample cohort to appropriately select a gene set for diagnosis, a discriminant formula, and a discrimination method having more universally high discriminant performance between ovarian cancer and benign ovarian tumor.

In preparing a discriminant formula using expression levels of a plurality of genes as an explanatory variable, it is not necessary to select a gene having a clear difference in expression level between the group of ovarian cancer patients and the group of benign ovarian tumor patients as described above. Specifically, if expression levels of a plurality of genes are used in combination even though the expression levels of individual genes do not clearly differ, a discriminant formula having high discriminant performance can be obtained. Because of this, it is possible to search a discriminant formula having high discriminant performance when discrimination based on the expression levels of a plurality of genes is carried out.

Split-sample method can be used for evaluating the performance (generality) of the discriminant formula. In this method, a data set is divided into a training cohort and a validation cohort, and gene selection by a statistical test and discriminant formula preparation are performed using the training cohort. Accuracy, sensitivity, specificity, and the like are calculated using a result of discriminating a validation cohort according to the discriminant formula, and a valid group to which the validation cohort belongs, to evaluate the discriminant performance. On the other hand, instead of dividing a data set, the gene selection by a statistical test and discriminant formula preparation may be performed using all of samples. In this case, accuracy, sensitivity, specificity, and the like can be calculated, for example, by the discriminant formula using a newly prepared sample cohort for evaluation of the discriminant performance.

The present invention provides a polynucleotide(s) for detection or for diagnosis of a disease useful for diagnosing and treating ovarian cancer, a method for discriminating between ovarian cancer and benign ovarian tumor using the polynucleotide(s), and a kit and device for discriminating between ovarian cancer and benign ovarian tumor, comprising the polynucleotide(s). Particularly, the method of the present invention selects a marker(s) for diagnosis and prepare a discriminant formula so as to exhibit accuracy beyond the ovarian cancer diagnosis method using the existing tumor marker CA-125. Hence, according to the method of present invention, a marker set for diagnosis and a discriminant formula can be constructed, which exhibit accuracy beyond CA-125, for example, by comparing gene expression in serum from a patient who had been initially confirmed to be negative using a known tumor marker such as CA-125 but finally found to have ovarian cancer by detailed examination such as computed tomography using a contrast medium, with gene expression in serum from a patient found to have benign ovarian tumor in the same way.

For example, the marker set for diagnosis is set to any combination selected from one or two or more of the markers based on a nucleotide sequence represented by any of SEQ ID NOs: 1 to 9 as described above or a complementary sequence thereof; and optionally one or two or more of the additional markers based on a nucleotide sequence represented by any of SEQ ID NOs: 10 to 235 or a complementary sequence thereof. Further, a discriminant formula is constructed using the expression levels of the marker set for diagnosis in samples from a patient diagnosed with ovarian cancer and samples from a patient diagnosed with benign ovarian tumor as a result of tissue diagnosis. As a result, whether a subject, from which an unknown sample is provided, has ovarian cancer or has benign ovarian tumor can be determined with high accuracy by measuring expression levels of the marker set for diagnosis in an unknown sample.

EXAMPLES

The present invention will be described further specifically with reference to Examples below. However, the scope of the present invention is not intended to be limited by these Examples.

[Reference Example]

<Samples>

The preserved sera collected from 44 ovarian cancer patients, 15 benign ovarian tumor patients, and 39 healthy subjects were used after obtainment of informed consent. All patients were confirmed the diagnosis as ovarian cancer or benign ovarian tumor by laparotomy.

<Extraction of total RNA>

Total RNA was obtained as a sample using a reagent for RNA extraction in 3D-Gene$^{(TM)}$ RNA extraction reagent from liquid sample kit (Toray Industries, Inc. (Japan)) according to the protocol provided by the manufacturer, from 300 μL of the preserved serum obtained from each of the above-mentioned 98 patients in total.

<Measurement of gene expression level>

First, miRNA in the total RNA, which was obtained from the sera of a total of 98 people consisting of 44 ovarian cancer patients, 15 benign ovarian tumor patients, and 39 healthy subjects, was fluorescently labeled by use of 3D-Gene$^{(TM)}$ miRNA Labeling kit (Toray Industries, Inc.) according to the protocol provided by the manufacturer. The oligo DNA chip used was 3D-Gene$^{(TM)}$ Human miRNA Oligo chip (Toray Industries, Inc.) with attached probes having sequences complementary to 2,565 miRNAs among the miRNAs registered in miRBase Release 22. The hybridization of the miRNA in the total RNA with the probes on the DNA chip and washing following the hybridization were performed under stringent conditions according to the protocol provided by the manufacturer. The DNA chip was scanned using 3D-Gene$^{(TM)}$ scanner (Toray Industries, Inc.) to obtain images. Fluorescence intensity was digitized using 3D-Gene$^{(TM)}$ Extraction (Toray Industries, Inc.). The digitized fluorescence intensity was converted to a logarithmic value having a base of 2 and used as a gene expression level, from which a blank value was subtracted. A missing value was replaced with a value obtained by subtracting 0.1 from the logarithmic value of the minimum gene expression level in each DNA chip. As a result, the comprehensive gene expression levels of the miRNAs in the sera were obtained for 44 ovarian cancer patients, 15 benign ovarian tumor patients, and 39 healthy subjects. Calculation and statistical analysis using the digitized gene expression levels of the miRNAs were carried out using R language 3.6.1 (R Core Team (2016). R: A language and environment for statistical computing. R Foundation for Statistical Computing, Vienna, Austria. URL https://www.R-project.org/.) and MASS package 7.3.45 (Venables, W. N. & Ripley, B. D. (2002) Modern Applied Statistics with S. Fourth Edition. Springer, New York. ISBN 0-387-95457-0).

[Example 1]

<Selection of gene marker for discrimination between ovarian cancer and benign ovarian tumor by comparison between ovarian cancer patient and benign ovarian tumor patient, and evaluation of discriminant performance for ovarian cancer and benign ovarian tumor by gene marker>

In this Example, miRNAs whose expression level significantly differs between ovarian cancer patients and benign ovarian tumor patients were selected as gene markers for the discrimination between ovarian cancer and benign ovarian tumor. The discrimination accuracy of cross-validation was calculated on the basis of a discriminant formula. Based on the calculation, the discriminant performance of each of the single above-mentioned gene marker to distinguish ovarian cancer from benign ovarian tumor was evaluated. Further, the discriminant performance of each combination of two to five of the above-mentioned gene markers to distinguish ovarian cancer from benign ovarian tumor was evaluated.

Specifically, first, the miRNA expression levels obtained in the preceding Reference Examples were normalized. Next, gene markers for diagnosis were selected. Here, in order to acquire diagnostic markers with higher reliability, only genes having the expression level of $2^5$ or higher in 90% or more of the samples in either of the ovarian cancer patient group or the benign ovarian tumor patient group were selected. Next, false positive and true positive rates were calculated when the ovarian cancer patient group was defined as a positive cohort and the benign ovarian tumor patient group was defined as a negative cohort as to the gene expression levels in the ovarian cancer patient group and the benign ovarian tumor patient group. AUC (area under the curve) was determined from ROC (receiver operating characteristic) curves. More specifically, the 10-fold (10-fraction) cross-validation of the two groups was conducted, and average AUC determined from the ROC curves was regarded as AUC of each gene marker. Genes that exhibited AUC of 0.8 or more were selected as gene markers capable of discriminating between ovarian cancer and benign ovarian tumor. As a result of carrying out a two-sided t-test assuming equal variance in the ovarian cancer patient group and the benign ovarian tumor patient group, the selected genes had a P value of less than 0.05 and thus had a statistically significant difference in gene expression. The results about AUC and P values of the selected genes are shown in Table 2. Table 2 also shows increase or decrease in the average expression level of each gene in the ovarian cancer patients compared to the average expression level (control) of the gene in the benign tumor patients measured according to the present invention.

For example, the polynucleotides consisting of the nucleotide sequences represented by SEQ ID NOs: 1, 2, 3, 4, 5, 6, 7, 8, and 9 exhibited AUC, which indicated discriminant performance, of 0.875, 0.805, 0.845, 0.825, 0.803, 0.818, 0.810, 0.885, and 0.815, respectively. Figure 2 shows the measurement values of the marker (hsa-miR-1908-5p) consisting of the nucleotide sequence represented by SEQ ID NO: 1 in the ovarian cancer patient group and the benign ovarian tumor patient group.

In this way, hsa-miR-1908-5p, hsa-miR-4723-5p, hsa-miR-4674, hsa-miR-939-5p, hsa-miR-6789-5p, hsa-miR-1268a, hsa-miR-1202, hsa-miR-4525, hsa-miR-128-1-5p genes, and the relevant polynucleotides consisting of the nucleotide sequences of SEQ ID NOs: 1 to 9 were newly found as markers for discriminating between ovarian cancer and benign ovarian tumor.

Two polynucleotides were selected from the polynucleotides represented by SEQ ID NOs: 1 to 9 and combined, and a discriminant formula was prepared by the Fisher's discriminant analysis. As a result, combinations that elevated discrimination accuracy for ovarian cancer and benign ovarian tumor compared to the discriminant formula based on the corresponding single marker were found for all the markers. Specifically, the discriminant formulas based on the combinations of the markers of SEQ ID NOs: 8 and 1, SEQ ID NOs: 8 and 3, SEQ ID NOs: 8 and 4, SEQ ID NOs: 1 and 4, SEQ ID NOs: 1 and 6, SEQ ID NOs: 1 and 5, SEQ ID NOs: 8 and 7, SEQ ID NOs: 1 and 7, SEQ ID NOs: 8 and 6, SEQ ID NOs: 1 and 2, SEQ ID NOs: 1 and 9, SEQ ID NOs: 3 and 7, SEQ ID NOs: 3 and 2, SEQ ID NOs: 4 and 7, SEQ ID NOs: 8 and 9, SEQ ID NOs: 3 and 4, SEQ ID NOs: 8 and 2, SEQ ID NOs: 6 and 7, SEQ ID NOs: 2 and 5, SEQ ID NOs: 7 and 2, SEQ ID NOs: 9 and 2, SEQ ID NOs: 3 and 9, SEQ ID NOs: 9 and 5, SEQ ID NOs: 8 and 5, SEQ ID NOs: 3 and 5, SEQ ID NOs: 4 and 2, and SEQ ID NOs: 6 and 5 offered AUC, which indicated discriminant performance, of 0.895, 0.885, 0.883, 0.875, 0.865, 0.865, 0.865, 0.855, 0.853, 0.850, 0.845, 0.845, 0.840, 0.838, 0.835, 0.835, 0.830, 0.825, 0.825, 0.820, 0.820, 0.820, 0.818, 0.815, 0.815, 0.810, and 0.805, respectively. SEQ ID NOs and AUC of the selected genes are shown in Table 3. In Table 3, the column "SEQ ID NO" depicts each combination of the polynucleotides used, which was indicated by SEQ ID NO (the same applies to the tables below.).

Three to five polynucleotides were selected from the polynucleotides represented by SEQ ID NOs: 1 to 9 and combined, and a discriminant formula was prepared by the Fisher's discriminant analysis. As a result, combinations that elevated discrimination accuracy for ovarian cancer and benign ovarian tumor compared to the discriminant formula based on the corresponding single marker were found for all the markers. The results of combinations about each of the markers of SEQ ID NOs: 1 and 8 are shown in Tables 4 and 5. Specifically, as for the marker of SEQ ID NO: 1, the discriminant formulas based on the combinations of the markers of SEQ ID NOs: 1, 6 and 8, SEQ ID NOs: 1, 4 and 8, SEQ ID NOs: 1, 8 and 9, SEQ ID NOs: 1, 3 and 8, SEQ ID NOs: 1, 5 and 8, SEQ ID NOs: 1, 2 and 8, SEQ ID NOs: 1, 4 and 7, SEQ ID NOs: 1, 2 and 4, SEQ ID NOs: 1, 2 and 9, SEQ ID NOs: 1, 5 and 6, SEQ ID NOs: 1, 3 and 5, SEQ ID NOs: 1, 3 and 4, SEQ ID NOs: 1, 7 and 8, SEQ ID NOs: 1, 6 and 7, SEQ ID NOs: 1, 5 and 7, SEQ ID NOs: 1, 5 and 9, SEQ ID NOs: 1, 4 and 9, SEQ ID NOs: 1, 5, 6 and 8, SEQ ID NOs: 1, 4, 6 and 8, SEQ ID NOs: 1, 2, 8 and 9, SEQ ID NOs: 1, 3, 4 and 6, SEQ ID NOs: 1, 3, 8 and 9, SEQ ID NOs: 1, 3, 5 and 8, SEQ ID NOs: 1, 5, 7 and 8, SEQ ID NOs: 1, 2, 6 and 8, SEQ ID NOs: 1, 4, 5 and 8, SEQ ID NOs: 1, 4, 7 and 8, SEQ ID NOs: 1, 4, 6 and 7, SEQ ID NOs: 1, 6, 8 and 9, SEQ ID NOs: 1, 3, 5 and 6, SEQ ID NOs: 1, 3, 4 and 8, SEQ ID NOs: 1, 2, 5 and 8, SEQ ID NOs: 1, 2, 4 and 8, SEQ ID NOs: 1, 5, 8 and 9, SEQ ID NOs: 1, 3, 4, 6 and 8, SEQ ID NOs: 1, 5, 6, 7 and 8, SEQ ID NOs: 1, 2, 5, 6 and 8, SEQ ID NOs: 1, 4, 6, 8 and 9, SEQ ID NOs: 1, 3, 4 and 7, SEQ ID NOs: 1, 2, 4, 6 and 8, SEQ ID NOs: 1, 3, 4, 6 and 9, and SEQ ID NOs: 1, 4, 6, 7 and 8 offered AUC, which indicated discriminant performance, of 0.895, 0.888, 0.875, 0.875, 0.875, 0.870, 0.868, 0.865, 0.865, 0.865, 0.865, 0.865, 0.855, 0.855, 0.855, 0.855, 0.855, 0.900, 0.898, 0.885, 0.880, 0.875, 0.875, 0.875, 0.870, 0.870, 0.868, 0.868, 0.865, 0.865, 0.865, 0.865, 0.865, 0.865, 0.913, 0.900, 0.890, 0.890, 0.883, 0.883, 0.880, and 0.878, respectively. As for the marker of SEQ ID NO: 8, the discriminants based on the combinations of the markers of SEQ ID NOs: 1, 6 and 8, SEQ ID NOs: 1, 4 and 8, SEQ ID NOs: 1, 8 and 9, SEQ ID NOs: 1, 3 and 8, SEQ ID NOs: 1, 5 and 8, SEQ ID NOs: 3, 7 and 8, SEQ ID NOs: 4, 7 and 8, SEQ ID NOs: 1, 2 and 8, SEQ ID NOs: 1, 7 and 8, SEQ ID NOs: 4, 6 and 8, SEQ ID NOs: 3, 8 and 9, SEQ ID NOs: 7, 8 and 9, SEQ ID NOs: 5, 6 and 8, SEQ ID NOs: 2, 3 and 8, SEQ ID NOs: 5, 8 and 9, SEQ ID NOs: 3, 4 and 8, SEQ ID NOs: 2, 4 and 8, SEQ ID NOs: 6, 8 and 9, SEQ ID NOs: 3, 5 and 8, SEQ ID NOs: 3, 6 and 8, SEQ ID NOs: 6, 7 and 8, SEQ ID NOs: 4, 8 and 9, SEQ ID NOs: 1, 5, 6 and 8, SEQ ID NOs: 1, 4, 6 and 8, SEQ ID NOs: 1, 2, 8 and 9, SEQ ID NOs: 1, 3, 8 and 9, SEQ ID NOs: 1, 3, 5 and 8, SEQ ID NOs: 1, 5, 7 and 8, SEQ ID NOs: 1, 2, 6 and 8, SEQ ID NOs: 1, 4, 5 and 8, SEQ ID NOs: 1, 4, 7 and 8, SEQ ID NOs: 1, 6, 8 and 9, SEQ ID NOs: 1, 5, 8 and 9, SEQ ID NOs: 1, 3, 4 and 8, SEQ ID NOs: 1, 2, 5 and 8, SEQ ID NOs: 1, 2, 4 and 8, SEQ ID NOs: 1, 4, 8 and 9, SEQ ID NOs: 1, 6, 7 and 8, SEQ ID NOs: 1, 3, 6 and 8, SEQ ID NOs: 1, 2, 3 and 8, SEQ ID NOs: 1, 7, 8 and 9, SEQ ID NOs: 4, 6, 7 and 8, SEQ ID NOs: 3, 7, 8 and 9, SEQ ID NOs: 2, 3, 8 and 9, SEQ ID NOs: 1, 2, 7 and 8, SEQ ID NOs: 2, 3, 6 and 8, SEQ ID NOs: 3, 4, 6 and 8, SEQ ID NOs: 5, 7, 8 and 9, SEQ ID NOs: 1, 3, 7 and 8, SEQ ID NOs: 3, 6, 8 and 9, SEQ ID NOs: 3, 5, 7 and 8, SEQ ID NOs: 2, 3, 5 and 8, SEQ ID NOs: 1, 4, 6, 8 and 9, SEQ ID NOs: 1, 3, 5, 8 and 9, SEQ ID NOs: 1, 5, 7, 8 and 9, SEQ ID NOs: 1, 3, 6, 8 and 9, SEQ ID NOs: 1, 6, 7, 8 and 9, SEQ ID NOs: 1, 3, 4, 8 and 9, SEQ ID NOs: 1, 3, 7, 8 and 9, and SEQ ID NOs: 1, 4, 5, 8 and 9 offered AUC, which indicated discriminant performance, of 0.895, 0.888, 0.875, 0.875, 0.875, 0.875, 0.872, 0.870, 0.855, 0.855, 0.850, 0.850, 0.840, 0.840, 0.840, 0.835, 0.830, 0.830, 0.825, 0.825, 0.825, 0.820, 0.900, 0.898, 0.885, 0.875, 0.875, 0.875, 0.870, 0.870, 0.868, 0.865, 0.865, 0.865, 0.865, 0.865, 0.855, 0.855, 0.850, 0.850, 0.845, 0.845, 0.840, 0.840, 0.840, 0.840, 0.835, 0.830, 0.830, 0.825, 0.825, 0.825, 0.890, 0.865, 0.865, 0.850, 0.845, 0.845, 0.845, and 0.835, respectively.

From the above, it was demonstrated that all polynucleotides consisting of the nucleotide sequences represented by SEQ ID NOs: 1 to 9 are from genes newly found to be related to ovarian cancer and are useful as gene markers capable of discriminating between ovarian cancer and benign ovarian tumor with high performance when these are used singly or in combinations of two or more.

[Table 2]

| SEQ ID NO. | Name of gene | p value of cancer/ benign | AUC | Increase or decrease in average expression level in cancer compared to benign |
|---|---|---|---|---|
| 1 | hsa-miR-1908-5p | 2.4E-04 | 0.875 | Decrease |
| 2 | hsa-miR-4723-5p | 9.1E-04 | 0.805 | Decrease |
| 3 | hsa-miR-4674 | 1.2E-03 | 0.845 | Increase |
| 4 | hsa-miR-939-5p | 1.4E-03 | 0.825 | Decrease |
| 5 | hsa-miR-6789-5p | 1.5E-03 | 0.803 | Increase |
| 6 | hsa-miR-1268a | 4.3E-03 | 0.818 | Decrease |
| 7 | hsa-miR-1202 | 1.1E-02 | 0.810 | Decrease |
| 8 | hsa-miR-4525 | 1.5E-02 | 0.885 | Decrease |
| 9 | hsa-miR-128-1-5p | 1.9E-02 | 0.815 | Increase |

[Table 3]

| SEQ ID NO. | AUC |
|---|---|
| 8_1 | 0.895 |
| 8_3 | 0.885 |
| 8_4 | 0.883 |
| 1_4 | 0.875 |
| 1_6 | 0.865 |
| 1_5 | 0.865 |
| 8_7 | 0.865 |
| 1_7 | 0.855 |
| 8_6 | 0.853 |
| 1_2 | 0.850 |

(continued)

| SEQ ID NO. | AUC |
|---|---|
| 1_9 | 0.845 |
| 3_7 | 0.845 |
| 3_2 | 0.840 |
| 4_7 | 0.838 |
| 8_9 | 0.835 |
| 3_4 | 0.835 |
| 8_2 | 0.830 |
| 6_7 | 0.825 |
| 2_5 | 0.825 |
| 7_2 | 0.820 |
| 9_2 | 0.820 |
| 3_9 | 0.820 |
| 9_5 | 0.818 |
| 8_5 | 0.815 |
| 3_5 | 0.815 |
| 4_2 | 0.810 |
| 6_5 | 0.805 |

[Table 4]

| SEQ ID NO. | The number of markers | AUC |
|---|---|---|
| 1 | 1 | 0.875 |
| 1_8 | 2 | 0.895 |
| 1_4 | 2 | 0.875 |
| 1_6 | 2 | 0.865 |
| 1_5 | 2 | 0.865 |
| 1_7 | 2 | 0.855 |
| 1_2 | 2 | 0.850 |
| 1_9 | 2 | 0.845 |
| 1_6_8 | 3 | 0.895 |
| 1_4_8 | 3 | 0.888 |
| 1_8_9 | 3 | 0.875 |
| 1_3_8 | 3 | 0.875 |
| 1_5_8 | 3 | 0.875 |
| 1_2_8 | 3 | 0.870 |
| 1_4_7 | 3 | 0.868 |
| 1_2_4 | 3 | 0.865 |
| 1_2_9 | 3 | 0.865 |

(continued)

| SEQ ID NO. | The number of markers | AUC |
|---|---|---|
| 1_5_6 | 3 | 0.865 |
| 1_3_5 | 3 | 0.865 |
| 1_3_4 | 3 | 0.865 |
| 1_7_8 | 3 | 0.855 |
| 1_6_7 | 3 | 0.855 |
| 1_5_7 | 3 | 0.855 |
| 1_5_9 | 3 | 0.855 |
| 1_4_9 | 3 | 0.855 |
| 1_5_6_8 | 4 | 0.900 |
| 1_4_6_8 | 4 | 0.898 |
| 1_2_8_9 | 4 | 0.885 |
| 1_3_4_6 | 4 | 0.880 |
| 1_3_8_9 | 4 | 0.875 |
| 1_3_5_8 | 4 | 0.875 |
| 1_5_7_8 | 4 | 0.875 |
| 1_2_6_8 | 4 | 0.870 |
| 1_4_5_8 | 4 | 0.870 |
| 1_4_7_8 | 4 | 0.868 |
| 1_4_6_7 | 4 | 0.868 |
| 1_6_8_9 | 4 | 0.865 |
| 1_3_5_6 | 4 | 0.865 |
| 1_3_4_8 | 4 | 0.865 |
| 1_2_5_8 | 4 | 0.865 |
| 1_2_4_8 | 4 | 0.865 |
| 1_5_8_9 | 4 | 0.865 |
| 1_3_4_6_8 | 5 | 0.913 |
| 1_5_6_7_8 | 5 | 0.900 |
| 1_2_5_6_8 | 5 | 0.890 |
| 1_4_6_8_9 | 5 | 0.890 |
| 1_3_4_6_7 | 5 | 0.883 |
| 1_2_4_6_8 | 5 | 0.883 |
| 1_3_4_6_9 | 5 | 0.880 |
| 1_4_6_7_8 | 5 | 0.878 |

[Table 5]

| SEQ ID NO. | The number of markers | AUC |
|---|---|---|
| 8 | 1 | 0.885 |

(continued)

| SEQ ID NO. | The number of markers | AUC |
|---|---|---|
| 8_1 | 2 | 0.895 |
| 8_3 | 2 | 0.885 |
| 8_4 | 2 | 0.883 |
| 8_7 | 2 | 0.865 |
| 8_6 | 2 | 0.853 |
| 8_9 | 2 | 0.835 |
| 8_2 | 2 | 0.830 |
| 8_5 | 2 | 0.815 |
| 1_6_8 | 3 | 0.895 |
| 1_4_8 | 3 | 0.888 |
| 1_8_9 | 3 | 0.875 |
| 1_3_8 | 3 | 0.875 |
| 1_5_8 | 3 | 0.875 |
| 3_7_8 | 3 | 0.875 |
| 4_7_8 | 3 | 0.872 |
| 1_2_8 | 3 | 0.870 |
| 1_7_8 | 3 | 0.855 |
| 4_6_8 | 3 | 0.855 |
| 3_8_9 | 3 | 0.850 |
| 7_8_9 | 3 | 0.850 |
| 5_6_8 | 3 | 0.840 |
| 2_3_8 | 3 | 0.840 |
| 5_8_9 | 3 | 0.840 |
| 3_4_8 | 3 | 0.835 |
| 2_4_8 | 3 | 0.830 |
| 6_8_9 | 3 | 0.830 |
| 3_5_8 | 3 | 0.825 |
| 3_6_8 | 3 | 0.825 |
| 6_7_8 | 3 | 0.825 |
| 4_8_9 | 3 | 0.820 |
| 1_5_6_8 | 4 | 0.900 |
| 1_4_6_8 | 4 | 0.898 |
| 1_2_8_9 | 4 | 0.885 |
| 1_3_8_9 | 4 | 0.875 |
| 1_3_5_8 | 4 | 0.875 |
| 1_5_7_8 | 4 | 0.875 |
| 1_2_6_8 | 4 | 0.870 |
| 1_4_5_8 | 4 | 0.870 |

(continued)

| SEQ ID NO. | The number of markers | AUC |
|---|---|---|
| 1_4_7_8 | 4 | 0.868 |
| 1_6_8_9 | 4 | 0.865 |
| 1_5_8_9 | 4 | 0.865 |
| 1_3_4_8 | 4 | 0.865 |
| 1_2_5_8 | 4 | 0.865 |
| 1_2_4_8 | 4 | 0.865 |
| 1_4_8_9 | 4 | 0.855 |
| 1_6_7_8 | 4 | 0.855 |
| 1_3_6_8 | 4 | 0.850 |
| 1_2_3_8 | 4 | 0.850 |
| 1_7_8_9 | 4 | 0.845 |
| 4_6_7_8 | 4 | 0.845 |
| 3_7_8_9 | 4 | 0.840 |
| 2_3_8_9 | 4 | 0.840 |
| 1_2_7_8 | 4 | 0.840 |
| 2_3_6_8 | 4 | 0.840 |
| 3_4_6_8 | 4 | 0.835 |
| 5_7_8_9 | 4 | 0.830 |
| 1_3_7_8 | 4 | 0.830 |
| 3_6_8_9 | 4 | 0.825 |
| 3_5_7_8 | 4 | 0.825 |
| 2_3_5_8 | 4 | 0.825 |
| 1_4_6_8_9 | 5 | 0.890 |
| 1_3_5_8_9 | 5 | 0.865 |
| 1_5_7_8_9 | 5 | 0.865 |
| 1_3_6_8_9 | 5 | 0.850 |
| 1_6_7_8_9 | 5 | 0.845 |
| 1_3_4_8_9 | 5 | 0.845 |
| 1_3_7_8_9 | 5 | 0.845 |
| 1_4_5_8_9 | 5 | 0.835 |

[Example 2]

<Selection of gene marker for discrimination between ovarian cancer and benign ovarian tumor by comparison between ovarian cancer patient and benign ovarian tumor patient and evaluation of discriminant performance for ovarian cancer and benign ovarian tumor by combination of plurality of gene markers>

In this Example, in order to find additional gene markers that could exert high discriminant performance for ovarian cancer patient and benign ovarian tumor by combination with the gene markers (SEQ ID NOs: 1 to 9) for the discrimination between ovarian cancer patient and benign ovarian tumor selected in Example 1, a discriminant formula was prepared from expression levels of two to five gene markers combined. The discrimination accuracy of cross-validation was further calculated on the basis of the discriminant formula. Based on the calculation, the discriminant performance to distinguish ovarian cancer patients from benign ovarian tumor patients was evaluated.

The selection of genes and the calculation of AUC were carried out in the same way as in Example 1.

The selected genes were combined with the marker represented by any one of SEQ ID NOs: 1 to 9, and a discriminant formula based on the expression levels of two gene markers was prepared by the Fisher's discriminant analysis. Genes that exhibited AUC of 0.8 or more were selected as additional markers capable of discriminating ovarian cancer patients from benign ovarian tumor patients by combination with the markers of SEQ ID NOs: 1 to 9. The combinations of SEQ ID NOs and AUC indicating the discriminant performance of each combination are shown in Table 6. Markers consisting of the nucleotide sequences represented by SEQ ID NOs: 10 to 235 were obtained as these additional markers.

At least one of the markers represented by SEQ ID NOs: 1 to 9 and a plurality of markers selected from the additional markers consisting of the nucleotide sequences represented by SEQ ID NOs: 10 to 235 were combined, and a discriminant formula was prepared from expression levels of three to five markers. As a result, combinations that exhibited excellent discriminant performance with AUC of 0.95 or more were found. Although any combination of the markers is considered useful as a marker for the discrimination between ovarian cancer and benign ovarian tumor, typical combinations and their discriminant performance indicated by SEQ ID NOs and AUC thereof are listed in Table 7. The discriminant formulas based on the combinations of the markers of SEQ ID NOs: 1, 11, 16 and 7, SEQ ID NOs: 1, 11, 16, 26 and 8, SEQ ID NOs: 1, 2, 11, 16 and 8, SEQ ID NOs: 1, 11, 16, 18 and 7, SEQ ID NOs: 1, 11, 16, 8 and 29, SEQ ID NOs: 1, 12, 13, 16 and 8, SEQ ID NOs: 1, 11, 16, 7 and 26, and SEQ ID NOs: 1, 10, 11, 16 and 7 offered AUC, which indicated discriminant performance, of 0.960, 0.970, 0.968, 0.960, 0.955, 0.955, 0.950, and 0.950, respectively.

Detection sensitivity for ovarian cancer and specificity when the benign ovarian tumor patients are used as a negative cohort are exemplified in Table 8 and Figure 3, together with the existing marker, as to the discriminant formula based on the combination of the markers consisting of the sequences represented by SEQ ID NOs: 1, 11, 16, 26 and 8 among the combinations described above. The healthy subjects serving as a negative cohort were discriminated using the discriminant formula based on this combination of the markers as trial validation to evaluate the discriminant performance. The obtained specificity is exemplified in Table 8 together with the existing marker. The discriminant formula based on this combination of the markers exhibited 81.8% sensitivity and 93.3% specificity against the benign ovarian tumor patient group. On the other hand, the existing marker CA-125 exhibited 90.9% sensitivity and 33.3% specificity against the benign ovarian tumor patient group. The discriminant formula based on this combination of the markers had the similar level of sensitivity as in CA-125, whereas it enables discrimination with a performance, a specificity against the benign ovarian tumor patient group, beyond the marker. Therefore, it was demonstrated that the markers of the present invention exert superior discriminant performance between ovarian cancer and benign ovarian tumor, though the existing marker is not sufficient for discriminating between ovarian cancer and benign ovarian tumor.

From the above, all the additional markers of SEQ ID NOs: 10 to 235 obtained in this Example are from newly found genes capable of discriminating between ovarian cancer patients from benign ovarian tumor patients with high performance when these are used in combination with the markers consisting of the sequences represented by SEQ ID NOs: 1 to 9. It was also demonstrated that a combination of a plurality of polynucleotides may obtain higher discriminant performance than that of a single polynucleotide.

[Table 6]

| SEQ ID NO. | AUC |
| --- | --- |
| 8_36 | 0.945 |
| 8_55 | 0.905 |
| 8_70 | 0.903 |
| 1_108 | 0.895 |
| 1_110 | 0.895 |
| 1_121 | 0.895 |
| 1_130 | 0.895 |
| 1_143 | 0.895 |
| 1_8 | 0.895 |
| 1_80 | 0.895 |

(continued)

| SEQ ID NO. | AUC |
|---|---|
| 8_177 | 0.895 |
| 8_65 | 0.895 |
| 8_170 | 0.893 |
| 8_64 | 0.890 |
| 1_166 | 0.888 |
| 1_87 | 0.888 |
| 8_80 | 0.888 |
| 2_63 | 0.885 |
| 3_13 | 0.885 |
| 3_8 | 0.885 |
| 8_193 | 0.885 |
| 8_53 | 0.885 |
| 8_67 | 0.885 |
| 4_8 | 0.883 |
| 8_81 | 0.883 |
| 1_59 | 0.880 |
| 4_110 | 0.880 |
| 8_13 | 0.880 |
| 8_35 | 0.880 |
| 8_66 | 0.880 |
| 1_37 | 0.878 |
| 3_161 | 0.878 |
| 8_126 | 0.878 |
| 8_32 | 0.878 |
| 8_75 | 0.878 |
| 1_101 | 0.875 |
| 1_103 | 0.875 |
| 1_105 | 0.875 |
| 1_106 | 0.875 |
| 1_109 | 0.875 |
| 1_112 | 0.875 |
| 1_122 | 0.875 |
| 1_124 | 0.875 |
| 1_13 | 0.875 |
| 1_133 | 0.875 |
| 1_144 | 0.875 |
| 1_151 | 0.875 |
| 1_152 | 0.875 |

(continued)

| SEQ ID NO. | AUC |
|---|---|
| 1_160 | 0.875 |
| 1_162 | 0.875 |
| 1_164 | 0.875 |
| 1_174 | 0.875 |
| 1_183 | 0.875 |
| 1_187 | 0.875 |
| 1_188 | 0.875 |
| 1_205 | 0.875 |
| 1_207 | 0.875 |
| 1_214 | 0.875 |
| 1_215 | 0.875 |
| 1_216 | 0.875 |
| 1_220 | 0.875 |
| 1_224 | 0.875 |
| 1_234 | 0.875 |
| 1_235 | 0.875 |
| 1_36 | 0.875 |
| 1_4 | 0.875 |
| 1_45 | 0.875 |
| 1_46 | 0.875 |
| 1_49 | 0.875 |
| 1_51 | 0.875 |
| 1_60 | 0.875 |
| 1_64 | 0.875 |
| 1_70 | 0.875 |
| 1_77 | 0.875 |
| 1_88 | 0.875 |
| 1_89 | 0.875 |
| 1_90 | 0.875 |
| 1_92 | 0.875 |
| 1_96 | 0.875 |
| 5_13 | 0.875 |
| 8_134 | 0.875 |
| 8_137 | 0.875 |
| 8_163 | 0.875 |
| 8_167 | 0.875 |
| 8_176 | 0.875 |
| 8_183 | 0.875 |

(continued)

| SEQ ID NO. | AUC |
|---|---|
| 8_212 | 0.875 |
| 8_46 | 0.875 |
| 2_33 | 0.872 |
| 7_63 | 0.872 |
| 8_110 | 0.872 |
| 8_115 | 0.872 |
| 8_24 | 0.872 |
| 8_68 | 0.872 |
| 8_71 | 0.872 |
| 8_93 | 0.872 |
| 1_154 | 0.870 |
| 1_20 | 0.870 |
| 4_53 | 0.870 |
| 7_187 | 0.870 |
| 8_136 | 0.870 |
| 8_186 | 0.870 |
| 8_34 | 0.870 |
| 1_10 | 0.868 |
| 1_145 | 0.868 |
| 1_161 | 0.868 |
| 1_171 | 0.868 |
| 1_193 | 0.868 |
| 1_102 | 0.865 |
| 1_118 | 0.865 |
| 1_142 | 0.865 |
| 1_146 | 0.865 |
| 1_158 | 0.865 |
| 1_163 | 0.865 |
| 1_165 | 0.865 |
| 1_167 | 0.865 |
| 1_18 | 0.865 |
| 1_181 | 0.865 |
| 1_182 | 0.865 |
| 1_185 | 0.865 |
| 1_19 | 0.865 |
| 1_192 | 0.865 |
| 1_200 | 0.865 |
| 1_208 | 0.865 |

(continued)

| SEQ ID NO. | AUC |
|---|---|
| 1_219 | 0.865 |
| 1_221 | 0.865 |
| 1_223 | 0.865 |
| 1_226 | 0.865 |
| 1_228 | 0.865 |
| 1_32 | 0.865 |
| 1_38 | 0.865 |
| 1_41 | 0.865 |
| 1_43 | 0.865 |
| 1_44 | 0.865 |
| 1_5 | 0.865 |
| 1_50 | 0.865 |
| 1_53 | 0.865 |
| 1_57 | 0.865 |
| 1_58 | 0.865 |
| 1_6 | 0.865 |
| 1_62 | 0.865 |
| 1_67 | 0.865 |
| 1_81 | 0.865 |
| 1_86 | 0.865 |
| 3_112 | 0.865 |
| 3_223 | 0.865 |
| 7_8 | 0.865 |
| 8_173 | 0.865 |
| 8_210 | 0.865 |
| 8_23 | 0.865 |
| 8_30 | 0.865 |
| 8_54 | 0.865 |
| 9_23 | 0.865 |
| 1_149 | 0.863 |
| 1_65 | 0.863 |
| 4_68 | 0.863 |
| 8_121 | 0.863 |
| 8_144 | 0.863 |
| 8_195 | 0.863 |
| 8_220 | 0.863 |
| 8_227 | 0.863 |
| 8_96 | 0.863 |

(continued)

| SEQ ID NO. | AUC |
|---|---|
| 1_140 | 0.860 |
| 1_189 | 0.860 |
| 3_172 | 0.860 |
| 3_210 | 0.860 |
| 3_235 | 0.860 |
| 6_37 | 0.860 |
| 8_10 | 0.860 |
| 8_142 | 0.860 |
| 8_214 | 0.860 |
| 1_159 | 0.857 |
| 1_210 | 0.857 |
| 3_154 | 0.857 |
| 3_19 | 0.857 |
| 4_26 | 0.857 |
| 7_34 | 0.857 |
| 7_36 | 0.857 |
| 7_81 | 0.857 |
| 8_78 | 0.857 |
| 1_111 | 0.855 |
| 1_113 | 0.855 |
| 1_114 | 0.855 |
| 1_116 | 0.855 |
| 1_117 | 0.855 |
| 1_119 | 0.855 |
| 1_12 | 0.855 |
| 1_123 | 0.855 |
| 1_127 | 0.855 |
| 1_134 | 0.855 |
| 1_137 | 0.855 |
| 1_139 | 0.855 |
| 1_141 | 0.855 |
| 1_150 | 0.855 |
| 1_155 | 0.855 |
| 1_156 | 0.855 |
| 1_16 | 0.855 |
| 1_169 | 0.855 |
| 1_170 | 0.855 |
| 1_172 | 0.855 |

(continued)

| SEQ ID NO. | AUC |
|---|---|
| 1_173 | 0.855 |
| 1_177 | 0.855 |
| 1_184 | 0.855 |
| 1_190 | 0.855 |
| 1_191 | 0.855 |
| 1_195 | 0.855 |
| 1_199 | 0.855 |
| 1_202 | 0.855 |
| 1_204 | 0.855 |
| 1_209 | 0.855 |
| 1_21 | 0.855 |
| 1_218 | 0.855 |
| 1_227 | 0.855 |
| 1_229 | 0.855 |
| 1_231 | 0.855 |
| 1_25 | 0.855 |
| 1_27 | 0.855 |
| 1_30 | 0.855 |
| 1_54 | 0.855 |
| 1_63 | 0.855 |
| 1_7 | 0.855 |
| 1_75 | 0.855 |
| 1_76 | 0.855 |
| 1_78 | 0.855 |
| 1_94 | 0.855 |
| 1_98 | 0.855 |
| 3_110 | 0.855 |
| 3_137 | 0.855 |
| 3_149 | 0.855 |
| 3_152 | 0.855 |
| 3_156 | 0.855 |
| 3_165 | 0.855 |
| 3_166 | 0.855 |
| 3_169 | 0.855 |
| 3_187 | 0.855 |
| 3_82 | 0.855 |
| 3_87 | 0.855 |
| 3_91 | 0.855 |

(continued)

| SEQ ID NO. | AUC |
| --- | --- |
| 3_98 | 0.855 |
| 4_13 | 0.855 |
| 4_130 | 0.855 |
| 4_16 | 0.855 |
| 7_161 | 0.855 |
| 7_231 | 0.855 |
| 8_12 | 0.855 |
| 8_14 | 0.855 |
| 8_145 | 0.855 |
| 8_19 | 0.855 |
| 8_228 | 0.855 |
| 8_231 | 0.855 |
| 8_92 | 0.855 |
| 1_148 | 0.853 |
| 1_217 | 0.853 |
| 1_33 | 0.853 |
| 3_10 | 0.853 |
| 3_148 | 0.853 |
| 3_37 | 0.853 |
| 6_8 | 0.853 |
| 8_122 | 0.853 |
| 8_160 | 0.853 |
| 8_194 | 0.853 |
| 8_61 | 0.853 |
| 8_94 | 0.853 |
| 1_168 | 0.850 |
| 1_179 | 0.850 |
| 1_2 | 0.850 |
| 1_211 | 0.850 |
| 1_222 | 0.850 |
| 1_225 | 0.850 |
| 1_69 | 0.850 |
| 1_79 | 0.850 |
| 1_82 | 0.850 |
| 1_97 | 0.850 |
| 1_99 | 0.850 |
| 3_51 | 0.850 |
| 4_103 | 0.850 |

(continued)

| SEQ ID NO. | AUC |
|---|---|
| 4_137 | 0.850 |
| 4_190 | 0.850 |
| 4_218 | 0.850 |
| 4_32 | 0.850 |
| 4_96 | 0.850 |
| 8_107 | 0.850 |
| 8_130 | 0.850 |
| 8_16 | 0.850 |
| 8_31 | 0.850 |
| 8_38 | 0.850 |
| 8_82 | 0.850 |
| 9_41 | 0.850 |
| 1_180 | 0.848 |
| 1_11 | 0.847 |
| 1_198 | 0.847 |
| 1_213 | 0.847 |
| 1_66 | 0.847 |
| 3_168 | 0.847 |
| 4_207 | 0.847 |
| 4_30 | 0.847 |
| 4_94 | 0.847 |
| 6_11 | 0.847 |
| 6_130 | 0.847 |
| 6_53 | 0.847 |
| 7_32 | 0.847 |
| 8_15 | 0.847 |
| 1_100 | 0.845 |
| 1_104 | 0.845 |
| 1_126 | 0.845 |
| 1_128 | 0.845 |
| 1_131 | 0.845 |
| 1_132 | 0.845 |
| 1_15 | 0.845 |
| 1_175 | 0.845 |
| 1_176 | 0.845 |
| 1_197 | 0.845 |
| 1_233 | 0.845 |
| 1_24 | 0.845 |

(continued)

| SEQ ID NO. | AUC |
|---|---|
| 1_35 | 0.845 |
| 1_61 | 0.845 |
| 1_68 | 0.845 |
| 1_74 | 0.845 |
| 1_9 | 0.845 |
| 3_100 | 0.845 |
| 3_102 | 0.845 |
| 3_116 | 0.845 |
| 3_121 | 0.845 |
| 3_122 | 0.845 |
| 3_130 | 0.845 |
| 3_131 | 0.845 |
| 3_150 | 0.845 |
| 3_158 | 0.845 |
| 3_160 | 0.845 |
| 3_176 | 0.845 |
| 3_177 | 0.845 |
| 3_188 | 0.845 |
| 3_190 | 0.845 |
| 3_200 | 0.845 |
| 3_214 | 0.845 |
| 3_220 | 0.845 |
| 3_32 | 0.845 |
| 3_34 | 0.845 |
| 3_50 | 0.845 |
| 3_53 | 0.845 |
| 3_63 | 0.845 |
| 3_7 | 0.845 |
| 3_75 | 0.845 |
| 3_79 | 0.845 |
| 3_90 | 0.845 |
| 3_94 | 0.845 |
| 4_108 | 0.845 |
| 4_203 | 0.845 |
| 4_80 | 0.845 |
| 6_108 | 0.845 |
| 7_139 | 0.845 |
| 7_196 | 0.845 |

(continued)

| SEQ ID NO. | AUC |
|---|---|
| 7_55 | 0.845 |
| 8_108 | 0.845 |
| 8_11 | 0.845 |
| 8_114 | 0.845 |
| 8_169 | 0.845 |
| 8_26 | 0.845 |
| 8_45 | 0.845 |
| 3_36 | 0.843 |
| 3_81 | 0.843 |
| 4_10 | 0.843 |
| 4_109 | 0.843 |
| 5_20 | 0.843 |
| 6_146 | 0.843 |
| 6_207 | 0.843 |
| 6_36 | 0.843 |
| 6_89 | 0.843 |
| 7_27 | 0.843 |
| 8_25 | 0.843 |
| 8_43 | 0.843 |
| 8_50 | 0.843 |
| 8_79 | 0.843 |
| 1_120 | 0.840 |
| 1_136 | 0.840 |
| 1_14 | 0.840 |
| 1_194 | 0.840 |
| 1_203 | 0.840 |
| 1_22 | 0.840 |
| 1_230 | 0.840 |
| 1_34 | 0.840 |
| 1_40 | 0.840 |
| 1_71 | 0.840 |
| 1_85 | 0.840 |
| 2_108 | 0.840 |
| 2_137 | 0.840 |
| 2_140 | 0.840 |
| 2_196 | 0.840 |
| 2_3 | 0.840 |
| 2_70 | 0.840 |

(continued)

| SEQ ID NO. | AUC |
|---|---|
| 2_80 | 0.840 |
| 3_14 | 0.840 |
| 3_16 | 0.840 |
| 3_22 | 0.840 |
| 3_31 | 0.840 |
| 3_97 | 0.840 |
| 4_165 | 0.840 |
| 4_63 | 0.840 |
| 5_203 | 0.840 |
| 5_70 | 0.840 |
| 6_128 | 0.840 |
| 6_13 | 0.840 |
| 6_135 | 0.840 |
| 6_217 | 0.840 |
| 6_51 | 0.840 |
| 7_130 | 0.840 |
| 7_46 | 0.840 |
| 8_103 | 0.840 |
| 8_129 | 0.840 |
| 8_199 | 0.840 |
| 8_20 | 0.840 |
| 8_216 | 0.840 |
| 8_234 | 0.840 |
| 8_39 | 0.840 |
| 8_56 | 0.840 |
| 2_207 | 0.838 |
| 4_199 | 0.838 |
| 4_45 | 0.838 |
| 4_7 | 0.838 |
| 4_79 | 0.838 |
| 6_121 | 0.838 |
| 6_122 | 0.838 |
| 6_75 | 0.838 |
| 6_80 | 0.838 |
| 8_143 | 0.838 |
| 8_151 | 0.838 |
| 8_161 | 0.838 |
| 8_185 | 0.838 |

(continued)

| SEQ ID NO. | AUC |
|---|---|
| 8_200 | 0.838 |
| 8_63 | 0.838 |
| 1_107 | 0.835 |
| 1_125 | 0.835 |
| 1_196 | 0.835 |
| 1_23 | 0.835 |
| 1_232 | 0.835 |
| 1_29 | 0.835 |
| 1_3 | 0.835 |
| 1_39 | 0.835 |
| 1_55 | 0.835 |
| 1_95 | 0.835 |
| 3_118 | 0.835 |
| 3_119 | 0.835 |
| 3_123 | 0.835 |
| 3_124 | 0.835 |
| 3_125 | 0.835 |
| 3_128 | 0.835 |
| 3_138 | 0.835 |
| 3_139 | 0.835 |
| 3_141 | 0.835 |
| 3_145 | 0.835 |
| 3_163 | 0.835 |
| 3_167 | 0.835 |
| 3_171 | 0.835 |
| 3_173 | 0.835 |
| 3_175 | 0.835 |
| 3_183 | 0.835 |
| 3_195 | 0.835 |
| 3_203 | 0.835 |
| 3_209 | 0.835 |
| 3_219 | 0.835 |
| 3_227 | 0.835 |
| 3_234 | 0.835 |
| 3_30 | 0.835 |
| 3_38 | 0.835 |
| 3_39 | 0.835 |
| 3_4 | 0.835 |

(continued)

| SEQ ID NO. | AUC |
|---|---|
| 3_41 | 0.835 |
| 3_55 | 0.835 |
| 3_58 | 0.835 |
| 3_65 | 0.835 |
| 3_68 | 0.835 |
| 3_69 | 0.835 |
| 3_74 | 0.835 |
| 3_78 | 0.835 |
| 3_80 | 0.835 |
| 3_83 | 0.835 |
| 3_88 | 0.835 |
| 3_96 | 0.835 |
| 4_105 | 0.835 |
| 4_12 | 0.835 |
| 4_15 | 0.835 |
| 4_182 | 0.835 |
| 4_193 | 0.835 |
| 4_196 | 0.835 |
| 4_40 | 0.835 |
| 4_49 | 0.835 |
| 4_87 | 0.835 |
| 5_17 | 0.835 |
| 6_190 | 0.835 |
| 6_25 | 0.835 |
| 7_201 | 0.835 |
| 8_113 | 0.835 |
| 8_135 | 0.835 |
| 8_205 | 0.835 |
| 8_215 | 0.835 |
| 8_57 | 0.835 |
| 8_9 | 0.835 |
| 9_110 | 0.835 |
| 9_121 | 0.835 |
| 9_163 | 0.835 |
| 9_176 | 0.835 |
| 9_24 | 0.835 |
| 9_37 | 0.835 |
| 9_80 | 0.835 |

(continued)

| SEQ ID NO. | AUC |
|---|---|
| 3_133 | 0.832 |
| 3_191 | 0.832 |
| 3_226 | 0.832 |
| 4_22 | 0.832 |
| 4_51 | 0.832 |
| 6_148 | 0.832 |
| 6_155 | 0.832 |
| 6_212 | 0.832 |
| 6_81 | 0.832 |
| 6_87 | 0.832 |
| 8_188 | 0.832 |
| 8_213 | 0.832 |
| 8_37 | 0.832 |
| 8_51 | 0.832 |
| 8_76 | 0.832 |
| 1_129 | 0.830 |
| 1_138 | 0.830 |
| 1_147 | 0.830 |
| 1_28 | 0.830 |
| 1_31 | 0.830 |
| 1_56 | 0.830 |
| 1_83 | 0.830 |
| 2_12 | 0.830 |
| 2_125 | 0.830 |
| 2_21 | 0.830 |
| 2_25 | 0.830 |
| 2_32 | 0.830 |
| 2_39 | 0.830 |
| 2_53 | 0.830 |
| 2_8 | 0.830 |
| 3_108 | 0.830 |
| 3_174 | 0.830 |
| 3_181 | 0.830 |
| 3_207 | 0.830 |
| 3_231 | 0.830 |
| 3_24 | 0.830 |
| 3_42 | 0.830 |
| 4_11 | 0.830 |

(continued)

| SEQ ID NO. | AUC |
|---|---|
| 4_144 | 0.830 |
| 4_171 | 0.830 |
| 4_220 | 0.830 |
| 4_43 | 0.830 |
| 4_55 | 0.830 |
| 5_107 | 0.830 |
| 5_24 | 0.830 |
| 5_34 | 0.830 |
| 5_96 | 0.830 |
| 6_152 | 0.830 |
| 6_16 | 0.830 |
| 6_188 | 0.830 |
| 6_35 | 0.830 |
| 7_151 | 0.830 |
| 7_186 | 0.830 |
| 7_22 | 0.830 |
| 7_96 | 0.830 |
| 8_128 | 0.830 |
| 8_155 | 0.830 |
| 8_162 | 0.830 |
| 8_164 | 0.830 |
| 8_209 | 0.830 |
| 8_48 | 0.830 |
| 8_90 | 0.830 |
| 8_97 | 0.830 |
| 1_26 | 0.828 |
| 2_37 | 0.828 |
| 3_33 | 0.828 |
| 3_71 | 0.828 |
| 4_184 | 0.828 |
| 4_212 | 0.828 |
| 4_235 | 0.828 |
| 4_36 | 0.828 |
| 5_57 | 0.828 |
| 5_59 | 0.828 |
| 5_65 | 0.828 |
| 5_81 | 0.828 |
| 5_82 | 0.828 |

(continued)

| SEQ ID NO. | AUC |
|---|---|
| 6_110 | 0.828 |
| 7_65 | 0.828 |
| 8_100 | 0.828 |
| 8_131 | 0.828 |
| 8_166 | 0.828 |
| 8_217 | 0.828 |
| 8_49 | 0.828 |
| 9_17 | 0.828 |
| 9_18 | 0.828 |
| 1_157 | 0.825 |
| 1_186 | 0.825 |
| 1_201 | 0.825 |
| 1_206 | 0.825 |
| 1_42 | 0.825 |
| 1_73 | 0.825 |
| 1_93 | 0.825 |
| 2_159 | 0.825 |
| 2_22 | 0.825 |
| 2_34 | 0.825 |
| 2_5 | 0.825 |
| 3_105 | 0.825 |
| 3_202 | 0.825 |
| 3_221 | 0.825 |
| 3_229 | 0.825 |
| 3_43 | 0.825 |
| 3_44 | 0.825 |
| 3_73 | 0.825 |
| 3_92 | 0.825 |
| 3_93 | 0.825 |
| 4_132 | 0.825 |
| 4_139 | 0.825 |
| 4_173 | 0.825 |
| 4_176 | 0.825 |
| 4_187 | 0.825 |
| 4_19 | 0.825 |
| 4_200 | 0.825 |
| 4_217 | 0.825 |
| 4_232 | 0.825 |

(continued)

| SEQ ID NO. | AUC |
|---|---|
| 4_24 | 0.825 |
| 4_34 | 0.825 |
| 4_38 | 0.825 |
| 4_56 | 0.825 |
| 4_61 | 0.825 |
| 4_64 | 0.825 |
| 4_69 | 0.825 |
| 4_82 | 0.825 |
| 4_88 | 0.825 |
| 5_14 | 0.825 |
| 5_66 | 0.825 |
| 6_199 | 0.825 |
| 6_55 | 0.825 |
| 6_7 | 0.825 |
| 7_175 | 0.825 |
| 7_202 | 0.825 |
| 7_219 | 0.825 |
| 7_24 | 0.825 |
| 7_43 | 0.825 |
| 7_53 | 0.825 |
| 7_56 | 0.825 |
| 7_57 | 0.825 |
| 8_124 | 0.825 |
| 8_148 | 0.825 |
| 8_208 | 0.825 |
| 8_21 | 0.825 |
| 8_225 | 0.825 |
| 8_59 | 0.825 |
| 9_167 | 0.825 |
| 9_27 | 0.825 |
| 9_51 | 0.825 |
| 1_115 | 0.823 |
| 1_212 | 0.823 |
| 2_11 | 0.823 |
| 2_231 | 0.823 |
| 3_143 | 0.823 |
| 4_149 | 0.823 |
| 4_191 | 0.823 |

(continued)

| SEQ ID NO. | AUC |
|---|---|
| 4_83 | 0.823 |
| 5_109 | 0.823 |
| 6_101 | 0.823 |
| 6_186 | 0.823 |
| 6_94 | 0.823 |
| 8_138 | 0.823 |
| 8_29 | 0.823 |
| 8_40 | 0.823 |
| 8_47 | 0.823 |
| 8_74 | 0.823 |
| 8_87 | 0.823 |
| 1_135 | 0.820 |
| 1_17 | 0.820 |
| 1_91 | 0.820 |
| 2_109 | 0.820 |
| 2_126 | 0.820 |
| 2_142 | 0.820 |
| 2_190 | 0.820 |
| 2_38 | 0.820 |
| 2_64 | 0.820 |
| 2_7 | 0.820 |
| 2_9 | 0.820 |
| 2_96 | 0.820 |
| 3_103 | 0.820 |
| 3_106 | 0.820 |
| 3_114 | 0.820 |
| 3_120 | 0.820 |
| 3_129 | 0.820 |
| 3_135 | 0.820 |
| 3_136 | 0.820 |
| 3_146 | 0.820 |
| 3_170 | 0.820 |
| 3_18 | 0.820 |
| 3_184 | 0.820 |
| 3_193 | 0.820 |
| 3_196 | 0.820 |
| 3_199 | 0.820 |
| 3_222 | 0.820 |

(continued)

| SEQ ID NO. | AUC |
|---|---|
| 3_225 | 0.820 |
| 3_46 | 0.820 |
| 3_60 | 0.820 |
| 3_66 | 0.820 |
| 3_76 | 0.820 |
| 3_89 | 0.820 |
| 3_9 | 0.820 |
| 4_59 | 0.820 |
| 5_12 | 0.820 |
| 5_19 | 0.820 |
| 6_156 | 0.820 |
| 6_180 | 0.820 |
| 6_77 | 0.820 |
| 7_122 | 0.820 |
| 7_155 | 0.820 |
| 7_167 | 0.820 |
| 7_176 | 0.820 |
| 7_181 | 0.820 |
| 7_204 | 0.820 |
| 7_212 | 0.820 |
| 7_220 | 0.820 |
| 7_232 | 0.820 |
| 7_76 | 0.820 |
| 7_80 | 0.820 |
| 7_85 | 0.820 |
| 7_92 | 0.820 |
| 8_141 | 0.820 |
| 8_171 | 0.820 |
| 8_196 | 0.820 |
| 8_58 | 0.820 |
| 8_95 | 0.820 |
| 9_14 | 0.820 |
| 2_13 | 0.818 |
| 3_217 | 0.818 |
| 5_15 | 0.818 |
| 5_77 | 0.818 |
| 5_9 | 0.818 |
| 6_103 | 0.818 |

(continued)

| SEQ ID NO. | AUC |
|---|---|
| 6_106 | 0.818 |
| 6_117 | 0.818 |
| 6_149 | 0.818 |
| 6_163 | 0.818 |
| 6_177 | 0.818 |
| 6_196 | 0.818 |
| 6_208 | 0.818 |
| 6_220 | 0.818 |
| 6_233 | 0.818 |
| 6_50 | 0.818 |
| 6_69 | 0.818 |
| 6_70 | 0.818 |
| 6_92 | 0.818 |
| 7_37 | 0.818 |
| 8_139 | 0.818 |
| 8_165 | 0.818 |
| 1_84 | 0.815 |
| 2_110 | 0.815 |
| 2_117 | 0.815 |
| 2_130 | 0.815 |
| 2_135 | 0.815 |
| 2_24 | 0.815 |
| 2_56 | 0.815 |
| 2_59 | 0.815 |
| 2_77 | 0.815 |
| 3_101 | 0.815 |
| 3_111 | 0.815 |
| 3_155 | 0.815 |
| 3_180 | 0.815 |
| 3_197 | 0.815 |
| 3_198 | 0.815 |
| 3_201 | 0.815 |
| 3_216 | 0.815 |
| 3_40 | 0.815 |
| 3_5 | 0.815 |
| 3_59 | 0.815 |
| 3_6 | 0.815 |
| 3_64 | 0.815 |

(continued)

| SEQ ID NO. | AUC |
|---|---|
| 3_95 | 0.815 |
| 4_122 | 0.815 |
| 4_145 | 0.815 |
| 4_166 | 0.815 |
| 4_177 | 0.815 |
| 4_198 | 0.815 |
| 4_231 | 0.815 |
| 4_234 | 0.815 |
| 4_29 | 0.815 |
| 4_41 | 0.815 |
| 4_65 | 0.815 |
| 4_71 | 0.815 |
| 4_73 | 0.815 |
| 5_105 | 0.815 |
| 5_139 | 0.815 |
| 5_23 | 0.815 |
| 5_47 | 0.815 |
| 5_49 | 0.815 |
| 5_51 | 0.815 |
| 5_53 | 0.815 |
| 5_56 | 0.815 |
| 5_8 | 0.815 |
| 6_140 | 0.815 |
| 7_109 | 0.815 |
| 7_110 | 0.815 |
| 7_144 | 0.815 |
| 7_213 | 0.815 |
| 7_89 | 0.815 |
| 7_99 | 0.815 |
| 8_106 | 0.815 |
| 8_112 | 0.815 |
| 8_154 | 0.815 |
| 8_191 | 0.815 |
| 8_203 | 0.815 |
| 8_204 | 0.815 |
| 8_229 | 0.815 |
| 8_27 | 0.815 |
| 8_33 | 0.815 |

(continued)

| SEQ ID NO. | AUC |
|---|---|
| 8_44 | 0.815 |
| 8_60 | 0.815 |
| 9_130 | 0.815 |
| 9_156 | 0.815 |
| 9_160 | 0.815 |
| 9_177 | 0.815 |
| 9_183 | 0.815 |
| 9_200 | 0.815 |
| 9_220 | 0.815 |
| 9_223 | 0.815 |
| 9_87 | 0.815 |
| 9_92 | 0.815 |
| 9_99 | 0.815 |
| 4_100 | 0.813 |
| 4_140 | 0.813 |
| 4_186 | 0.813 |
| 4_208 | 0.813 |
| 4_50 | 0.813 |
| 4_77 | 0.813 |
| 4_84 | 0.813 |
| 4_91 | 0.813 |
| 5_137 | 0.813 |
| 5_22 | 0.813 |
| 5_220 | 0.813 |
| 5_227 | 0.813 |
| 5_54 | 0.813 |
| 5_79 | 0.813 |
| 6_109 | 0.813 |
| 6_160 | 0.813 |
| 6_166 | 0.813 |
| 6_197 | 0.813 |
| 6_198 | 0.813 |
| 6_201 | 0.813 |
| 7_12 | 0.813 |
| 7_31 | 0.813 |
| 8_152 | 0.813 |
| 8_179 | 0.813 |
| 3_126 | 0.810 |

(continued)

| SEQ ID NO. | AUC |
|---|---|
| 3_189 | 0.810 |
| 1_178 | 0.810 |
| 1_52 | 0.810 |
| 1_72 | 0.810 |
| 2_150 | 0.810 |
| 2_154 | 0.810 |
| 2_161 | 0.810 |
| 2_19 | 0.810 |
| 2_4 | 0.810 |
| 2_74 | 0.810 |
| 2_75 | 0.810 |
| 3_115 | 0.810 |
| 3_117 | 0.810 |
| 3_132 | 0.810 |
| 3_142 | 0.810 |
| 3_144 | 0.810 |
| 3_147 | 0.810 |
| 3_153 | 0.810 |
| 3_164 | 0.810 |
| 3_17 | 0.810 |
| 3_178 | 0.810 |
| 3_208 | 0.810 |
| 3_215 | 0.810 |
| 3_224 | 0.810 |
| 3_233 | 0.810 |
| 3_26 | 0.810 |
| 3_35 | 0.810 |
| 3_45 | 0.810 |
| 3_56 | 0.810 |
| 3_57 | 0.810 |
| 3_61 | 0.810 |
| 3_77 | 0.810 |
| 4_116 | 0.810 |
| 4_119 | 0.810 |
| 4_121 | 0.810 |
| 4_123 | 0.810 |
| 4_151 | 0.810 |
| 4_155 | 0.810 |

(continued)

| SEQ ID NO. | AUC |
|---|---|
| 4_168 | 0.810 |
| 4_175 | 0.810 |
| 4_179 | 0.810 |
| 4_181 | 0.810 |
| 4_185 | 0.810 |
| 4_9 | 0.810 |
| 4_97 | 0.810 |
| 5_142 | 0.810 |
| 5_18 | 0.810 |
| 6_114 | 0.810 |
| 6_119 | 0.810 |
| 6_125 | 0.810 |
| 6_165 | 0.810 |
| 6_195 | 0.810 |
| 6_20 | 0.810 |
| 6_210 | 0.810 |
| 6_54 | 0.810 |
| 6_9 | 0.810 |
| 7_105 | 0.810 |
| 7_115 | 0.810 |
| 7_116 | 0.810 |
| 7_121 | 0.810 |
| 7_124 | 0.810 |
| 7_125 | 0.810 |
| 7_129 | 0.810 |
| 7_132 | 0.810 |
| 7_138 | 0.810 |
| 7_142 | 0.810 |
| 7_154 | 0.810 |
| 7_157 | 0.810 |
| 7_165 | 0.810 |
| 7_169 | 0.810 |
| 7_177 | 0.810 |
| 7_206 | 0.810 |
| 7_209 | 0.810 |
| 7_211 | 0.810 |
| 7_214 | 0.810 |
| 7_218 | 0.810 |

(continued)

| SEQ ID NO. | AUC |
|---|---|
| 7_234 | 0.810 |
| 7_33 | 0.810 |
| 7_61 | 0.810 |
| 7_74 | 0.810 |
| 7_91 | 0.810 |
| 7_95 | 0.810 |
| 8_105 | 0.810 |
| 8_226 | 0.810 |
| 8_88 | 0.810 |
| 9_46 | 0.810 |
| 2_148 | 0.807 |
| 2_15 | 0.807 |
| 3_20 | 0.807 |
| 3_25 | 0.807 |
| 5_169 | 0.807 |
| 5_208 | 0.807 |
| 5_25 | 0.807 |
| 5_32 | 0.807 |
| 6_124 | 0.807 |
| 6_132 | 0.807 |
| 6_141 | 0.807 |
| 6_142 | 0.807 |
| 6_154 | 0.807 |
| 6_167 | 0.807 |
| 6_176 | 0.807 |
| 6_181 | 0.807 |
| 6_193 | 0.807 |
| 6_211 | 0.807 |
| 6_214 | 0.807 |
| 6_226 | 0.807 |
| 6_31 | 0.807 |
| 6_56 | 0.807 |
| 6_63 | 0.807 |
| 7_100 | 0.807 |
| 7_103 | 0.807 |
| 7_182 | 0.807 |
| 8_104 | 0.807 |
| 8_156 | 0.807 |

(continued)

| SEQ ID NO. | AUC |
|---|---|
| 8_190 | 0.807 |
| 8_202 | 0.807 |
| 8_223 | 0.807 |
| 9_13 | 0.807 |
| 2_103 | 0.805 |
| 2_116 | 0.805 |
| 2_119 | 0.805 |
| 2_121 | 0.805 |
| 2_122 | 0.805 |
| 2_124 | 0.805 |
| 2_146 | 0.805 |
| 2_152 | 0.805 |
| 2_156 | 0.805 |
| 2_157 | 0.805 |
| 2_158 | 0.805 |
| 2_165 | 0.805 |
| 2_167 | 0.805 |
| 2_173 | 0.805 |
| 2_175 | 0.805 |
| 2_176 | 0.805 |
| 2_177 | 0.805 |
| 2_182 | 0.805 |
| 2_185 | 0.805 |
| 2_186 | 0.805 |
| 2_187 | 0.805 |
| 2_192 | 0.805 |
| 2_200 | 0.805 |
| 2_201 | 0.805 |
| 2_202 | 0.805 |
| 2_208 | 0.805 |
| 2_226 | 0.805 |
| 2_228 | 0.805 |
| 2_41 | 0.805 |
| 2_46 | 0.805 |
| 2_49 | 0.805 |
| 2_50 | 0.805 |
| 2_6 | 0.805 |
| 2_65 | 0.805 |

(continued)

| SEQ ID NO. | AUC |
|---|---|
| 2_66 | 0.805 |
| 2_82 | 0.805 |
| 2_86 | 0.805 |
| 2_92 | 0.805 |
| 2_94 | 0.805 |
| 2_99 | 0.805 |
| 3_109 | 0.805 |
| 3_192 | 0.805 |
| 3_205 | 0.805 |
| 3_48 | 0.805 |
| 4_147 | 0.805 |
| 4_161 | 0.805 |
| 4_167 | 0.805 |
| 4_189 | 0.805 |
| 4_206 | 0.805 |
| 4_213 | 0.805 |
| 4_46 | 0.805 |
| 4_75 | 0.805 |
| 5_136 | 0.805 |
| 5_16 | 0.805 |
| 5_191 | 0.805 |
| 5_36 | 0.805 |
| 5_6 | 0.805 |
| 5_64 | 0.805 |
| 5_87 | 0.805 |
| 6_182 | 0.805 |
| 6_59 | 0.805 |
| 6_74 | 0.805 |
| 7_107 | 0.805 |
| 7_136 | 0.805 |
| 7_164 | 0.805 |
| 7_191 | 0.805 |
| 7_199 | 0.805 |
| 7_35 | 0.805 |
| 7_41 | 0.805 |
| 7_47 | 0.805 |
| 8_119 | 0.805 |
| 8_132 | 0.805 |

(continued)

| SEQ ID NO. | AUC |
|---|---|
| 8_184 | 0.805 |
| 8_189 | 0.805 |
| 8_218 | 0.805 |
| 8_41 | 0.805 |
| 9_114 | 0.805 |
| 9_161 | 0.805 |
| 9_170 | 0.805 |
| 9_178 | 0.805 |
| 9_193 | 0.805 |
| 9_198 | 0.805 |
| 9_205 | 0.805 |
| 9_214 | 0.805 |
| 9_22 | 0.805 |
| 9_221 | 0.805 |
| 9_222 | 0.805 |
| 9_38 | 0.805 |
| 9_53 | 0.805 |
| 2_131 | 0.803 |
| 4_120 | 0.803 |
| 4_124 | 0.803 |
| 4_133 | 0.803 |
| 4_162 | 0.803 |
| 4_20 | 0.803 |
| 4_216 | 0.803 |
| 4_223 | 0.803 |
| 4_226 | 0.803 |
| 4_57 | 0.803 |
| 4_66 | 0.803 |
| 4_81 | 0.803 |
| 4_98 | 0.803 |
| 5_103 | 0.803 |
| 5_110 | 0.803 |
| 5_115 | 0.803 |
| 5_118 | 0.803 |
| 5_121 | 0.803 |
| 5_125 | 0.803 |
| 5_129 | 0.803 |
| 5_130 | 0.803 |

(continued)

| SEQ ID NO. | AUC |
|---|---|
| 5_131 | 0.803 |
| 5_149 | 0.803 |
| 5_156 | 0.803 |
| 5_158 | 0.803 |
| 5_163 | 0.803 |
| 5_165 | 0.803 |
| 5_166 | 0.803 |
| 5_167 | 0.803 |
| 5_168 | 0.803 |
| 5_170 | 0.803 |
| 5_174 | 0.803 |
| 5_176 | 0.803 |
| 5_177 | 0.803 |
| 5_179 | 0.803 |
| 5_181 | 0.803 |
| 5_184 | 0.803 |
| 5_187 | 0.803 |
| 5_194 | 0.803 |
| 5_199 | 0.803 |
| 5_200 | 0.803 |
| 5_202 | 0.803 |
| 5_204 | 0.803 |
| 5_21 | 0.803 |
| 5_214 | 0.803 |
| 5_226 | 0.803 |
| 5_228 | 0.803 |
| 5_231 | 0.803 |
| 5_234 | 0.803 |
| 5_41 | 0.803 |
| 5_44 | 0.803 |
| 5_48 | 0.803 |
| 5_60 | 0.803 |
| 5_61 | 0.803 |
| 5_69 | 0.803 |
| 5_74 | 0.803 |
| 5_76 | 0.803 |
| 5_80 | 0.803 |
| 5_93 | 0.803 |

(continued)

| SEQ ID NO. | AUC |
|---|---|
| 5_98 | 0.803 |
| 6_147 | 0.803 |
| 6_15 | 0.803 |
| 6_174 | 0.803 |
| 7_18 | 0.803 |
| 7_30 | 0.803 |
| 8_149 | 0.803 |
| 8_159 | 0.803 |
| 8_172 | 0.803 |
| 8_222 | 0.803 |
| 8_91 | 0.803 |
| 9_30 | 0.803 |
| 9_36 | 0.803 |
| 2_171 | 0.800 |
| 2_26 | 0.800 |
| 2_43 | 0.800 |
| 3_151 | 0.800 |
| 3_157 | 0.800 |
| 3_182 | 0.800 |
| 3_194 | 0.800 |
| 3_204 | 0.800 |
| 3_206 | 0.800 |
| 3_213 | 0.800 |
| 3_23 | 0.800 |
| 3_29 | 0.800 |
| 3_54 | 0.800 |
| 3_62 | 0.800 |
| 3_99 | 0.800 |
| 4_101 | 0.800 |
| 4_104 | 0.800 |
| 4_106 | 0.800 |
| 4_107 | 0.800 |
| 4_112 | 0.800 |
| 4_117 | 0.800 |
| 4_125 | 0.800 |
| 4_131 | 0.800 |
| 4_136 | 0.800 |
| 4_14 | 0.800 |

(continued)

| SEQ ID NO. | AUC |
|---|---|
| 4_156 | 0.800 |
| 4_157 | 0.800 |
| 4_169 | 0.800 |
| 4_174 | 0.800 |
| 4_201 | 0.800 |
| 4_202 | 0.800 |
| 4_214 | 0.800 |
| 4_219 | 0.800 |
| 4_221 | 0.800 |
| 4_228 | 0.800 |
| 4_229 | 0.800 |
| 4_25 | 0.800 |
| 4_89 | 0.800 |
| 4_99 | 0.800 |
| 6_116 | 0.800 |
| 6_131 | 0.800 |
| 6_161 | 0.800 |
| 6_185 | 0.800 |
| 6_23 | 0.800 |
| 6_32 | 0.800 |
| 6_38 | 0.800 |
| 6_44 | 0.800 |
| 6_47 | 0.800 |
| 6_97 | 0.800 |
| 7_10 | 0.800 |
| 7_101 | 0.800 |
| 7_117 | 0.800 |
| 7_119 | 0.800 |
| 7_123 | 0.800 |
| 7_131 | 0.800 |
| 7_134 | 0.800 |
| 7_145 | 0.800 |
| 7_158 | 0.800 |
| 7_163 | 0.800 |
| 7_171 | 0.800 |
| 7_173 | 0.800 |
| 7_183 | 0.800 |
| 7_194 | 0.800 |

(continued)

| SEQ ID NO. | AUC |
|---|---|
| 7_198 | 0.800 |
| 7_222 | 0.800 |
| 7_226 | 0.800 |
| 7_42 | 0.800 |
| 7_49 | 0.800 |
| 7_50 | 0.800 |
| 7_51 | 0.800 |
| 7_66 | 0.800 |
| 7_78 | 0.800 |
| 7_83 | 0.800 |
| 8_111 | 0.800 |
| 8_158 | 0.800 |
| 8_168 | 0.800 |
| 8_224 | 0.800 |
| 8_28 | 0.800 |
| 8_69 | 0.800 |
| 8_83 | 0.800 |
| 9_12 | 0.800 |
| 9_57 | 0.800 |

[Table 7]

| SEQ ID NO. | The number of markers | AUC |
|---|---|---|
| 1_11_16_7 | 4 | 0.960 |
| 1_11_16_26_8 | 5 | 0.970 |
| 1_2_11_16_8 | 5 | 0.968 |
| 1_11_16_18_7 | 5 | 0.960 |
| 1_11_16_8_29 | 5 | 0.955 |
| 1_12_13_16_8 | 5 | 0.955 |
| 1_11_16_7_26 | 5 | 0.950 |
| 1_10_11_16_7 | 5 | 0.950 |

[Table 8]

| SEQ ID NO. | AUC |
|---|---|
| 8_52 | 0.993 |
| 84_22 | 0.950 |
| 46_72 | 0.943 |
| 159_8 | 0.943 |

(continued)

| SEQ ID NO. | AUC |
|------------|-------|
| 8_187 | 0.938 |
| 22_72 | 0.938 |
| 52_22 | 0.937 |
| 56_22 | 0.935 |
| 234_72 | 0.935 |
| 8_84 | 0.933 |
| 157_22 | 0.930 |
| 46_33 | 0.927 |
| 64_135 | 0.923 |
| 234_33 | 0.923 |
| 64_85 | 0.922 |
| 209_72 | 0.922 |
| 184_22 | 0.922 |
| 46_52 | 0.920 |
| 46_159 | 0.920 |
| 234_52 | 0.920 |
| 222_22 | 0.918 |
| 230_8 | 0.918 |
| 231_22 | 0.917 |
| 84_15 | 0.915 |
| 75_22 | 0.913 |
| 8_72 | 0.913 |
| 213_22 | 0.913 |
| 22_74 | 0.913 |
| 52_87 | 0.912 |
| 150_8 | 0.912 |
| 22_232 | 0.912 |
| 46_84 | 0.912 |
| 33_22 | 0.910 |
| 52_26 | 0.910 |
| 117_22 | 0.910 |
| 84_53 | 0.910 |
| 7_52 | 0.910 |
| 209_52 | 0.908 |
| 22_15 | 0.908 |
| 64_84 | 0.908 |
| 19_52 | 0.907 |
| 202_85 | 0.907 |

(continued)

| SEQ ID NO. | AUC |
|---|---|
| 88_72 | 0.907 |
| 159_22 | 0.907 |
| 90_22 | 0.907 |
| 187_22 | 0.907 |
| 20_52 | 0.905 |
| 64_22 | 0.905 |
| 209_84 | 0.905 |
| 230_22 | 0.903 |
| 234_159 | 0.903 |
| 219_22 | 0.903 |
| 63_22 | 0.903 |
| 53_72 | 0.903 |
| 46_150 | 0.903 |
| 44_84 | 0.903 |
| 13_84 | 0.902 |
| 203_22 | 0.902 |
| 72_70 | 0.902 |
| 102_8 | 0.902 |
| 22_206 | 0.902 |
| 234_186 | 0.900 |
| 22_147 | 0.900 |
| 196_22 | 0.900 |
| 182_22 | 0.900 |
| 22_191 | 0.898 |
| 56_8 | 0.898 |
| 37_84 | 0.898 |
| 31_72 | 0.897 |
| 84_33 | 0.897 |
| 105_72 | 0.897 |
| 22_165 | 0.897 |
| 68_22 | 0.897 |
| 84_16 | 0.895 |
| 175_22 | 0.895 |
| 20_72 | 0.893 |
| 234_232 | 0.893 |
| 78_84 | 0.893 |
| 71_22 | 0.893 |
| 150_22 | 0.893 |

(continued)

| SEQ ID NO. | AUC |
|---|---|
| 130_72 | 0.893 |
| 73_22 | 0.892 |
| 14_22 | 0.892 |
| 234_187 | 0.892 |
| 25_52 | 0.892 |
| 194_64 | 0.892 |
| 80_72 | 0.890 |
| 61_22 | 0.890 |
| 48_52 | 0.890 |
| 22_123 | 0.890 |
| 52_188 | 0.890 |
| 23_84 | 0.890 |
| 152_8 | 0.890 |
| 31_84 | 0.890 |
| 80_84 | 0.888 |
| 52_93 | 0.888 |
| 20_84 | 0.888 |
| 146_22 | 0.888 |
| 23_72 | 0.888 |
| 87_72 | 0.888 |
| 46_187 | 0.888 |
| 159_87 | 0.888 |
| 150_53 | 0.888 |
| 161_84 | 0.887 |
| 56_65 | 0.887 |
| 26_72 | 0.887 |
| 234_84 | 0.887 |
| 52_85 | 0.887 |
| 25_72 | 0.887 |
| 22_67 | 0.887 |
| 84_67 | 0.887 |
| 125_84 | 0.887 |
| 8_226 | 0.887 |
| 152_22 | 0.887 |
| 192_22 | 0.887 |
| 87_84 | 0.887 |
| 230_53 | 0.887 |
| 131_22 | 0.885 |

(continued)

| SEQ ID NO. | AUC |
|---|---|
| 84_11 | 0.885 |
| 230_64 | 0.885 |
| 197_22 | 0.885 |
| 8_64 | 0.885 |
| 234_150 | 0.885 |
| 8_33 | 0.885 |
| 52_84 | 0.885 |
| 162_84 | 0.885 |
| 1_84 | 0.885 |
| 146_84 | 0.885 |
| 31_52 | 0.885 |
| 9_84 | 0.885 |
| 84_117 | 0.885 |
| 84_178 | 0.885 |
| 75_84 | 0.885 |
| 44_52 | 0.885 |
| 61_84 | 0.885 |
| 22_151 | 0.883 |
| 224_22 | 0.883 |
| 221_84 | 0.883 |
| 84_30 | 0.883 |
| 195_64 | 0.883 |
| 53_22 | 0.883 |
| 212_22 | 0.883 |
| 115_22 | 0.883 |
| 202_22 | 0.883 |
| 38_84 | 0.883 |
| 13_56 | 0.883 |
| 68_84 | 0.883 |
| 72_15 | 0.883 |
| 143_22 | 0.882 |
| 86_22 | 0.882 |
| 84_130 | 0.882 |
| 25_84 | 0.882 |
| 84_76 | 0.882 |
| 56_64 | 0.882 |
| 84_5 | 0.882 |
| 52_29 | 0.882 |

(continued)

| SEQ ID NO. | AUC |
|---|---|
| 64_66 | 0.882 |
| 102_22 | 0.882 |
| 84_157 | 0.882 |
| 22_132 | 0.882 |
| 84_74 | 0.882 |
| 40_84 | 0.880 |
| 52_53 | 0.880 |
| 84_17 | 0.880 |
| 2_72 | 0.880 |
| 84_73 | 0.880 |
| 187_87 | 0.880 |
| 39_72 | 0.880 |
| 19_72 | 0.880 |
| 118_64 | 0.880 |
| 44_72 | 0.880 |
| 105_84 | 0.880 |
| 84_123 | 0.880 |
| 84_85 | 0.878 |
| 228_22 | 0.878 |
| 106_8 | 0.878 |
| 186_8 | 0.878 |
| 207_84 | 0.878 |
| 52_45 | 0.878 |
| 52_115 | 0.878 |
| 84_110 | 0.878 |
| 84_191 | 0.878 |
| 166_53 | 0.878 |
| 84_71 | 0.878 |
| 132_72 | 0.878 |
| 84_77 | 0.878 |
| 2_84 | 0.878 |
| 171_84 | 0.878 |
| 56_46 | 0.878 |
| 84_88 | 0.878 |
| 84_103 | 0.878 |
| 21_72 | 0.877 |
| 22_29 | 0.877 |
| 48_22 | 0.877 |

(continued)

| SEQ ID NO. | AUC |
|---|---|
| 88_33 | 0.877 |
| 18_84 | 0.877 |
| 185_85 | 0.877 |
| 3_52 | 0.877 |
| 22_82 | 0.877 |
| 22_76 | 0.877 |
| 234_73 | 0.877 |
| 90_84 | 0.877 |
| 49_84 | 0.877 |
| 184_84 | 0.877 |
| 84_14 | 0.877 |
| 38_53 | 0.877 |
| 8_11 | 0.877 |
| 66_52 | 0.877 |
| 97_84 | 0.877 |
| 22_10 | 0.877 |
| 52_135 | 0.877 |
| 84_29 | 0.877 |
| 56_72 | 0.877 |
| 7_22 | 0.877 |
| 178_72 | 0.877 |
| 78_22 | 0.877 |
| 52_105 | 0.877 |
| 186_22 | 0.877 |
| 61_52 | 0.875 |
| 52_54 | 0.875 |
| 171_22 | 0.875 |
| 62_84 | 0.875 |
| 56_33 | 0.875 |
| 22_153 | 0.875 |
| 222_33 | 0.875 |
| 22_199 | 0.875 |
| 22_11 | 0.875 |
| 39_84 | 0.875 |
| 182_84 | 0.875 |
| 121_84 | 0.875 |
| 84_45 | 0.875 |
| 46_230 | 0.875 |

(continued)

| SEQ ID NO. | AUC |
|---|---|
| 231_84 | 0.875 |
| 160_84 | 0.875 |
| 84_54 | 0.875 |
| 19_84 | 0.875 |
| 97_52 | 0.873 |
| 84_163 | 0.873 |
| 22_70 | 0.873 |
| 142_72 | 0.873 |
| 22_139 | 0.873 |
| 108_84 | 0.873 |
| 69_84 | 0.873 |
| 84_213 | 0.873 |
| 201_22 | 0.873 |
| 84_145 | 0.873 |
| 84_172 | 0.873 |
| 24_84 | 0.873 |
| 7_84 | 0.873 |
| 84_222 | 0.873 |
| 52_153 | 0.873 |
| 11_72 | 0.873 |
| 72_4 | 0.873 |
| 220_84 | 0.873 |
| 56_234 | 0.873 |
| 32_22 | 0.872 |
| 67_72 | 0.872 |
| 84_164 | 0.872 |
| 33_15 | 0.872 |
| 46_11 | 0.872 |
| 23_52 | 0.872 |
| 226_22 | 0.872 |
| 56_36 | 0.872 |
| 84_93 | 0.872 |
| 21_52 | 0.872 |
| 84_104 | 0.872 |
| 6_84 | 0.872 |
| 7_72 | 0.872 |
| 154_52 | 0.872 |
| 142_22 | 0.872 |

(continued)

| SEQ ID NO. | AUC |
|------------|-------|
| 75_72 | 0.872 |
| 66_84 | 0.872 |
| 8_73 | 0.872 |
| 120_84 | 0.872 |
| 56_200 | 0.872 |
| 52_131 | 0.872 |
| 84_70 | 0.872 |
| 187_26 | 0.872 |
| 52_5 | 0.872 |
| 22_45 | 0.872 |
| 86_84 | 0.872 |
| 229_84 | 0.872 |
| 152_84 | 0.872 |
| 162_22 | 0.872 |
| 114_84 | 0.872 |
| 49_22 | 0.870 |
| 217_84 | 0.870 |
| 22_168 | 0.870 |
| 43_72 | 0.870 |
| 56_91 | 0.870 |
| 84_72 | 0.870 |
| 84_122 | 0.870 |
| 115_84 | 0.870 |
| 159_29 | 0.870 |
| 64_72 | 0.870 |
| 96_22 | 0.870 |
| 124_84 | 0.870 |
| 74_72 | 0.870 |
| 200_84 | 0.870 |
| 167_84 | 0.870 |
| 102_46 | 0.870 |
| 60_22 | 0.870 |
| 166_64 | 0.870 |
| 222_11 | 0.870 |
| 75_52 | 0.870 |
| 95_84 | 0.870 |
| 222_72 | 0.870 |
| 61_72 | 0.870 |

(continued)

| SEQ ID NO. | AUC |
|---|---|
| 64_27 | 0.870 |
| 84_27 | 0.870 |
| 52_10 | 0.870 |
| 5_72 | 0.870 |
| 50_84 | 0.868 |
| 90_52 | 0.868 |
| 149_84 | 0.868 |
| 22_16 | 0.868 |
| 222_16 | 0.868 |
| 33_147 | 0.868 |
| 192_84 | 0.868 |
| 106_84 | 0.868 |
| 60_84 | 0.868 |
| 169_84 | 0.868 |
| 189_84 | 0.868 |
| 93_72 | 0.868 |
| 28_72 | 0.868 |
| 45_72 | 0.868 |
| 84_42 | 0.868 |
| 174_84 | 0.868 |
| 65_84 | 0.868 |
| 56_53 | 0.868 |
| 201_84 | 0.868 |
| 57_84 | 0.868 |
| 56_84 | 0.868 |
| 78_72 | 0.868 |
| 69_22 | 0.868 |
| 234_117 | 0.868 |
| 166_22 | 0.868 |
| 12_84 | 0.868 |
| 21_84 | 0.868 |
| 22_110 | 0.868 |
| 99_84 | 0.867 |
| 53_33 | 0.867 |
| 84_168 | 0.867 |
| 159_84 | 0.867 |
| 109_72 | 0.867 |
| 19_159 | 0.867 |

(continued)

| SEQ ID NO. | AUC |
|---|---|
| 3_72 | 0.867 |
| 52_76 | 0.867 |
| 54_72 | 0.867 |
| 106_22 | 0.867 |
| 84_223 | 0.867 |
| 84_126 | 0.867 |
| 176_84 | 0.867 |
| 110_72 | 0.867 |
| 48_72 | 0.867 |
| 180_72 | 0.867 |
| 84_196 | 0.867 |
| 8_100 | 0.867 |
| 8_53 | 0.867 |
| 84_4 | 0.867 |
| 2_52 | 0.867 |
| 38_81 | 0.867 |
| 84_147 | 0.867 |
| 84_131 | 0.867 |
| 225_22 | 0.867 |
| 22_145 | 0.867 |
| 152_72 | 0.867 |
| 195_52 | 0.867 |
| 22_208 | 0.867 |
| 84_206 | 0.867 |
| 220_22 | 0.867 |
| 142_52 | 0.867 |
| 56_52 | 0.867 |
| 138_84 | 0.865 |
| 47_84 | 0.865 |
| 84_59 | 0.865 |
| 38_64 | 0.865 |
| 84_165 | 0.865 |
| 194_84 | 0.865 |
| 23_64 | 0.865 |
| 108_64 | 0.865 |
| 84_153 | 0.865 |
| 180_84 | 0.865 |
| 210_84 | 0.865 |

(continued)

| SEQ ID NO. | AUC |
|---|---|
| 52_165 | 0.865 |
| 219_84 | 0.865 |
| 84_193 | 0.865 |
| 56_210 | 0.865 |
| 83_84 | 0.865 |
| 52_14 | 0.865 |
| 114_22 | 0.865 |
| 99_22 | 0.865 |
| 99_72 | 0.865 |
| 1_22 | 0.863 |
| 109_84 | 0.863 |
| 56_222 | 0.863 |
| 212_84 | 0.863 |
| 195_84 | 0.863 |
| 84_188 | 0.863 |
| 235_84 | 0.863 |
| 207_22 | 0.863 |
| 173_84 | 0.863 |
| 60_232 | 0.863 |
| 96_84 | 0.863 |
| 190_84 | 0.863 |
| 84_100 | 0.863 |
| 84_41 | 0.863 |
| 187_53 | 0.863 |
| 224_84 | 0.863 |
| 55_84 | 0.863 |
| 97_22 | 0.863 |
| 84_10 | 0.863 |
| 181_84 | 0.863 |
| 175_84 | 0.863 |
| 101_84 | 0.863 |
| 52_213 | 0.863 |
| 114_72 | 0.863 |
| 109_52 | 0.863 |
| 112_84 | 0.863 |
| 194_202 | 0.863 |
| 142_84 | 0.863 |
| 38_46 | 0.863 |

(continued)

| SEQ ID NO. | AUC |
|---|---|
| 84_208 | 0.863 |
| 221_22 | 0.863 |
| 133_84 | 0.863 |
| 27_72 | 0.863 |
| 2_22 | 0.863 |
| 68_72 | 0.862 |
| 64_98 | 0.862 |
| 22_130 | 0.862 |
| 48_84 | 0.862 |
| 143_84 | 0.862 |
| 233_84 | 0.862 |
| 84_98 | 0.862 |
| 86_72 | 0.862 |
| 161_8 | 0.862 |
| 3_84 | 0.862 |
| 154_84 | 0.862 |
| 29_72 | 0.862 |
| 184_16 | 0.862 |
| 81_84 | 0.862 |
| 56_169 | 0.862 |
| 60_8 | 0.862 |
| 79_64 | 0.862 |
| 227_84 | 0.862 |
| 114_64 | 0.862 |
| 46_186 | 0.862 |
| 209_33 | 0.862 |
| 56_128 | 0.862 |
| 66_202 | 0.862 |
| 56_227 | 0.862 |
| 129_22 | 0.862 |
| 158_22 | 0.862 |
| 56_134 | 0.862 |
| 12_22 | 0.862 |
| 92_84 | 0.860 |
| 22_100 | 0.860 |
| 94_84 | 0.860 |
| 9_72 | 0.860 |
| 62_22 | 0.860 |

(continued)

| SEQ ID NO. | AUC |
|---|---|
| 64_52 | 0.860 |
| 155_72 | 0.860 |
| 58_84 | 0.860 |
| 84_113 | 0.860 |
| 234_230 | 0.860 |
| 52_72 | 0.860 |
| 75_150 | 0.860 |
| 52_70 | 0.860 |
| 121_22 | 0.860 |
| 64_10 | 0.860 |
| 43_84 | 0.860 |
| 60_72 | 0.860 |
| 22_135 | 0.860 |
| 234_22 | 0.860 |
| 20_64 | 0.860 |
| 84_26 | 0.860 |
| 8_200 | 0.860 |
| 9_22 | 0.860 |
| 84_36 | 0.860 |
| 44_22 | 0.860 |
| 84_183 | 0.860 |
| 64_32 | 0.860 |
| 102_72 | 0.860 |
| 84_170 | 0.858 |
| 22_17 | 0.858 |
| 56_24 | 0.858 |
| 118_84 | 0.858 |
| 56_1 | 0.858 |
| 129_72 | 0.858 |
| 25_64 | 0.858 |
| 122_72 | 0.858 |
| 108_52 | 0.858 |
| 210_22 | 0.858 |
| 33_70 | 0.858 |
| 7_159 | 0.858 |
| 147_72 | 0.858 |
| 84_158 | 0.858 |
| 46_226 | 0.858 |

(continued)

| SEQ ID NO. | AUC |
|---|---|
| 200_22 | 0.858 |
| 60_52 | 0.858 |
| 230_84 | 0.858 |
| 52_143 | 0.858 |
| 134_22 | 0.858 |
| 60_64 | 0.858 |
| 84_91 | 0.858 |
| 56_147 | 0.858 |
| 34_22 | 0.858 |
| 53_85 | 0.858 |
| 25_159 | 0.858 |
| 79_84 | 0.858 |
| 215_84 | 0.858 |
| 206_72 | 0.858 |
| 161_72 | 0.858 |
| 105_22 | 0.858 |
| 52_4 | 0.858 |
| 55_22 | 0.858 |
| 8_206 | 0.857 |
| 233_22 | 0.857 |
| 128_84 | 0.857 |
| 102_64 | 0.857 |
| 195_72 | 0.857 |
| 141_84 | 0.857 |
| 127_22 | 0.857 |
| 79_72 | 0.857 |
| 56_18 | 0.857 |
| 18_72 | 0.857 |
| 56_38 | 0.857 |
| 80_22 | 0.857 |
| 234_206 | 0.857 |
| 102_53 | 0.857 |
| 93_232 | 0.857 |
| 84_132 | 0.857 |
| 56_196 | 0.857 |
| 230_52 | 0.857 |
| 212_8 | 0.857 |
| 56_73 | 0.857 |

(continued)

| SEQ ID NO. | AUC |
|---|---|
| 28_64 | 0.857 |
| 52_77 | 0.857 |
| 18_22 | 0.857 |
| 43_22 | 0.857 |
| 119_84 | 0.857 |
| 195_22 | 0.857 |
| 187_84 | 0.857 |
| 10_72 | 0.857 |
| 150_64 | 0.857 |
| 43_33 | 0.857 |
| 230_15 | 0.857 |
| 85_222 | 0.857 |
| 84_232 | 0.857 |
| 20_159 | 0.857 |
| 177_84 | 0.857 |
| 148_84 | 0.857 |
| 137_72 | 0.857 |
| 127_64 | 0.857 |
| 161_56 | 0.857 |
| 127_84 | 0.857 |
| 84_135 | 0.857 |
| 213_72 | 0.855 |
| 28_84 | 0.855 |
| 81_22 | 0.855 |
| 194_52 | 0.855 |
| 234_192 | 0.855 |
| 171_72 | 0.855 |
| 52_222 | 0.855 |
| 89_84 | 0.855 |
| 14_72 | 0.855 |
| 84_82 | 0.855 |
| 11_70 | 0.855 |
| 102_84 | 0.855 |
| 37_22 | 0.855 |
| 227_22 | 0.855 |
| 183_72 | 0.855 |
| 66_22 | 0.855 |
| 84_134 | 0.855 |

(continued)

| SEQ ID NO. | AUC |
|---|---|
| 218_84 | 0.855 |
| 150_31 | 0.855 |
| 145_72 | 0.855 |
| 162_72 | 0.855 |
| 71_72 | 0.855 |
| 21_64 | 0.855 |
| 129_84 | 0.855 |
| 169_22 | 0.855 |
| 62_72 | 0.855 |
| 13_22 | 0.855 |
| 22_91 | 0.855 |
| 215_22 | 0.855 |
| 31_22 | 0.855 |
| 56_16 | 0.855 |
| 62_52 | 0.853 |
| 155_84 | 0.853 |
| 190_72 | 0.853 |
| 84_151 | 0.853 |
| 138_22 | 0.853 |
| 234_151 | 0.853 |
| 84_144 | 0.853 |
| 234_106 | 0.853 |
| 54_22 | 0.853 |
| 52_74 | 0.853 |
| 174_22 | 0.853 |
| 64_197 | 0.853 |
| 113_22 | 0.853 |
| 52_164 | 0.853 |
| 52_39 | 0.853 |
| 6_150 | 0.853 |
| 129_52 | 0.853 |
| 52_37 | 0.853 |
| 217_52 | 0.853 |
| 28_22 | 0.853 |
| 201_52 | 0.853 |
| 8_171 | 0.853 |
| 79_52 | 0.853 |
| 102_234 | 0.853 |

(continued)

| SEQ ID NO. | AUC |
|---|---|
| 161_52 | 0.853 |
| 84_202 | 0.853 |
| 108_22 | 0.853 |
| 95_22 | 0.853 |
| 84_137 | 0.853 |
| 49_52 | 0.853 |
| 23_22 | 0.853 |
| 111_84 | 0.853 |
| 198_84 | 0.853 |
| 56_87 | 0.853 |
| 90_72 | 0.853 |
| 224_72 | 0.852 |
| 60_196 | 0.852 |
| 156_64 | 0.852 |
| 222_10 | 0.852 |
| 84_199 | 0.852 |
| 64_33 | 0.852 |
| 192_72 | 0.852 |
| 219_52 | 0.852 |
| 214_22 | 0.852 |
| 159_26 | 0.852 |
| 28_52 | 0.852 |
| 34_72 | 0.852 |
| 203_84 | 0.852 |
| 64_11 | 0.852 |
| 37_72 | 0.852 |
| 40_22 | 0.852 |
| 116_22 | 0.852 |
| 148_22 | 0.852 |
| 41_22 | 0.852 |
| 180_73 | 0.852 |
| 38_8 | 0.852 |
| 168_72 | 0.852 |
| 23_232 | 0.850 |
| 38_22 | 0.850 |
| 3_22 | 0.850 |
| 66_222 | 0.850 |
| 218_22 | 0.850 |

(continued)

| SEQ ID NO. | AUC |
|---|---|
| 52_96 | 0.850 |
| 38_72 | 0.850 |
| 56_70 | 0.850 |
| 136_84 | 0.850 |
| 128_22 | 0.850 |
| 201_72 | 0.850 |
| 12_72 | 0.850 |
| 27_22 | 0.850 |
| 52_130 | 0.850 |
| 146_52 | 0.850 |
| 56_172 | 0.850 |
| 64_226 | 0.850 |
| 178_22 | 0.850 |
| 163_72 | 0.850 |
| 150_84 | 0.850 |
| 79_22 | 0.850 |
| 185_22 | 0.850 |
| 21_22 | 0.850 |
| 125_72 | 0.850 |
| 84_179 | 0.850 |
| 63_84 | 0.850 |
| 42_22 | 0.850 |
| 56_209 | 0.850 |
| 140_84 | 0.850 |
| 24_22 | 0.850 |
| 80_52 | 0.850 |
| 167_72 | 0.850 |
| 5_22 | 0.850 |
| 222_73 | 0.850 |
| 46_22 | 0.850 |
| 229_22 | 0.850 |
| 157_72 | 0.850 |
| 150_44 | 0.850 |
| 46_166 | 0.850 |
| 22_188 | 0.850 |
| 150_72 | 0.850 |
| 140_64 | 0.848 |
| 84_197 | 0.848 |

(continued)

| SEQ ID NO. | AUC |
|---|---|
| 231_72 | 0.848 |
| 198_22 | 0.848 |
| 125_22 | 0.848 |
| 69_72 | 0.848 |
| 52_110 | 0.848 |
| 38_202 | 0.848 |
| 229_52 | 0.848 |
| 83_52 | 0.848 |
| 25_187 | 0.848 |
| 119_64 | 0.848 |
| 57_64 | 0.848 |
| 56_193 | 0.848 |
| 56_159 | 0.848 |
| 72_16 | 0.848 |
| 56_6 | 0.848 |
| 73_72 | 0.848 |
| 51_84 | 0.848 |
| 185_84 | 0.848 |
| 164_22 | 0.848 |
| 140_52 | 0.848 |
| 39_33 | 0.848 |
| 36_22 | 0.848 |
| 126_22 | 0.848 |
| 19_22 | 0.848 |
| 56_188 | 0.848 |
| 217_22 | 0.848 |
| 152_52 | 0.848 |
| 211_64 | 0.848 |
| 52_173 | 0.848 |
| 52_163 | 0.848 |
| 22_59 | 0.848 |
| 22_26 | 0.848 |
| 232_72 | 0.848 |
| 56_203 | 0.848 |
| 64_95 | 0.847 |
| 93_226 | 0.847 |
| 46_73 | 0.847 |
| 124_22 | 0.847 |

(continued)

| SEQ ID NO. | AUC |
|---|---|
| 234_90 | 0.847 |
| 49_72 | 0.847 |
| 189_22 | 0.847 |
| 93_196 | 0.847 |
| 212_52 | 0.847 |
| 56_217 | 0.847 |
| 32_53 | 0.847 |
| 119_22 | 0.847 |
| 173_72 | 0.847 |
| 56_206 | 0.847 |
| 202_11 | 0.847 |
| 6_72 | 0.847 |
| 56_77 | 0.847 |
| 64_77 | 0.847 |
| 25_22 | 0.847 |
| 75_230 | 0.847 |
| 66_72 | 0.847 |
| 35_84 | 0.847 |
| 207_52 | 0.847 |
| 161_222 | 0.847 |
| 39_22 | 0.847 |
| 142_11 | 0.847 |
| 47_22 | 0.847 |
| 8_22 | 0.847 |
| 228_84 | 0.847 |
| 216_84 | 0.847 |
| 8_85 | 0.847 |
| 166_70 | 0.847 |
| 84_226 | 0.847 |
| 184_72 | 0.847 |
| 78_52 | 0.847 |
| 124_72 | 0.847 |
| 56_235 | 0.847 |
| 153_72 | 0.847 |
| 52_145 | 0.847 |
| 56_96 | 0.847 |
| 127_72 | 0.847 |
| 33_4 | 0.847 |

(continued)

| SEQ ID NO. | AUC |
|---|---|
| 58_22 | 0.845 |
| 175_72 | 0.845 |
| 88_22 | 0.845 |
| 56_184 | 0.845 |
| 220_72 | 0.845 |
| 65_22 | 0.845 |
| 84_204 | 0.845 |
| 177_72 | 0.845 |
| 64_187 | 0.845 |
| 203_72 | 0.845 |
| 53_98 | 0.845 |
| 56_118 | 0.845 |
| 93_33 | 0.845 |
| 52_69 | 0.845 |
| 45_232 | 0.845 |
| 60_33 | 0.845 |
| 186_52 | 0.845 |
| 116_64 | 0.845 |
| 83_22 | 0.845 |
| 176_72 | 0.845 |
| 148_72 | 0.845 |
| 38_234 | 0.845 |
| 81_85 | 0.845 |
| 116_52 | 0.845 |
| 8_151 | 0.845 |
| 140_22 | 0.845 |
| 76_72 | 0.845 |
| 116_56 | 0.845 |
| 34_84 | 0.845 |
| 106_72 | 0.845 |
| 72_91 | 0.845 |
| 56_30 | 0.845 |
| 33_26 | 0.843 |
| 56_69 | 0.843 |
| 8_213 | 0.843 |
| 22_172 | 0.843 |
| 25_33 | 0.843 |
| 56_202 | 0.843 |

(continued)

| SEQ ID NO. | AUC |
|---|---|
| 234_213 | 0.843 |
| 56_40 | 0.843 |
| 42_72 | 0.843 |
| 92_72 | 0.843 |
| 218_72 | 0.843 |
| 75_8 | 0.843 |
| 1_166 | 0.843 |
| 52_191 | 0.843 |
| 52_11 | 0.843 |
| 159_93 | 0.843 |
| 55_72 | 0.843 |
| 149_52 | 0.843 |
| 173_22 | 0.843 |
| 177_52 | 0.843 |
| 141_72 | 0.843 |
| 115_93 | 0.843 |
| 196_72 | 0.843 |
| 52_33 | 0.843 |
| 8_232 | 0.843 |
| 134_72 | 0.843 |
| 176_52 | 0.843 |
| 215_72 | 0.843 |
| 1_72 | 0.843 |
| 38_222 | 0.843 |
| 115_72 | 0.843 |
| 109_22 | 0.843 |
| 13_72 | 0.843 |
| 137_22 | 0.843 |
| 43_52 | 0.843 |
| 56_141 | 0.843 |
| 77_22 | 0.843 |
| 181_72 | 0.843 |
| 184_52 | 0.843 |
| 56_230 | 0.843 |
| 8_66 | 0.843 |
| 160_72 | 0.843 |
| 230_11 | 0.843 |
| 22_204 | 0.843 |

(continued)

| SEQ ID NO. | AUC |
|---|---|
| 190_22 | 0.843 |
| 160_22 | 0.843 |
| 111_22 | 0.843 |
| 22_163 | 0.842 |
| 164_72 | 0.842 |
| 117_72 | 0.842 |
| 97_72 | 0.842 |
| 60_134 | 0.842 |
| 56_142 | 0.842 |
| 52_197 | 0.842 |
| 22_30 | 0.842 |
| 226_33 | 0.842 |
| 191_72 | 0.842 |
| 8_96 | 0.842 |
| 167_22 | 0.842 |
| 35_22 | 0.842 |
| 176_22 | 0.842 |
| 186_72 | 0.842 |
| 217_72 | 0.842 |
| 128_72 | 0.842 |
| 156_84 | 0.842 |
| 59_72 | 0.842 |
| 223_72 | 0.842 |
| 83_72 | 0.842 |
| 52_126 | 0.842 |
| 53_117 | 0.842 |
| 33_72 | 0.842 |
| 18_52 | 0.842 |
| 22_170 | 0.842 |
| 56_75 | 0.842 |
| 96_33 | 0.842 |
| 75_222 | 0.842 |
| 55_52 | 0.842 |
| 101_22 | 0.842 |
| 193_22 | 0.842 |
| 56_187 | 0.842 |
| 161_22 | 0.842 |
| 149_22 | 0.842 |

(continued)

| SEQ ID NO. | AUC |
|---|---|
| 136_22 | 0.842 |
| 52_15 | 0.842 |
| 104_22 | 0.842 |
| 52_167 | 0.842 |
| 150_15 | 0.842 |
| 64_76 | 0.842 |
| 89_72 | 0.842 |
| 159_44 | 0.842 |
| 149_72 | 0.842 |
| 103_72 | 0.842 |
| 75_53 | 0.842 |
| 116_84 | 0.842 |
| 211_84 | 0.842 |
| 223_22 | 0.842 |
| 133_22 | 0.842 |
| 123_72 | 0.842 |
| 56_93 | 0.842 |
| 235_22 | 0.842 |
| 234_212 | 0.842 |
| 50_22 | 0.842 |
| 118_22 | 0.842 |
| 57_22 | 0.842 |
| 216_22 | 0.842 |
| 234_196 | 0.842 |
| 120_22 | 0.842 |
| 86_52 | 0.842 |
| 52_183 | 0.842 |
| 60_202 | 0.842 |
| 56_110 | 0.842 |
| 25_73 | 0.840 |
| 30_72 | 0.840 |
| 119_52 | 0.840 |
| 25_232 | 0.840 |
| 52_71 | 0.840 |
| 32_72 | 0.840 |
| 52_123 | 0.840 |
| 127_33 | 0.840 |
| 75_159 | 0.840 |

(continued)

| SEQ ID NO. | AUC |
|---|---|
| 112_22 | 0.840 |
| 53_197 | 0.840 |
| 226_72 | 0.840 |
| 64_93 | 0.840 |
| 56_85 | 0.840 |
| 22_144 | 0.840 |
| 203_53 | 0.840 |
| 188_72 | 0.840 |
| 96_72 | 0.840 |
| 107_22 | 0.840 |
| 23_33 | 0.840 |
| 22_98 | 0.840 |
| 52_226 | 0.840 |
| 180_52 | 0.840 |
| 200_52 | 0.840 |
| 64_216 | 0.840 |
| 56_151 | 0.840 |
| 127_52 | 0.840 |
| 84_139 | 0.840 |
| 229_72 | 0.840 |
| 155_52 | 0.840 |
| 33_132 | 0.840 |
| 56_175 | 0.840 |
| 56_31 | 0.840 |
| 121_52 | 0.840 |
| 56_66 | 0.840 |
| 101_72 | 0.840 |
| 143_72 | 0.840 |
| 159_45 | 0.840 |
| 131_72 | 0.840 |
| 165_72 | 0.840 |
| 33_67 | 0.838 |
| 56_132 | 0.838 |
| 37_226 | 0.838 |
| 97_64 | 0.838 |
| 205_84 | 0.838 |
| 46_206 | 0.838 |
| 56_55 | 0.838 |

(continued)

| SEQ ID NO. | AUC |
|---|---|
| 56_17 | 0.838 |
| 218_52 | 0.838 |
| 150_209 | 0.838 |
| 56_226 | 0.838 |
| 152_33 | 0.838 |
| 72_208 | 0.838 |
| 224_52 | 0.838 |
| 47_72 | 0.838 |
| 182_52 | 0.838 |
| 136_52 | 0.838 |
| 106_53 | 0.838 |
| 6_22 | 0.838 |
| 75_11 | 0.838 |
| 162_52 | 0.838 |
| 114_52 | 0.838 |
| 18_33 | 0.838 |
| 56_180 | 0.838 |
| 52_88 | 0.838 |
| 85_22 | 0.838 |
| 220_52 | 0.838 |
| 46_106 | 0.838 |
| 56_71 | 0.838 |
| 56_232 | 0.838 |
| 158_72 | 0.838 |
| 19_64 | 0.838 |
| 22_122 | 0.838 |
| 60_53 | 0.838 |
| 230_72 | 0.838 |
| 56_50 | 0.838 |
| 180_22 | 0.837 |
| 72_98 | 0.837 |
| 56_150 | 0.837 |
| 197_72 | 0.837 |
| 51_22 | 0.837 |
| 203_8 | 0.837 |
| 56_166 | 0.837 |
| 142_73 | 0.837 |
| 147_16 | 0.837 |

(continued)

| SEQ ID NO. | AUC |
|---|---|
| 108_72 | 0.837 |
| 146_72 | 0.837 |
| 118_72 | 0.837 |
| 159_52 | 0.837 |
| 85_72 | 0.837 |
| 118_52 | 0.837 |
| 197_16 | 0.837 |
| 75_106 | 0.837 |
| 56_137 | 0.837 |
| 60_222 | 0.837 |
| 52_17 | 0.837 |
| 52_157 | 0.837 |
| 89_22 | 0.837 |
| 102_56 | 0.837 |
| 212_72 | 0.837 |
| 52_193 | 0.837 |
| 52_16 | 0.837 |
| 64_180 | 0.837 |
| 56_10 | 0.837 |
| 119_56 | 0.837 |
| 234_11 | 0.837 |
| 214_84 | 0.837 |
| 121_72 | 0.837 |
| 32_84 | 0.837 |
| 56_80 | 0.837 |
| 72_17 | 0.837 |
| 234_231 | 0.837 |
| 184_11 | 0.837 |
| 177_22 | 0.837 |
| 234_75 | 0.837 |
| 66_53 | 0.837 |
| 175_52 | 0.837 |
| 64_126 | 0.837 |
| 155_22 | 0.837 |
| 8_87 | 0.837 |
| 56_104 | 0.837 |
| 103_22 | 0.837 |
| 133_72 | 0.835 |

(continued)

| SEQ ID NO. | AUC |
|---|---|
| 64_134 | 0.835 |
| 52_208 | 0.835 |
| 22_183 | 0.835 |
| 159_109 | 0.835 |
| 52_181 | 0.835 |
| 127_36 | 0.835 |
| 94_72 | 0.835 |
| 221_72 | 0.835 |
| 181_22 | 0.835 |
| 41_72 | 0.835 |
| 64_144 | 0.835 |
| 234_74 | 0.835 |
| 186_53 | 0.835 |
| 64_179 | 0.835 |
| 200_72 | 0.835 |
| 56_229 | 0.835 |
| 64_153 | 0.835 |
| 60_150 | 0.835 |
| 207_33 | 0.835 |
| 52_30 | 0.835 |
| 56_174 | 0.835 |
| 60_147 | 0.835 |
| 56_5 | 0.835 |
| 52_134 | 0.835 |
| 13_203 | 0.835 |
| 56_126 | 0.835 |
| 186_84 | 0.835 |
| 33_10 | 0.835 |
| 56_4 | 0.835 |
| 75_33 | 0.835 |
| 22_179 | 0.835 |
| 219_72 | 0.833 |
| 53_151 | 0.833 |
| 93_22 | 0.833 |
| 102_15 | 0.833 |
| 234_207 | 0.833 |
| 182_72 | 0.833 |
| 56_35 | 0.833 |

(continued)

| SEQ ID NO. | AUC |
|---|---|
| 53_222 | 0.833 |
| 169_72 | 0.833 |
| 56_165 | 0.833 |
| 52_100 | 0.833 |
| 22_4 | 0.833 |
| 161_64 | 0.833 |
| 142_32 | 0.833 |
| 111_72 | 0.833 |
| 154_22 | 0.833 |
| 150_52 | 0.833 |
| 52_91 | 0.833 |
| 233_72 | 0.833 |
| 126_72 | 0.833 |
| 8_117 | 0.833 |
| 20_22 | 0.833 |
| 156_22 | 0.833 |
| 56_130 | 0.833 |
| 194_72 | 0.833 |
| 216_72 | 0.833 |
| 7_8 | 0.833 |
| 203_16 | 0.833 |
| 75_64 | 0.833 |
| 113_72 | 0.833 |
| 140_72 | 0.833 |
| 1_33 | 0.833 |
| 209_22 | 0.833 |
| 64_113 | 0.833 |
| 211_22 | 0.833 |
| 52_92 | 0.833 |
| 198_72 | 0.833 |
| 87_226 | 0.833 |
| 175_93 | 0.833 |
| 52_27 | 0.833 |
| 92_22 | 0.833 |
| 185_72 | 0.833 |
| 56_11 | 0.833 |
| 38_147 | 0.833 |
| 187_72 | 0.833 |

(continued)

| SEQ ID NO. | AUC |
|---|---|
| 127_16 | 0.832 |
| 56_57 | 0.832 |
| 64_45 | 0.832 |
| 77_72 | 0.832 |
| 38_142 | 0.832 |
| 75_85 | 0.832 |
| 64_145 | 0.832 |
| 8_90 | 0.832 |
| 108_56 | 0.832 |
| 56_122 | 0.832 |
| 93_73 | 0.832 |
| 56_157 | 0.832 |
| 6_52 | 0.832 |
| 21_33 | 0.832 |
| 94_22 | 0.832 |
| 40_72 | 0.832 |
| 56_37 | 0.832 |
| 211_52 | 0.832 |
| 52_82 | 0.832 |
| 234_166 | 0.832 |
| 180_33 | 0.832 |
| 60_184 | 0.832 |
| 52_122 | 0.832 |
| 135_72 | 0.832 |
| 119_72 | 0.832 |
| 56_155 | 0.832 |
| 44_33 | 0.832 |
| 86_234 | 0.832 |
| 100_72 | 0.832 |
| 225_84 | 0.832 |
| 111_64 | 0.832 |
| 221_52 | 0.830 |
| 81_72 | 0.830 |
| 180_53 | 0.830 |
| 225_56 | 0.830 |
| 33_130 | 0.830 |
| 179_72 | 0.830 |
| 38_15 | 0.830 |

(continued)

| SEQ ID NO. | AUC |
|---|---|
| 93_222 | 0.830 |
| 199_72 | 0.830 |
| 172_72 | 0.830 |
| 193_72 | 0.830 |
| 46_16 | 0.830 |
| 141_52 | 0.830 |
| 159_85 | 0.830 |
| 127_53 | 0.830 |
| 127_210 | 0.830 |
| 106_52 | 0.830 |
| 104_72 | 0.830 |
| 56_26 | 0.830 |
| 150_105 | 0.830 |
| 166_72 | 0.830 |
| 184_64 | 0.830 |
| 13_52 | 0.830 |
| 228_56 | 0.830 |
| 25_150 | 0.830 |
| 205_72 | 0.830 |
| 155_166 | 0.830 |
| 202_72 | 0.830 |
| 152_64 | 0.830 |
| 38_52 | 0.830 |
| 109_33 | 0.830 |
| 234_64 | 0.830 |
| 79_159 | 0.830 |
| 52_172 | 0.830 |
| 56_25 | 0.830 |
| 56_121 | 0.830 |
| 75_142 | 0.830 |
| 205_22 | 0.830 |
| 56_49 | 0.830 |
| 56_106 | 0.830 |
| 56_219 | 0.830 |
| 170_72 | 0.828 |
| 227_52 | 0.828 |
| 32_222 | 0.828 |
| 53_226 | 0.828 |

(continued)

| SEQ ID NO. | AUC |
|---|---|
| 187_105 | 0.828 |
| 105_33 | 0.828 |
| 25_96 | 0.828 |
| 157_153 | 0.828 |
| 33_110 | 0.828 |
| 152_53 | 0.828 |
| 112_72 | 0.828 |
| 56_15 | 0.828 |
| 194_22 | 0.828 |
| 226_45 | 0.828 |
| 85_226 | 0.828 |
| 159_72 | 0.828 |
| 52_104 | 0.828 |
| 234_96 | 0.828 |
| 128_52 | 0.828 |
| 56_58 | 0.828 |
| 68_52 | 0.828 |
| 156_46 | 0.828 |
| 56_81 | 0.828 |
| 46_200 | 0.828 |
| 166_84 | 0.828 |
| 52_178 | 0.828 |
| 142_66 | 0.828 |
| 56_140 | 0.828 |
| 144_72 | 0.828 |
| 57_52 | 0.828 |
| 147_11 | 0.828 |
| 95_72 | 0.828 |
| 159_53 | 0.828 |
| 72_82 | 0.828 |
| 73_26 | 0.828 |
| 72_151 | 0.828 |
| 89_52 | 0.828 |
| 234_115 | 0.828 |
| 174_72 | 0.828 |
| 64_5 | 0.828 |
| 23_150 | 0.828 |
| 219_85 | 0.828 |

(continued)

| SEQ ID NO. | AUC |
|------------|-------|
| 116_72 | 0.828 |
| 46_152 | 0.828 |
| 60_142 | 0.827 |
| 1_85 | 0.827 |
| 5_33 | 0.827 |
| 210_72 | 0.827 |
| 156_53 | 0.827 |
| 52_202 | 0.827 |
| 75_25 | 0.827 |
| 52_67 | 0.827 |
| 166_8 | 0.827 |
| 56_125 | 0.827 |
| 20_33 | 0.827 |
| 1_93 | 0.827 |
| 214_72 | 0.827 |
| 56_100 | 0.827 |
| 52_42 | 0.827 |
| 203_64 | 0.827 |
| 31_33 | 0.827 |
| 227_72 | 0.827 |
| 37_85 | 0.827 |
| 189_72 | 0.827 |
| 64_129 | 0.827 |
| 154_72 | 0.827 |
| 56_173 | 0.827 |
| 159_66 | 0.827 |
| 19_187 | 0.827 |
| 60_175 | 0.827 |
| 56_45 | 0.827 |
| 142_187 | 0.827 |
| 56_105 | 0.827 |
| 52_73 | 0.827 |
| 56_47 | 0.825 |
| 150_70 | 0.825 |
| 218_8 | 0.825 |
| 75_44 | 0.825 |
| 87_22 | 0.825 |
| 1_197 | 0.825 |

(continued)

| SEQ ID NO. | AUC |
|---|---|
| 93_213 | 0.825 |
| 56_109 | 0.825 |
| 63_72 | 0.825 |
| 56_83 | 0.825 |
| 52_206 | 0.825 |
| 56_112 | 0.825 |
| 225_72 | 0.825 |
| 56_114 | 0.825 |
| 56_154 | 0.825 |
| 228_72 | 0.825 |
| 12_52 | 0.825 |
| 234_61 | 0.825 |
| 138_72 | 0.825 |
| 8_32 | 0.825 |
| 56_117 | 0.825 |
| 56_208 | 0.825 |
| 44_187 | 0.825 |
| 60_81 | 0.825 |
| 33_16 | 0.825 |
| 234_200 | 0.825 |
| 234_2 | 0.825 |
| 75_166 | 0.825 |
| 24_72 | 0.825 |
| 8_98 | 0.825 |
| 8_16 | 0.825 |
| 211_72 | 0.825 |
| 56_145 | 0.825 |
| 159_23 | 0.825 |
| 60_230 | 0.823 |
| 28_33 | 0.823 |
| 196_85 | 0.823 |
| 25_196 | 0.823 |
| 60_157 | 0.823 |
| 58_52 | 0.823 |
| 46_100 | 0.823 |
| 38_185 | 0.823 |
| 81_226 | 0.823 |
| 64_109 | 0.823 |

(continued)

| SEQ ID NO. | AUC |
|---|---|
| 44_64 | 0.823 |
| 13_75 | 0.823 |
| 47_52 | 0.823 |
| 226_188 | 0.823 |
| 18_60 | 0.823 |
| 56_153 | 0.823 |
| 234_53 | 0.823 |
| 190_52 | 0.823 |
| 54_33 | 0.823 |
| 85_15 | 0.823 |
| 1_150 | 0.823 |
| 56_94 | 0.823 |
| 85_132 | 0.823 |
| 150_39 | 0.823 |
| 141_22 | 0.823 |
| 52_117 | 0.823 |
| 56_48 | 0.823 |
| 200_53 | 0.823 |
| 234_226 | 0.823 |
| 207_72 | 0.823 |
| 56_171 | 0.823 |
| 102_75 | 0.823 |
| 202_33 | 0.823 |
| 150_93 | 0.822 |
| 181_33 | 0.822 |
| 43_73 | 0.822 |
| 107_84 | 0.822 |
| 56_163 | 0.822 |
| 56_133 | 0.822 |
| 56_212 | 0.822 |
| 203_52 | 0.822 |
| 75_16 | 0.822 |
| 56_113 | 0.822 |
| 150_26 | 0.822 |
| 56_51 | 0.822 |
| 86_56 | 0.822 |
| 56_82 | 0.822 |
| 56_79 | 0.822 |

(continued)

| SEQ ID NO. | AUC |
|---|---|
| 46_75 | 0.822 |
| 226_11 | 0.822 |
| 80_33 | 0.822 |
| 28_232 | 0.822 |
| 75_32 | 0.822 |
| 114_33 | 0.822 |
| 8_214 | 0.822 |
| 18_66 | 0.822 |
| 79_187 | 0.822 |
| 56_90 | 0.822 |
| 120_56 | 0.822 |
| 87_33 | 0.822 |
| 43_56 | 0.822 |
| 234_175 | 0.822 |
| 56_167 | 0.822 |
| 56_182 | 0.822 |
| 18_150 | 0.822 |
| 56_143 | 0.822 |
| 56_3 | 0.822 |
| 52_132 | 0.822 |
| 79_53 | 0.822 |
| 235_72 | 0.822 |
| 64_53 | 0.822 |
| 52_147 | 0.822 |
| 56_192 | 0.822 |
| 53_206 | 0.822 |
| 234_157 | 0.822 |
| 75_233 | 0.822 |
| 85_144 | 0.822 |
| 121_33 | 0.820 |
| 150_224 | 0.820 |
| 87_73 | 0.820 |
| 225_8 | 0.820 |
| 64_41 | 0.820 |
| 46_151 | 0.820 |
| 120_52 | 0.820 |
| 46_213 | 0.820 |
| 148_52 | 0.820 |

(continued)

| SEQ ID NO. | AUC |
|---|---|
| 53_10 | 0.820 |
| 210_52 | 0.820 |
| 234_143 | 0.820 |
| 142_98 | 0.820 |
| 52_59 | 0.820 |
| 38_1 | 0.820 |
| 225_52 | 0.820 |
| 83_64 | 0.820 |
| 94_64 | 0.820 |
| 33_11 | 0.820 |
| 125_52 | 0.820 |
| 231_52 | 0.820 |
| 8_178 | 0.820 |
| 56_127 | 0.820 |
| 38_232 | 0.820 |
| 18_75 | 0.820 |
| 56_12 | 0.820 |
| 40_52 | 0.820 |
| 79_33 | 0.820 |
| 192_8 | 0.820 |
| 57_72 | 0.820 |
| 19_56 | 0.820 |
| 231_11 | 0.820 |
| 234_214 | 0.820 |
| 159_11 | 0.820 |
| 180_100 | 0.820 |
| 161_33 | 0.820 |
| 34_52 | 0.820 |
| 102_52 | 0.820 |
| 56_9 | 0.820 |
| 117_16 | 0.820 |
| 120_72 | 0.820 |
| 46_85 | 0.820 |
| 18_93 | 0.820 |
| 233_52 | 0.820 |
| 56_115 | 0.818 |
| 64_3 | 0.818 |
| 136_72 | 0.818 |

(continued)

| SEQ ID NO. | AUC |
|---|---|
| 56_95 | 0.818 |
| 8_147 | 0.818 |
| 56_207 | 0.818 |
| 225_234 | 0.818 |
| 202_98 | 0.818 |
| 81_52 | 0.818 |
| 166_52 | 0.818 |
| 58_72 | 0.818 |
| 56_67 | 0.818 |
| 56_61 | 0.818 |
| 38_75 | 0.818 |
| 56_129 | 0.818 |
| 112_52 | 0.818 |
| 56_76 | 0.818 |
| 159_209 | 0.818 |
| 60_182 | 0.818 |
| 23_53 | 0.818 |
| 38_73 | 0.818 |
| 46_32 | 0.818 |
| 161_60 | 0.818 |
| 203_33 | 0.818 |
| 38_150 | 0.818 |
| 60_206 | 0.818 |
| 159_39 | 0.818 |
| 18_166 | 0.818 |
| 129_157 | 0.817 |
| 150_12 | 0.817 |
| 154_8 | 0.817 |
| 203_11 | 0.817 |
| 32_110 | 0.817 |
| 215_52 | 0.817 |
| 184_53 | 0.817 |
| 64_59 | 0.817 |
| 166_16 | 0.817 |
| 79_222 | 0.817 |
| 8_222 | 0.817 |
| 150_132 | 0.817 |
| 44_96 | 0.817 |

(continued)

| SEQ ID NO. | AUC |
|---|---|
| 60_128 | 0.817 |
| 60_226 | 0.817 |
| 159_14 | 0.817 |
| 38_132 | 0.817 |
| 56_195 | 0.817 |
| 109_53 | 0.817 |
| 25_226 | 0.817 |
| 161_234 | 0.817 |
| 32_16 | 0.817 |
| 230_222 | 0.817 |
| 220_11 | 0.817 |
| 142_226 | 0.817 |
| 204_72 | 0.817 |
| 60_166 | 0.817 |
| 7_234 | 0.817 |
| 32_130 | 0.817 |
| 52_24 | 0.817 |
| 9_52 | 0.817 |
| 56_68 | 0.817 |
| 56_135 | 0.817 |
| 60_213 | 0.817 |
| 149_56 | 0.817 |
| 56_123 | 0.817 |
| 60_187 | 0.817 |
| 9_33 | 0.817 |
| 56_176 | 0.817 |
| 46_53 | 0.817 |
| 64_80 | 0.817 |
| 36_10 | 0.817 |
| 56_131 | 0.817 |
| 109_226 | 0.817 |
| 8_110 | 0.817 |
| 64_70 | 0.815 |
| 210_10 | 0.815 |
| 21_150 | 0.815 |
| 150_109 | 0.815 |
| 56_146 | 0.815 |
| 56_144 | 0.815 |

(continued)

| SEQ ID NO. | AUC |
|---|---|
| 65_72 | 0.815 |
| 171_222 | 0.815 |
| 33_29 | 0.815 |
| 150_30 | 0.815 |
| 56_152 | 0.815 |
| 64_67 | 0.815 |
| 64_15 | 0.815 |
| 33_153 | 0.815 |
| 124_52 | 0.815 |
| 80_11 | 0.815 |
| 175_85 | 0.815 |
| 1_52 | 0.815 |
| 56_97 | 0.815 |
| 95_52 | 0.815 |
| 56_54 | 0.815 |
| 1_230 | 0.815 |
| 224_33 | 0.815 |
| 56_231 | 0.815 |
| 53_11 | 0.815 |
| 1_75 | 0.815 |
| 189_52 | 0.815 |
| 89_75 | 0.815 |
| 56_213 | 0.815 |
| 187_70 | 0.815 |
| 99_52 | 0.815 |
| 228_53 | 0.815 |
| 148_33 | 0.815 |
| 67_16 | 0.815 |
| 93_123 | 0.815 |
| 234_210 | 0.815 |
| 19_73 | 0.815 |
| 234_80 | 0.815 |
| 46_192 | 0.815 |
| 85_232 | 0.815 |
| 230_33 | 0.815 |
| 56_162 | 0.815 |
| 234_152 | 0.815 |
| 73_70 | 0.815 |

(continued)

| SEQ ID NO. | AUC |
|---|---|
| 52_98 | 0.815 |
| 56_216 | 0.815 |
| 156_72 | 0.815 |
| 56_160 | 0.815 |
| 60_73 | 0.815 |
| 68_33 | 0.815 |
| 64_82 | 0.815 |
| 64_214 | 0.815 |
| 8_223 | 0.815 |
| 56_74 | 0.815 |
| 46_96 | 0.815 |
| 56_29 | 0.815 |
| 56_23 | 0.815 |
| 56_14 | 0.815 |
| 56_220 | 0.815 |
| 64_178 | 0.813 |
| 194_222 | 0.813 |
| 162_33 | 0.813 |
| 232_26 | 0.813 |
| 18_226 | 0.813 |
| 171_93 | 0.813 |
| 64_200 | 0.813 |
| 150_67 | 0.813 |
| 53_82 | 0.813 |
| 38_130 | 0.813 |
| 60_192 | 0.813 |
| 145_16 | 0.813 |
| 150_88 | 0.813 |
| 56_197 | 0.813 |
| 78_56 | 0.813 |
| 129_202 | 0.813 |
| 23_222 | 0.813 |
| 38_70 | 0.813 |
| 28_222 | 0.813 |
| 20_56 | 0.813 |
| 56_32 | 0.813 |
| 8_157 | 0.813 |
| 187_52 | 0.813 |

(continued)

| SEQ ID NO. | AUC |
|---|---|
| 56_190 | 0.813 |
| 79_226 | 0.813 |
| 64_26 | 0.813 |
| 60_218 | 0.813 |
| 10_232 | 0.813 |
| 56_215 | 0.813 |
| 78_159 | 0.813 |
| 52_223 | 0.813 |
| 234_222 | 0.813 |
| 196_76 | 0.813 |
| 60_159 | 0.813 |
| 75_66 | 0.813 |
| 157_93 | 0.813 |
| 230_16 | 0.813 |
| 75_226 | 0.812 |
| 213_45 | 0.812 |
| 60_227 | 0.812 |
| 159_64 | 0.812 |
| 55_64 | 0.812 |
| 56_44 | 0.812 |
| 53_178 | 0.812 |
| 150_10 | 0.812 |
| 28_53 | 0.812 |
| 152_166 | 0.812 |
| 56_59 | 0.812 |
| 187_85 | 0.812 |
| 219_93 | 0.812 |
| 38_216 | 0.812 |
| 180_226 | 0.812 |
| 96_53 | 0.812 |
| 149_64 | 0.812 |
| 1_110 | 0.812 |
| 105_226 | 0.812 |
| 56_183 | 0.812 |
| 148_73 | 0.812 |
| 186_26 | 0.812 |
| 169_52 | 0.812 |
| 53_15 | 0.812 |

(continued)

| SEQ ID NO. | AUC |
|---|---|
| 161_75 | 0.812 |
| 52_171 | 0.812 |
| 56_214 | 0.812 |
| 159_88 | 0.812 |
| 192_52 | 0.812 |
| 56_88 | 0.812 |
| 56_158 | 0.812 |
| 52_103 | 0.812 |
| 194_75 | 0.812 |
| 62_56 | 0.812 |
| 56_2 | 0.812 |
| 7_56 | 0.812 |
| 56_60 | 0.812 |
| 56_27 | 0.812 |
| 234_178 | 0.810 |
| 8_74 | 0.810 |
| 44_226 | 0.810 |
| 181_11 | 0.810 |
| 187_188 | 0.810 |
| 1_11 | 0.810 |
| 234_29 | 0.810 |
| 38_33 | 0.810 |
| 157_85 | 0.810 |
| 63_11 | 0.810 |
| 52_185 | 0.810 |
| 99_33 | 0.810 |
| 234_201 | 0.810 |
| 53_93 | 0.810 |
| 96_85 | 0.810 |
| 124_56 | 0.810 |
| 125_222 | 0.810 |
| 187_93 | 0.810 |
| 159_76 | 0.810 |
| 19_33 | 0.810 |
| 75_129 | 0.810 |
| 64_110 | 0.810 |
| 184_75 | 0.810 |
| 52_232 | 0.810 |

(continued)

| SEQ ID NO. | AUC |
|---|---|
| 56_139 | 0.810 |
| 189_56 | 0.810 |
| 46_60 | 0.810 |
| 50_52 | 0.810 |
| 234_219 | 0.810 |
| 155_110 | 0.810 |
| 150_37 | 0.810 |
| 56_21 | 0.810 |
| 142_10 | 0.810 |
| 46_232 | 0.810 |
| 166_80 | 0.810 |
| 66_232 | 0.810 |
| 73_147 | 0.810 |
| 155_33 | 0.810 |
| 20_150 | 0.810 |
| 1_8 | 0.810 |
| 60_65 | 0.810 |
| 60_231 | 0.810 |
| 56_224 | 0.810 |
| 56_223 | 0.810 |
| 73_15 | 0.810 |
| 234_100 | 0.810 |
| 56_42 | 0.810 |
| 110_11 | 0.810 |
| 93_117 | 0.810 |
| 66_226 | 0.810 |
| 187_11 | 0.808 |
| 230_37 | 0.808 |
| 8_15 | 0.808 |
| 38_219 | 0.808 |
| 159_31 | 0.808 |
| 215_32 | 0.808 |
| 37_33 | 0.808 |
| 85_33 | 0.808 |
| 48_33 | 0.808 |
| 64_171 | 0.808 |
| 229_64 | 0.808 |
| 64_122 | 0.808 |

(continued)

| SEQ ID NO. | AUC |
|---|---|
| 1_187 | 0.808 |
| 28_56 | 0.808 |
| 89_8 | 0.808 |
| 56_99 | 0.808 |
| 18_230 | 0.808 |
| 227_64 | 0.808 |
| 214_53 | 0.808 |
| 75_153 | 0.808 |
| 73_110 | 0.808 |
| 32_52 | 0.808 |
| 60_123 | 0.808 |
| 73_45 | 0.808 |
| 34_33 | 0.808 |
| 234_224 | 0.808 |
| 155_150 | 0.808 |
| 60_11 | 0.808 |
| 156_142 | 0.808 |
| 21_159 | 0.808 |
| 60_15 | 0.808 |
| 184_234 | 0.808 |
| 75_201 | 0.808 |
| 212_93 | 0.808 |
| 75_191 | 0.808 |
| 56_201 | 0.808 |
| 53_204 | 0.808 |
| 229_73 | 0.808 |
| 56_111 | 0.808 |
| 198_33 | 0.808 |
| 38_16 | 0.808 |
| 46_212 | 0.808 |
| 38_175 | 0.808 |
| 56_211 | 0.808 |
| 133_52 | 0.808 |
| 132_11 | 0.807 |
| 234_39 | 0.807 |
| 234_26 | 0.807 |
| 75_232 | 0.807 |
| 231_8 | 0.807 |

(continued)

| SEQ ID NO. | AUC |
|---|---|
| 68_8 | 0.807 |
| 56_194 | 0.807 |
| 60_91 | 0.807 |
| 215_11 | 0.807 |
| 60_200 | 0.807 |
| 46_66 | 0.807 |
| 154_73 | 0.807 |
| 216_52 | 0.807 |
| 60_219 | 0.807 |
| 234_197 | 0.807 |
| 80_73 | 0.807 |
| 52_137 | 0.807 |
| 64_170 | 0.807 |
| 75_172 | 0.807 |
| 3_33 | 0.807 |
| 60_152 | 0.807 |
| 52_196 | 0.807 |
| 56_168 | 0.807 |
| 73_33 | 0.807 |
| 106_64 | 0.807 |
| 31_213 | 0.807 |
| 75_98 | 0.807 |
| 90_11 | 0.807 |
| 56_186 | 0.807 |
| 145_11 | 0.807 |
| 38_144 | 0.807 |
| 20_187 | 0.807 |
| 60_171 | 0.807 |
| 60_130 | 0.807 |
| 53_73 | 0.807 |
| 109_73 | 0.807 |
| 11_15 | 0.807 |
| 19_226 | 0.807 |
| 38_96 | 0.807 |
| 106_15 | 0.807 |
| 192_53 | 0.807 |
| 52_168 | 0.807 |
| 137_33 | 0.807 |

(continued)

| SEQ ID NO. | AUC |
|---|---|
| 93_206 | 0.807 |
| 60_115 | 0.807 |
| 8_130 | 0.807 |
| 56_177 | 0.807 |
| 223_33 | 0.807 |
| 56_233 | 0.807 |
| 159_105 | 0.807 |
| 168_11 | 0.807 |
| 60_106 | 0.805 |
| 56_89 | 0.805 |
| 129_33 | 0.805 |
| 64_123 | 0.805 |
| 63_85 | 0.805 |
| 173_11 | 0.805 |
| 96_93 | 0.805 |
| 75_93 | 0.805 |
| 234_203 | 0.805 |
| 176_11 | 0.805 |
| 75_187 | 0.805 |
| 75_210 | 0.805 |
| 32_33 | 0.805 |
| 72_139 | 0.805 |
| 33_45 | 0.805 |
| 35_52 | 0.805 |
| 222_226 | 0.805 |
| 111_53 | 0.805 |
| 75_24 | 0.805 |
| 75_17 | 0.805 |
| 194_8 | 0.805 |
| 234_66 | 0.805 |
| 64_104 | 0.805 |
| 86_8 | 0.805 |
| 224_16 | 0.805 |
| 150_11 | 0.805 |
| 75_209 | 0.805 |
| 107_72 | 0.805 |
| 60_36 | 0.805 |
| 167_11 | 0.805 |

(continued)

| SEQ ID NO. | AUC |
|---|---|
| 85_147 | 0.805 |
| 71_33 | 0.805 |
| 159_222 | 0.805 |
| 142_33 | 0.805 |
| 64_31 | 0.805 |
| 62_33 | 0.805 |
| 56_148 | 0.805 |
| 56_39 | 0.805 |
| 102_222 | 0.805 |
| 159_10 | 0.805 |
| 60_110 | 0.805 |
| 53_168 | 0.803 |
| 14_33 | 0.803 |
| 109_11 | 0.803 |
| 156_222 | 0.803 |
| 71_11 | 0.803 |
| 160_52 | 0.803 |
| 78_33 | 0.803 |
| 36_72 | 0.803 |
| 234_199 | 0.803 |
| 75_69 | 0.803 |
| 8_93 | 0.803 |
| 38_37 | 0.803 |
| 150_4 | 0.803 |
| 79_8 | 0.803 |
| 38_139 | 0.803 |
| 60_75 | 0.803 |
| 8_174 | 0.803 |
| 52_199 | 0.803 |
| 119_10 | 0.803 |
| 94_53 | 0.803 |
| 66_17 | 0.803 |
| 38_159 | 0.803 |
| 28_150 | 0.803 |
| 121_64 | 0.803 |
| 60_201 | 0.803 |
| 150_16 | 0.803 |
| 144_11 | 0.803 |

(continued)

| SEQ ID NO. | AUC |
|---|---|
| 187_209 | 0.803 |
| 8_207 | 0.803 |
| 101_52 | 0.803 |
| 222_98 | 0.803 |
| 53_113 | 0.803 |
| 53_213 | 0.803 |
| 19_150 | 0.803 |
| 56_179 | 0.803 |
| 56_191 | 0.803 |
| 187_45 | 0.803 |
| 161_53 | 0.803 |
| 62_150 | 0.803 |
| 112_64 | 0.803 |
| 46_171 | 0.803 |
| 33_168 | 0.803 |
| 85_73 | 0.803 |
| 234_93 | 0.803 |
| 64_202 | 0.803 |
| 13_226 | 0.803 |
| 38_206 | 0.803 |
| 8_80 | 0.803 |
| 88_11 | 0.803 |
| 230_202 | 0.803 |
| 156_56 | 0.803 |
| 200_37 | 0.803 |
| 8_175 | 0.803 |
| 38_65 | 0.803 |
| 117_33 | 0.803 |
| 46_10 | 0.803 |
| 194_53 | 0.803 |
| 60_74 | 0.802 |
| 8_10 | 0.802 |
| 64_168 | 0.802 |
| 142_113 | 0.802 |
| 75_173 | 0.802 |
| 121_73 | 0.802 |
| 159_70 | 0.802 |
| 116_60 | 0.802 |

(continued)

| SEQ ID NO. | AUC |
|---|---|
| 93_74 | 0.802 |
| 33_76 | 0.802 |
| 75_167 | 0.802 |
| 234_14 | 0.802 |
| 61_33 | 0.802 |
| 33_208 | 0.802 |
| 163_11 | 0.802 |
| 183_11 | 0.802 |
| 127_24 | 0.802 |
| 142_166 | 0.802 |
| 177_11 | 0.802 |
| 23_206 | 0.802 |
| 60_151 | 0.802 |
| 156_8 | 0.802 |
| 150_2 | 0.802 |
| 25_213 | 0.802 |
| 64_219 | 0.802 |
| 152_66 | 0.802 |
| 38_110 | 0.802 |
| 142_204 | 0.802 |
| 182_11 | 0.802 |
| 150_222 | 0.802 |
| 64_151 | 0.802 |
| 64_16 | 0.802 |
| 73_130 | 0.802 |
| 213_33 | 0.802 |
| 32_151 | 0.802 |
| 56_170 | 0.802 |
| 137_11 | 0.802 |
| 187_10 | 0.802 |
| 150_180 | 0.802 |
| 1_222 | 0.802 |
| 56_198 | 0.802 |
| 75_146 | 0.802 |
| 64_130 | 0.802 |
| 56_164 | 0.802 |
| 50_72 | 0.802 |
| 66_33 | 0.802 |

(continued)

| SEQ ID NO. | AUC |
|------------|-------|
| 53_232 | 0.802 |
| 228_52 | 0.802 |
| 64_204 | 0.802 |
| 134_85 | 0.802 |
| 219_11 | 0.802 |
| 156_75 | 0.802 |
| 184_33 | 0.802 |
| 56_221 | 0.802 |
| 234_171 | 0.802 |
| 234_91 | 0.802 |
| 38_123 | 0.802 |
| 161_150 | 0.802 |
| 8_181 | 0.800 |
| 85_16 | 0.800 |
| 8_127 | 0.800 |
| 28_75 | 0.800 |
| 166_132 | 0.800 |
| 56_92 | 0.800 |
| 212_180 | 0.800 |
| 52_204 | 0.800 |
| 75_99 | 0.800 |
| 155_11 | 0.800 |
| 115_85 | 0.800 |
| 202_10 | 0.800 |
| 234_63 | 0.800 |
| 234_49 | 0.800 |
| 220_64 | 0.800 |
| 231_16 | 0.800 |
| 230_226 | 0.800 |
| 52_65 | 0.800 |
| 25_11 | 0.800 |
| 64_147 | 0.800 |
| 234_55 | 0.800 |
| 226_73 | 0.800 |
| 60_172 | 0.800 |
| 75_73 | 0.800 |
| 142_159 | 0.800 |
| 150_130 | 0.800 |

(continued)

| SEQ ID NO. | AUC |
|---|---|
| 64_29 | 0.800 |
| 196_11 | 0.800 |
| 20_232 | 0.800 |
| 28_227 | 0.800 |
| 150_202 | 0.800 |
| 38_226 | 0.800 |
| 178_33 | 0.800 |
| 93_16 | 0.800 |
| 103_33 | 0.800 |
| 44_213 | 0.800 |
| 60_186 | 0.800 |
| 60_1 | 0.800 |
| 80_222 | 0.800 |
| 64_198 | 0.800 |
| 182_85 | 0.800 |
| 171_53 | 0.800 |
| 234_202 | 0.800 |
| 1_66 | 0.800 |
| 1_106 | 0.800 |
| 53_196 | 0.800 |
| 95_16 | 0.800 |
| 8_165 | 0.800 |
| 79_150 | 0.800 |
| 234_36 | 0.800 |
| 31_187 | 0.800 |
| 159_2 | 0.800 |
| 162_11 | 0.800 |
| 234_15 | 0.800 |
| 108_16 | 0.800 |
| 63_52 | 0.800 |
| 60_164 | 0.800 |
| 52_41 | 0.800 |
| 44_73 | 0.800 |
| 1_203 | 0.800 |
| 52_36 | 0.800 |
| 56_181 | 0.800 |
| 230_187 | 0.800 |
| 94_52 | 0.800 |

(continued)

| SEQ ID NO. | AUC |
|---|---|
| 8_188 | 0.800 |

[Example 3]

<Evaluation of discriminant performance of gene marker for discrimination between ovarian cancer and benign ovarian tumor by comparison of ovarian cancer patient with benign ovarian tumor patient and healthy subject>

In this Example, the discrimination accuracy of cross-validation was calculated on the basis of a discriminant formula based on two gene markers for the discrimination between ovarian cancer and benign ovarian tumor (SEQ ID NOs: 1 to 235). Based on the calculation, whether the gene markers for the discrimination between ovarian cancer and benign ovarian tumor selected in Examples 1 and 2 could discriminate ovarian cancer patients from benign ovarian tumor patients even when healthy subjects are included in a negative cohort, was evaluated.

The selection of genes and the calculation of AUC were carried out in the same way as in Example 1 except that healthy subjects were included in a negative cohort.

Combinations of SEQ ID NOs and AUC of two of the markers of SEQ ID NOs: 1 to 235 that exhibited AUC of 0.8 or more are shown in Table 9.

For example, the discriminant formula based on the combination of the genes represented by SEQ ID NOs: 8 and 52 among the combinations in Table 9 offered AUC as high as 0.993, revealing that a positive cohort involving ovarian cancer patients can be discriminated with high performance from a negative cohort involving benign ovarian tumor patients even when healthy subjects are included in the negative cohort.

From the above, it was demonstrated that all polynucleotides consisting of the nucleotide sequences represented by SEQ ID NOs: 1 to 235 are genes or nucleic acids that can discriminate a positive cohort including ovarian cancer patients from a negative cohort including benign ovarian tumor patients and healthy subjects with high performance.

[Table 9]

| | Discriminant | CA-125 | CA19-9 | CEA |
|---|---|---|---|---|
| Sensitivity | 81.8 | 90.9 | 29.5 | 11.4 |
| Specificity (negative = healthy) | 71.1 | 100.0 | 89.7 | 94.7 |
| Specificity (negative = benign) | 93.3 | 33.3 | 66.7 | 85.7 |

Specifically, as shown in Tables 2 to 9 in Examples 1 to 3 above, all polynucleotides consisting of the nucleotide sequences represented by SEQ ID NOs: 1 to 235 are excellent diagnostic markers which exhibit discriminant performance equal to or better than that of the existing tumor marker when these are used alone or in combinations of two, three, four, or five in the discrimination of ovarian cancer.

[Example 4]

<Evaluation of gene marker for discrimination by comparison between ovarian cancer patient and benign ovarian tumor patient by use of quantitative RT-PCR>

In this Example, expression levels of two gene markers for the discrimination between ovarian cancer and benign ovarian tumor (SEQ ID NOs: 1 and 8) selected in Example 1 were compared between ovarian cancer patients and benign ovarian tumor patients by use of quantitative RT-PCR. Whether the gene markers found in the preceding Example were useful even when quantitative RT-PCR was used, was thereby evaluated.

Specifically, first, cDNA was obtained by the reverse transcription of miRNA in total RNA, which was obtained from each of three ovarian cancer patients and three benign ovarian tumor patients, by use of TaqMan(TM) Advanced microRNA cDNA Synthesis Kit (Thermo Fisher Scientific Inc.) according to the protocol provided by the manufacturer. The obtained cDNA was subjected to real-time PCR based on a real-time PCR system (CFX96, Bio-Rad Laboratories, Inc.) by use of TaqMan(TM) Advanced microRNA Assays according to the protocol provided by the manufacturer. After the completion of real-time PCR reaction, two-group comparison was carried out by the ΔΔCt method based on the obtained Ct values. As a result of carrying out a two-sided t-test assuming equal variance in the ovarian cancer patient group and the benign ovarian tumor patient group, the marker of SEQ ID NO: 1 had a P value of less than 0.01 and the marker of SEQ ID NO: 8 had a P value of less than 0.1. It was thus demonstrated that these gene markers for discrimination can statistically discriminate ovarian cancer patient groups from benign ovarian tumor patient groups when their expression levels measured by quantitative RT-PCR are used. The obtained results about the P values are shown in Table 10.

[Table 10]

| SEQ ID NO. | Name of gene | p value of cancer/benign |
|---|---|---|
| 1 | hsa-miR-1908-5p | 5.6E-03 |
| 8 | hsa-miR-4525 | 7.3E-02 |

From the above, the gene markers shown in SEQ ID NOs: 1 to 9 and SEQ ID NOs: 10 to 235 and combinations thereof can discriminate ovarian cancer patients from benign ovarian tumor patients even when their expression levels measured by a gene assay method other than microarrays, such as quantitative RT-PCR, are used.

As shown in Examples above, the kit, the device and the method of the present invention enable early preoperative discrimination between ovarian cancer and benign ovarian tumor, and conducting an appropriate response for each case because ovarian cancer and benign ovarian tumor can be discriminated with higher performance than that of the existing tumor marker. As a result, early treatment of ovarian cancer or the avoidance of unnecessary surgery for benign ovarian tumor patients can be attained, leading to improvement in survival rate and reduction in emotional, physical, and economical burdens on subjects.

Industrial Applicability

According to the target nucleic acid(s) of the present invention, ovarian cancer and benign ovarian tumor can be effectively discriminated by a simple and inexpensive method. This enables early detection, diagnosis and treatment of an ovarian cancer. Also, the method of the present invention enables less-invasive, easy and rapid preoperative discrimination between ovarian cancer and benign ovarian tumor using a sample, such as blood, from a patient and can therefore avoid unnecessary surgery for benign ovarian tumor patients while enabling early detection and treatment of ovarian cancer.

All publications, patents, and patent applications cited herein are incorporated herein by reference in their entirety.

**Claims**

1. A kit for discrimination between ovarian cancer and benign ovarian tumor, comprising a probe(s) and/or a primer(s) for detecting at least one polynucleotide selected from the group consisting of the following markers for discriminating between ovarian cancer and benign ovarian tumor: miR-1908-5p, miR-4723-5p, miR-4674, miR-939-5p, miR-6789-5p, miR-1268a, miR-1202, miR-4525, and miR-128-1-5p.

2. The kit according to claim 1, wherein the probe(s) and/or primer(s) is a polynucleotide(s) selected from the group consisting of the following polynucleotides (a) to (e):

   (a) a polynucleotide consisting of a nucleotide sequence represented by any of SEQ ID NOs: 1 to 9, or a nucleotide sequence derived from the nucleotide sequence by the replacement of u with t, a variant thereof, a derivative thereof, or a fragment thereof comprising 15 or more consecutive nucleotides;
   (b) a polynucleotide comprising a nucleotide sequence represented by any of SEQ ID NOs: 1 to 9;
   (c) a polynucleotide consisting of a nucleotide sequence complementary to a nucleotide sequence represented by any of SEQ ID NOs: 1 to 9, or a nucleotide sequence derived from the nucleotide sequence by the replacement of u with t, a variant thereof, a derivative thereof, or a fragment thereof comprising 15 or more consecutive nucleotides;
   (d) a polynucleotide comprising a nucleotide sequence complementary to a nucleotide sequence represented by any of SEQ ID NOs: 1 to 9, or a nucleotide sequence derived from the nucleotide sequence by the replacement of u with t; and
   (e) a polynucleotide hybridizing under stringent conditions to any of the polynucleotides (a) to (d).

3. The kit according to claim 1 or 2, wherein the kit further comprises a probe(s) and/or a primer(s) for detecting at least one polynucleotide selected from the group consisting of the following additional markers for discriminating between ovarian cancer and benign ovarian tumor: miR-6806-5p, miR-7845-5p, miR-4632-5p, miR-10396b-5p, miR-6768-5p, miR-8059, miR-8072, miR-9901, miR-1231, miR-1225-5p, miR-12114, miR-3178, miR-6798-5p, miR-4276, miR-6125, miR-3652, miR-7111-5p, miR-6749-5p, miR-1199-5p, miR-6802-5p, miR-6816-5p, miR-4706, miR-5008-5p, miR-6797-5p, miR-4516, miR-4508, miR-6729-5p, miR-564, miR-1233-5p, miR-6127, miR-1469, miR-6738-5p, miR-6785-5p, miR-10401-5p, miR-4430, miR-6889-5p, miR-1236-5p, miR-3176, miR-3141, miR-3928-3p, miR-1237-5p, miR-1915-3p, miR-5195-3p, miR-6743-5p, miR-6746-5p, miR-4446-3p, miR-1228-5p, miR-1268b, miR-1260a, miR-6879-3p, miR-149-3p, miR-3162-5p, miR-1207-5p, miR-4747-3p, miR-4651, miR-638, miR-4736, miR-6845-5p, miR-1343-3p, miR-6126, miR-92b-5p, miR-6774-5p, miR-7847-3p, miR-6795-5p, miR-7109-5p, miR-3197, miR-6824-5p, miR-6771-5p, miR-11399, miR-2861, miR-4707-3p, miR-4638-5p, miR-8073, miR-328-5p, miR-665, miR-6778-5p, miR-10398-3p, miR-5698, miR-6794-5p, miR-1247-3p, miR-4697-5p, miR-8069, miR-572, miR-6751-5p, miR-3180-3p, miR-486-3p, miR-6086, miR-30c-1-3p, miR-8063, miR-3621, miR-6887-5p, miR-3191-3p, miR-11181-3p, miR-6722-5p, miR-6781-5p, miR-5739, miR-3937, miR-1343-5p, miR-1181, miR-4725-3p, miR-6865-5p, miR-375-5p, miR-3196, miR-6762-5p, miR-4258, miR-5196-5p, miR-10401-3p, miR-675-5p, miR-4488, miR-10527-5p, miR-10396a-5p, miR-4269, miR-6800-5p, miR-6819-5p, miR-10396b-3p, miR-4688, miR-6786-5p, miR-4634, miR-3940-5p, miR-4655-5p, miR-7155-5p, miR-6769b-5p, miR-6810-5p, miR-4665-3p, miR-6727-5p, miR-6803-5p, miR-4640-5p, miR-6735-5p, miR-4535, miR-8089, miR-1292-3p, miR-5088-5p, miR-3622a-5p, miR-6124, miR-6820-5p, miR-6805-3p, miR-4513, miR-760, miR-4665-5p, miR-10400-3p, miR-4298, miR-8085, miR-4463, miR-6807-5p, miR-4433b-3p, miR-3185, miR-12121, miR-671-5p, miR-6752-5p, miR-371a-5p, miR-3917, miR-1224-5p, miR-498-5p, miR-7704, miR-6741-5p, miR-765, miR-4486, miR-6090, miR-718, miR-4767, miR-6851-5p, miR-5572, miR-6850-5p, miR-6089, miR-5787, miR-4534, miR-3665, miR-4787-5p, miR-6754-5p, miR-6825-3p, miR-4728-5p, miR-6088, miR-3154, miR-6869-5p, miR-187-5p, miR-6165, miR-4447, miR-4731-5p, miR-6805-5p, miR-12118, miR-4270, miR-7846-3p, miR-4443, miR-6737-5p, miR-197-5p, miR-1229-5p, miR-6757-5p, miR-6765-5p, miR-4722-5p, miR-6891-5p, miR-5006-5p, miR-345-3p, miR-6726-5p, miR-3195, miR-6877-5p, miR-4462, miR-6812-5p, miR-483-5p, miR-9899, miR-4800-5p, miR-4734, miR-3135b, miR-4433a-3p, miR-6769a-5p, miR-4743-5p, miR-1909-3p, miR-4741, miR-4685-5p, miR-3147, miR-4726-5p, miR-3180, miR-3188, miR-6782-5p, miR-6776-5p, miR-4484, miR-1185-1-3p, miR-6790-3p, miR-4466, miR-10394-3p, miR-1275, miR-4478, miR-3175, miR-7106-5p, miR-4667-5p, miR-193b-5p, and miR-602.

4. The kit according to claim 3, wherein the probe(s) and/or primer(s) is a polynucleotide(s) selected from the group consisting of the following polynucleotides (a) to (e):

(a) a polynucleotide consisting of a nucleotide sequence represented by any of SEQ ID NOs: 10 to 235, or a nucleotide sequence derived from the nucleotide sequence by the replacement of u with t, a variant thereof, a derivative thereof, or a fragment thereof comprising 15 or more consecutive nucleotides;

(b) a polynucleotide comprising a nucleotide sequence represented by any of SEQ ID NOs: 10 to 235;

(c) a polynucleotide consisting of a nucleotide sequence complementary to a nucleotide sequence represented by any of SEQ ID NOs: 10 to 235, or a nucleotide sequence derived from the nucleotide sequence by the replacement of u with t, a variant thereof, a derivative thereof, or a fragment thereof comprising 15 or more consecutive nucleotides;

(d) a polynucleotide comprising a nucleotide sequence complementary to a nucleotide sequence represented by any of SEQ ID NOs: 10 to 235, or a nucleotide sequence derived from the nucleotide sequence by the replacement of u with t; and

(e) a polynucleotide hybridizing under stringent conditions to any of the polynucleotides (a) to (d).

5. A device for discrimination between ovarian cancer and benign ovarian tumor, comprising a probe(s) and/or a primer(s) for detecting at least one polynucleotide selected from the group consisting of the following markers for discriminating between ovarian cancer and benign ovarian tumor: miR-1908-5p, miR-4723-5p, miR-4674, miR-939-5p, miR-6789-5p, miR-1268a, miR-1202, miR-4525, and miR-128-1-5p.

6. The device according to claim 5, wherein the probe(s) and/or primer(s) is a polynucleotide(s) selected from the group consisting of the following polynucleotides (a) to (e):

(a) a polynucleotide consisting of a nucleotide sequence represented by any of SEQ ID NOs: 1 to 9, or a nucleotide sequence derived from the nucleotide sequence by the replacement of u with t, a variant thereof, a derivative thereof, or a fragment thereof comprising 15 or more consecutive nucleotides;

(b) a polynucleotide comprising a nucleotide sequence represented by any of SEQ ID NOs: 1 to 9;

(c) a polynucleotide consisting of a nucleotide sequence complementary to a nucleotide sequence represented by any of SEQ ID NOs: 1 to 9, or a nucleotide sequence derived from the nucleotide sequence by the replacement of u with t, a variant thereof, a derivative thereof, or a fragment thereof comprising 15 or more consecutive nucleotides;

(d) a polynucleotide comprising a nucleotide sequence complementary to a nucleotide sequence represented by any of SEQ ID NOs: 1 to 9, or a nucleotide sequence derived from the nucleotide sequence by the replacement of u with t; and

(e) a polynucleotide hybridizing under stringent conditions to any of the polynucleotides (a) to (d).

7. The device according to claim 5 or 6, wherein the device further comprises a probe(s) and/or a primer(s) for detecting at least one polynucleotide selected from the group consisting of the following additional markers for discriminating between ovarian cancer and benign ovarian tumor: miR-6806-5p, miR-7845-5p, miR-4632-5p, miR-10396b-5p, miR-6768-5p, miR-8059, miR-8072, miR-9901, miR-1231, miR-1225-5p, miR-12114, miR-3178, miR-6798-5p, miR-4276, miR-6125, miR-3652, miR-7111-5p, miR-6749-5p, miR-1199-5p, miR-6802-5p, miR-6816-5p, miR-4706, miR-5008-5p, miR-6797-5p, miR-4516, miR-4508, miR-6729-5p, miR-564, miR-1233-5p, miR-6127, miR-1469, miR-6738-5p, miR-6785-5p, miR-10401-5p, miR-4430, miR-6889-5p, miR-1236-5p, miR-3176, miR-3141, miR-3928-3p, miR-1237-5p, miR-1915-3p, miR-5195-3p, miR-6743-5p, miR-6746-5p, miR-4446-3p, miR-1228-5p, miR-1268b, miR-1260a, miR-6879-3p, miR-149-3p, miR-3162-5p, miR-1207-5p, miR-4747-3p, miR-4651, miR-638, miR-4736, miR-6845-5p, miR-1343-3p, miR-6126, miR-92b-5p, miR-6774-5p, miR-7847-3p, miR-6795-5p, miR-7109-5p, miR-3197, miR-6824-5p, miR-6771-5p, miR-11399, miR-2861, miR-4707-3p, miR-4638-5p, miR-8073, miR-328-5p, miR-665, miR-6778-5p, miR-10398-3p, miR-5698, miR-6794-5p, miR-1247-3p, miR-4697-5p, miR-8069, miR-572, miR-6751-5p, miR-3180-3p, miR-486-3p, miR-6086, miR-30c-1-3p, miR-8063, miR-3621, miR-6887-5p, miR-3191-3p, miR-11181-3p, miR-6722-5p, miR-6781-5p, miR-5739, miR-3937, miR-1343-5p, miR-1181, miR-4725-3p, miR-6865-5p, miR-375-5p, miR-3196, miR-6762-5p, miR-4258, miR-5196-5p, miR-10401-3p, miR-675-5p, miR-4488, miR-10527-5p, miR-10396a-5p, miR-4269, miR-6800-5p, miR-6819-5p, miR-10396b-3p, miR-4688, miR-6786-5p, miR-4634, miR-3940-5p, miR-4655-5p, miR-7155-5p, miR-6769b-5p, miR-6810-5p, miR-4665-3p, miR-6727-5p, miR-6803-5p, miR-4640-5p, miR-6735-5p, miR-4535, miR-8089, miR-1292-3p, miR-5088-5p, miR-3622a-5p, miR-6124, miR-6820-5p, miR-6805-3p, miR-4513, miR-760, miR-4665-5p, miR-10400-3p, miR-4298, miR-8085, miR-4463, miR-6807-5p, miR-4433b-3p, miR-3185, miR-12121, miR-671-5p, miR-6752-5p, miR-371a-5p, miR-3917, miR-1224-5p, miR-498-5p, miR-7704, miR-6741-5p, miR-765, miR-4486, miR-6090, miR-718, miR-4767, miR-6851-5p, miR-5572, miR-6850-5p, miR-6089, miR-5787, miR-4534, miR-3665, miR-4787-5p, miR-6754-5p, miR-6825-3p, miR-4728-5p, miR-6088, miR-3154, miR-6869-5p, miR-187-5p, miR-6165, miR-4447, miR-4731-5p, miR-6805-5p, miR-12118, miR-4270, miR-7846-3p, miR-4443, miR-6737-5p, miR-197-5p, miR-1229-5p, miR-6757-5p, miR-6765-5p, miR-4722-5p, miR-6891-5p, miR-5006-5p, miR-345-3p, miR-6726-5p, miR-3195, miR-6877-5p, miR-4462, miR-6812-5p, miR-483-5p, miR-9899, miR-4800-5p, miR-4734, miR-3135b, miR-4433a-3p, miR-6769a-5p, miR-4743-5p, miR-1909-3p, miR-4741, miR-4685-5p, miR-3147, miR-4726-5p, miR-3180, miR-3188, miR-6782-5p, miR-6776-5p, miR-4484, miR-1185-1-3p, miR-6790-3p, miR-4466, miR-10394-3p, miR-1275, miR-4478, miR-3175, miR-7106-5p, miR-4667-5p, miR-193b-5p, and miR-602.

8. The device according to claim 7, wherein the probe(s) and/or primer(s) is a polynucleotide(s) selected from the group consisting of the following polynucleotides (a) to (e):

(a) a polynucleotide consisting of a nucleotide sequence represented by any of SEQ ID NOs: 10 to 235, or a nucleotide sequence derived from the nucleotide sequence by the replacement of u with t, a variant thereof, a derivative thereof, or a fragment thereof comprising 15 or more consecutive nucleotides;

(b) a polynucleotide comprising a nucleotide sequence represented by any of SEQ ID NOs: 10 to 235;

(c) a polynucleotide consisting of a nucleotide sequence complementary to a nucleotide sequence represented by any of SEQ ID NOs: 10 to 235, or a nucleotide sequence derived from the nucleotide sequence by the replacement of u with t, a variant thereof, a derivative thereof, or a fragment thereof comprising 15 or more consecutive nucleotides;

(d) a polynucleotide comprising a nucleotide sequence complementary to a nucleotide sequence represented by any of SEQ ID NOs: 10 to 235, or a nucleotide sequence derived from the nucleotide sequence by the replacement of u with t; and

(e) a polynucleotide hybridizing under stringent conditions to any of the polynucleotides (a) to (d).

9. The device according to any one of claims 5 to 8, wherein the device is a device for measurement by a hybridization technique.

10. The device according to claim 9, wherein the hybridization technique is a nucleic acid array technique.

**11.** A method for discriminating between ovarian cancer and benign ovarian tumor, comprising: measuring an expression level(s) of at least one polynucleotide selected from the group consisting of the following markers for discriminating between ovarian cancer and benign ovarian tumor: miR-1908-5p, miR-4723-5p, miR-4674, miR-939-5p, miR-6789-5p, miR-1268a, miR-1202, miR-4525, and miR-128-1-5p in a sample from a subject; and evaluating *in vitro* whether the subject has ovarian cancer or is a benign ovarian tumor patient using the measured expression level(s) and a control expression level(s) from a benign ovarian tumor patient measured in the same manner.

**12.** The method according to claim 11, comprising assigning the expression level(s) of the target gene(s) in the sample from the subject to a discriminant formula, which is capable of discriminatorily determining ovarian cancer and benign ovarian tumor and which is prepared with gene expression levels in a sample(s) from a patient(s) known to have ovarian cancer and a sample(s) from a benign ovarian tumor patient(s) as training samples, and thereby evaluating the presence of ovarian cancer or the presence of benign ovarian tumor.

**13.** The method according to claim 11 or 12, comprising measuring an expression level(s) of the polynucleotide using a kit according to any one of claims 1 to 4 or a device according to any one of claims 5 to 10, wherein the kit or the device comprises a probe(s) and/or a primer(s) for detecting the polynucleotide.

**14.** The method according to claim 13, wherein the probe(s) and/or primer(s) is a polynucleotide(s) selected from the group consisting of the following polynucleotides (a) to (e):

(a) a polynucleotide consisting of a nucleotide sequence represented by any of SEQ ID NOs: 1 to 9, or a nucleotide sequence derived from the nucleotide sequence by the replacement of u with t, a variant thereof, a derivative thereof, or a fragment thereof comprising 15 or more consecutive nucleotides;
(b) a polynucleotide comprising a nucleotide sequence represented by any of SEQ ID NOs: 1 to 9;
(c) a polynucleotide consisting of a nucleotide sequence complementary to a nucleotide sequence represented by any of SEQ ID NOs: 1 to 9, or a nucleotide sequence derived from the nucleotide sequence by the replacement of u with t, a variant thereof, a derivative thereof, or a fragment thereof comprising 15 or more consecutive nucleotides;
(d) a polynucleotide comprising a nucleotide sequence complementary to a nucleotide sequence represented by any of SEQ ID NOs: 1 to 9, or a nucleotide sequence derived from the nucleotide sequence by the replacement of u with t; and
(e) a polynucleotide hybridizing under stringent conditions to any of the polynucleotides (a) to (d).

**15.** The method according to claim 13 or 14, wherein the probe(s) and/or the primer(s) further comprises a probe(s) and/or a primer(s) for detecting at least one polynucleotide selected from the group consisting of the following additional markers for discriminating between ovarian cancer and benign ovarian tumor: miR-6806-5p, miR-7845-5p, miR-4632-5p, miR-10396b-5p, miR-6768-5p, miR-8059, miR-8072, miR-9901, miR-1231, miR-1225-5p, miR-12114, miR-3178, miR-6798-5p, miR-4276, miR-6125, miR-3652, miR-7111-5p, miR-6749-5p, miR-1199-5p, miR-6802-5p, miR-6816-5p, miR-4706, miR-5008-5p, miR-6797-5p, miR-4516, miR-4508, miR-6729-5p, miR-564, miR-1233-5p, miR-6127, miR-1469, miR-6738-5p, miR-6785-5p, miR-10401-5p, miR-4430, miR-6889-5p, miR-1236-5p, miR-3176, miR-3141, miR-3928-3p, miR-1237-5p, miR-1915-3p, miR-5195-3p, miR-6743-5p, miR-6746-5p, miR-4446-3p, miR-1228-5p, miR-1268b, miR-1260a, miR-6879-3p, miR-149-3p, miR-3162-5p, miR-1207-5p, miR-4747-3p, miR-4651, miR-638, miR-4736, miR-6845-5p, miR-1343-3p, miR-6126, miR-92b-5p, miR-6774-5p, miR-7847-3p, miR-6795-5p, miR-7109-5p, miR-3197, miR-6824-5p, miR-6771-5p, miR-11399, miR-2861, miR-4707-3p, miR-4638-5p, miR-8073, miR-328-5p, miR-665, miR-6778-5p, miR-10398-3p, miR-5698, miR-6794-5p, miR-1247-3p, miR-4697-5p, miR-8069, miR-572, miR-6751-5p, miR-3180-3p, miR-486-3p, miR-6086, miR-30c-1-3p, miR-8063, miR-3621, miR-6887-5p, miR-3191-3p, miR-11181-3p, miR-6722-5p, miR-6781-5p, miR-5739, miR-3937, miR-1343-5p, miR-1181, miR-4725-3p, miR-6865-5p, miR-375-5p, miR-3196, miR-6762-5p, miR-4258, miR-5196-5p, miR-10401-3p, miR-675-5p, miR-4488, miR-10527-5p, miR-10396a-5p, miR-4269, miR-6800-5p, miR-6819-5p, miR-10396b-3p, miR-4688, miR-6786-5p, miR-4634, miR-3940-5p, miR-4655-5p, miR-7155-5p, miR-6769b-5p, miR-6810-5p, miR-4665-3p, miR-6727-5p, miR-6803-5p, miR-4640-5p, miR-6735-5p, miR-4535, miR-8089, miR-1292-3p, miR-5088-5p, miR-3622a-5p, miR-6124, miR-6820-5p, miR-6805-3p, miR-4513, miR-760, miR-4665-5p, miR-10400-3p, miR-4298, miR-8085, miR-4463, miR-6807-5p, miR-4433b-3p, miR-3185, miR-12121, miR-671-5p, miR-6752-5p, miR-371a-5p, miR-3917, miR-1224-5p, miR-498-5p, miR-7704, miR-6741-5p, miR-765, miR-4486, miR-6090, miR-718, miR-4767, miR-6851-5p, miR-5572, miR-6850-5p, miR-6089, miR-5787, miR-4534, miR-3665, miR-4787-5p, miR-6754-5p, miR-6825-3p, miR-4728-5p, miR-6088, miR-3154, miR-6869-5p, miR-187-5p, miR-6165, miR-4447, miR-4731-5p, miR-6805-5p, miR-12118, miR-4270, miR-7846-3p, miR-4443, miR-6737-5p, miR-197-5p, miR-1229-5p, miR-6757-5p, miR-6765-5p, miR-4722-5p, miR-6891-5p, miR-5006-5p, miR-345-3p, miR-6726-5p, miR-3195, miR-6877-5p, miR-4462, miR-6812-5p, miR-483-5p, miR-9899, miR-4800-5p, miR-4734, miR-3135b, miR-4433a-3p, miR-6769a-5p, miR-4743-5p, miR-1909-3p, miR-4741, miR-4685-5p, miR-3147, miR-4726-5p, miR-3180, miR-3188, miR-6782-5p, miR-6776-5p, miR-4484, miR-1185-1-3p, miR-6790-3p, miR-4466, miR-10394-3p, miR-1275, miR-4478, miR-3175, miR-7106-5p, miR-4667-5p, miR-193b-5p, and miR-602.

16. The method according to claim 15, wherein the probe(s) and/or primer(s) is a polynucleotide(s) selected from the group consisting of the following polynucleotides (a) to (e):

(a) a polynucleotide consisting of a nucleotide sequence represented by any of SEQ ID NOs: 10 to 235, or a nucleotide sequence derived from the nucleotide sequence by the replacement of u with t, a variant thereof, a derivative thereof, or a fragment thereof comprising 15 or more consecutive nucleotides;
(b) a polynucleotide comprising a nucleotide sequence represented by any of SEQ ID NOs: 10 to 235;
(c) a polynucleotide consisting of a nucleotide sequence complementary to a nucleotide sequence represented by any of SEQ ID NOs: 10 to 235, or a nucleotide sequence derived from the nucleotide sequence by the replacement of u with t, a variant thereof, a derivative thereof, or a fragment thereof comprising 15 or more consecutive nucleotides;
(d) a polynucleotide comprising a nucleotide sequence complementary to a nucleotide sequence represented by any of SEQ ID Nos: 10 to 235, or a nucleotide sequence derived from the nucleotide sequence by the replacement of u with t; and
(e) a polynucleotide hybridizing under stringent conditions to any of the polynucleotides (a) to (d).

17. The method according to any one of claims 11 to 16, wherein the subject is a human.

18. The method according to any one of claims 11 to 17, wherein the sample is blood, serum, or plasma.

# Fig. 1

hsa-miR-10396b-5p
(SEQ ID NO: 13)

hsa-mir-10396b
(SEQ ID NO: 248)

hsa-miR-10396b-3p
(SEQ ID NO: 124)

# Fig. 2

**hsa-miR-1908-5p**

# Fig. 3

## INTERNATIONAL SEARCH REPORT

| International application No. |
|---|
| **PCT/JP2022/039032** |

### A. CLASSIFICATION OF SUBJECT MATTER

*C12N 15/11*(2006.01)i; *C12Q 1/6837*(2018.01)i; *C12Q 1/6886*(2018.01)i
FI:   C12N15/11 Z ZNA; C12Q1/6886 Z; C12Q1/6837 Z

According to International Patent Classification (IPC) or to both national classification and IPC

### B. FIELDS SEARCHED

Minimum documentation searched (classification system followed by classification symbols)

C12N15/11; C12Q1/6837; C12Q1/6886

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| X | CN 112626209 A (PEOPLE'S HOSPITAL OF MIANZHU CITY) 09 April 2021 (2021-04-09) claims, examples | 1-2, 4-6, 8-10, 13-14, 16-18 |
| Y | | 1-18 |
| X | US 2016/0312301 A1 (THE FEINSTEIN INSTITUTE FOR MEDICAL RESEARCH) 27 October 2016 (2016-10-27) claims, paragraphs [0010], [0061]-[0063], examples | 1-2, 4-6, 8-10, 13-14, 16-18 |
| Y | | 1-18 |
| Y | WO 2018/199275 A1 (TORAY INDUSTRIES, INC.) 01 November 2018 (2018-11-01) claims, examples | 1-18 |

☐ Further documents are listed in the continuation of Box C.   ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **01 December 2022** | **13 December 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

International application No.

**PCT/JP2022/039032**

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

   a. ☑ forming part of the international application as filed:

      ☑ in the form of an Annex C/ST.25 text file.

      ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

      ☐ in the form of an Annex C/ST.25 text file (Rule 13*ter*.1(a)).

      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

   "The form of Annex C/ST.25 text file" above shall read as "the form of ST.26.".

Form PCT/ISA/210 (continuation of first sheet) (January 2015)

# EP 4 421 171 A1

**INTERNATIONAL SEARCH REPORT**
Information on patent family members

International application No.

**PCT/JP2022/039032**

| Patent document cited in search report | | | Publication date (day/month/year) | Patent family member(s) | | | Publication date (day/month/year) |
|---|---|---|---|---|---|---|---|
| CN | 112626209 | A | 09 April 2021 | (Family: none) | | | |
| US | 2016/0312301 | A1 | 27 October 2016 | WO | 2015/095862 | A2 | |
| WO | 2018/199275 | A1 | 01 November 2018 | US | 2020/0140956 | A1 | |
| | | | | claims, examples | | | |
| | | | | EP | 3628736 | A1 | |

Form PCT/ISA/210 (patent family annex) (January 2015)

172

## REFERENCES CITED IN THE DESCRIPTION

**Patent documents cited in the description**

- WO 2018199275 A
- JP 2010154843 A
- US 20200123614 A
- JP 2021171945 A

**Non-patent literature cited in the description**

- **WHITEHOUSE C. et al.** *Gynecologic Oncology,* 2003, vol. 88, S152
- **ZHENG ZHANG et al.** *J. Comput. Biol.,* 2000, vol. 7, 203-214
- **ALTSCHUL, S.F. et al.** *Journal of Molecular Biology,* vol. 215, 403-410
- **PEARSON, W.R. et al.** *Proc. Natl. Acad. Sci. U. S. A.,* 1988, vol. 85, 2444-2448
- **NIELSEN, P.E. et al.** *Science,* 1991, vol. 254, 1497-500
- **OBIKA, S. et al.** *Tetrahedron Lett.,* 1998, vol. 39, 5401-5404
- **BAR M et al.** *Stem Cells,* 2008, vol. 26, 2496-2505
- **PERSSON H et al.** *Cancer Res,* vol. 71, 78-86
- **PERSSON H et al.** *Cancer Res,* 2011, vol. 71, 78-86
- **LUI WO et al.** *Cancer Res,* 2007, vol. 67, 6031-6043
- **LADEWIG E et al.** *Genome Res,* 2012, vol. 22, 1634-1645
- **MORIN RD et al.** *Genome Res,* 2008, vol. 18, 610-621
- **MARTON S et al.** *Leukemia,* 2008, vol. 22, 330-338
- **JIMA DD et al.** *Blood,* 2010, vol. 116, e118-e127
- **LAGOS-QUINTANA M et al.** *Curr Biol,* 2002, vol. 12, 735-739
- **PLE H et al.** *PLoS One,* 2012, vol. 7, e50746
- **LIM EL et al.** *Genome Biol,* 2015, vol. 16, 18
- **WANG HJ et al.** *Shock,* 2013, vol. 39, 480-487
- **BOELE J et al.** *Proc Natl Acad Sci USA,* 2014, vol. 111, 11467-11472
- **BEREZIKOV E et al.** *Mol Cell,* 2007, vol. 28, 328-336
- **OZATA DM et al.** *Cell Death Dis,* 2017, vol. 8, e2759
- **STARK MS et al.** *PLoS One,* 2010, vol. 5, e9685
- **GOFF LA et al.** *PLoS One,* 2009, vol. 4, e7192
- **SMITH JL et al.** *J Virol,* 2012, vol. 86, 5278-5287
- **MEIRI E et al.** *Nucleic Acids Res,* 2010, vol. 38, 6234-6246
- **SALVI A et al.** *Int J Oncol,* 2013, vol. 42, 391-402
- **HANSEN TB et al.** *RNA Biol,* 2011, vol. 8, 378-383
- **CUMMINS JM et al.** *Proc Natl Acad Sci USA,* 2006, vol. 103, 3687-3692
- **DANNEMANN M et al.** *Genome Biol Evol,* 2012, vol. 4, 552-564
- **KAWAJI H et al.** *BMC Genomics,* 2008, vol. 9, 157
- **CREIGHTON CJ et al.** *PLoS One,* 2010, vol. 5, e9637
- **SCHOTTE D et al.** *Leukemia,* 2011, vol. 25, 1389-1399
- **HUPPI K et al.** *Mol Cancer Res,* 2008, vol. 6, 212-221
- **CUMMINS JM et al.** *Proc Natl Acad Sci USA,* vol. 103, 3687-3692
- **SUN Q et al.** *Exp Cell Res,* 2015, vol. 333, 220-227

- **LI H.** *J Clin Invest,* 2009, vol. 119, 3666-3677
- **KIM J et al.** *Proc Natl Acad Sci USA,* 2004, vol. 101, 360-365
- **BEREZIKOV E et al.** *Genome Res,* 2006, vol. 16, 1289-1298
- **WATAHIKI A.** *PLoS One,* 2011, vol. 6, e24950
- **FU H et al.** *FEBS Lett,* 2005, vol. 579, 3849-3854
- **YOO JK et al.** *Stem Cells Dev,* 2012, vol. 21, 2049-2057
- **WITTEN D et al.** *BMC Biol,* 2010, vol. 8, 58
- **DOKANEHIIFARD S et al.** *Cell Mol Life Sci,* 2015, vol. 72, 2613-2625
- **LI Y et al.** *Gene,* 2012, vol. 497, 330-335
- **YOO JK et al.** *Biochem Biophys Res Commun,* 2011, vol. 415, 258-262
- **LIAO JY et al.** *PLoS One,* 2010, vol. 5, e10563
- **SUBRAMANIAN S et al.** *Oncogene,* 2008, vol. 27, 2015-2026
- **POY MN et al.** *Nature,* 2004, vol. 432, 226-230
- **CAI X et al.** *RNA,* 2007, vol. 13, 313-316
- **ASIKAINEN S et al.** *PLoS One,* 2015, vol. 10, e0116668
- **MEUNIER J et al.** *Genome Res,* 2013, vol. 23, 34-45
- **DING N et al.** *J Radiat Res,* 2011, vol. 52, 425-432
- **SUH MR et al.** *Dev Biol,* 2004, vol. 270, 488-498
- **BENTWICH I et al.** *Nat Genet,* 2005, vol. 37, 766-770
- **SWAMINATHAN S et al.** *Biochem Biophys Res Commun,* 2013, vol. 434, 228-234
- **ARTZI S et al.** *BMC Bioinformatics,* 2008, vol. 9, 39
- **TANDON M et al.** *Oral Dis,* 2012, vol. 18, 127-131
- **YOO H et al.** *Biochem Biophys Res Commun,* 2011, vol. 415, 567-572
- **XIE X et al.** *Nature,* 2005, vol. 434, 338-345
- **LIM LP et al.** *Science,* 2003, vol. 299, 1540
- **PARSI S et al.** *PLoS One,* 2012, vol. 7, e35561
- **LAGOS-QUINTANA M et al.** *RNA,* 2003, vol. 9, 175-179
- **MORIN RD. et al.** *Genome Res.,* 2008, vol. 18, 610-621
- **AUSUBEL et al.** Current Protocols in Molecular Biology. John Willey & Sons, 1993
- **SAMBROOK et al.** Molecular Cloning - A Laboratory Manual. Cold Spring Harbor Laboratory Press, 1989
- **SAMBROOK, J. ; RUSSEL, D.** Molecular Cloning, A LABORATORY MANUAL. Cold Spring Harbor Laboratory Press, 15 January 2001, vol. 1,2, 7.42-7.45, 8.9-8.17
- **BOLSTAD, B. M. et al.** *Bioinformatics,* 2003, vol. 19, 185-193
- **VENABLES, W.N. et al.** Modern Applied Statistics with S. Springer, 2002
- **TAKAFUMI KANAMORI et al.** Pattern Recognition. KYORITSU SHUPPAN CO., LTD, 2009
- **RICHARD O. et al.** Pattern Classification. Wiley-Interscience, 2000
- **V. VAPNIK.** The Nature of Statistical Leaning Theory. Springer, 1995
- Statistics of pattern recognition and learning - New concepts and approaches. **HIDEKI ASO et al.** Frontier of Statistical Science. Iwanami Shoten, Publishers, 2004, vol. 6
- **NELLO CRISTIANINI et al.** Introduction to SVM. Kyoritsu Shuppan Co., Ltd, 2008
- **C. CORTES et al.** *Machine Learning,* 1995, vol. 20, 273-297
- **YASUSHI NAGATA et al.** Basics of statistical multiple comparison methods. Scientist Press Co., Ltd, 2007
- **FUREY TS. et al.** *Bioinformatics,* 2000, vol. 16, 906-14
- R: A language and environment for statistical computing. R Foundation for Statistical Computing, 2016
- **VENABLES, W. N. ; RIPLEY, B. D.** Modern Applied Statistics with S. Springer, 2002